# EUROPÄISCHE PATENTSCHRIFT

(11) **EP 1 697 400 B1**
(45) Veröffentlichungstag und Bekanntmachung des Hinweises auf die Patenterteilung: **25.05.2011**
(21) Anmeldenummer: 04801192.8
(22) Anmeldetag: 02.12.2004
(51) Int. Cl.: C07K 5/08, C07K 5/10, C07K 7/06, A61K 38/04, A61P 31/04

(54) **ANTIBAKTERIELLE MAKROZYKLEN MIT SUBSTITUIERTEM BIPHENYL**
BIPHENYL-SUBSTITUTED ANTIBACTERIAL MACROCYCLES
MACROCYCLES ANTIBACTERIENS A SUBSTITUTION BIPHENYLE

(30) Priorität: 16.12.2003 DE 10358824
(43) Veröffentlichungstag der Anmeldung: 06.09.2006
(73) Patentinhaber: AiCuris GmbH & Co. KG, 42117 Wuppertal (DE)
(72) Erfinder: ENDERMANN, Rainer, 42113 Wuppertal (DE); EHLERT, Kerstin, 42553 Velbert (DE); RADDATZ, Siegfried, 51065 Köln (DE); CANCHO GRANDE, Yolanda, 51373 Leverkusen (DE); MICHELS, Martin, 51109 Köln (DE); WEIGAND, Stefan, 82377 Penzberg (DE); ADELT, Isabelle, 42781 Haan (DE); LAMPE, Thomas, 40545 Düsseldorf (DE)
(74) Vertreter: Weller, Wolfgang
(86) Internationale Anmeldenummer: PCT/EP2004/013688
(87) Internationale Veröffentlichungsnummer: WO 2005/058943

(56) Entgegenhaltungen:
- WO-A-03/106480
- SCHMIDT U ET AL: "Amino acids and peptides. 84. Synthesis of biologically active cyclopeptides. 24. Total synthesis of the biphenomycins. III. Synthesis of biphenomycin B" SYNTHESIS, GEORG THIEME VERLAG. STUTTGART, DE, Nr. 10, Oktober 1992 (1992-10), Seiten 1025-1030, XP001155274 ISSN: 0039-7881 in der Anmeldung erwähnt
- KRENITSKY P J ET AL: "Synthesis of the (S,S,S)-diastereomer of the 15-membered biaryl ring system of RP 66453" TETRAHEDRON LETTERS, ELSEVIER SCIENCE PUBLISHERS, AMSTERDAM, NL, Bd. 44, Nr. 21, 19. Mai 2003 (2003-05-19), Seiten 4019-4022, XP004423025 ISSN: 0040-4039
- SCHMIDT U ET AL: "Amino acids and peptides. 88. Synthesis of biologically active cyclopeptides. 26. Total synthesis of the biphenomycins. V. Synthesis of biphenomycin A" SYNTHESIS, GEORG THIEME VERLAG. STUTTGART, DE, Nr. 12, Dezember 1992 (1992-12), Seiten 1248-1254, XP001155271 ISSN: 0039-7881 in der Anmeldung erwähnt
- CHANG C C ET AL: "LL-AF283 antibiotics, cyclic biphenyl peptides" JOURNAL OF ANTIBIOTICS, JAPAN ANTIBIOTICS RESEARCH ASSOCIATION. TOKYO, JP, Bd. 44, Nr. 6, Juni 1991 (1991-06), Seiten 674-677, XP009018534 ISSN: 0021-8820

## Beschreibung

Die Erfindung betrifft antibakterielle Makrozyklen mit substituiertem Biphenyl und Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von bakteriellen Infektionen.

In US 3,452,136, Dissertation R. U. Meyer, Universität Stuttgart, Deutschland 1991, Dissertation V. Leitenberger, Universität Stuttgart, Deutschland 1991, Synthesis (1992), (10), 1025-30, J. Chem. Soc., Perkin Trans. 1 (1992), (1), 123-30, J. Chem. Soc., Chem. Commun. (1991), (10), 744, Synthesis (1991), (5), 409-13, J. Chem. Soc., Chem. Commun. (1991), (5), 275-7, J. Antibiot. (1985), 38(11), 1462-8, J. Antibiot. (1985), 38(11), 1453-61, wird der Naturstoff Biphenomycin B als antibakteriell wirksam beschrieben. Teilschritte der Synthese von Biphenomycin B werden in Synlett (2003), 4, 522-526 beschrieben.

Chirality (1995), 7(4), 181-92, J. Antibiot. (1991), 44(6), 674-7, J. Am. Chem. Soc. (1989), 111(19), 7323-7, J. Am. Chem. Soc. (1989), 111(19), 7328-33, J. Org. Chem. (1987), 52(24), 5435-7, Anal. Biochem. (1987), 165(1), 108-13, J. Org. Chem. (1985), 50(8), 1341-2, J. Antibiot. (1993), 46(3), C-2, J. Antibiot. (1993), 46(1), 135-40, Synthesis (1992), (12), 1248-54, Appl. Environ. Microbiol. (1992), 58(12), 3879-8, J. Chem. Soc., Chem. Commun. (1992), (13), 951-3 beschreiben einen strukturell verwandten Naturstoff, Biphenomycin A, der am Makrozyklus eine weitere Substitution mit einer Hydroxygruppe aufweist.

Die Naturstoffe entsprechen hinsichtlich ihrer Eigenschaften nicht den Anforderungen, die an antibakterielle Arzneimittel gestellt werden. Auf dem Markt sind zwar strukturell andersartige antibakteriell wirkende Mittel vorhanden, es kann aber regelmäßig zu einer Resistenzentwicklung kommen. Neue Mittel für eine gute und wirksamere Therapie sind daher wünschenswert.

Eine Aufgabe der vorliegenden Erfindung ist es daher, neue und alternative Verbindungen mit gleicher oder verbesserter antibakterieller Wirkung zur Behandlung von bakteriellen Erkrankungen bei Menschen und Tieren zur Verfügung zu stellen.

Überraschenderweise wurde gefunden, dass bestimmte Derivate dieser Naturstoffe, worin die Carboxylgruppe des Naturstoffs gegen eine Amidgruppe ausgetauscht wird, die eine basische Gruppe enthält, gegen Biphenomycin resistente *S. aureus* Stämme (RN4220Bi^{R} und T17) antibakteriell" wirksam sind.

Weiterin zeigen diese Derivate gegen *S. aureus* Wildtyp-Stämme und Biphenomycin resistente *S. aureus* Stämme eine verbesserte Spontanresistenz-Frequenz.

Gegenstand der Erfindung sind Verbindungen der Formel 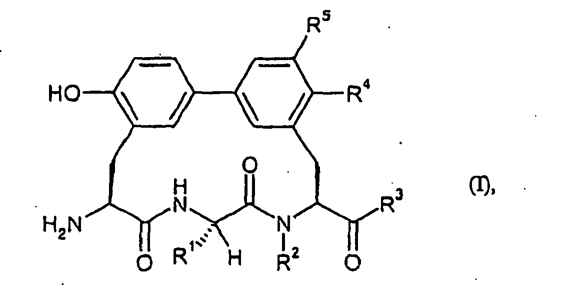 worin
- R¹: gleich Alkyl ist, wobei Alkyl substituiert ist mit 1 bis 3 Substituenten, wobei die Substi- tuenten unabhängig voneinander ausgewählt werden aus der Gruppe, bestehend aus Ami- no, Hydroxy, Aminocarbonyl, Hydroxycarbonyl, (C₁-C₆)-Alkoxy, (C₁-C₆)-Alkylamino, 5- bis 7-gliedriges Heterocyclyl, 5- bis 7-gliedriges Heteroaryl, (C₁-C₆)-Alkylaminocarbonyl, Guanidino und Amidino, worin Heterocyclyl und Heteroaryl substituiert sein können mit 1 bis 2 Substituen- ten, wobei die Substituenten unabhängig voneinander ausgewählt werden aus der Gruppe, bestehend aus Amino und (C₁-C₆)-Alkyl,
- R²: gleich Wasserstoff, (C₁-C₆)-Alkyl oder (C₃-C₇)-Cycloalkyl ist,
- R³: gleich -NR⁶R⁷ ist,
- R⁴: gleich Wasserstoff, Halogen, Amino, Hydroxy, Aminocarbonyl, Hydroxycarbonyl, Nitro, Trifluormethyl, Trifluormethoxy, (C₁-C₄)-Alkyl, (C₁-C₄)-Alkoxy, (C₁-C₆)-Alkylamino, Amino oder mono-(C₁-C₄)-Alkylamino substituiertes mono-(C₁-C₆)-Alkylaminocarbonyl oder Amino oder mono-(C₁-C₄)-Alkylamino substituiertes (C₁-C₆)-Alkylcarbonylamino ist,
- R⁵: gleich Wasserstoff, Halogen. Amino, Hydroxy, Aminocarbonyl, Hydroxycurbonyl, Nitro, Trifluormethyl, Trifluormethoxy, (C₁-C₄)-Alkyl, (C₁-C₄)-Alkoxy, (C₁-C₆)-Alkylamino, Amino oder mono-(C₁-C₄)-Alkylamino substituiertes mono-(C₁-C₆)-Alkylaminocarbonyl oder Amino oder mono-(C₁-C₄)-Alkylamino substituiertes (C₁-C₆)-Alkylcarbonylamino ist, wobei R⁵ gleich Halogen, Amino, Hydroxy, Aminocarbonyl, Hydroxycarbonyl, Nitro, Trifluormethyl, Trifluormethoxy, (C₁-C₄)-Alkyl, (C₁-C₄)-Alkoxy, (C₁-C₆)-Alkylamino, Amino oder mono-(C₁-C₄)-Alkylamino substituiertes mono-(C₁-C₆)-Alkylaminocarbonyl
- R⁶: oder Amino oder mono-(C₁-C₄)-Alkylamino substituiertes (C₁-C₆)-Alkylcarbonylamino ist, wenn R⁴ gleich Hydroxy ist, gleich Wasserstoff, (C₁-C₆)-Alkyl, (C₃-C₇)-Cycloalkyl, 5- bis 7-gliedriges Heterocyclyl, (C₆-C₁₀)-Aryl oder 5- bis 7-gliedriges Heteroaryl ist, wobei Alkyl, Cycloalkyl, Heterocyclyl, Aryl und Heteroaryl substituiert sein können mit 1 bis 3 Substituenten, wobei die Substituenten unabhängig voneinander ausgewählt werden aus der Gruppe, bestehend aus Halogen, Amino, Hydroxy, Aminocarbonyl, Hydroxycar- bonyl, Nitro, Cyano, Trifluormethyl, Trifluormethoxy, (C₁-C₆)-Alkyl, (C₁-C₆)-Alkoxy, (C₁- C₆)-Alkylamino, (C₃-C₇)-Cycloalkyl, 5- bis 7-gliedriges Heterocyclyl, (C₆-C₁₀)-Aryl, 5- bis 7-gliedriges Heteroaryl, (C₁-C₆)-Alkoxycarbonyl, (C₁-C₅)-Alkylaminocarbonyl, (C₁-C₆)- Alkylsulfonylamino und (C₆-C₁₀)-Arylsulfonylamino, worin Alkyl, Alkylamino, Cycloalkyl, Heterocyclyl, Aryl, Heteroaryl, Alkyl- aminocarbonyl, Alkylsulfonylamino und Arylsulfonylamino substituiert sein kön- nen mit 1 bis 3 Substituenten, wobei die Substituenten unabhängig voneinander ausgewählt werden aus der Gruppe, bestehend aus Amino, Hydroxy, Aminocarbo- nyl und Hydroxycarbonyl,
- R⁷: gleich Wasserstoff, (C₁-C₆)-Alkyl oder (C₃-C₇)-Cycloalkyl ist, wobei Alkyl substituiert sein kann mit 1 bis 2 Substituenten, wobei die Substituenten un- abhängig voneinander ausgewählt werden aus der Gruppe, bestehend aus Amino, Hydroxy und (C₁-C₆)-Alkylamino,
oder
R⁶ und R⁷ zusammen mit dem Stickstoffatom, an das sie gebunden sind, ein Piperidinyl, Morpholinyl, Piperazinyl oder Pyrrolidinyl bilden, wobei Piperidinyl, Morpholinyl, Piperazinyl und Pyrrolidinyl substituiert sein können mit 1 bis 3 Substituenten, wobei die Substituenten unabhängig voneinander ausgewählt werden aus der Gruppe, bestehend aus Amino, Hydroxy, Aminocarbonyl, Hydroxycarbonyl, gegebenenfalls Amino oder Hydroxy substituierten (C₁-C₆)-Alkyl, (C₁-C₆)-Alkoxy, (C₁-C₆)-Alkylamino und (C₁-C₆)-Alkoxycarbonyl,
und ihre Salze, ihre Solvate und die Solvate ihrer Salze.

Erfindungsgemäße Verbindungen sind die Verbindungen der Formel (I) und deren Salze, Solvate und Solvate der Salze, sowie die von Formel (I) umfassten, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich durch bekannte Verfahren wie Chromatographie an chiraler Phase oder Kristallisation mit chiralen Aminen oder chiralen Säuren die stereoisomer einheitlichen Bestandteile in bekannter Weise isolieren.

Die Erfindung betrifft in Abhängigkeit von der Struktur der Verbindungen auch Tautomere der Verbindungen.

Als Salze sind im Rahmen der Erfindung physiologisch unbedenkliche Salze der erfindungsgemäßen Verbindungen bevorzugt.

Physiologisch unbedenkliche Salze der Verbindungen (I) umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Äpfelsäure, Zitronensäure, Fumarsäure, Maleinsäure, Trifluoressigsäure und Benzoesäure.

Physiologisch unbedenkliche Salze der Verbindungen (I) umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Dihydroabietylamin, Arginin, Lysin, Ethylendiamin und Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:
Alkyl per se und "Alk" und "Alkyl" in Alkoxy, Alkylamino, Alkoxycarbonyl, Alkylaminocarbonyl und Alkylsulfonylamino stehen für einen linearen oder verzweigten Alkylrest mit in der Regel 1 bis 6 ("C₁-C₆-Alkyl"), vorzugsweise 1 bis 4, besonders bevorzugt 1 bis 3 Kohlenstoffatomen, beispielhaft und vorzugsweise für Methyl, Ethyl, n-Propyl, Isopropyl, *tert-*Butyl, n-Pentyl und n-Hexyl.
Alkoxy steht beispielhaft und vorzugsweise für Methoxy, Ethoxy, n-Propoxy, Isopropoxy, *tert-*Butoxy, n-Pentoxy und n-Hexoxy.
Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, *tert-*Butylamino, n-Pentylamino, n-Hexylamino, *N*,*N*-Dimethylamino, *N*,*N*-Diethylamino, *N*-Ethyl-*N*-methylamino, *N*-Methyl-*N*-n-propylamino, *N*-Isopropyl-*N*-n-propylamino, *N*-t-Butyl-*N*-methylamino, *N*-Ethyl-*N*-n-pentylamino und *N*-n-Hexyl-*N*-methylamino. C₁-C₃-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.
Alkoxycarbonyl steht beispielhaft und vorzugsweise für Methoxycarbonyl, Ethoxycarbonyl, n-Propoxycarbonyl, Isopropoxycarbonyl, *tert-*Butoxycarbonyl, n-Pentoxycarbonyl und n-Hexoxycarbonyl.
Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, Isopropylaminocarbonyl, *tert-*Butylaminocarbonyl, n-Pentylaminocarbonyl, n-Hexylaminocarbonyl, *N*,*N*-Dimethylaminocarbonyl, *N,N-*Diethylaminocarbonyl, *N*-Ethyl-*N*-methylaminocarbonyl, M-Methyl-*N*-n-propylaminocarbonyl, *N*-Isopropyl-*N*-n-propylaminocarbonyl, *N-tert*-Butyl-*N*-methylaminocarbonyl, *N-*Ethyl-*N-*n-pentylaminocarbonyl und *N*-n-Hexyl-*N*-methylaminocarbonyl. C₁-C₃-Alkylaminocarbonyl steht beispielsweise für einen Monoalkylaminocarbonylrest mit 1 bis 3 Kohlenstoffatomen oder für einen Dialkylaminocarbonylrest mit jeweils 1 bis 3 Kohlenstoffatomen pro Alkylsubstituent.
Alkylsulfonylamino steht beispielhaft und vorzugsweise für. Methylsulfonylamino, Ethylsulfonylamino, n-Propylsulfonylamino, Isopropylsulfonylamino, *tert-*Butylsulfonylamino, n-Pentylsulfonylamino und n-Hexylsulfonylamino.
Cycloalkyl steht für eine Cycloalkylgruppe mit in der Regel 3 bis 7, bevorzugt 5 bis 6 Kohlenstoffatomen, beispielhaft und vorzugsweise für Cyclopropyl, Cyclobutyl, Cyclopentyl, Cyclohexyl und Cycloheptyl.
Aryl steht für einen mono- oder bicyclischen aromatischen, carbocyclischen Rest mit in der Regel 6 bis 10 Kohlenstoffatomen; beispielhaft und vorzugsweise für Phenyl und Naphthyl.
Arylsulfonylamino steht beispielhaft und vorzugsweise für Phenylsulfonylamino und Naphthylsulfonylamino.
5- bis 7-gliedriges Heterocyclyl steht im Rahmen der Erfindung für einen mono- oder bicyclischen, gesättigten oder partiell ungesättigten Heterocyclus mit bis zu drei Heteroatomen aus der Reihe N, O und/oder S, der über ein Ringkohlenstoffatom oder ein Stickstoffatom des Heterocyclus verknüpft ist. Beispielhaft und vorzugsweise seien genannt: Tetrahydrofuryl, Dihydrofuryl, Imidazolidinyl, Thiolanyl, Dioxolanyl, Pyrrolidinyl, Pyrrolinyl, Tetrahydropyranyl, Dihydropyranyl, Piperidinyl, Piperazinyl, Morpholinyl, Thiomorpholinyl und 1,4-Diazepanyl.
5- bis 7-gliedrieges Heteroaryl steht im Rahmen der Erfindung im allgemeinen für einen aromatischen, mono- oder bicyclischen Rest mit 5 bis 7 Ringatomen und bis zu 4 Heteroatomen aus der Reihe S, O und/oder N. Bevorzugt sind 5- bis 6-gliedrige Heteroaryle mit bis zu 4 Heteroatomen. Der Heteroarylrest kann über ein Kohlenstoff- oder Heteroatom gebunden sein. Beispielsweise und vorzugsweise seien genannt: Thienyl, Furyl, Pyrrolyl, Thiazolyl, Oxazolyl, Imidazolyl, Pyridyl, Pyrimidyl, Pyridazinyl, Indolyl, Indazolyl, Benzofuranyl und Benzothiophenyl.

Halogen steht für Fluor, Chlor, Brom und Jod.

Ein Symbol # an einem Kohlenstoffatom bedeutet, dass die Verbindung hinsichtlich der Konfiguration an diesem Kohlenstoffatom in enantiomerenreiner Form vorliegt, worunter im Rahmen der vorliegenden Erfindung ein Enantiomerenüberschuss (enantiomeric excess) von mehr als 90 % verstanden wird (> 90 % ee).

In den Formeln der Gruppen, für die R⁶ stehen kann, steht der Endpunkt der Linie, neben der jeweils ein * steht, nicht für ein Kohlenstoffatom beziehungsweise eine CH₂-Gruppe sondern ist Bestandteil der Bindung zu dem Stickstoffatom, an das R⁶ gebunden ist. R⁶ ist also beispielsweise 2-Aminoethyl im Falle von k = 0,1= 1 und R⁹ = H, 3-Amino-2-hydroxypropyl im Falle von k = 1, R⁸ = OH, 1 = 1 und R⁹ = H, Piperidin-4-yl-methyl im Falle von q = 1 und r = 1 oder Piperidin-4-yl im Falle von q=0 und r=1.

Bevorzugt im Rahmen der vorliegenden Erfindung sind Verbindungen der Formel (I), worin
- R¹: gleich Alkyl ist, wobei Alkyl substituiert ist mit 1 bis 3 Substituenten, wobei die Substi- tuenten unabhängig voneinander ausgewählt werden aus der Gruppe, bestehend aus Ami- no, Hydroxy, Aminocarbonyl, Hydroxycarbonyl, (C₁-C₆)-Alkoxy, (C₁-C₆)-Alkylamino, 5- bis 7-gliedriges Heterocyclyl, 5- bis 7-gliedriges Heteroaryl, (C₁-C₆)-Alkylaminocarbonyl, Guanidino und Amidino, worin Heterocyclyl und Heteroaryl substituiert sein können mit t bis 2 Substituen- ten, wobei die Substituenten unabhängig voneinander ausgewählt werden aus der Gruppe, bestehend aus Amino und (C₁-C₆)-Alkyl,
- R²: gleich Wasserstoff, (C₁-C₆)-Alkyl oder (C₃-C₇)-Cycloalkyl ist,
- R³: gleich -NR⁶R⁷ ist,
- R⁴: gleich Wasserstoff, Halogen, Amino, Hydroxy, Aminocarbonyl, Hydroxycarbonyl, Nitro, Trifluormethyl, Trifluormethoxy, (C₁-C₄)-Alkyl, (C₁-C₄)-Alkoxy oder (C₁-C₆)-Alkylamino ist,
- R⁵: gleich Wasserstoff, Halogen, Amino, Hydroxy, Aminocarbonyl, Hydroxycarbonyl, Nitro, Trifluormethyl, Trifluormethoxy, (C₁-C₄)-Alkyl, (C₁-C₄)-Alkoxy oder (C₁-C₆)-Alkylamino ist, wobei R⁵ gleich Halogen, Amino, Hydroxy, Aminocarbonyl, Hydroxycarbonyl, Nitro, Trifluormethyl, Trifluarmethoxy, (C₁-C₄)-Alkyl, (C₁-C₄)-Alkoxy oder (C₁-C₆)-Alkylamino ist, wenn R⁴ gleich Hydroxy ist,
- R⁶: gleich Wasserstoff, (C₁-C₆)-Alkyl, (C₃-C₇)-Cycloalkyl, 5- bis 7-gliedriges Heterocyclyl, (C₆-C₁₀)-Aryl oder 5-bis 7-gliedriges Heteroaryl ist, wobei Alkyl, Cycloalkyl, Heterocyclyl, Aryl und Heteroaryl substituiert sein können mit 1 bis 3 Substituenten, wobei die Substituenten unabhängig voneinander ausgewählt werden aus der Gruppe, bestehend aus Halogen, Amino, Hydroxy, Aminocarbonyl, Hydroxycar- bonyl, Nitro, Cyano, Trifluormethyl, Trifluormethoxy, (C₁-C₆)-Alkyl, (C₁-C₆)-Alkoxy, (C₁- C₆)-Alkylamino, (C₃-C₇)-Cycloalkyl, 5- bis 7-gliedriges Heterocyclyl, (C₆-C₁₀)-Aryl, 5- bis 7-gliedriges Heteroaryl, (C₁-C₆)-Alkoxycarbonyl, (C₁-C₆)-Alkylaminocarbonyl, (C₁-C₆)- Alkylsulfonylamino und (C₆-C₁₀)-Arylsulfonylamino, worin Alkyl, Alkylamino, Cycloalkyl, Heterocyclyl, Aryl, Heteroaryl, Alkyl- aminocarbonyl, Alkylsulfonylamino und Arylsulfonylamino substituiert sein kön- nen mit 1 bis 3 Substituenten, wobei die Substituenten unabhängig voneinander ausgewählt werden aus der Gruppe, bestehend aus Amino, Hydroxy, Aminocarbo- nyl und Hydroxycarbonyl,
- R⁷: gleich Wasserstoff, (C₁-Cₑ)-Alkyl oder (C₃-C₇)-Cycloalkyl ist, wobei Alkyl substituiert sein kann mit 1 bis 2 Substituenten, wobei die Substituenten un- abhängig voneinander ausgewählt werden aus der Gruppe, bestehend aus Amino, Hydroxy und (C₁-C₆)-Alkylamino,
oder
R⁶ und R⁷ zusammen mit dem Stickstoffatom, an das sie gebunden sind, ein Piperidinyl, Morpholinyl, Piperazinyl oder Pyrrolidinyl bilden, wobei Piperidinyl, Morpholinyl, Piperazinyl und Pyrrolidinyl substituiert sein können mit 1 bis 3 Substituenten, wobei die Substituenten unabhängig voneinander ausgewählt werden aus der Gruppe, bestehend aus Amino, Hydroxy, Aminocarbonyl, Hydroxycarbonyl, gegebenenfalls Amino oder Hydroxy substituierten (C₁-C₆)-Alkyl, (C₁-C₆)-Alkoxy, (C₁-C₆)-Alkylamino und (C₁-C₆)-Alkoxycarbonyl,
und ihre Salze, ihre Solvate und die Solvate ihrer Salze.

Bevorzugt im Rahmen der vorliegenden Erfindung sind auch Verbindungen der Formel (I), worin
- R¹: gleich Aminomethyl, 2-Aminoethyl, 3-Aminoprop-1-yl, 4-Aminobut-1-yl, Hydroxymethyl, 2-Hydroxy-ethyl, Aminocarbonylmethyl, 2-Aminocarbonylethyl, 2-Hydroxycarbonylethyl, 3-Guanidinoprop-1-yl, 3-Amino-2-hydroxyprop-1-yl oder 4-Amino-3-hydroxybut-1-yl ist,
- R²: gleich Wasserstoff, Methyl, Ethyl oder Cyclopropyl ist,
- R³: gleich -NR⁶R⁷ ist,
- R⁴: gleich Wasserstoff, Halogen, Amino, Hydroxy, Hydroxycarbonyl, Aminocarbonyl, Nitro oder Methyl ist,
- R⁵: gleich Wasserstoff, Halogen, Amino, Hydroxy, Aminocarbonyl, Hydroxycarbonyl, Nitro oder Methyl ist, wobei R⁵ gleich Halogen, Amino, Hydroxy, Aminocarbonyl, Hydroxycarbonyl, Nitro oder Methyl ist, wenn R⁴ gleich Hydroxy ist,
- R⁶: gleich Wasserstoff, (C₁-C₆)-Alkyl, (C₅-C₆)-Cycloalkyl, 5- bis 7-gliedriges Heterocyclyl oder Phenyl ist, wobei Alkyl, Cycloalkyl, Heterocyclyl, und Phenyl substituiert sein können mit 1 bis 3 Substituenten, wobei die Substituenten unabhängig voneinander ausgewählt werden aus der Gruppe, bestehend aus Amino, Hydroxy, Aminocarbonyl, Hydroxycarbonyl, (C₁-C₄)- Alkyl, (C₁-C₆)-Alkylamino, 5- bis 7-gliedriges Heterocyclyl, Phenyl, 5- oder 6-gliedriges Heteroaryl, (C₁-C₆)-Alkoxycarbonyl und (C₁-C₆)-Alkylaminocarbonyl, worin Alkyl, Alkylamino, Heterocyclyl, Aryl, Heteroaryl und Alkylaminocarbonyl substituiert sein können mit 1 bis 3 Substituenten, wobei die Substituenten unab- hängig voneinander ausgewählt werden aus der Gruppe, bestehend aus Amino, Hydroxy, Aminocarbonyl und Hydroxycarbonyl,
- R⁷: gleich Wasserstoff oder (C₁-C₄)-Alkyl ist, wobei Alkyl substituiert sein kann mit 1 bis 2 Substituenten, wobei die Substituenten un- abhängig voneinander ausgewählt werden aus der Gruppe, bestehend aus Amino, Hydroxy und (C₁-C₆)-Alkylamino,
oder
R⁶ und R⁷ zusammen mit dem Stickstoffatom, an das sie gebunden sind, ein Piperazinyl bilden, wobei Piperazinyl substituiert sein kann mit 1 bis 3 Substituenten, wobei die Substituenten unabhängig voneinander ausgewählt werden aus der Gruppe, bestehend aus Amino, Hydroxy, gegebenenfalls Amino substituiertem (C₁-C₆)-Alkyl, und (C₁-C₆)-Alkylamino,
und ihre Salze, ihre Solvate und die Solvate ihrer Salze.

Bevorzugt im Rahmen der vorliegenden Erfindung sind auch Verbindungen der Formel (I), worin
- R¹: gleich 2-Aminoethyl, 3-Aminoprop-1-yl, 4-Aminobut-1-yl oder 3-Amino-2-hydroxyprop- 1-yl ist,
- R²: gleich Wasserstoff, Methyl oder Ethyl ist,
- R³: gleich -NR⁶R⁷ ist,
- R⁴: gleich Wasserstoff, Fluor, Chlor, Amino, Hydroxy oder Methyl ist,
- R⁵: gleich Wasserstoff, Fluor oder Hydroxy ist, wobei R⁵ gleich Fluor ist, wenn R⁴ gleich Hydroxy ist,
- R⁶: gleich eine Gruppe der Formel 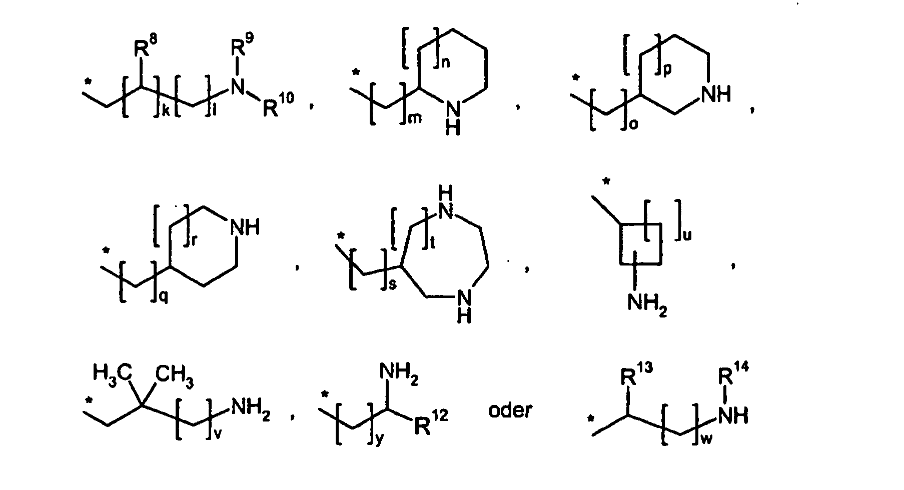
ist,
wobei
- R⁸: gleich Wasserstoff oder Hydroxy ist,
R⁹ und R¹⁴ unabhängig voneinander Wasserstoff, Methyl oder eine Gruppe der Formel 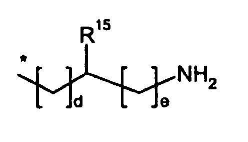 sind,
worin
* die Anknüpfstelle an das Stickstoffatom ist,
- R¹⁵: gleich Wasserstoff oder *-(CH₂)_{f}-NH₂ ist, worin f eine Zahl 1, 2 oder 3 ist,
- d: eine Zahl 0, 1, 2 oder 3 ist
und e eine Zahl 1, 2 oder 3 ist,
- R¹⁰: gleich Wasserstoff oder Aminoethyl ist,
oder
und R¹⁰ bilden zusammen mit dem Stickstoffatom an das sie gebunden sind einen Piperazin-Ring,
R¹² und R¹³ unabhängig voneinander eine Gruppe der Formel *-(CH₂)_{Z1}-OH oder *-(CH₂)_{Z2}-NH₂ sind,
worin
* die Anknüpfstelle an das Stickstoffatom ist,

Z1 und Z2 unabhängig voneinander eine Zahl 1, 2, 3 oder 4 sind,
k und t unabhängig voneinander eine Zahl 0 oder 1 sind,
l, w und y unabhängig voneinander eine Zahl 1, 2, 3 oder 4 ist,
m, r, s und v unabhängig voneinander eine Zahl 1 oder 2 sind,
n, o, p und q unabhängig voneinander eine Zahl 0, 1 oder 2 sind,
- u: eine Zahl 0, 1, 2 oder 3 ist, 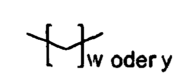
unabhängig voneinander bei w oder y gleich 3 eine Hydroxy-Gruppe am mittleren Kohlenstoffatom der Dreierkette tragen kann,
* die Anknüpfstelle an das Stickstoffatom ist,
- R⁷: gleich Wasserstoff ist,
und ihre Salze, ihre Solvate und die Solvate ihrer Salze.

Bevorzugt im Rahmen der vorliegenden Erfindung sind auch Verbindungen der Formel (I), worin
- R¹: gleich 2-Aminoethyl, 3-Aminoprop-1-yl, 4-Aminobut-1-yl oder 3-Amino-2-hydroxyprop- 1-yl ist,
- R²: gleich Wasserstoff, Methyl oder Ethyl ist,
- R³: gleich -NR⁶R⁷ ist,
- R⁴: gleich Wasserstoff, Fluor, Chlor, Amino, Hydroxy oder Methyl ist,
- R⁵: gleich Wasserstoff, Fluor oder Hydroxy ist, wobei R⁵ gleich Fluor ist, wenn R⁴ gleich Hydroxy ist,
- R⁶: gleich eine Gruppe der Formel 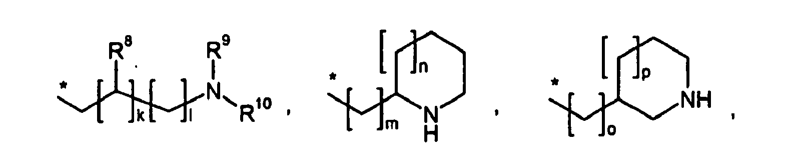 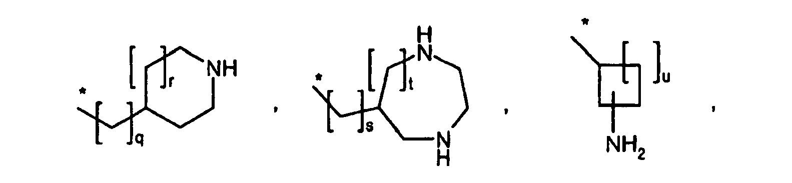 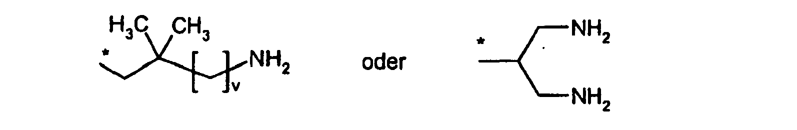
ist,
wobei
- R⁸: gleich Wasserstoff oder Hydroxy ist,
- R⁹: gleich Wasserstoff oder Methyl ist,
- R¹⁰: gleich Wasserstoff ist,
oder
R⁹ und R¹⁰ bilden zusammen mit dem Stickstoffatom an das sie gebunden sind einen Piperazin-Ring
k und t unabhängig voneinander eine Zahl 0 oder 1 sind,
- l: eine Zahl 1, 2, 3 oder 4 ist,
m, r, s und v unabhängig voneinander eine Zahl 1 oder 2 sind,
n, o, p und q unabhängig voneinander eine Zahl 0, 1 oder 2 sind,
- u: eine Zahl 0, 1, 2 oder 3 ist,
* die Anknüpfstelle an das Stickstoffatom ist,
- k⁷: gleich Wasserstoff ist,
und ihre Salze, ihre Solvate und die Solvate ihrer Salze.

Bevorzugt im Rahmen der vorliegenden Erfindung sind auch Verbindungen der Formel (I), bei denen
- R¹: gleich 3-Aminoprop-1-yl oder 3-Amino-2-hydroxyprop-1-yl ist,
- R²: gleich Wasserstoff oder Methyl ist,
- R³: gleich -NR⁶R⁷ ist,
- R⁴: gleich Wasserstoff, Fluor, Chlor oder Methyl ist,
- R⁵: gleich Wasserstoff ist,
- R⁶: gleich eine Gruppe der Formel 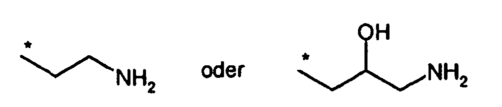
ist,
wobei
* gleich die Anknüpfstelle an das Stickstoffatom ist,
- R⁷: gleich Wasserstoff ist,
und ihre Salze, ihre Solvate und die Solvate ihrer Salze.

Gegenstand der Erfindung ist weiterhin ein Verfahren zur Herstellung der Verbindungen der Formel (I) oder ihrer Salze, ihrer Solvate oder der Solvate ihrer Salze, wobei nach Verfahren

### [A] Verbindungen der Formel

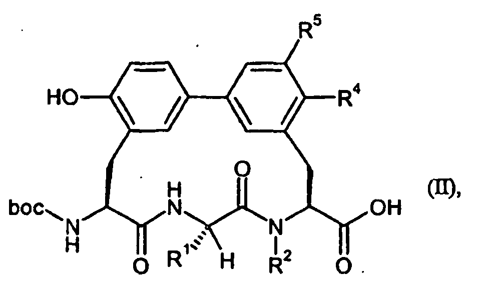

worin R¹, R², R⁴ und R⁵ die oben angegebene Bedeutung haben und boc gleich *tert-*Butoxycarbonyl ist,
in einem zweistufigen Verfahren zunächst in Gegenwart von einem oder mehreren Dehydratisierungsreagenzien mit Verbindungen der Formel

HNR⁶R⁷ (III),

worin R⁶ und R⁷ die oben angegebene Bedeutung haben,
und anschließend mit einer Säure zu Verbindungen der Formel 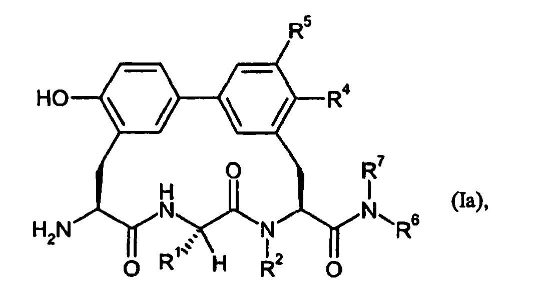 worin R¹ R², R⁴ und R⁵ die oben angegebene Bedeutung haben,
oder

### [B] Verbindungen der Formel

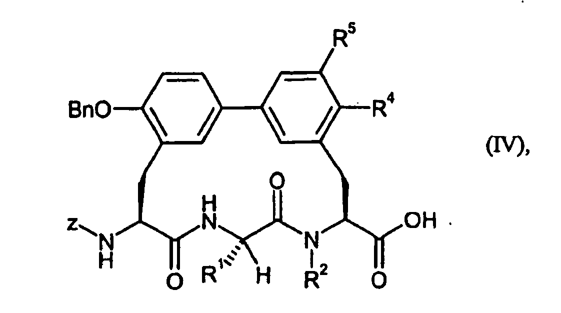

worin R¹, R², R⁴ und R⁵ die oben angegebene Bedeutung haben und Z gleich Benzyloxycarbonyl ist,
in einem zweistufigen Verfahren zunächst in Gegenwart von einem oder mehreren Dehydratisierungsreagenzien mit Verbindungen der Formel (III) und anschließend mit einer Säure oder durch Hydrogenolyse zu Verbindungen der Formel (Ia),
oder

### [C] Verbindungen der Formel (IV) mit einer Säure oder durch Hydrogenolyse zu Verbindungen der Formel

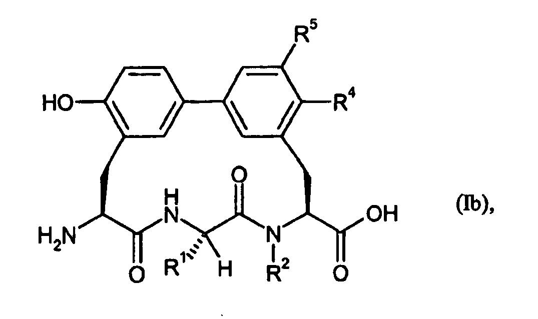

worin R¹, R², R⁴ und R⁵ die oben angegebene Bedeutung haben,
oder

### [D] Verbindungen der Formel

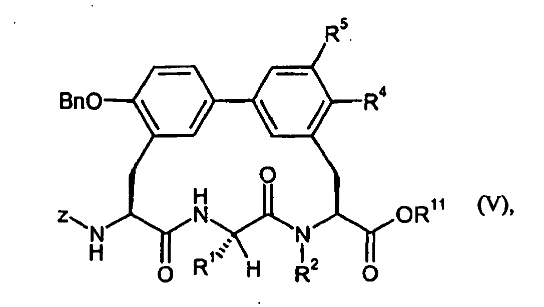

worin R¹, R², R⁴ und R⁵ die oben angegebene Bedeutung haben, und
- R¹¹: gleich Benzyl, Methyl oder Ethyl ist,
mit einer Säure oder durch Hydrogenolyse, gegebenenfalls durch anschließende Umsetzung mit einer Base zur Verseifung des Methyl- oder Ethylesters, zu Verbindungen der Formel (Ib), umgesetzt werden.

Verbindungen der Formel (I) sind Verbindungen der Formeln (Ia) und (Ib).

Die freie Base der Salze kann zum Beispiel durch Chromatographie an einer Reversed Phase Säule mit einem Acetonitril-Wasser-Gradienten unter Zusatz einer Base erhalten werden, insbesondere durch Verwendung einer RP18 Phenomenex Luna C18(2) Säule und Diethylamin als Base.

Weiterer Gegenstand der Erfindung ist ein Verfahren zur Herstellung der Verbindungen der Formel (I) oder ihrer Solvate nach Anspruch 1, bei dem Salze der Verbindungen oder Solvate der Salze der Verbindungen durch Chromatographie unter Zusatz einer Base in die Verbindungen überfährt werden.

Eine Hydroxygruppe im Rest R¹ ist gegebenenfalls während der Umsetzung mit Verbindungen der Formel (III) mit einer *tert-*Butyldimethylsilyl-Gruppe geschützt, die im zweiten Reaktionsschritt abgespalten wird.

Reaktive Funktionalitäten in den Resten R⁶ und R⁷ von Verbindungen der Formel (III) werden bereits geschützt mit in die Synthese eingebracht, bevorzugt sind säurelabile Schutzgruppen (z.B. boc).

Die Umsetzung der ersten Stufe der Verfahren [A] und [B] erfolgt im Allgemeinen in inerten Lösungsmitteln, gegebenenfalls in Gegenwart einer Base, bevorzugt in einem Temperaturbereich von 0°C bis 40°C bei Normaldruck.

Als Dehydratisierungsreagenzien eignen sich hierbei beispielsweise Carbodiimide wie z.B. *N,N'-*Diethyl-, *N,N*'-Dipropyl-, *N*,*N*-Diisopropyl-, *N*,*N*'-Dicyclohexylcarbodiimid, *N*-(3-Dimethylamino-isopropyl)-*N*'-ethylcarbodiimid-Hydrochlorid (EDC), *N*-Cyclohexylcarbodiimid-*N*'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert-*Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexa-fluorophosphat, oder O-(Benzotriazol-1-yl)-*N*,*N*,*N*',*N*'-tetra-methyluroniumhexafluorophosphat (HBTU), 2-(2-Oxo-1-(2H)-pyridyl)-1,1,3,3-tetramethyl-uroniumtetrafluoroborat (TPTU) oder O-(7-Azabenzotriazol-1-yl)-*N*,*N*,*N*,'*N*'-tetramethyl-uroniumhexafluorophosphat (HATU), oder 1-Hydroxybenztriazol (HOBt), oder Benzotriazol-1-yloxytris(dimethylamino)-phosphoniumhexafluoro-phosphat (BOP), oder Mischungen aus diesen, oder Mischung aus diesen zusammen mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, *N*-Methylpiperidin, 4-Dimethylaminopyridin oder Diisopropylethylamin.

Vorzugsweise wird die Kondensation mit HATU in Gegenwart einer Base, insbesondere Diisopropylethylamin, durchgeführt.

Inerte Lösungsmittel sind beispielsweise Halogenkohlenwasserstoffe wie Dichlormethan oder Trichlormethan, Kohlenwasserstoff wie Benzol, oder Nitromethan, Dioxan, Dimethylformamid oder Acetonitril. Ebenso ist es möglich, Gemische der Lösemittel einzusetzen. Besonders bevorzugt ist Dimethylformamid.

Die Umsetzung mit einer Säure in der zweiten Stufe der Verfahren [A] und [B] sowie die Umsetzung mit einer Säure in den Verfahren [C] und [D] erfolgt bevorzugt in einem Temperaturbereich von 0°C bis 40°C bei Normaldruck.

Als Säuren eignen sich hierbei Chlorwasserstoff in Dioxan, Bromwasserstoff in Essigsäure oder Trifluoressigsäure in Methylenchlorid.

Die Hydrogenolyse in der zweiten Stufe des Verfahrens [B] sowie die Hydrogenolyse in den Verfahren [C] und [D] erfolgt im Allgemeinen in einem Lösungsmittel in Gegenwart von Wasserstoff und Palladium auf Aktivkohle, bevorzugt in einem Temperaturbereich von 0°C bis 40°C bei Normaldruck.

Lösungsmittel sind beispielsweise Alkohole wie Methanol, Ethanol, n-Propanol oder iso-Propanol, in einem Gemisch mit Wasser und Eisessig, bevorzugt ist ein Gemisch aus Ethanol, Wasser und Eisessig.

Die Verseifung in Verfahrem [D] kann zum Beispiel erfolgen, wie bei der Umsetzung von Verbindungen der Formel (V) zu Verbindungen der Formel (IV) beschrieben.

Die Verbindungen der Formel (III) sind bekannt oder können analog bekannten Verfahren hergestellt werden.

Die Verbindungen der Formel (II) sind bekannt oder können hergestellt werden, indem Verbindungen der Formel (Ib) mit Di-(*tert-*butyl)-dicarbonat in Gegenwart einer Base umgesetzt werden.

Die Umsetzung erfolgt im Allgemeinen in einem Lösungsmittel, bevorzugt in einem Temperaturbereich von 0°C bis 40°C bei Normaldruck.

Basen sind beispielsweise Alkalihydroxide wie Natrium- oder Kaliumhydroxid, oder Alkalicarbonate wie Cäsiumcarbonat, Natrium- oder Kaliumcarbonat, oder andere Basen wie DBU, Triethylamin oder Diisopropylethylamin, bevorzugt ist Natriumhydroxid oder Natriumcarbonat.

Lösungsmittel sind beispielsweise Halogenkohlenwasserstoffe wie Methylenchlorid oder 1,2-Dichlorethan, Alkohole wie Methanol, Ethanol oder iso-Propanol, oder Wasser.

Vorzugsweise wird die Umsetzung mit Natriumhydroxid in Wasser oder Natriumcarbonat in Methanol durchgeführt.

Die Verbindungen der Formel (IV) sind bekannt oder können hergestellt werden, indem in Verbindungen der Formel (V) der Benzyl-, Methyl- oder Ethylester verseift wird.

Die Umsetzung erfolgt im Allgemeinen in einem Lösungsmitteln, in Gegenwart einer Base, bevorzugt in einem Temperaturbereich von 0°C bis 40°C bei Normaldruck.

Basen sind beispielsweise Alkalihydroxide wie Lithium-, Natrium- oder Kaliumhydroxid, bevorzugt ist Lithiumhydroxid.

Lösungsmittel sind beispielsweise Halogenkohlenwasserstoffe wie Dichlormethan oder Trichlormethan, Ether wie Tetrahydrofuran oder Dioxan, oder Alkohole wie Methanol, Ethanol oder 1-sopropanol, oder Dimethylformamid. Ebenso ist es möglich, Gemische der Lösungsmittel oder Gemische der Lösungsmittel mit Wasser einzusetzen. Besonders bevorzugt sind Tetrahydrofuran oder ein Gemisch aus Methanol und Wasser.

Die Verbindungen der Formel (V) sind bekannt oder können hergestellt werden, indem Verbindungen der Formel 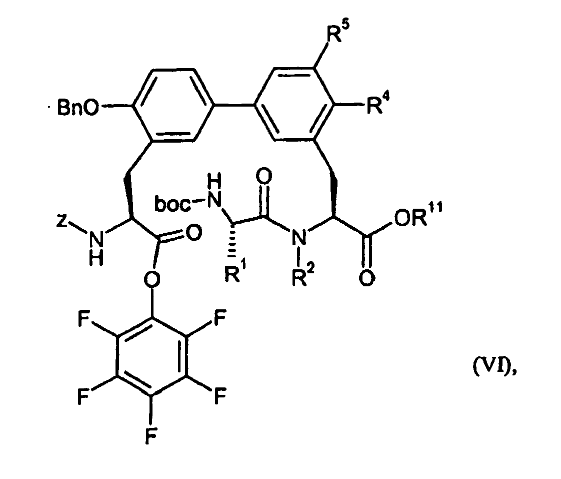 worin R¹, R², R⁴, R⁵ und R¹¹ die oben angegebene Bedeutung haben,
in der ersten Stufe mit Säuren, wie für die zweite Stufe der Verfahren [A] und [B] beschrieben, und in der zweiten Stufe mit Basen umgesetzt werden.

In der zweiten Stufe erfolgt die Umsetzung mit Basen im Allgemeinen in einem Lösungsmittel, bevorzugt in einem Temperaturbereich von 0°C bis 40°C bei Normaldruck.

Basen sind beispielsweise Alkalihydroxide wie Natrium- oder Kaliumhydroxid, oder Alkalicarbonate wie Cäsiumcarbonat, Natrium- oder Kaliumcarbonat, oder andere Basen wie DBU, Triethylamin oder Diisopropylethylamin, bevorzugt ist Triethylamin.

Lösungsmittel sind beispielsweise Halogenkohlenwasserstoffe wie Chloroform, Methylenchlorid oder 1,2-Dichlorethan, oder Tetrahydrofuran, oder Gemische der Lösungsmittel, bevorzugt ist Methylenchlorid oder Tetrahydrofuran.

Die Verbindungen der Formel (VI) sind bekannt oder können hergestellt werden, indem Verbindungen der Formel 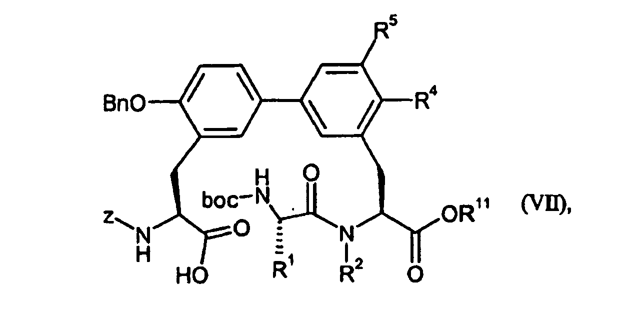 worin R¹, R², R⁴, R⁵ und R¹¹ die oben angegebene Bedeutung haben,
mit Pentafluorphenol in Gegenwart von Dehydratisierungsreagenzien, wie für die erste Stufe der Verfahren [A] und [B] beschrieben, umgesetzt werden.
Die Umsetzung erfolgt bevorzugt mit DMAP und EDC in Dichlormethan in einem Temperaturbereich von -40°C bis 40°C bei Normaldruck.
Die Verbindungen der Formel (VII) sind bekannt oder können hergestellt werden, indem Verbindungen der Formel 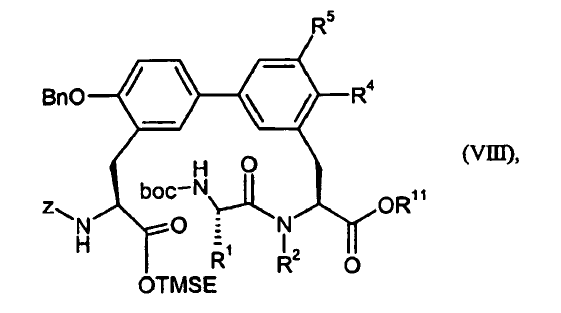 worin R¹, R², R⁴, R⁵ und R¹¹ die oben angegebene Bedeutung haben,
mit Fluorid, insbesondere mit Tetrabutylammoniumfluorid, umgesetzt werden.

Die Umsetzung erfolgt im Allgemeinen in einem Lösungsmitteln, bevorzugt in einem Temperaturbereich von -10°C bis 30°C bei Normaldruck.

Inerte Lösungsmittel sind beispielsweise Halogenkohlenwasserstoffe wie Dichlormethan, oder Kohlenwasserstoffe wie Benzol oder Toluol, oder Ether wie Tetrahydrofuran oder Dioxan, oder Dimethylformamid. Ebenso ist es möglich, Gemische der Lösemittel einzusetzen. Bevorzugte Lösungsmittel sind Tetrahydrofuran und Dimethylformamid.

Die Verbindungen der Formel (VIII) sind bekannt oder können hergestellt werden, indem Verbindungen der Formel 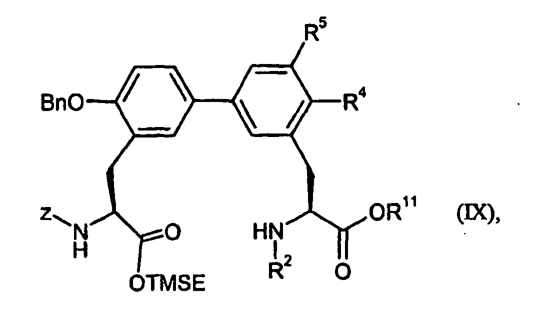 worin R², R⁴, R⁵ und R¹¹ die oben angegebene Bedeutung haben,
mit Verbindungen der Formel 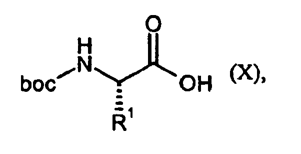 worin R¹ die oben angegebene Bedeutung hat,
in Gegenwart von Dehydratisierungsreagenzien, wie für die erste Stufe der Verfahren [A] und [B] beschrieben, umgesetzt werden.

Die Verbindungen der Formel (IX) sind bekannt oder können analog den im Beispielteil beschriebenen Verfahren hergestellt werden.

Die Verbindungen der Formel (X) sind bekannt oder können analog bekannten Verfahren hergestellt werden.

Die erfindungsgemäßen Verbindungen zeigen ein nicht vorhersehbares, wertvolles pharmakologisches und pharmakokinetisches Wirkspektrum.

Sie eignen sich daher zur Verwendung als Arzneimittel zur Behandlung und/oder Prophylaxe von Krankheiten bei Menschen und Tieren.

Die erfindungsgemäßen Verbindungen können aufgrund ihrer pharmakologischen Eigenschaften allein oder in Kombination mit anderen Wirkstoffen zur Behandlung und/oder Prophylaxe von Infektionskrankheiten, insbesondere von bakteriellen Infektionen, eingesetzt werden. Beispielsweise können lokale und/oder systemische Erkrankungen behandelt und/oder verhindert werden, die durch die folgenden Erreger oder durch Mischungen der folgenden Erreger verursacht werden:
Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxytocy), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen topisch anwendbaren Zubereitungen verhindert, gebessert oder geheilt werden können, seien beispielsweise genannt:
Infektionskrankheiten beim Menschen wie z. B. septische Infektionen, Knochen- und Gelenkinfektionen, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, septische Arthritis, Mastitis, Tonsillitis, Genital-Infektionen und Augeninfektionen.

Außer beim Menschen können bakterielle Infektionen auch bei anderen Spezies behandelt werden. Beispielhaft seien genannt:
Schwein: Coli-diarrhoe, Enterotoxamie, Sepsis, Dysenterie, Salmonellose, Metritis-Mastitis-Agalaktiae-Syndrom, Mastitis;
Wiederkäuer (Rind, Schaf, Ziege): Diarrhoe, Sepsis, Bronchopneumonie, Salmonellose, Pasteurellose, Mykoplasmose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;
Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz-und Zierfischen behandelt werden, wobei sich das antibakterielle Spektrum über die vorher genannten Erreger hinaus auf weitere Erreger wie z.B. Pasteurella, Brucella, Campylobacter, Listeria, Erysipelothris, Corynebakterien, Borellia, Treponema, Nocardia, Rikettsie, Yersinia, erweitert.

Weiterer Gegenstand der vorliegenden Erfindung ist der Einsatz der erfindungsgemäßen Verbindungen zur Behandlung und/oder Prophylaxe von Erkrankungen, vorzugsweise von bakteriellen Krankheiten, insbesondere von bakteriellen Infektionen.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist die Verwendung einer antibakteriell wirksamen Menge der erfindungsgemäßen Verbindungen zur Herstellung eines

Medikaments zur Behandlung und/oder Prophylaxe von Erkrankungen, insbesondere der zuvor genannten Erkrankungen.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei parenteraler Applikation Mengen von etwa 5 bis 250 mg/kg Körpergewicht je 24 h zur Erzielung wirksamer Ergebnisse zu verabreichen. Bei oraler Applikation beträgt die Menge etwa 5 bis 100 mg/kg Körpergewicht je 24 h.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

**A. Beispiele**

| **Verwendete Abkürzungen:** | |
|---|---|
| abs. | absolut |
| aq. | wässrig |
| Bn | Benzyl |
| Boc | *tert-*Butoxycarbonyl |
| CDCl₃ | Deuterochloroform |
| CH | Cyclohexan |
| D | dublett (im¹H-NMR) |
| Dd | dublett von dublett (im ¹H-NMR) |
| DCC | Dicyclohexylcarbodiimid |
| DIC | Diisopropylcarbodiimid |
| DIEA | Diisopropylethylamin (Hünig-Base) |
| DMSO | Dimethylsulfoxid |
| DMAP | 4-*N*,*N*-Dimethylaminopyridin |
| DMF | Dimethylformamid |
| d. Th. | der Theorie |
| EDC | *N*'-(3-Dimethylaminopropyl)-*N*-ethylcarbodiimid x HCl |
| EE | Ethylacetat (Essigsäureethylester) |
| ESI | Elektrospray-Ionisation (bei MS) |
| ges. | gesättigt |
| HATU | *O-*(7-Azabenzotriazol-1-yl)-*N*,*N*,*N*',*N*'-tetramethyluronium-hexafluorophosphat |
| HBTU | *O-*(Benzotriazol-1-yl)-*N*,*N*,*N*;*N*'-tetramethyluronium-hexafluorophosphat |
| HOBt | 1-Hydroxy-1H-benzotriazol x H₂O |
| H | Stunde(n) |
| HPLC | Hochdruck-, Hochleistungsflüssigchromatographie |
| LC-MS | Flüssigchromatographie-gekoppelte Massenspektroskopie |
| M | multiplett (im ¹H-NMR) |
| Min | Minute |
| MS | Massenspektroskopie |
| NMR | Kernresonanzspektroskopie |
| MTBE | Methyl-*tert-*butylether |
| Pd/C | Palladium/Kohle |
| Proz. | Prozent |
| Q | quartett (im ¹H-NMR) |
| R_{f} | Retentionsindex (bei DC) |
| RP | Reverse Phase (bei HPLC) |
| RT | Raumtemperatur |
| Rₜ | Retentionszeit (bei HPLC) |
| S | singulett (im ¹H-NMR) |
| T | triplett (im ¹H-NMR) |
| TBS | *tert-*Butyldimethylsilyl |
| TFA | Trifluoressigsäure |
| THF | Tetrahydrofuran |
| TMSE | 2-(Trimethylsilyl)-ethyl |
| TPTU | 2-(2-Oxo-1(2H)-pyridyl)-1,1,3,3-tetramethyluroniumtetrafluoroborat |
| Z | Benzyloxycarbonyl |

### LC-MS- und HPLC-Methoden:

**Methode 1 (LC-MS):** Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%ige Ameisensäure, Eluent B: 11 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 208- 400 nm.
**Methode 2 (LC-MS):** Gerätetyp MS: Micromass ZQ; Gerätetyp HPLC: Waters Alliance 2795; Säule: Phenomenex Synergi 2µ Hydro-RP Mercury 20 mm x 4mm; Eluent A: 11 Wasser + 0.5 ml 50%ige Ameisensäure, Eluent B: 11 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.
**Methode 3 (LC-MS):** Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 11 Wasser + 0.5 ml 50%ige Ameisensäure, Eluent B: 11 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 mi/min; Ofen: 50°C; UV-Detektion: 210 nm.
**Methode 4 (LC-MS):** Instrument: Micromass Platform LCZ mit HPLC Agilent Serie 1100; Säule: Grom-SIL120 ODS-4 HE, 50 mm x 2.0 mm, 3 µm; Eluent A: 11 Wasser + 1 ml 50%ige Ameisensäure, Eluent B: 11 Acetonitril + 1 ml 50%ige Ameisensäure; Gradient: 0.0 min 100%A → 0.2 min 100%A → 2.9 min 30%A → 3.1 min 10%A → 4.5 min 10%A; Ofen: 55°C; Fluss: 0.8 ml/min; UV-Detektion: 208-400 nm.
**Methode 5 (LC-MS):** Gerätetyp MS: Micromass ZQ; Gerätetyp HPLC: Waters Alliance 2795; Säule: Merck Chromolith SpeedROD RP-18e 50 mm x 4.6mm; Eluent A: Wasser + 500 µl 50%ige Ameisensäure / 1; Eluent B: Acetonitril + 500 µl 50%ige Ameisensäure / 1; Gradient: 0.0 min 10%B→ 3.0 min 95%B→ 4.0 min 95%B; Ofen: 35°C; Fluss: 0.0 min 1.0 ml/min→ 3.0 min 3.0 ml/min→ 4.0 min 3.0 ml/min; UV-Detektion: 210 nm.
**Methode 6 (LC-MS):** Gerätetyp MS: Micromass ZQ; Gerätetyp HPLC: HP 1100 Series; UV DAD; Säule: Grom-Sil 120 ODS-4 HE 50 mm x 2 mm, 3.0 µm; Eluent A: Wasser + 500 µl 50%ige Ameisensäure / 1, Eluent B: Acetonitril + 500 µl 50%ige Ameisensäure / 1; Gradient: 0.0 min 70%B → 4.5 min 90%B; Ofen: 50°C; Fluss: 0.8 ml/min; UV-Detektion: 210 nm.
**Methode 7 (LC-MS):** Gerätetyp MS: Micromass ZQ; Gerätetyp HPLC: Waters Alliance 2790; Säule: Grom-Sil 120 ODS-4 HE 50 mm x 2 mm, 3.0 µm; Eluent A: Wasser + 500 µl 50%ige Ameisensäure / 1, Eluent B: Acetonitril + 500 µl 50%ige Ameisensäure /1; Gradient: 0.0 min 70%B → 4.5 min 90%B; Ofen: 45°C; Fluss: 0.8 ml/min; UV-Detektion: 210 nm.
**Methode 8 (LC-MS):** Gerätetyp MS: Micromass ZQ; Gerätetyp HPLC: HP 1100 Series; UV DAD; Säule: Grom-Sil 120 ODS-4 HE 50 mm x 2 mm, 3.0 µm; Eluent A: Wasser + 500 µl 50%ige Ameisensäure /1, Eluent B: Acetonitril + 500 µl 50%ige Ameisensäure /1; Gradient: 0.0 min 0%B → 2.9 min 70%B → 3.1 min 90%B → 4.5 min 90%B; Ofen: 50 °C; Fluss: 0.8 ml/min; UV-Detektion: 210 nm.
**Methode 9 (LC-MS):** Gerätetyp MS: Micromass ZQ; Gerätetyp HPLC: Waters Alliance 2790; Säule: Grom-Sil 120 ODS-4 HE 50 mm x 2 mm, 3.0 µm; Eluent A: Wasser + 500 µl 50%ige Ameisensäure; Eluent B: Acetonitril + 500 µl 50%ige Ameisensäure / 1; Gradient: 0.0 min 5%B → 2.0 min 40%B → 4.5 min 90%B → 5.5 min 90%B; Ofen: 45°C; Fluss: 0.0 min 0.75 ml/min → 4.5 min 0.75 ml/min 5.5 min → 5.5 min 1.25 ml/min; UV-Detektion: 210 nm.
**Methode 10 (LC-MS):** Gerätetyp MS: Micromass ZQ; Gerätetyp HPLC: Waters Alliance 2790; Säule: Grom-Sil 120 ODS-4 HE 50 mm x 2 mm, 3.0 µm; Eluent A: Wasser + 500 µl 50%ige Ameisensäure /1, Eluent B: Acetonitril + 500 µl 50%ige Ameisensäure / 1; Gradient: 0.0 min 90%B → 4.5 min 90%B; Ofen: 45°C; Fluss: 0.8 ml/min; UV-Detektion: 210 nm.
**Methode 11 (HPLC):** Instrument: HP 1100 mit DAD,Detektion; Säule: Kromasil RP-18, 60 mm x 2 mm, 3.5 µm; Eluent A: 5 ml HClO₄/l Wasser, Eluent B: Acetonitril; Gradient: 0 min 2%B, 0.5 min 2%B, 4.5 min 90%B, 6.5 min 90%B; Fluss: 0.75 ml/min; Ofen: 30°C; UV-Detektion: 210 nm. **Methode 12 (HPLC):** Instrument: HP 1100 mit DAD-Detektion; Säule: Kromasil RP-18, 60 mm x 2 mm, 3.5 µm; Eluent A: 5 ml HClO₄/l Wasser, Eluent B: Acetonitril; Gradient: 0 min 2%B, 0.5 min 2%B, 4.5 min 90%B, 15 min 90%B; Fluss: 0.75ml/min; Ofen: 30°C; UV-Detektion: 210 nm.
**Methode 13 (HPLC):** Instrument: HP 1100 mit DAD-Detektion; Säule: Kromasil RP-18, 60 mm x 2 mm, 3.5 µm; Eluent A: 5 ml HClO₄/l Wasser, Eluent B: Acetonitril; Gradient: 0 min 2%B, 0.5 min 2%B, 4.5 min 90%B, 9 min 90%B; Fluss: 0.75 ml/min; Ofen: 30°C; UV-Detektion: 210 nm.
**Methode 14 (LC-MS)**: Gerätetyp MS: Micromass ZQ; Gerätetyp HPLC: Waters Alliance 2790; Säule: Grom-Sil 120 ODS-4 HE 50 mm x 2 mm, 3.0 µm; Eluent A: Wasser + 500µl 50%ige Ameisensäure /l; Eluent B.: Acetonitril + 500µl 50%ige Ameisensäure /l; Gradient: 0.0 min 0%B → 0.2 min 0%B → 2.9 min 700/oB → 3.1 min 90%B → 4.5 min 90%B; Fluss: 0.8 ml/min; Ofen: 45°C; W-Detektion: 210 nm.
**Methode 15 (LC-MS)**: Instrument: Micromass Quattro LCZ, mit HPLC Agilent Serie 1100; Säule: Grom-SIL120 ODS-4 HE, 50 mm x 2.0 mm, 3 µm; Eluent A: 11 Wasser + 1 ml 50%ige Ameisensäure, Eluent B: 11 Acetonitril + 1 ml 50%ige Ameisensäure; Gradient: 0.0 min 100%A → 0.2 min 100%A → 2.9 min 30%A → 3.1 min 10%A → 4.5 min 10%A; Ofen: 55°C; Fluss: 0.8 ml/min; UV-Detektion: 208-400 nm.
**Methode 16 (LC-MS)**: Gerätetyp MS: Micromass ZQ; Gerätetyp HPLC: Waters Alliance 2795; Säule: Merck Chromolith SpeedROD RP-18e 50 mm x 4.6 mm; Eluent A: Wasser + 500 µl 50%ige Ameisensäure /l, Eluent B: Acetonitril + 500 µl 50%ige Ameisensäure / l; Gradient: 0.0 min 10%B → 2.0 min 95%B → 4.0 min 95%B; Ofen: 35°C; Fluss: 0.0 min 1.0 ml/min → 2.0 min 3.0 ml/min → 4.0 min 3.0 ml/min; UV-Detektion: 210 nm.
**Methode 17 (LC-MS)**: Instrument: Micromass Platform LCZ mit HPLC Agilent Serie 1100; Säule: Thermo HyPURITY Aquastar 3µ 50 mm x 2.1 mm; Eluent A: 11 Wasser + 0.5 ml 50%ige Ameisensäure, Eluent B: 11 Acetonitril + 0.5 ml 50%ige Ameisensäure; Gradient: 0.0 min 100%A → 0.2 min 100%A → 2.9 min 30%A → 3.1 min 10%A → 5.5 min 10%A; Ofen: 50°C; Fluss: 0.8 ml/min; UV-Detektion: 210 nm.
**Methode 18 (LC-MS)**: Gerätetyp MS: Micromass ZQ; Gerätetyp HPLC: Waters Alliance 2790; Säule: Grom-Sil 120 ODS-4 HE 50 x 2 mm, 3.0 µm; Eluent B: Acetonitril + 0.05% Ameisensäure, Eluent A: Wasser + 0.05% Ameisensäure; Gradient: 0.0 min 70%B → 4.5 min 90%B → 5.5 min 90%B; Ofen: 45°C; Fluss: 0.0 min 0.75 ml/min → 4.5 min 0.75 ml/min→ 5.5 min 1.25 ml/min; UV-Detektion: 210 nm.

### Ausgangsverbindungen

Die Buchstabenbezeichnung in den Ausgangsverbindungen stehen für das Strukturelement des Kopfes der erfindungsgemäßen Verbindungen in Verbindung mit einem Rest R¹ (z.B. Ornithin (3-Aminoprop-1-yl) oder Hydroxy-Ornithin (3-Amino-2-hydroxyprop-1-yl)), wie in der folgenden Tabelle beschrieben. Die Zahlen stehen für verschiedene Reaktionstypen.

| **Serie** | **Strukturelement** | **R¹** | **R²** |
|---|---|---|---|
| **A** | 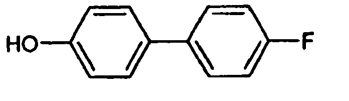 | Ornithin | H |
| **B** | 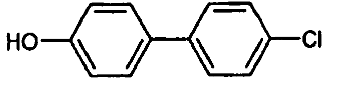 | Hydroxy-Ornithin | Methyl |
| **C** | 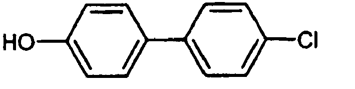 | Hydroxy-Ornithin | H |
| **D** | 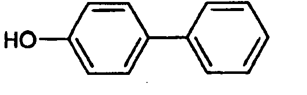 | Hydroxy-Ornithin | H |
| **E** | 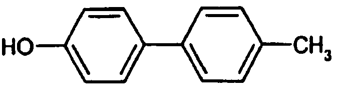 | Ornithin | H |
| **F** | 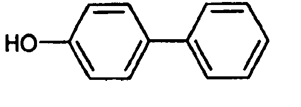 | Ornithin | H |
| **G** | 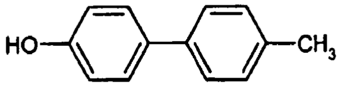 | Hydroxy-Ornithin | H |
| **H** | 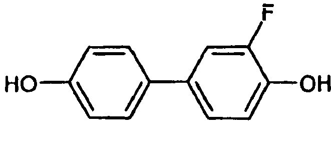 | Hydroxy-Ornithin | H |
| **I** | 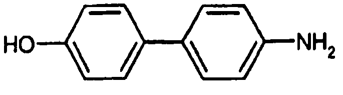 | Hydroxy-Ornithin | H |
| **J** | 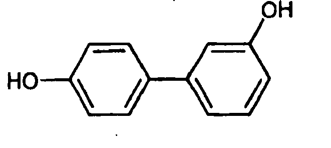 | Hydroxy-Ornithin | H |
| **K** | 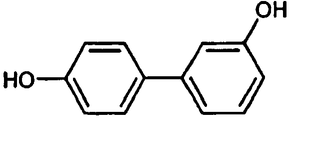 | Ornithin | H |
| **L** | 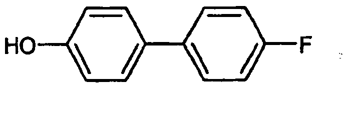 | Hydroxy-Ornithin | H |
| **M** | 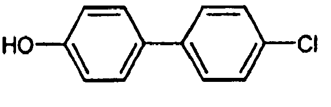 | Ornithin | Methyl |
| **N** | 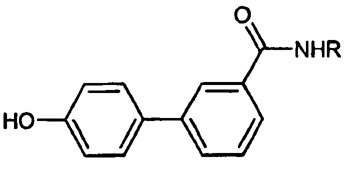 | Hydroxy-Ornithin | H |
| **O** | 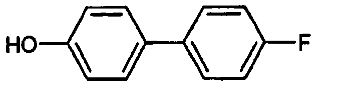 | Ornithin | Methyl |
| **P** | 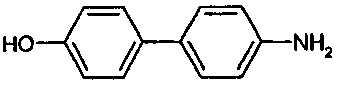 | Ornithin | H |

### Beispiel 1B

### (5-Brom-2-chlorphenyl)methanol

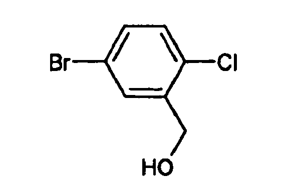

10 g (42.5 mmol) 5-Brom-3-chlorbenzoesäure werden in 135 ml THF gelöst. Bei 0°C werden 4.2 g (55.2 mmol, 5.24 ml) Boran-Dimethylsulfid-Komplex zugetropft. Anschließend wird 25 min bei RT, dann 30 min unter Rückfluss nachgerührt. Unter Eiskühlung tropft man nacheinander 40 ml Wasser und 15 ml 2N Salzsäure zu und rührt für 45 min bei RT nach. Nach emeuter Zugabe von 15 ml 2N Salzsäure wird mehrmals mit Diethylether extrahiert. Die vereinigte organische Phase wird nacheinander mit 1N Salzsäure, Wasser, gesättigter Natriumhydrogencarbonat-Lösung und gesättigter Natriumchlorid-Lösung gewaschen, über Magnesiumsulfat getrocknet und im Vakuum eingeengt. Der Feststoff wird bis zur Gewichtskonstanz im Hochvakuum getrocknet.
Ausbeute: 9.1 g (97% d. Th.)
HPLC (Methode 11): Rₜ = 4.22 min
MS (EI): m/z = 220 (M)⁺
¹H-NMR (300 MHz, CDCl₃): δ = 1.90 (t, 1H), 4.75 (d, 2H), 7.20 (d, 1H), 7.35 (dd, 1H), 7.67 (d, 1H).

### Beispiel 2B

### 5-Brom-2-chlorbenzaldehyd

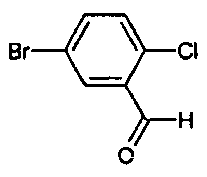

18 ml (0.26 mol) DMSO werden in 64 ml Dichlormethan vorgelegt und bei -78°C mit 16.1 g (0.127 mol, 11.1 ml) Oxalylchlorid versetzt. Nach 30 min wird eine Lösung von 13.1 g (59 mmol) (5-Brom-2-chlorphenyl)methanol in 100 ml Chloroform zugetropft. Nach 20 min wird mit 40 ml Triethylamin versetzt und die Reaktionsmischung wird langsam auf RT erwärmt. Nach Zugabe von 50 ml Wasser wird mehrmals mit Essigsäureethylester extrahiert. Die vereinigten organischen Phasen werden nacheinander mit 2N Salzsäure, Wasser, gesättigter Natriumhydrogencarbonat-Lösung und gesättigter Natriumchlorid-Lösung gewaschen. Die organische Phase wird über Magnesiumsulfat getrocknet und im Vakuum eingeengt. Der erhaltene Feststoff wird bis zur Gewichtskonstanz im Hochvakuum getrocknet.
Ausbeute: 12.7 g (94% d. Th.)
HPLC (Methode 11): Rₜ = 4.65 min
MS (EI): m/z = 218 (M)+
¹H-NMR (300 MHz, CDCl₃): δ = 7.32 (d, 1H), 7.65 (dd, 1H), 8.03 (d, 1H), 10.4 (s, 1H).

### Beispiel 3H

### 2-(Benzyloxy)-3-fluorbenzaldehyd

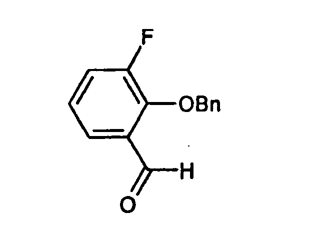

9.0 g (64 mmol) 3-Fluor-2-hydroxybenzaldehyd werden in 200 ml DMF gelöst, mit 10.7 g (77.1 mmol) Kaliumcarbonat und 8.4 ml (12 g, 71 mmol) Benzylbromid versetzt und 24 h bei 80°C gerührt. Man gießt auf 600 ml Wasser, extrahiert mehrfach mit Essigsäureethylester, trocknet die organische Phase über Natriumsulfat, engt im Vakuum ein und trocknet am Hochvakuum. Das Rohprodukt wird per Kieselgelchromatographie (Cyclohexan:Essigsäureethylester 2:1) gereinigt.
Ausbeute: 14.3 g (97% d.Th.)
HPLC (Methode 11): Rₜ = 4.82 min.
MS (DCI): m/z = 231 (M+H)⁺
¹H-NMR (300 MHz, CDCl₃): δ = 5.26 (s, 2H), 7.10 (m_{c}, 1H), 7.31-7.43 (m, 6H), 7.58 (dd, 1H), 10.25 (s, 1H).

### Beispiel 3J

### 3-(Benzyloxy)-5-hydroxybenzaldehyd

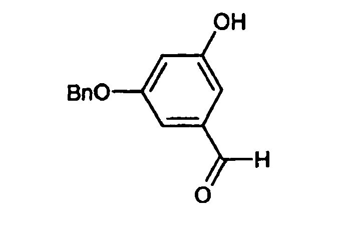

Die Herstellung erfolgt analog zu Beispiel 3H aus 5.0 g (36.2 mmol) 3,5-Dihydroxybenzaldehyd, 6.81 g (39.8 mmol) Benzylbromid und 11.8 g (36.2 mmol) Cäsiumcarbonat.
Ausbeute: 2.8 g (34% d. Th.)
LC-MS (Methode 8): Rₜ = 3.31 min
MS (EI): m/z = 227 (M+H)⁺
¹H-NMR (300 MHz, CDCl₃): δ = 5.10 (s, 2H), 5.21 (s, 1H), 6.75 (s, 1H), 6.96 (s, 1H), 7.08 (s, 1H), 7.25-7.45 (m, 5H), 9.88 (s, 1H).

### Beispiel 4A

### Benzyl-(2Z)-3-(5-brom-2-fluorphenyl)-2-[(tert-butoxycarbonyl)amino]acrylat

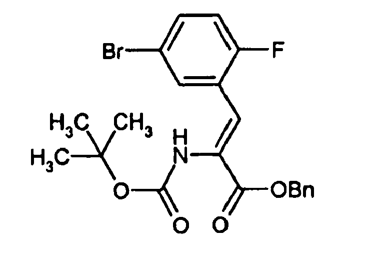

6.0 g (30 mmol) 5-Brom-2-fluorbenzaldehyd und 12.7 g (34 mmol) Benzyl-[(*tert*-butoxycarbonyl)-amino]-(dimethoxyphosphoryl)acetat werden in 90 ml THF vorgelegt und unter Aceton/Trockeneis-Kühlung bei -78°C mit 3.91 g (34 mmol) 1,1,3,3-Tetramethylguanidin versetzt. Nach 4 h im Kältebad wird langsam auf RT erwärmt und für weitere 12 h bei RT gerührt. Das Lösungsmittel wird im Vakuum abdestilliert, das Rohprodukt mit Essigsäureethylester aufgenommen und je einmal mit gesättigter Natriumhydrogencarbonat- und gesättigter Natriumchlorid-Lösung gewaschen.

Die organische Phase wird über Magnesiumsulfat getrocknet und im Vakuum zur Trockne eingeengt. Das Rohprodukt wird chromatographisch (Kieselgel, Cyclohexan/Essigsäureethylester 2:1) gereinigt.
Ausbeute: 14 g (95% d.Th.)
HPLC (Methode 11): Rₜ = 5.47 min.
MS (EI⁺): m/z = 450 (M+H)⁺
¹H-NMR (300 MHz, EDCl₃): δ = 1.39 (s, 9H), 5.30 (s, 2H), 6.53 (br. s, 1H), 6.94 (m, 1H), 7.28-7.46 (m, 6H), 7.67 (m, 1H).

Analog zu obiger Vorschrift werden die in der folgenden Tabelle aufgeführten Beispiele 4B, 4C, 4E, 4H bis 4J und 4N und 4P aus den entsprechenden Edukten hergestellt:

| **Beispiel-Nr.** | **Struktur** | **Hergestellt analog Beispiel-Nr.** | **Analytische Daten** |
|---|---|---|---|
| **4B** | 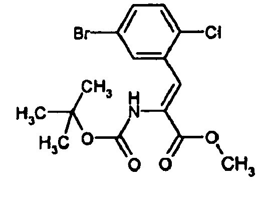 | 4A aus Beispiel 2B und Methyl-[(*tert*-butoxycarbonyl)-amino]-(dimethoxyphosphoryl)acetat | |
| **4C** | 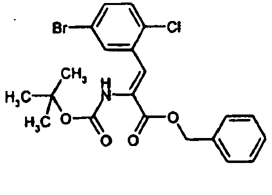 | 4A aus Beispiel 2B | LC-MS (Methode 2): Rₜ = 2.97 min. MS (ES): m/z = 466 (M+H)^{+ 1}H-NMR (300 MHz, CDCl₃): δ = 1.36 (s, 9H), 5.32 (s, 2H), 6.47 (br. s, 1H), 7.2-7.5 (m, 7H), 7.70 (d, 1H). |
| **4E** | 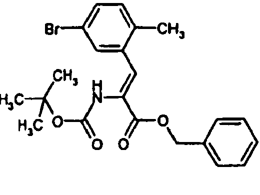 | 4A aus 5-Brom-2-methylbenzaldehyd | LC-MS (Methode 4): Rₜ = 3.38 min. MS (ES): m/z = 446 (M+H)^{+ 1}H-NMR (300 MHz, CDCl₃): δ = 1.35 (s, 9H), 2.28 (s, 3H), 5.30 (s, 2H), 6.21 (br. s, 1H), 7.04 (d, 1H), 7.21-7.46 (m, 7H), 7.10 (d, 1H). |
| **4H** | 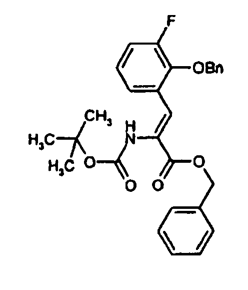 | 4A aus Beispiel 3H | LCMS (Methode 13): Rₜ = 5.52 min. MS (ESI): m/z = 478 (M+H)^{+ 1}H-NMR (300 MHz, CDCl₃): δ = 1.36 (s, 9H), 5.05 (s, 2H), 5.27 (s, 2H), 6.57 (br. s, 1H), 7.03 (m_{c}, 1H), 7.12-7.43 (m, 13H). |
| **4I** | 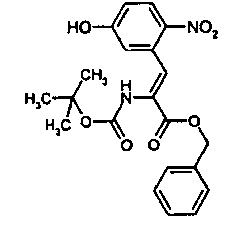 | 4A aus 5-Hydroxy-2-nitrobenzaldehyd | LC-MS (Methode 4): Rₜ = 4.27 min. MS (ES): m/z = 415 (M+H)^{+ 1}H-NMR (300 MHz, CDCl₃): δ = 1.28 (s, 9H), 5.30 (s, 2H), 6.29 (s, 1H), 6.63 (br. s, 1H), 6.84 (dd, 1H), 6.96 (d, 1H), 7.3-7.45 (m, 4H), 7.65 (s, 1H), 8.12 (d, 1H). |
| **4J** | 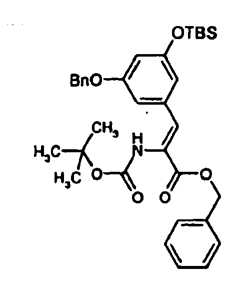 | 4A aus Beispiel 11J | HPLC (Methode 12): Rₜ = 6.80 min. MS (ES): m/z = 590 (M+H)⁺ |
| **4N** | 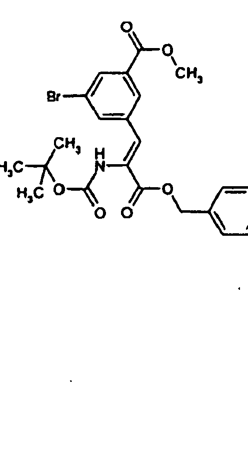 | 4A aus Beispiel 10N | HPLC (Methode 11): Rₜ = 5.51 min. MS (DCI): m/z = 507 (M+NH₄)^{+ 1}H-NMR (400 MHz, CDCl₃): δ = 1.4 (s, 9H), 3.9 (s, 3H), 5.29 (s, 2H), 6.57 (br. s, 1H), 7.23 (s, 1H), 7.31-7.45 (m, 5H), 7.81 (s, 1H), 8.04 (s, 1H), 8.07 (s, 1H). |
| **4P** | 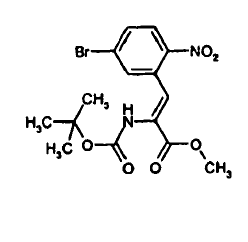 | 4A aus 5-Brom-2-nitrobenzaldehyd (*Chem. Ber.* **1905**, *38*, 2812) | LC-MS (Methode 3): Rₜ = 2.69 min. MS (ES): m/z = 402 (M+H)^{+ 1}H-NMR (300 MHz, DMSO-d₆): δ = 1.3 (s, 9H), 3.75 (s, 3H), 7.47 (s, 1H), 7.64 (s, 1H), 7.72 (d, 1H), 8.10 (d, 1H), 8.72 (br. s, 1H). |

### Beispiel 40

### Methyl-(2Z)-3-(5-brom-2-fluorphenyl)-2-[(tert-butoxycarbonyl)amino]acrylat

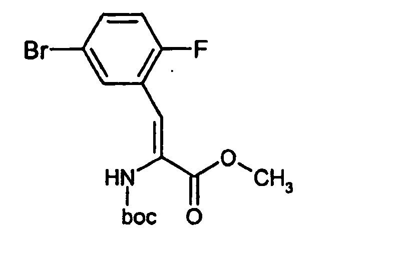

Zu einer auf -70°C gekühlten Lösung von 30 g (147.8 mmol) 5-Brom-2-fluorbenzaldehyd und 50.51 g (169.9 mmol) Methyl-[(*tert*-butoxycarbonyl)amino](dimethoxyphosphoryl)acetat in 450 ml wasserfreiem Tetrahydrofuran werden 21.32 ml (169.9 mmol) *N,N,N,N-*Tetramethylguanidin hinzugegeben. Nach 4 h Rühren bei -70°C wird das Reaktionsgemisch 15 h bei RT gerührt. Die Mischung wird mit 1000 ml Wasser und 1000 ml Essigsäureethylester versetzt. Die organische Phase wird mit Wasser gewaschen, über Natriumsulfat getrocknet und eingeengt. Das Rohprodukt wird säulenchromatographisch an Silicagel (Laufmittel: Cyclohexan:Essigsäureethylester 4:1) gereinigt.
Ausbeute: quant.
HPLC (Methode 11): Rₜ = 5.0 min.
MS (DCI(NH₃)): m/z = 391 (M+NH₄)⁺.
¹H-NMR (300 MHz, CDCl₃): δ = 1.40 (s, 9H), 3.85 (s, 3H), 6.55 (br.s, 1H), 6.95 (dd, 1H), 7.26 (s, 1H), 7.35 (m, 1H), 7.58 (d, 1H).

### Beispiel 5A

### Benzyl-3-brom-N-(tert-butoxycarbonyl)-6-fluor-L-phenylalaninat

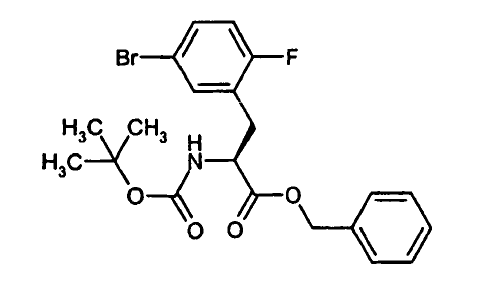

6.0 g (13.3 mmol) Benzyl-(2Z)-3-(5-brom-2-fluorphenyl)-2-[(*tert*-butoxycarbonyl)amino]acrylat werden in 100 ml Ethanol gelöst. Unter Argonatmosphäre gibt man 40 mg (0.055 mmol) (+)-1,2-Bis((2*S*,5*S*)-2,5-diethylphospholano)benzol(cyclooctadien)rhodium(I)trifluormethansulfonat hinzu und leitet 30 min Argon durch die Lösung. Anschließend wird für 4 Tage unter einem Wasserstoffdruck von 3 bar hydriert. Es wird über Kieselgel filtriert und sorgfältig mit Ethanol nachgewaschen. Das Filtrat wird im Vakuum eingeengt und das Rohprodukt am Hochvakuum getrocknet.
Ausbeute: 5.2 g (86% d.Th.)
HPLC (Methode 11): Rₜ = 5.40 min.
MS (DCI(NH₃)): m/z = 469 (M+NH₄)⁺
¹H-NMR (300 MHz, CDCl₃): δ = 1.41 (s, 9H), 3.01 (m_{c}, 1H), 3.19 (m_{c}, 1H), 4.60 (m_{c}, 1H), 5.09
(br. m, 1H), 5.09 (m, 2H), 6.87 (m_{c}, 1H), 7.20-7.42 (m, 7H).

Analog zu obiger Vorschrift werden die in der folgenden Tabelle aufgeführten Beispiele 5B, 5C, 5E, 5H bis 5J und 5N und 5P aus den entsprechenden Edukten hergestellt:

| **Beispiel-Nr.** | **Struktur** | **Hergestellt analog Beispiel-Nr.** | **Analytische Daten** |
|---|---|---|---|
| **5B** | 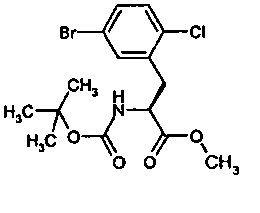 | 5A aus Beispiel 4B | LC-MS (Methode 2): Rₜ = 2.59 min. MS (EI): m/z = 392 (M+H)^{+ 1}H-NMR (400 MHz, DMSO-d₆): δ = 1.30 (s, 9H), 2.89 (m_{c}, 1H), 3.22 (m_{c}, 1H), 3.65 (s, 3H), 4.30 (m_{c}, 1H), 7.3-7.5 (m, 2H), 7.57 (m, 1H). |
| **5C** | 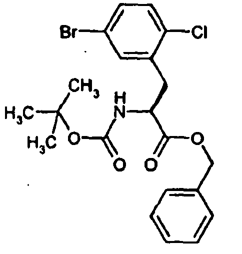 | 5A aus Beispiel 4C | LC-MS (Methode 1): Rₜ = 3.07 min. MS (EI): m/z = 468 (M+H)^{+ 1}H-NMR (300 MHz, DMSO-d₆): δ = 1.30 (s, 9H), 2.95 (m_{c}, 1H), 3.23 (m_{c}, 1H), 4.33 (m_{c}, 1H), 5.13 (s, 2H), 7.27-7.52 (m, 7H), 7.57 (m, 1H). |
| **5E** | 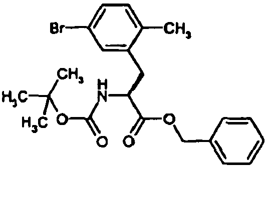 | 5A aus Beispiel 4E | LC-MS (Methode 8): Rₜ = 3.81 min. MS (ES): m/z = 448 (M+H)^{+ 1}H-NMR (300 MHz, CDCl₃): δ = 1.39 (s, 9H), 2.24 (s, 3H), 2.83-3.15 (m, 2H), 4.57 (m_{c}, 1H), 5.00 (br. s, 1H), 5.09 (dd, 2H), 6.97 (d, 1H), 7.14-7.48 (m, 7H). |
| **5H** | 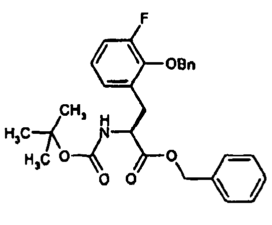 | 5A aus Beispiel 4H | ¹H.NMR (200 MHz, CDCl₃): δ = 1.38 (s, 9H), 3.00 (m, 2H), 4.51 (m_{c}, 1H), 5.03 (s, 2H), 5.10 (s, 2H), überlagert zum Teil 5.18 (m, 1H), 6.78-7.15 (m, 3H), 7.17-7.44 (m, 10H). |
| **5I** | 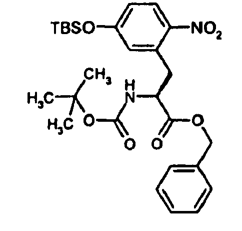 | 5A aus Beispiel 11I | LCMS (Methode 5): Rₜ = 3.19 min. MS (ES): m/z = 531 (M+H)^{+ 1}H-NMR (300 MHz, DMSO-d₆): δ = 0.25 (m, 6H), 0.95 (s, 9H), 1.28 (s, 9H), 2.97 (m_{c}, 1H), 3.53 (m_{c}, 1H), 4.42 (m_{c}, 1H), 5.14 (s, 2H), 6.87-7.0 (m, 2H), 7.27-7.41 (m, 5H), 7.98 (d, 1H). |
| **5J** | 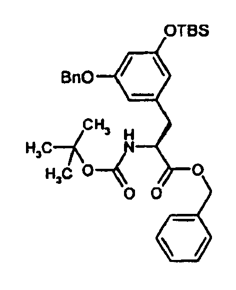 | 5A aus Beispiel 4J | LCMS (Methode 7): Rₜ = 2.88 min. MS (ES): m/z = 592 (M+H)^{+ 1}H-NMR (300 MHz, CDCl₃): δ = 0.0 (m, 6H), 0.82 (s, 9H), 1.28 (s, 9H), 3.36 (m_{c}, 2H), 4.45 (m_{c}, 1H), 4.47 (s, 2H), 4.85 (br. m, 1H), 4.96 (s, 2H), 6.09 (m, 1H), 6.18 (m, 2H), 7.15-7.35 (m, 10H). |
| **5N** | 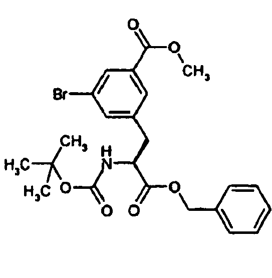 | 5A aus Beispiel 4N | HPLC (Methode 11): Rₜ = 5.37 min. MS (DCI): m/z = 509 (M+NH₄)^{+ 1}H-NMR (300 MHz, CDCl₃): δ = 1.43 (s, 9H), 2.95-3.25 (m, 2H), 3.9 (s, 3H), 4.60 (m_{c}, 1H), 5.05 (m_{c}, 1H), 5.15 (s, 2H), 7.23-7.42 (m, 5H), 7.44 (s, 1H), 7.75 (s, 1H), 8.06 (s, 1H). |
| **5P** | 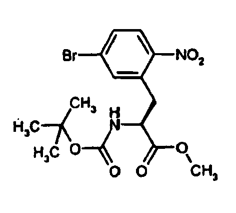 | 5A aus Beispiel 4P | LC-MS (Methode 3): Rₜ = 2.69 min. MS (ES): m/z = 402 (M+H)^{+ 1}H-NMR (300 MHz, DMSO-d₆): δ = 1.28 (s, 9H), 2.95 (m_{c}, 1H), 3.48 (m_{c}, 1H), 3.65 (s, 3H), 4.42 (m_{c}, 1H), 7.31 (br. d, 1H), 7.7-7.8 (m, 2H), 7.95 (m, 1H). |

### Beispiel 50

### Methyl-3-brom-N-(tert-butoxycarbonyl)-6-fluor-L-phenylalaninat

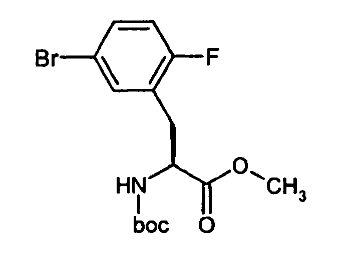

20 g (53.45 mmol) Methyl-(2Z)-3-(5-brom-2-fluorphenyl)-2-[(*tert*-butoxycarbonyl)amino]acrylat (Beispiel 40) werden in 300 ml Ethanol/Dioxan (3:1) gelöst. Unter Argonatmosphäre gibt man 200 mg (+)-1,2-Bis((2*S*,5*S*)-2,5-diethylphospolano)benzol(cyclooctadien)rodhium(I)trifluoromethansulfonat hinzu und leitet 30 min Argon durch die Lösung. Anschließend wird für 3 Tage unter einem Wasserstoffdruck von 3.5 bar hydriert. Das Lösungsmittel wird einrotiert und das Rohprodukt wird säulenchromatographisch an Silicagel (Laufmittel: Cyclohexan: Essigsäureethylester 2:2) gereinigt.
Ausbeute: quant.
HPLC (Methode 11): Rₜ = 4.9 min.
MS (DCI(NH₃)): m/z = 393 (M+NH₄)⁺.
¹H-NMR (400 MHz, CDCl₃): δ = 1.40 (s, 9H), 2.98 (dd, 1H), 3.20 (dd, 1H), 3.73 (s, 3H), 4.57 (m, 1H), 5.05 (m, 1H), 6,80 (dd, 1H), 7.25-7.40 (m, 2H).

### Beispiel 6H

### Henzyl-2-(benzyloxy)-N-(tert-butoxycarbonyl)-3-fluor-5-iod-L-phenylalaninat

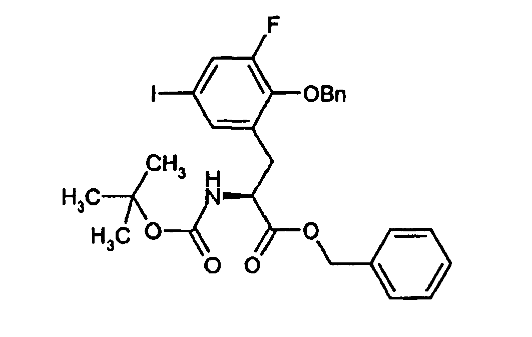

12.5 g (26.1 mmol) Benzyl-2-(benzyloxy)-*N*-(*tert*-butoxycarbonyl)-3-fluor-*L-*phenylalaninat werden in 200 ml Dichlormethan vorgelegt und mit 8.76 g (104 mmol) Natriumhydrogencarbonat versetzt. 8.46 g (52.4 mmol) Iodmonochlorid in 10 ml Dichlormethan werden langsam zugetropft. Nach 72 h versetzt man mit 300 ml einer 5%-igen Natriumbisulfit-Lösung. Die Phasen werden getrennt und die organische Phase wird mit Wasser extrahiert. Die organische Phase wird eingeengt und der Rückstand über Kieselgel (Cyclohexan:Essigsäureethylester 6:1) gereinigt.
Ausbeute: 7.0 g (35% d.Th.)
HPLC (Methode 11): Rₜ = 6.06 min.
MS (ESI): m/z = 606 [M+H]⁺
¹H-NMR (200 MHz, CDCl₃): δ = 1.38 (s, 9H), 2.70-3.11 (m, 2H), 4.52 (m_{c}, 1H), 5.04 (m_{c}, 4H) überlagert 5.05 (m, 1H), 6.78-7.09 (m, 2H), 7.15-7.48 (m, 10H).

### Beispiel 7D

### Diethyl-[(tert-butoxycarbonyl)amino](3-iodbenzyl)malonet

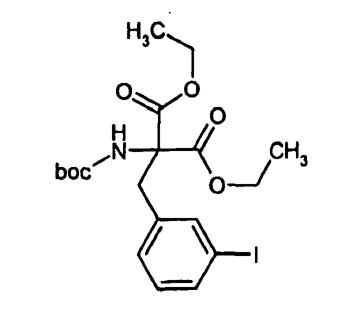

Zu einer Lösung von 24.3 g (88.4 mmol) Diethyl-[(*tert*-butoxycarbonyl)amino]malonat und 3.7 g (92.6 mmol) Natriumhydrid in 300 ml DMF werden unter Eiskühlung 25 g (84.2 mmol) 3-Iodbenzylbromid hinzugegeben. Nach 4 h Rühren bei RT wird unter Eiskühlung vorsichtig mit 5 ml Wasser versetzt. Es wird mehrmals mit Essigsäureethylester extrahiert, die vereinigten organischen Phasen werden mit gesättigter Natriumchlorid-Lösung und Wasser gewaschen, über Magnesiumsulfat getrocknet und im Vakuum eingeengt. Das Rohprodukt wird im Hochvakuum getrocknet.
Ausbeute: 43g (99% d. Th.)
HPLC (Methode 11): Rₜ = 5.60 min.
1H-NMR (300 MHz, DMSO-d₆): δ = 1.18 (t, 6 H), 1.44 (s, 9H), 3.40 (s, 2H), 4.05-4.25 (m, 2H), 6.4 (br. s, 1H), 7.02 (d, 1H), 7.10 (t, 1H), 7.35 (s, 1H), 7.61(d, 1H).

### Beispiel 8D

### N-(tert-Butoxycarbonyl)-3-iodphenylalanin

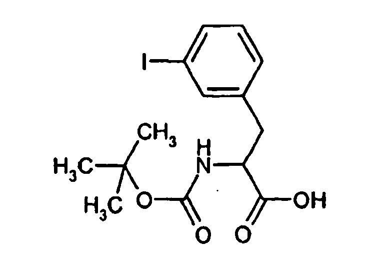

Zu einer Suspension von 30.3 g (62 mmol) Diethyl-[(*tert*-butoxycarbonyl)amino](3-iodbenzyl)malonat (Beispiel 7D) in 440 ml eines Gemisches von Ethanol und Wasser (3:1) werden 240 ml 1N Natronlauge hinzugegeben. Nach 3 h unter Rückfluss wird der pH-Wert der Reaktionsmischung nach Abkühlung auf RT mit konzentrierter Salzsäure auf ca. pH 2 eingestellt. Die Reaktionsmischung wird im Vakuum eingeengt. Der Rückstand wird in MTBE und Wasser aufgenommen. Die wässrige Phase wird dreimal mit MTBE extrahiert. Die vereinigten organischen Phasen werden über Natriumsulfat getrocknet, filtriert und eingeengt. Der Rückstand wird durch RP-HPLC (Acetonitril/Wasser Gradient) gereinigt.
Ausbeute: 16.2 g (67% d. Th.)
HPLC (Methode 11): Rₜ = 4.53 min.
¹H-NMR (400 MHz, DMSO-d₆): δ = 1.42 (s, 9H), 3.02 (m_{c}, 1H], 3.18 (m_{c}, 1H), 4.57 (br. m_{c}, 1H), 4.94 (br. m_{c}, 1H), 7.05 (t, 1H), 7.17 (d, 1H), 7.5 (s, 1H), 7.60 (d, 1H).

### Beispiel 9D

### N-(tert-Butoxycarbonyl)-3-iod-L-phenylalanin

Das Racemat aus Beispiel 8D wird an einer chiralen stationären Kieselgelphase, basierend auf dem Selektor aus Poly(*N*-methacryloyl-*L-*leucin-dicyclopropylmethylamid), mit einem Gemisch aus i-Hexan/Essigsäureethylester als Elutionsmittel getrennt. Nach chromatographischem Vergleich entspricht das zuerst eluierte Enantiomer dem (*R*)-Enantiomer (97% ee), das zweite, rechtsdrehende ([α]_{D}²⁰: +18.8°, c = 0.57, Dichlormethan) Enantiomer dem (*S*)-Enantiomer (97% ee).

### Beispiel 10D

### Benzyl-N-(tert-butoxycarbonyl)-3-iod-L-phenylalaninat

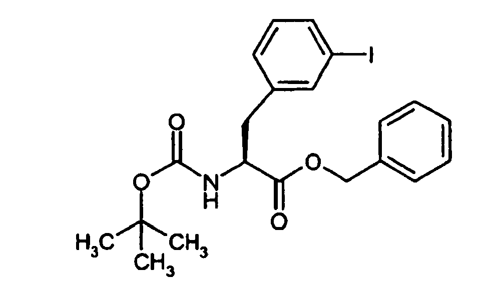

Unter Argon werden 5.33 g (13.6 mmol) *N*-(*tert*-Butoxycarbonyl)-3-iod-*L-*phenylalanin (aus Beispiel 9D) in 110 ml Acetonitril gelöst. Dazu werden 166 mg (1.36 mmol) 4-Dimethylaminopyridin und 2.82 ml (27.2 mmol) Benzylalkohol hinzugefügt. Die Mischung wird auf -10°C abgekühlt und mit 3.13 g (16.35 mmol) EDC versetzt. Man lässt alles langsam auf RT kommen und rührt über Nacht. Nach ca. 16 h wird das Gemisch im Vakuum einrotiert und der Rückstand chromatographisch an RP-HPLC (Laufmittel: Acetonitril/Wasser Gradient) gereinigt.
Ausbeute: 4.78 g (73% d. Th.).
LC-MS (Methode 8): Rₜ = 3.77 min.
MS (EI): m/z = 482 (M+H)⁺.

### Beispiel 111

### Benzyl-(22)-2-[(tert-butoxycarbonyl)amino]-3-(5-{[tert-butyl(dimethyl)silyl]oxy}-2-nitrophenyl)acrylat

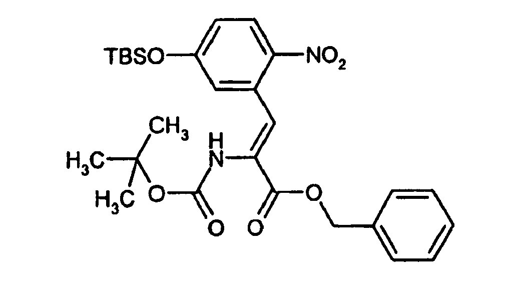

Eine Lösung von 6.76 g (9.8 mmol) Benzyl-(2Z)-2-[(*tert*-butoxycarbonyl)amino]-3-(5-hydroxy)-2-nitrophenyl)acrylat (Beispiel 4I) in 100 ml Dichlormethan wird unter Eiskühlung mit 1.67 g (24.5 mol) Imidazol und 1.77 g (11.7 mmol) *tert*-Butyldimethylsilylchlorid versetzt. Es wird auf RT erwärmt und 18 h gerührt. Die organische Phase wird mehrfach mit Wasser gewaschen, über Magnesiumsulfat getrocknet und im Vakuum eingeengt. Das Rohprodukt mittels RP-HPLC (Laufmittel: Acetonitril/Wasser Gradient) gereinigt.
Ausbeute: 2.82 g (55% d. Th.)
LC-MS (Methode 8): Rₜ = 4.19 min.
MS (EI⁺): m/z = 529 (M+H)⁺
¹H-NMR (400 MHz, CDCl₃): δ = 0.24 (s, 6H), 0.97 (s, 9H), 1.30 (s, 9H), 5.30 (s, 2H), 6.25 (br. s, 1H), 6.82 (dd, 1H), 6.93 (d, 1H), 7.30-7.46 (m, 5H), 7.60 (s, 1H), 8.12 (d, 1H).

### Beispiel 11J

### 3-(Benzyloxy)-5-{[tert-butyl(dimethyl)silyl]oxy}benzaldehyd

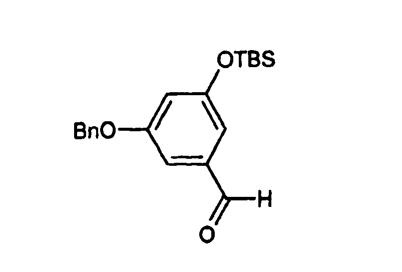

Die Herstellung erfolgt analog zu Beispiel 11I aus 2.8 g (12.3 mmol) 3-(Benzyloxy)-5-hydroxybenzaldehyd (Beispiel 3J), 1.67 g (24.5 mmol) Imidazol und 3.57 g (13.5 mmol) Trifluormethansulfonsäure-*tert*-butyl-dimethylsilylester.
Ausbeute: 4.0 g (95% d. Th.)
LC-MS (Methode 18): Rₜ = 2.05 min
MS (EI): m/z = 343 (M+H)⁺
¹H-NMR (400 MHz, CDCl₃): δ = 0.21 (s, 6H), 0.97 (s, 9H), 5.10 (s, 2H), 6.72 (s, 1H), 6.95 (s, 1H), 7.10 (s, 1H), 7.28-7.48 (m, 5H).

### Beispiel 10N

### Methyl-3-brom-5-formylbenzoat

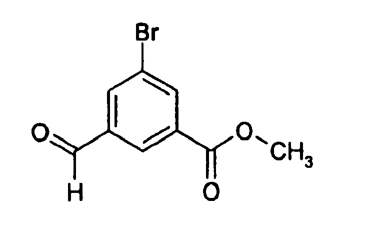

Unter Argon werden 6.2 g (27.1 mmol) 3-Brom-5-formylbenzoesäure (J. Org. Chem., 2002, 67, 3548-3554) in 110 ml Acetonitril gelöst. Dazu werden 330 mg (2.7 mmol) 4-Dimethylaminopyridin und 1.73 ml (54.2 mmol) Methanol hinzugefügt. Die Mischung wird auf -10°C abgekühlt und mit 6.23 g (32.5 mmol) EDC versetzt. Man lässt alles langsam auf RT kommen und rührt 20 h nach. Anschließend wird das Lösungsmittel im Vakuum eingedampft und der Rückstand chromatographisch an Kieselgel (Laufmittel: Cyclohexan/Essigsäureethylester (6:1)) gereinigt.
Ausbeute: 5.1 g (77% d. Th.).
HPLC (Methode 11): Rₜ = 4.41 min.
¹H-NMR (200 MHz, CDCl₃): δ = 3.95 (s, 3H), 8.20 (m, 1H), 8.43 (m, 1H), 8.47 (m, 1H), 10.03 (s, 1H).

### Beispiel 12I

### Benzyl-N-(tert-butoxycarbonyl)-3-hydroxy-6-nitro-L-phenylalaninat

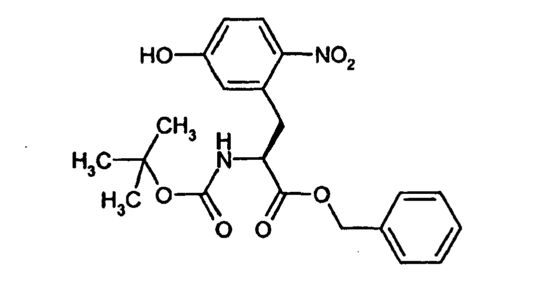

Eine Lösung von 2.77 g (5.22 mmol) Benzyl-*N*-(*tert*-butoxycarbonyl)-3-{[*tert-*butyl(dimethyl)silyl]oxy}-6-nitro-*L-*phenylataninat (Beispiel 5I) in 50 ml THF wird mit 10.4 ml (10.4 mmol) einer 1N Tetrabutylammoniumflurid-Lösung in THF versetzt und 30 min bei RT gerührt. Anschließend gießt man die Lösung auf Eiswasser und extrahiert mehrmals mit Essigsäureethylester. Die organische Phase wird über Magnesiumsulfat getrocknet und im Vakuum eingeengt. Das Rohprodukt wird im Hochvakuum bis zur Gewichtskonstanz getrocknet.
Ausbeute: 2.1 g (97% d. Th.)
LC-MS (Methode 8): Rₜ = 3.23 min.
MS (EI⁺): m/z = 417 (M+H)⁺

### Beispiel 12J

### Henzyl-3-(benzyloxy)-N-(tert-butoxycarbonyl)-5-hydroxy-L-phenylalaninat

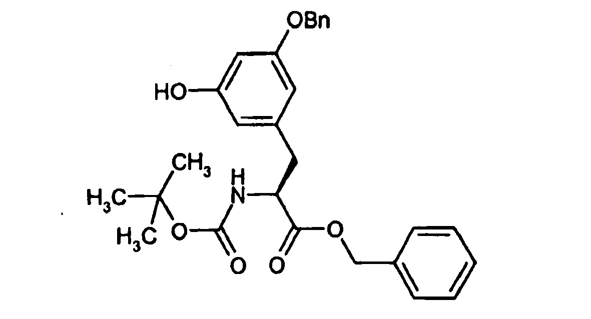

Die Herstellung erfolgt analog zu Beispiel 12I aus 1.30 g (2.2 mmol) Benzyl-3-(benzyloxy)-*N-*(*tert*-butoxycarbonyl)-5-{[*tert*-butyl(dimethyl)silyl]oxy}-*L-*phenylalaninat (Beispiel 5J) und 4.4 ml (4.39 mmol) einer 1N Tetrabutylammoniumfluorid-Lösung in THF.
Ausbeute: 1.15 g (95% d. Th.)
LC-MS (Methode 14): Rₜ = 3.82 min
MS (EI): m/z = 478 (M+H)⁺

### Beispiel 13I

### Benzyl-N-(tert-butoxycarbonyl)-2-nitro-5-{[(trifluonnethyl)sulfonyl]oxy}-L-phenylalaninat

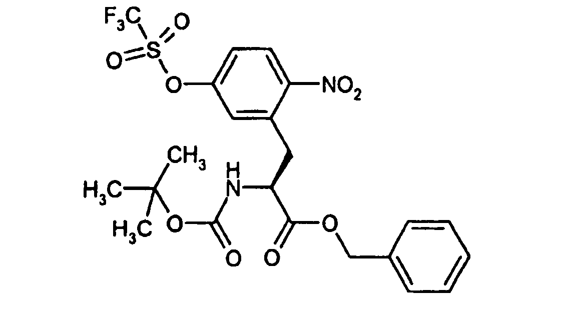

Eine Lösung von 2.27 g (5.46 mmol) Benzyl-*N*-(*tert*-butoxycarbonyl)-3-hydroxy-6-nitro-*L-*phenylalaninat (Beispiel 12I) und 1.52 ml (10.9 mmol) Triethylamin in 150 ml Dichlormethan wird bei -15°C (Aceton/Trockeneis Bad) tropfenweise mit 1.0 ml (6.01 mmol) Trifluormethansulfonsäureanhydrid versetzt. Nach 15 min wird auf RT erwärmt und mit Wasser versetzt. Die organische Phase wird getrennt, mehrmals mit Wasser gewaschen, über Magnesiumsulfat getrocknet und im Vakuum eingeengt. Das Rohprodukt wird mittels RP-HPLC (Laufmittel: Acetonitril/Wasser Gradient) gereinigt.
Ausbeute: 1.88 g (63% d. Th.)
HPLC (Methode 13): Rₜ = 5.35 min.
MS (DCI): m/z = 566 (M+NH₄)⁺
¹H-NMR (200 MHz, DMSO-d₆): δ = 1.29 (s, 9H), 3.08 (m_{c}, 1H), 3.50 (m_{c}, 1H), 4.46 (m_{c}, 1H), 5.15 (s, 2H), 7.25-7.50 (m, 5H), 7.65-7.80 (m, 2H), 8.20 (m_{c}, 1H).

### Beispiel 13J

### Benzyl-3-(benzyloxy)-N-(tert-butoxycarbonyl)-5-{[(trifluormethyl)sulfonyl]oxy-L-phenylalaninat

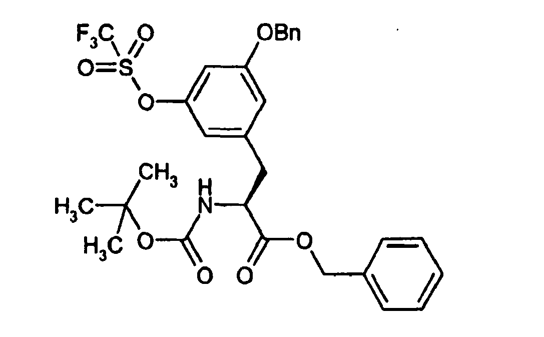

Die Herstellung erfolgt analog zu Beispiel 13I aus 1.15 g (2.41 mmol) Benzyl-3-(benzyloxy)-*N-*(*tert*-butoxycarbonyl)-5-hydroxy-*L-*phenylalaninat (Beispiel 12J), 0.67 ml (4.82 mmol) Triethylamin und 0.45 ml (2.65 mmol) Trifluormethansulfonsäureanhydrid.
Ausbeute: 1.4 g (95% d. Th.)
LC-MS (Methode 14): Rₜ = 4.24 min
MS (EI): m/z = 610 (M+H)⁺

### Beispiel 14B

### Methyl-3-brom-N-(tert-butoxycarbonyl)-6-hlor-N-methyl-L-phenylalaninat

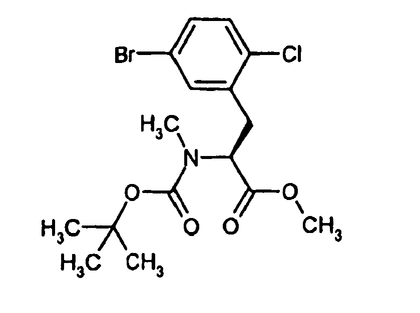

100 mg (0.25 mmol) der Verbindung aus Beispiel 5B werden unter Argon in 5 ml THF gelöst und bei RT mit 30 mg (0.76 mmol) Natriumhydrid (60%ige Dispersion in Mineralöl) versetzt. Nach Zugabe von 290 mg (130 µl, 2.04 mmol) Methyliodid wird für 12 h bei RT gerührt. Anschließend gibt man je 20 ml Essigsäureethylester und Wasser hinzu und stellt durch Zugabe von 0.1N Salzsäure einen pH-Wert von 3 ein. Es wird mehrmals mit Essigsäureethylester extrahiert, die organische Phase wird getrennt, über Magnesiumsulfat getrocknet und im Vakuum zur Trockne eingeengt. Das Rohprodukt wird in 10 ml Methanol gelöst und unter Argon bei 0°C (Eisbadkühlung) mit 50 mg (0.25 mmol) EDC und 8 mg (0.057 mmol) HOBt versetzt. Es wird 36 h bei RT gerührt. Methanol wird im Vakuum abgedampft und der Rohansatz wird mit Wasser versetzt und mehrmals mit Essigsäureethylester extrahiert. Die organische Phase wird über Magnesiumsulfat getrocknet, im Vakuum zur Trockne eingeengt und durch RP-HPLC (Laufmittel: Acetonitril/Wasser Gradient) gereinigt.
Ausbeute: 0.06 g (59% d. Th.)
LC-MS (Methode 3): Rₜ = 2.96 min.
MS (EI): m/z = 306 (M-boc+H)⁺
¹H-NMR (400 MHz, DMSO-d₆): RT-Spektrum zeigt Rotamere: δ = 1.25 (s_{c}, 9H), 2.65 (s_{c}, 3H), 3.18 (m_{c}, 1H), 3.38 (m_{c}, 1H), 3.70 (s_{c}, 3H), 4.83 (m_{c}, 1H), 7.35-7.60 (m, 3H).

### Beispiel 14O

### Methyl-3-brom-N-(tert-butoxycarbonyl)-6-fluor-N-methyl-L-phenylataninat

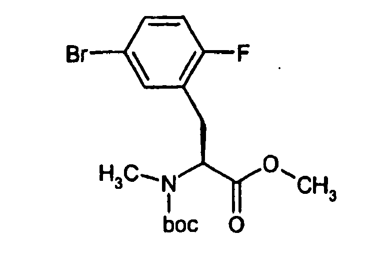

Zu einer Lösung von 16.5 g (43.86 mmol) Methyl-3-brom-*N*-(*tert*-butoxycarbonyl)-6-fluor-*L-*phenylalaninat (Beispiel 5O) in 220 ml wasserfreiem Tetrahydrofuran werden 49.8 g (350.86 mmol) Iodmethan und 2.28 g (57.01 mmol) Natriumhydrid hinzugegeben. Die Reaktionsmischung wird bei RT über Nacht gerührt. Die Mischung wird mit 1000 ml Wasser und 1000 ml Essigsäure-ethylester versetzt. Die organische Phase wird nacheinander mit Wasser und gesättigter Natriumchlorid-lösung gewaschen, über Natriumsulfat getrocknet und eingeengt. Das Rohprodukt wird säulenchromatographisch an Silicagel (Laufmittel: Cyclohexan:Essigsäureethylester 3:1) gereinigt.
Ausbeute: quant.
HPLC (Methode 11): Rₜ = 5.1 min.
MS (DCI(NH₃)): m/z = 390 (M+M)⁺.
¹H-NMR (400 MHz, CDCl₃): δ = 1.48 (d, 9H), 2.23 (d, 3H), 3.09 (dd, 1H), 3.30 (dd, 1H), 3.75 (s, 3H), 4.70 (ddd, 1H), 6.92 (dd, 1H), 7.30 (m, 2H).

### Beispiel 15E

### 5-Brom-2-methylbenzaldehyd

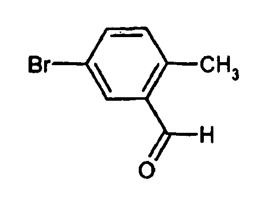

77.7 g (583 mmol) Aluminiumtrichlorid werden in 200 ml Dichlormethan suspendiert und auf 0°C gekühlt. 40.0 g (333 mmol) 2-Methylbenzaldehyd werden innerhalb von 30 min zugetropft. Anschließend gibt man 53.2 g (333 mmol) Brom innerhalb von 6 h bei 0°C zu, lässt auf RT erwärmen und rührt 12 h nach. Die Reaktionslösung wird auf 500 ml Eiswasser gegeben. Die wässrige Phase wird mehrfach mit Dichlormethan extrahiert. Die vereinigten organischen Phasen werden nacheinander mit 2N Salzsäure, gesättigter wässriger Natriumhydrogencarbonat-Lösung und gesättigter wässriger Natriumchlorid-Lösung gewaschen. Die organische Phase wird über Natriumsulfat getrocknet und im Vakuum eingeengt. Man reinigt per Kieselgelchromatographie und anschließend über Kristallisation aus Cyclohexan. Das ausgefallene Produkt wird abfiltriert.
Ausbeute: 3.2 g (5% d.Th.)
LC-MS (Methode 9): Rₜ = 3.26 min
MS (EI): m/z = 199 (M+H)⁺

### Beispiel 16A

### 2-(Trimethylsilyl)ethyl-2-(benzyloxy)-N-[(benzyloxy)carbonyl]-5-(4,4,5,5-tetramethyl-1,3,2-dioxaborolan-2-yl)-L-phenylalaninat

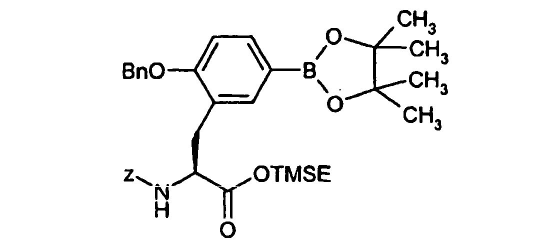

Zu einer Lösung von 2.00 g (3.17 mmol) 2(*S*)-Benzyloxycarbonylamino-3-(2-benzyloxy-5-iod-phenyl)-propionsäure-(2-trimethylsilyl)-ethylester in 30 ml DMSO werden 0.932 g (9.50 mmol) Kaliumacetat zugegeben. Die Mischung wird deoxygeniert, indem durch die kräftig gerührte Lösung 15 min lang Argon durchgeleitet wird. Dann werden 0.924 g (3.64 mmol) 4,4,4',4',5,5,5',5'-Octamethyl-2,2'-bi-1,3,2-dioxaborolan und 0.116 g (0.160 mmol, 0.05 Äquivalente) Bis-(diphenylphosphino)ferrocenpalladium(II)chlorid zugegeben. Unter leichtem Argonstrom wird auf 80°C erhitzt und nach 6 h wieder abgekühlt. Die Mischung wird über Silicagel (Laufmittel: Dichlormethan) filtriert. Der Rückstand wird säulenchromatographisch an Silicagel (Laufmittel: Cyclohexan:Essigsäureethylester 4:1) gereinigt.
LC-MS (Methode 22): Rₜ = 4.50 min
MS (EI): m/z = 632 (M+H)⁺
¹H-NMR (200 MHz, CDCl₃): δ = 0.92 (dd, 2H), 1.31 (s, 12H), 2.95-3.95 (m, 2H), 4.11 (m_{c}, 2H), 4.55 (11 (m_{c}, 1H), 4.99 (s, 2H), 5.08 (s, 2H), 5.53 (d, 1H), 6.90 (d, 1H), 7.15-7.47 (m, 10 H), 7.58 (d, 1H), 7.67 (dd, 1H).

### Beispiel 17A

### 2-(Trimethylsilyl)ethyl-(2S)-3-(3'-{(2S)-3-(benzyloxy)-2-[(tert-butoxycarbonyl)amino]-3-oxopropyl}-4'-fluor-4-hydroxybiphenyl-3-yl)-2-{[(benzyloxy)carbonyl]amino}propanoat

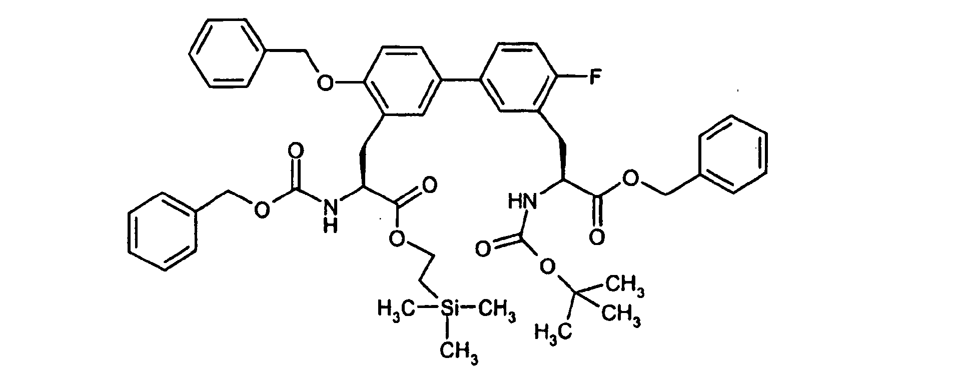

Man löst 358 mg (0.79 mmol) Benzyl-3-brom-*N*-(*tert*-butoxycarbonyl)-6-fluor-*L-*phenylalaninat (Beispiel 5A) in 3 ml abs. DMF, entgast die Lösung 5 min im Vakuum, belüftet mit Argon und führt die nachfolgende Reaktion auch unter Argondurchfluss durch. Zu dieser Lösung gibt man unter Rühren 58 mg (0.08 mmol) Bis(diphenylphosphino)ferrocen-palladium(II)-chlorid und 516 mg (1.58 mmol) Cäsiumcarbonat. Parallel dazu löst man 500 mg (0.79 mmol) 2-(Trimethylsilyl)ethyl-2-[(benzyloxy)carbonyl]-5-(4,4,5,5-tetramethyl-1,3,2-dioxaborolan-2-yl)-*L-*phenylalaninat (Beispiel 16A) in 3 ml abs. DMF, entgast 5 min. im Vakuum und leitet anschließend 15 min. Argon durch die Lösung. Die so hergestellte Lösung gibt man unter Argonatmosphäre zu der ersten Lösung, erwärmt auf 40°C und lässt unter Rühren über Nacht reagieren. Man engt im Vakuum zur Trockne ein, nimmt den Rückstand in 10 ml Essigsäureethylester auf, filtriert vom Ungelösten und extrahiert die organische Phase dreimal mit 3 ml Wasser. Die organische Phase wird getrocknet (Natriumsulfat), im Vakuum eingeengt und der Rückstand säulenchromatographisch getrennt (Kieselgel 60, Laufmittel: Cyclohexan / Essigsäure-ethylester = 4/1). Die Hauptfraktion liefert 389 mg Produkt (47% Ausbeute); aus der Mischfraktion werden mittels HPLC (Acetonitril / Wasser) weitere 72 mg (10% Ausbeute) gewonnen.
Gesamtausbeute: 57% d.Th.
LC-MS (Methode 4): Rₜ = 5.0 min.
MS (EI): m/z = 876 [M+H]⁺

Analog zu obiger Vorschrift werden die in der folgenden Tabelle aufgeführten Beispiele 17B bis 17E, 17H bis 17J, 17N und 17P aus den entsprechenden Edukten hergestellt:

| **Beispiel-Nr.** | **Struktur** | **Hergestellt analog Beispiel-Nr.** | **Analytische Daten** |
|---|---|---|---|
| **17B** | 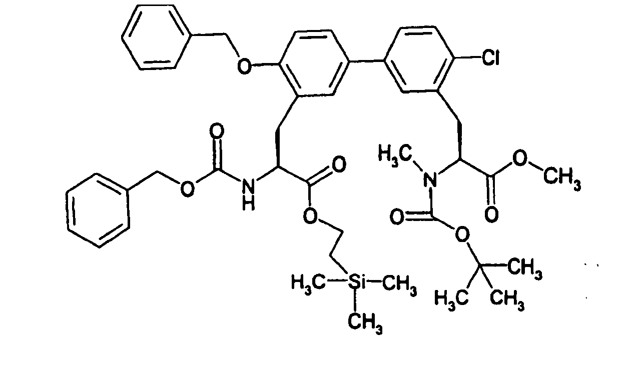 | 17A aus Beispiel 14B und 16A | LC-MS (Methode 3): R₁ = 3.55 min. MS (ES): m/z = 853 (M+Na)⁺ |
| **17C** | 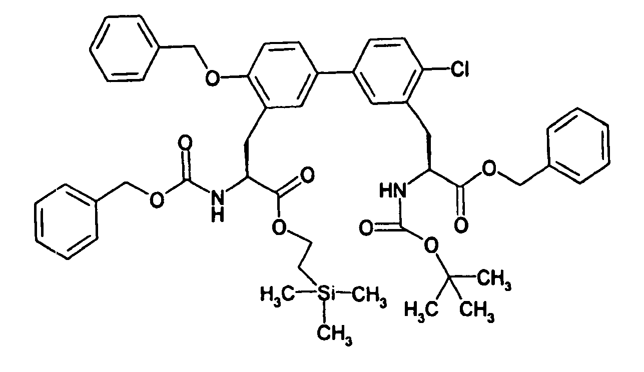 | 17A aus Beispiel 5C und 16A | HPLC (Methode 12): Rₜ = 7.20 min. MS (ES): m/z = 893 (M+H)^{+ 1}H-NMR (200 MHz, DMSO-d₆): δ = 0.0 (s, 9H), 0.83 (m_{c}, 2H), 1.30 (s, 9H), 2.75-3.15 (m, 2H), 3.30 (m_{c}, 2H), 3.88 (s, 3H), 4.09 (m_{c}, 2H), 4.44 (m_{c}, 2H), 4.97 (m_{c}, 2H), 5.10-5.30 (m, 4H), 7.1-7.9 (m, 21H). |
| **17D** | 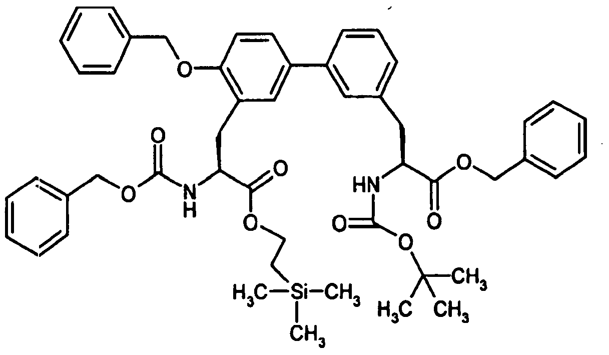 | 17A aus Beispiel 10D und 16A | LC-MS (Methode 9): Rₜ = 5.35 min. MS (ES): m/z = 859 (M+H)⁺ |
| **17E** | 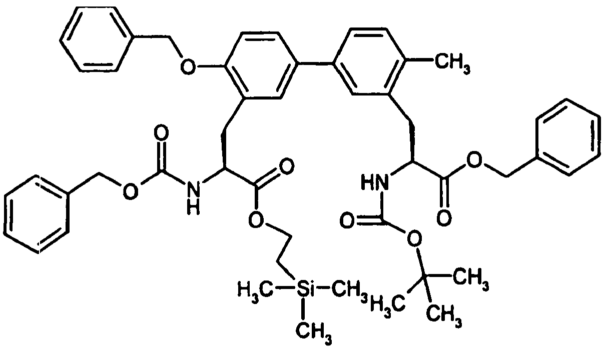 | 17A aus Beispiel 5E und 16A | LC-MS (Methode 18): Rₜ = 4.01 min. MS (ES): m/z = 873 (M+H)⁺ |
| **17H** | 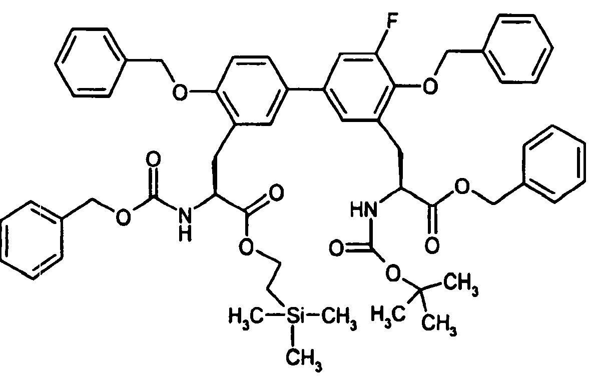 | 17A aus Beispiel 6H und 16A | LC-MS (Methode 18): Rₜ = 3.79 min. MS (ES): m/z = 983 (M+H)⁺ |
| **17I** | 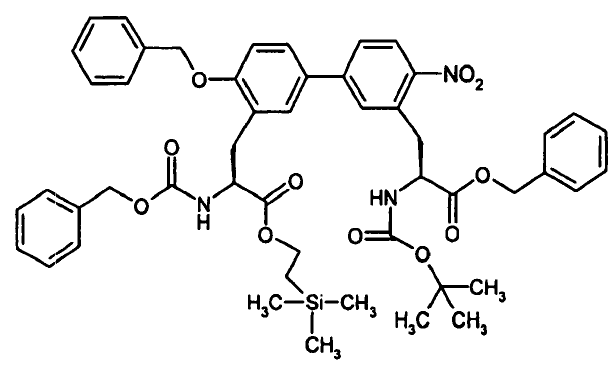 | 17A aus Beispiel 13I und 16A | LC-MS (Methode 7): Rₜ = 2.93 min. MS (ES): m/z = 904 (M+H)⁺ |
| **17J** | 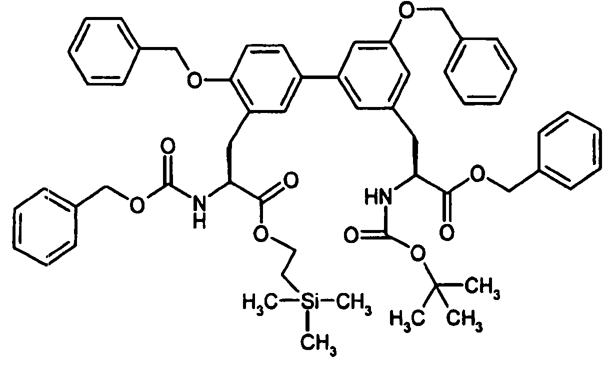 | 17A aus Beispiel 13J und 16A | LC-MS (Methode 7): Rₜ = 3.73 min. MS (ES): m/z = 965 (M+H)⁺ |
| **17N** | 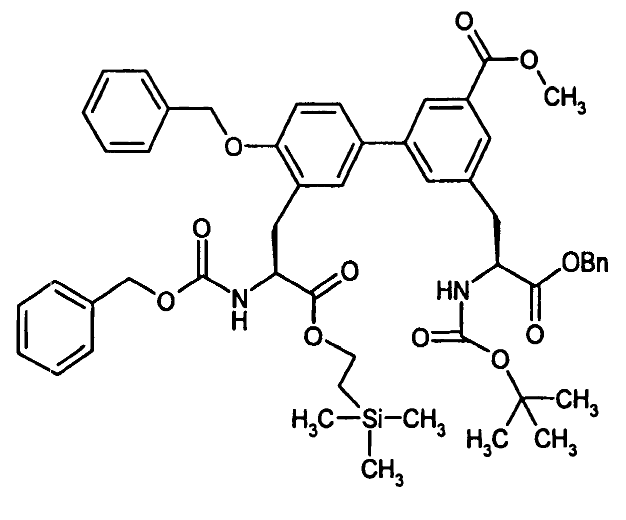 | 17A aus Beispiel 5N und 16A | LC-MS (Methode 5): Rₜ = 3.36 min. MS (ES): m/z = 917 (M+H)⁺ |
| **17P** | 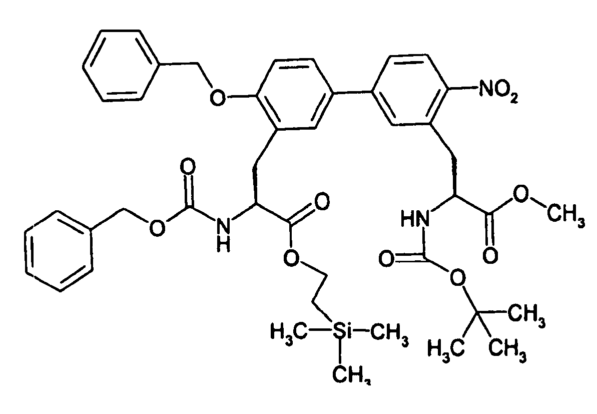 | 17A aus Beispiel 5P und 16A | LC-MS (Methode 1): Rₜ = 3.40 min. MS (ES): m/z = 828 (M+H)⁺ |

### Beispiel 17O

### Methyl-(2S)-3-(4'-(benzyloxy)-3'-{(2S)-2-{[(benzyloxy)carbonyl]amino}-3-oxo-3-[2-(trimethylsilyl)ethoxy]propyl}-4-fluorbiphenyl-3-yl)-2-[(tert-butoxycarbonyl)(methyl)amino]propanoat

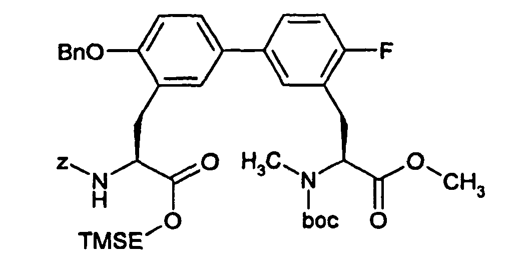

Eine Lösung von 1.68 g (4.09 mmol) Methyl-3-brom-N-(*tert-*butoxycarbonyl)-6-fluor-N methyl-*L-*phenylalaninat (Beispiel 140) in 8 ml 1-Methyl-2-pyrrolidon wird inertisiert und mit Argon gesättigt (ca. 30 min Argon durchleiten). Anschließend gibt man 334 mg (0.41 mmol) Bis(diphenylphosphino)ferrocen-palladium(II)chlorid (PdCl₂(dppf)) dazu, und die Mischung wird 10 min bei RT gerührt. Danach werden 3.45 g (4.92 mmol) 2-(Trimethylsilyl)ethyl-2-(benzyloxy)-*N*-[(benzyloxy)carbonyl]-5-(4,4,5,5-tetramethyl-1,3,2-dioxaborolan-2-yl)-*L*-phenylalaninat (Beispiel 16A) in 8 ml 1-Methyl-2-pyrrolidon und 2.67 g (8.19 mmol) Cäsiumcarbonat hinzugegeben. Das Reaktionsgemisch wird mit Argon leicht überströmt und für 20 h bei 50°C gerührt. Die Mischung wird abgekühlt, in Dichlormethan aufgenommen und mit Wasser gewaschen. Die organische Phase wird über Magnesiumsulfat getrocknet und das Lösungsmittel wird im Vakuum eingeengt. Der Rückstand wird säulenchromatographisch an Kieselgel gereinigt (Cyclohexan:Essigsäureethylester 7:3).
Ausbeute: 3.6 g (86% d. Th.).
LC-MS (Methode 1): Rₜ = 2.49 min
MS (EI): m/z = 1140 (M+H)⁺.

### Beispiel 18A

### Benzyl-(2S)-2-amino-3-(4'-(benzyloxy)-3'-{(2S)-2-{[(benzyloxy)carbonyl]amino}-3-oxo-3-[2-(trimethylsilyl)ethoxy]propyl}-4-fluorbiphenyl-3-yl)propanoat-Hydrochlorid

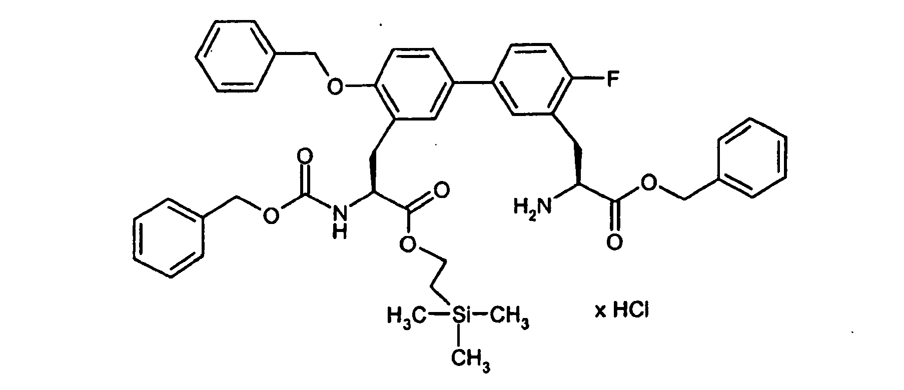

405 mg (0.46 mmol) der Verbindung aus Beispiel 17A werden in 2 ml abs. Dioxan suspendiert, auf 0°C gekühlt und unter Rühren mit 12 ml 4N Dioxan / Chlorwasserstoff-Lüsung versetzt. Nach 3 h dampft man alles im Vakuum zur Trockne ein und trocknet im Hochvakuum bis zur Gewichtskonstanz.
Ausbeute: 395 mg (88% d.Th.)
LC-MS (Methode 5): Rₜ = 2.45 min.
MS (EI): m/z = 776 [M+H]⁺

Analog zu obiger Vorschrift werden die in der folgenden Tabelle aufgeführten Beispiele 18B bis 18E, 18H bis 18J und 18N und 18P aus den entsprechenden Edukten hergestellt:

| Beispiel-Nr. | Struktur | Hergestellt analog Beispiel-Nr. | Analytische Daten |
|---|---|---|---|
| **18B** | 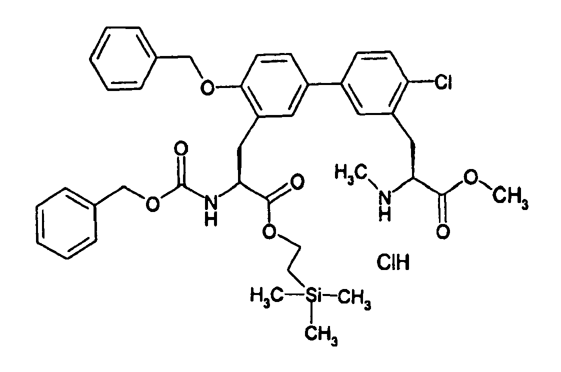 | 18A aus Beispiel 17B | LC-MS (Methode 1): Rₜ = 2.69 min. MS (ES): m/z = 731 (M-HCl+H)⁺ |
| **18C** | 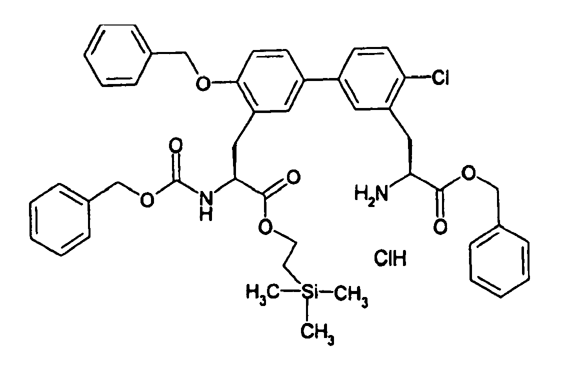 | 18A aus Beispiel 17C | LC-MS (Methode 5): Rₜ = 2.50 min. MS (ES): m/z = 793 (M-HCl+H)⁺ |
| **18D** | 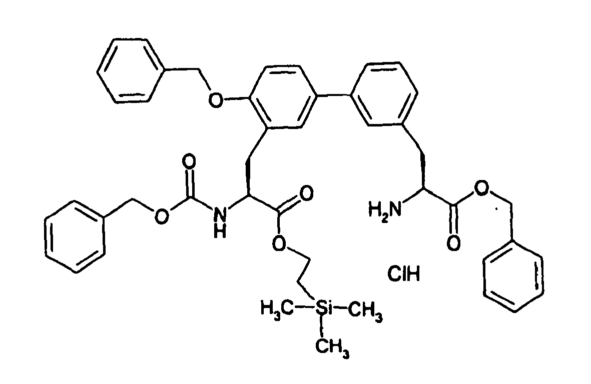 | 18A aus Beispiel 17D | LC-MS (Methode 5): Rₜ = 2.36 min. MS (ES): m/z = 759 (M-HCl+H)⁺ |
| **18E** | 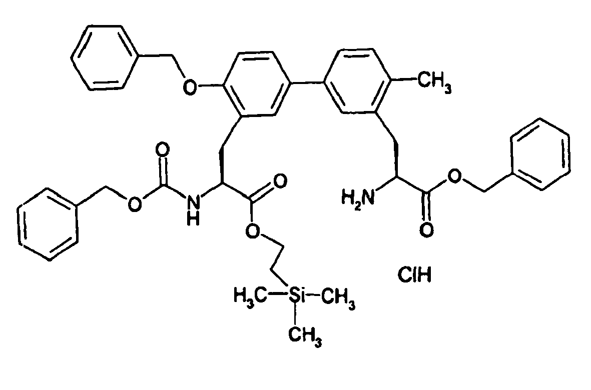 | 18A aus Beispiel 17E | LC-MS (Methode 8): Rₜ = 3.10 min. MS (ES): m/z = 773 (M-HCl+H)⁺ |
| **18H** | 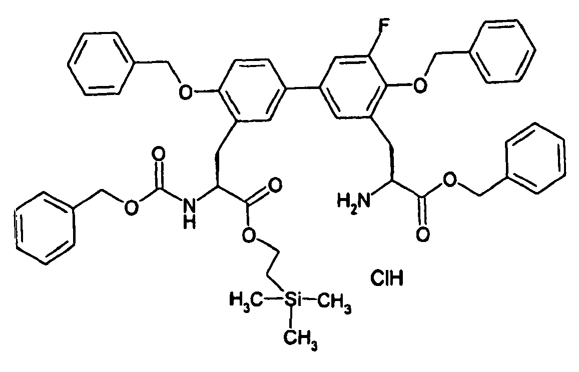 | 18A aus Beispiel 17H | LC-MS (Methode 18): Rₜ =1.28 min. MS (ES): m/z = 883 (M-HCl+H)⁺ |
| **181** | 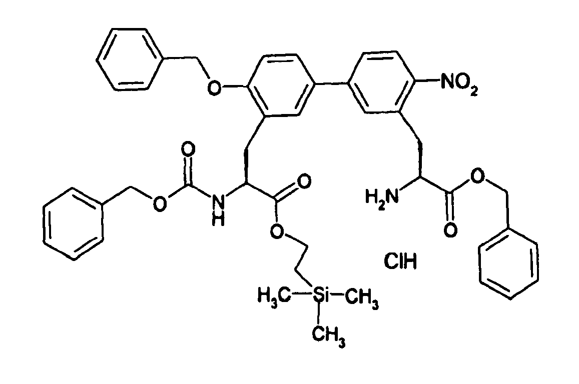 | 18A aus Beispiel 17I | LC-MS (Methode 14): Rₜ = 3.45 min. MS (ES): m/z = 804 (M-HCkl+H)⁺ |
| **18J** | 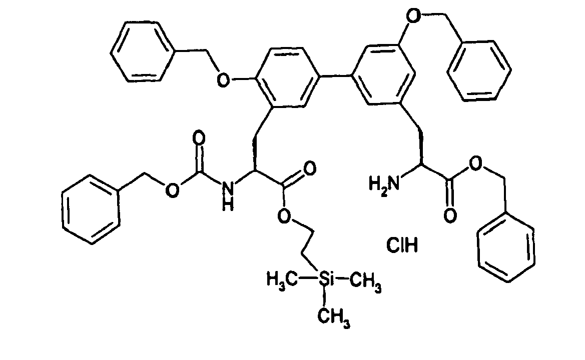 | 18A aus Beispiel 17J | LC-MS (Methode 14): Rₜ = 3.61 min. MS (ES): m/z = 865 (M-HCl+H)⁺ |
| **18N** | 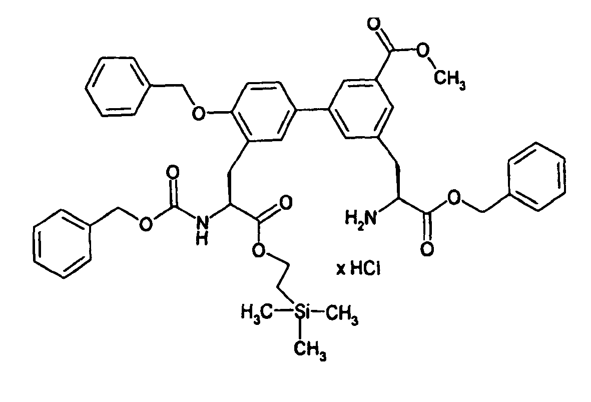 | 18A aus Beispiel 17N | LC-MS (Methode 5): Rₜ = 2.42 min. MS (ES): m/z = 817 (M-HCl+H)+ |
| **18P** | 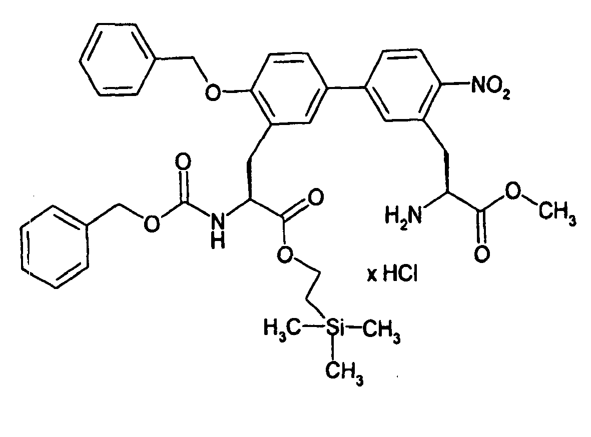 | **18A** aus Beispiel 17P | LC-MS (Methode 1): Rₜ = 2.39 min. MS (ES): m/z = 728 (M-HCl+H)⁺ |

### Beispiel 18O

### 2-(Trimethylsilyl)ethyl-(2S)-2-{[(benzyloxy)carbonyl]amino}-3-{4-(benzyloxy)-4'-fluor-3'-[(2S)-3-methoxy-2-(methylamino)propyl]biphenyl-3-yl}propanoat-Hydrochlorid

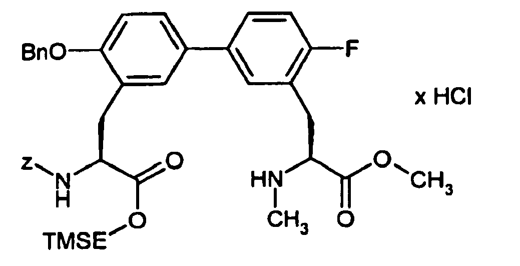

Zu einer auf 0°C gekühlten Lösung von 1.2 g (1.47 mmol) Methyl-(2*S*)-3-(4'-(benzyloxy)-3'-{(2*S*)-2-{[(benzyloxy)carbonyl]amino}-3-oxo-3-[2-(trimethylsilyl)ethoxy]propyl}-4-fluorbiphenyl-3-yl)-2-[(*tert-*butoxycarbonyl)(methyl)amino]propanoat (Beispiel 170) in 6 ml wasserfreiem Dioxan werden 23 ml einer 4M Chlorwasserstoff-Dioxan-Lösung hinzugegeben. Nach 3 h Rühren wird das Lösungsmittel im Vakuum eingedampft, mehrmals mit Dichlormethan coevaporiert und im Hochvakuum getrocknet. Das Rohprodukt wird ohne weitere Reinigung umgesetzt.
Ausbeute: quant.
LC-MS (Methode 2): Rₜ = 2.62 min.
MS (EI): m/z = 715 (M+H)⁺.

### Beispiel 19A

### 2-(Trimethylsilyl)ethyl-(2S)-3-{3'-[(2S)-3-(benzyloxy)-2-({(2S)-5-{[(benzyloxy)carbonyl]amino}-2-[(tert-butoxycarbonyl)amino]pentanoyl}amino)-3-oxopropyl]-4'-fluor-4-hydroxybiphenyl-3-y}-2-{[(benzyloxy)carbonyl]amino}propanoat

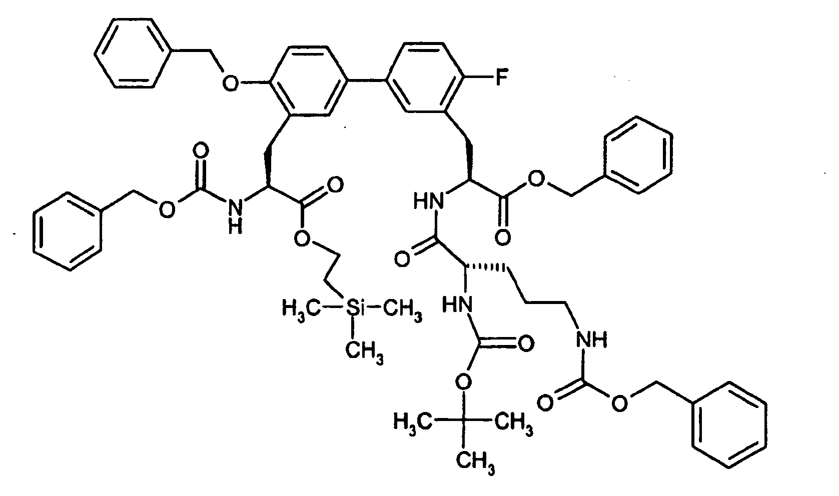

90 mg (0.12 mmol) der Verbindung aus Beispiel 18A und 42.4 mg (0.12 mmol) *N*⁵-[(Benzyloxy)carbonyl]-*N*²-(*tert-*butoxycarbonyl)-*L-*ornithin werden in 3 ml abs. DMF gelöst, auf 0°C gekühlt, mit 44 mg (0.12 mmol) HATU und 16.2 mg (0.13 mmol) Hünig-Base versetzt. Man rührt 30 min. bei dieser Temperatur, versetzt dann mit weiteren 32.4 mg (0.26 mmol) Hünig-Base und lässt die Temperatur auf RT ansteigen. Nach Reaktion über Nacht dampft man alles im Vakuum zur Trockne ein und reinigt den Rückstand säulenchromatographisch (Kieselgel 60, Laufmittel: Dichlormethan / Methanol = 100/5).
Ausbeute: 98 mg (75% d.Th.)
**LC-MS (Methode 6): Rₜ = 3.84 min.**
**MS (EI): m/z = 1124 (M+H)⁺**

### Beispiel 19B

### 2-(Trimethylsilyl)ethyl-(2S)-3-(4-(benzyloxy)-3'-{(2S)-2-[((2S,4R)-5-{[(benzyloxy)carbonyl]-amino}-2-[(tert-butoxycarbonyl)amino]-4-{[tert-butyl(dimethyl)silyl]oxy}pentanoyl)(methyl)-amino]-3-methoxy-3-oxopropyl}-4'-chlorbiphenyl-3-yl)-2-{[(benzyloxy)carbonyl]amino}-propanoat

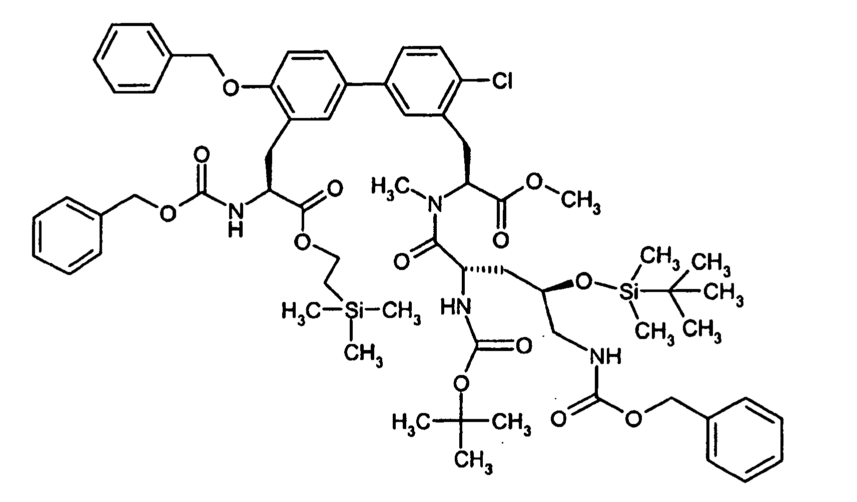

320 mg (0.43 mmol) der Verbindung aus Beispiel 18B und 320 mg (0.52 mmol) (2*S*,4R)-5-{[(Benzyloxy)carbonyl]amino}-2-[(*tert-*butoxycarbonyl)amino]-4-{[*tert-*butyl(dimethyl)silyl]-oxy}pentansäure werden in 6 ml abs. DMF gelöst, auf 0°C gekühlt, mit 20 mg (0.52 mmol) HATU und 19.2 mg (1.51 mmol) Hünig-Base versetzt. Man rührt 30 min. bei dieser Temperatur, versetzt dann mit weiteren 3.2 mg (0.06 mmol) Hünig-Base und lässt die Temperatur auf RT ansteigen. Nach Reaktion über Nacht engt man alles im Vakuum zur Trockne ein und reinigt das Rohprodukt über die HLPC (Laufmittel: Acetonitril / Methanol Gradient).
Ausbeute: 303 mg (58% d.Th.)
LC-MS (Methode 3): Rₜ = 3.81 min.
MS (EI): m/z = 1209 (M+H)⁺

Analog zu den angegebenen Vorschriften werden die in der folgenden Tabelle aufgeführten Beispiele 19C bis 19N und 19P aus den entsprechenden Edukten hergestellt:

| Beispiel-Nr. | Struktur | Hergestellt analog Beispiel-Nr. | Analytische Daten |
|---|---|---|---|
| **19C** | 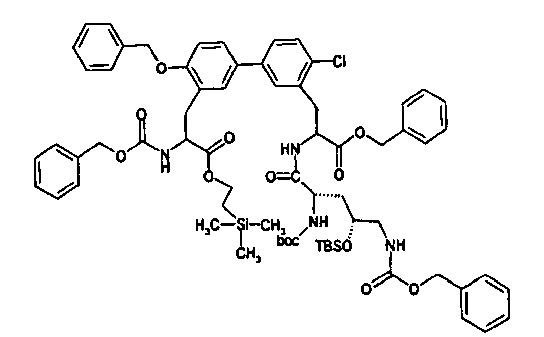 | 19B aus Beispiel 18C | LCMS (Methode 2): Rₜ = 3.69 min. MS (ES): m/z = 1271 (M+H)⁺ |
| **19D** | 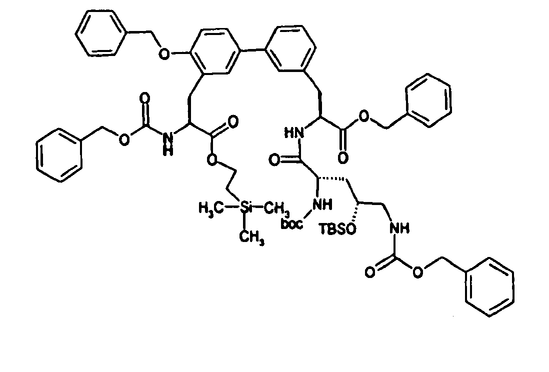 | 19B aus Beispiel 18D | LCMS (Methode 2): Rₜ = 3.64 min. MS (ES): m/z = 1237 (M+H)⁺ |
| **19E** | 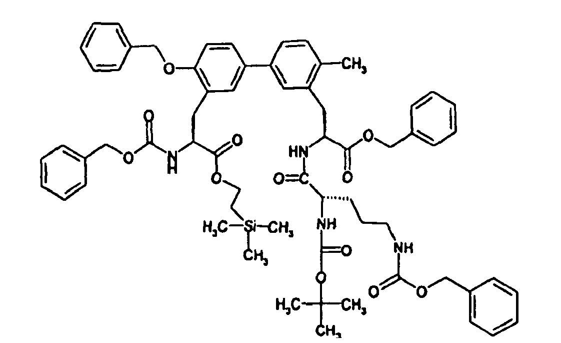 | 19A aus Beispiel 18E | LCMS (Methode 3): Rₜ = 3.54 min. MS (ES): m/z = 1121 (M+H)⁺ |
| **19F** | 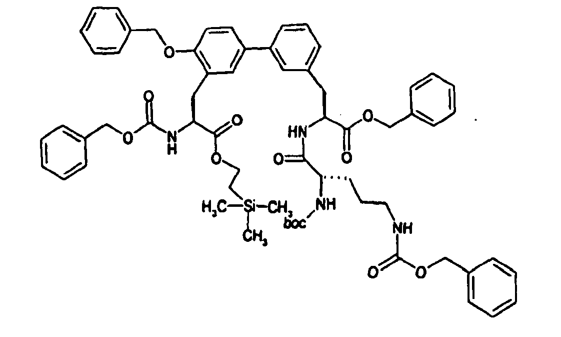 | 19A aus Beispiel 18D | LCMS (Methode 9): Rₜ = 5.35 min. MS (ES): m/z = 1107 (M+H)⁺ |
| **19G** | 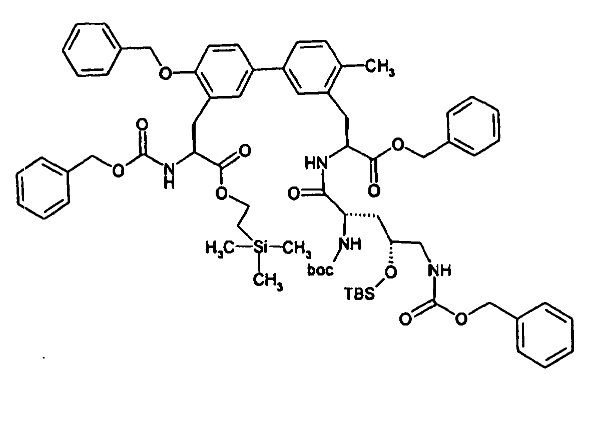 | 19B aus Beispiel 18E | LCMS (Methode 5): Rₜ = 3.89 min. MS (ES): m/z = 1252 (M+H)⁺ |
| **19H** | 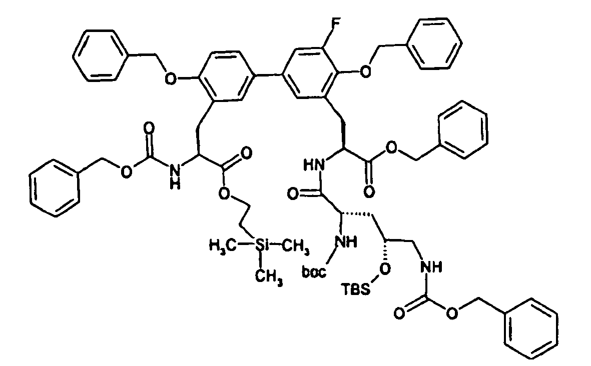 | 19B aus Beispiel 18H | LCMS (Methode 10): Rₜ = 1.34 min. MS (ES): m/z = 1362 (M+H)⁺ |
| **19I** | 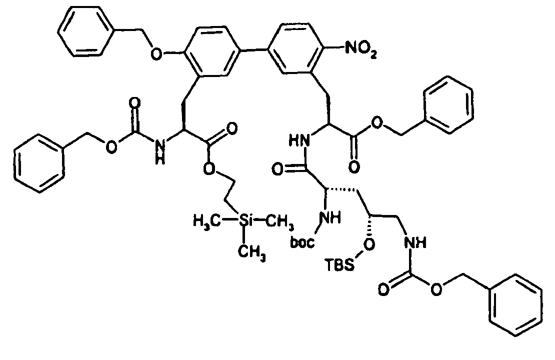 | 19B aus Beispiel 18I | LCMS (Methode 10): Rₜ = 1.04 min. MS (ES): m/z = 1282 (M+H)⁺ |
| **19J** | 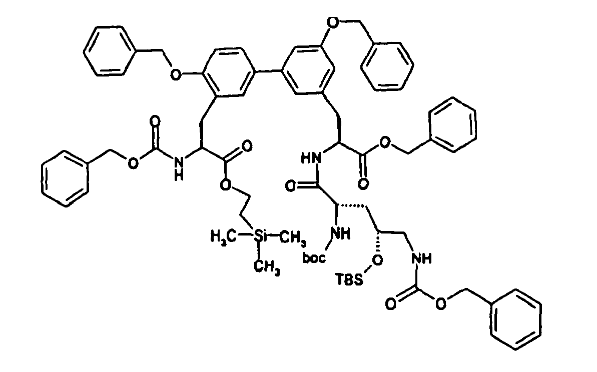 | 19B aus Beispiel 18J | LCMS (Methode 10): Rₜ = 1.38 min. MS (ES): m/z = 1343 (M+H)⁺ |
| **19K** | 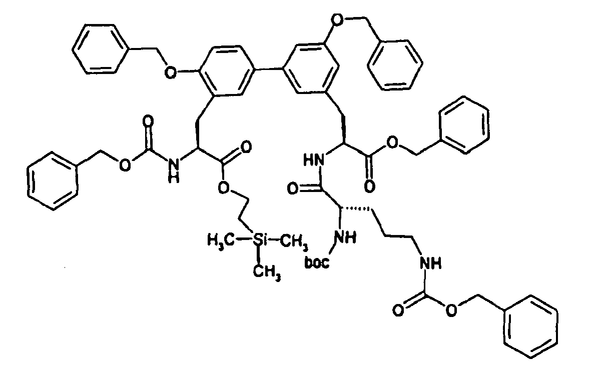 | 19A aus Beispiel 18J | LCMS (Methode 7): Rₜ = 3.58 min. MS (ES): m/z = 1213 (M+H)⁺ |
| **19L** | 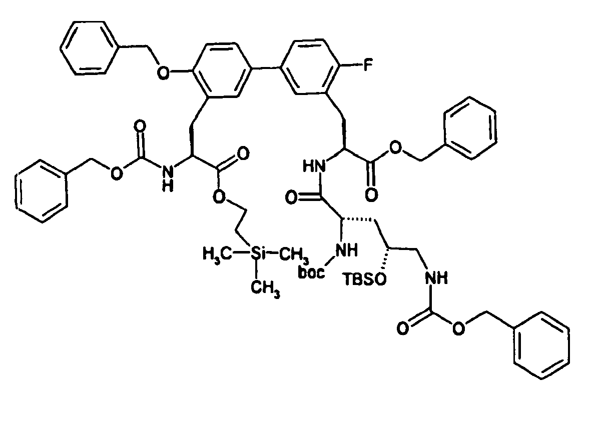 | 19B aus Beispiel 18A | LCMS (Methode 3): Rₜ = 3.87 min. MS (ES): m/z = 1255 (M+H)⁺ |
| **19M** | 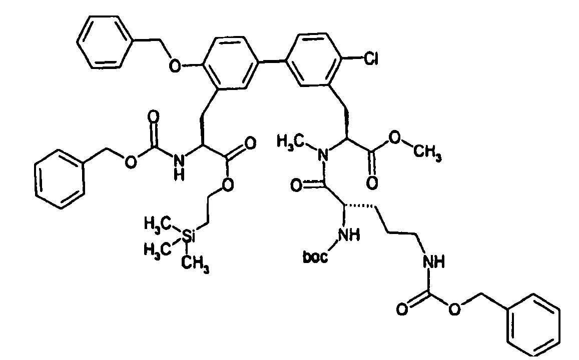 | 19A aus Beispiel 18B | LC-MS (Methode 2): Rₜ = 3.38 min. MS (ES): m/z = 1079 (M+H)⁺ |
| **19N** | 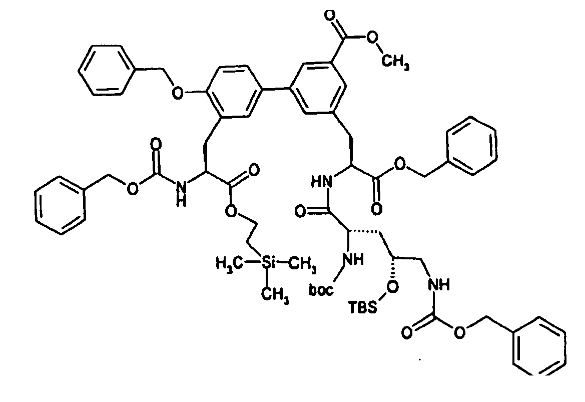 | 19B aus Beispiel 18N | LC-MS (Methode 16): Rₜ = 2.88 min. MS (ES): m/z = 1295 (M+H)⁺ |
| **19P** | 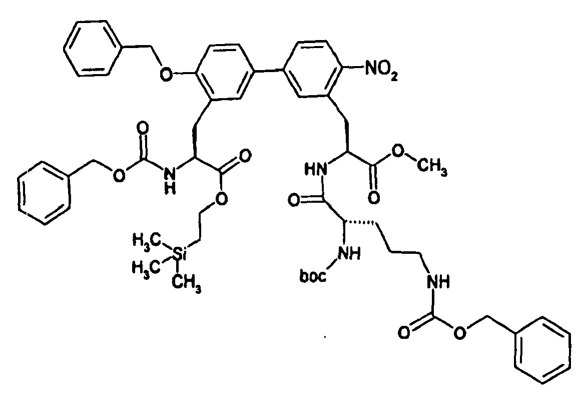 | 19A aus Beispiel 18P | LC-MS (Methode 2): Rₜ = 3.23 min. MS (ES): m/z = 1076 (M+H)⁺ |

### Beispiel 19O

### 2-(Trimethylsilyl)ethyl-(2S)-3-(4-(benzyloxy)-3'-{(2S)-2-[{(2R)-5-{[(benzyloxy)carbonyl]amino}-2-[(tert-butoxycarbonyl)amino]pentanoyl}(methyl)amino]-3-methoxy-3-oxopropyl}-4'-fluorbiphenyl-3-yl)-2-{[(benzyloxy)carbonyl]amino}propanoat

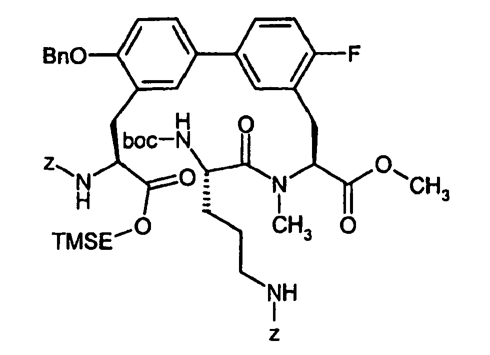

Zu einer auf 0°C gekühlten Lösung von 1.05 g (Rohprodukt, ca. 1.47 mmol) 2-(Trimethylsilyl)ethyl-(2*S*)-2-{[(benzyloxy)carbonyl]amino}-3-{4-(benzyloxy)-4'-fluor-3'-[(2*S*)-3-methoxy-2-(methylamino)propyl]biphenyl-3-yl}propanoat-Hydrochlorid (Beispiel 180) und 0.64 g (1.77 mmol) *N*⁵-[(Benzyloxy)carbonyl]-*N*²-(*tert-*butoxycarbonyl)-*L*-ornithin in 20 ml wasserfreiem DMF werden 0.73 g (1.91 mmol) HATU und 0.22 g (1.72 mmol) *N*,*N*-Diisopropylethylamin hinzugegeben. Nach 30 min Rühren bei 0°C werden zusätzliche 0.44 g (3.45 mmol) *N,N-*Diisopropylethylamin hinzugegeben. Das Reaktionsgemisch wird 15 h bei RT gerührt. Das Lösungsmittel wird dann eingedampft und der Rückstand in Dichlormethan aufgenommen. Die organische Phase wird mit Wasser gewaschen, über Natriumsulfat getrocknet und eingeengt. Das Rohprodukt wird durch präparative HPLC gereinigt.
Ausbeute 0.89 g (57% d. Th.)
LC-MS (Methode 3): Rₜ = 3.45 min.
MS (El): m/z = 1064 (M+H)⁺.

### Beispiel 20B

### (2S)-3-(4-(Benzyloxy)-3'- {(2S-2-[ {(2S,4R)-5-{[(benzyloxy)carbonyl]amino} -2-[(tert-butoxycarbonyl)amino]-4-hydroxypentanoyl}(methyl)amino]-3-methoxy-3-oxopropyl}-4'-chlorbiphenyl-3-yl)-2-{[(benzyloxy)carbonyl]amino}propansäure

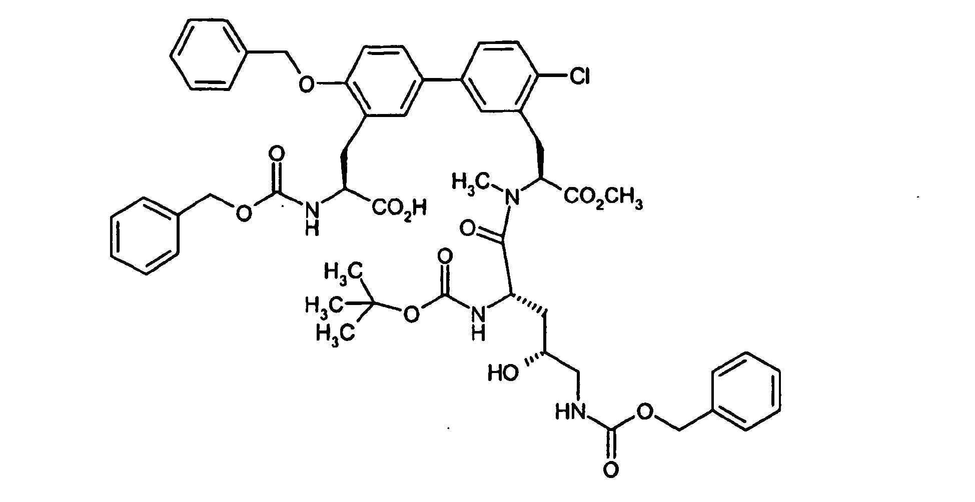

282.3 mg (0.23 mmol) 2-(Trimethylsilyl)ethyl-(2*S*)-3-(4-benyzloxy)-3'-{(2*S*)-2-[((2*S*,4*R*)-5-{[(benzyloxy)carbonyl]amino}-2-[(*tert-*butoxycarbonyl)amino]-4-{[*tert-*butyl(dimethyl)silyl]oxy}-pentanoyl)(methyl)amino]-3-methoxy-3-oxopropyl} -4'-chlorbiphenyl-3-yl)-2- {[benzyloxy)-carbonyl]amino}propanoat (Beispiel 19B) werden in 20 ml abs. DMF vorgelegt, unter Rühren mit 0.7 ml (183 mg, 0.7 mmol) Tetra-n-butylammoniumfluorid-Lösung versetzt und 20 min bei RT gerührt. Man kühlt auf 0°C und versetzt mit 60 ml Wasser und 0.5 ml 1N Salzsäure. Es fällt ein Niederschlag aus. Man rührt noch 60 min., filtriert den Niederschlag ab, wäscht mit wenig Wasser nach und trocknet den Niederschlag im Vakuum bis zur Gewichtskonstanz.
Ausbeute: 236 mg (98% d.Th.)
LC-MS (Methode 1): Rₜ = 3.07 min.
MS (EI): m/z = 994 [M+H]⁺

Analog zu den angegebenen Vorschriften werden die in der folgenden Tabelle aufgeführten Beispiele 20A, 20C bis 20N und 20P aus den entsprechenden Edukten hergestellt:

| Beispiel-Nr. | Struktur | Hergestellt analog Beispiel-Nr. | Analytische Daten |
|---|---|---|---|
| **20A** | 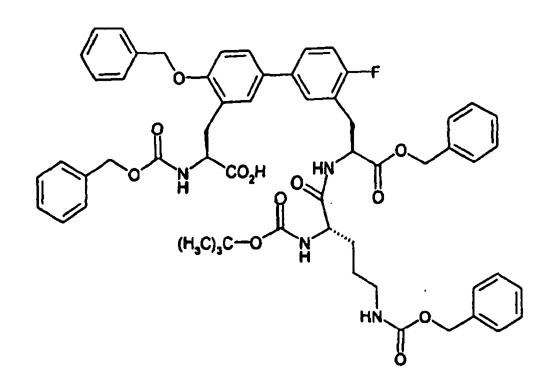 | 20B aus Beispiel 19A | LC-MS (Methode 7): Rₜ = 0.91 min. MS (EI): m/z = 1024 (M+H)⁺ |
| **20C** | 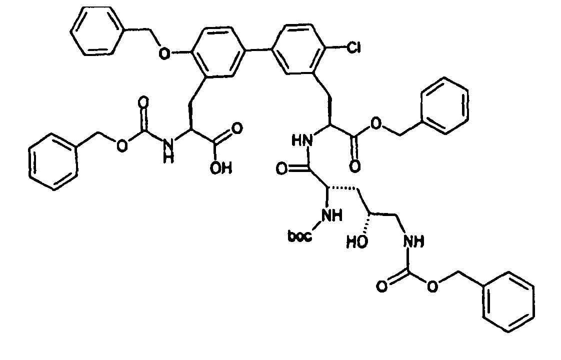 | 20B aus Beispiel 19C | LC-MS (Methode 5): Rₜ = 2.93 min. MS (ES): m/z = 1057 (M+H)⁺ |
| **20D** | 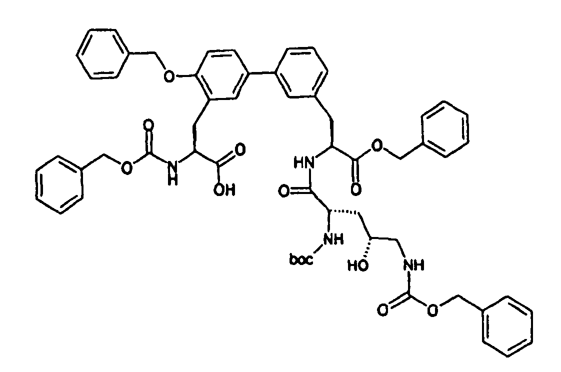 | 20B aus Beispiel 19D | LC-MS (Methode 15): Rₜ = 4.59 min. MS (ES): m/z = 1023 (M+H)⁺ |
| **20E** | 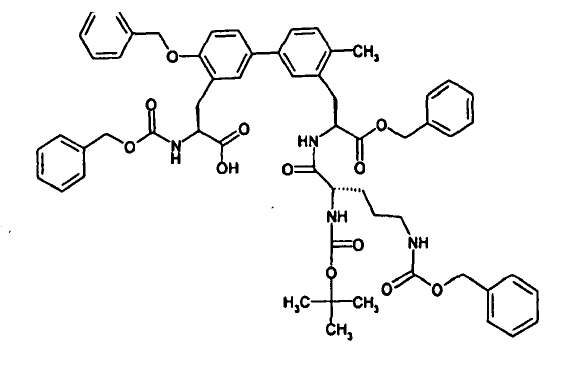 | 20B aus Beispiel 19E | LC-MS (Methode 8): Rₜ = 3.90 min. MS (ES): m/z = 1021 (M+H)⁺ |
| **20F** | 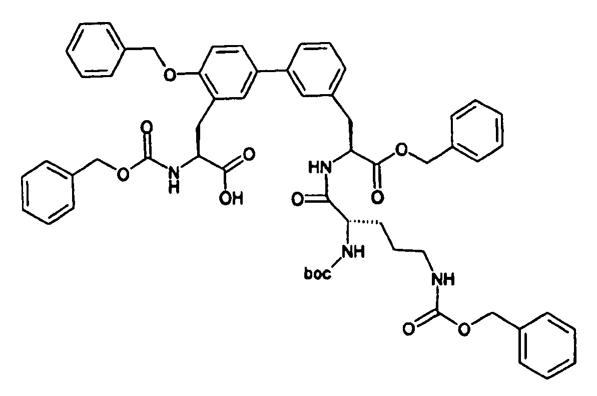 | 20B aus Beispiel 19F | LC-MS (Methode 8): Rₜ = 4.23 min. MS (ES): m/z = 1007 (M+H)⁺ |
| **20G** | 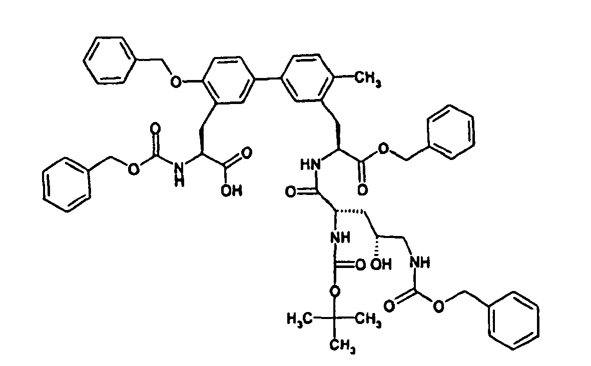 | 20B aus Beispiel 19G | LC-MS (Methode 8): Rₜ = 3.84 min. MS (ES): m/z = 1037 (M+H)⁺ |
| **20H** | 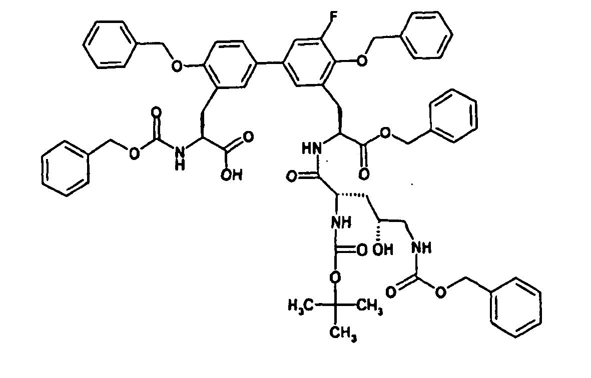 | 20B aus Beispiel 19H | |
| **20I** | 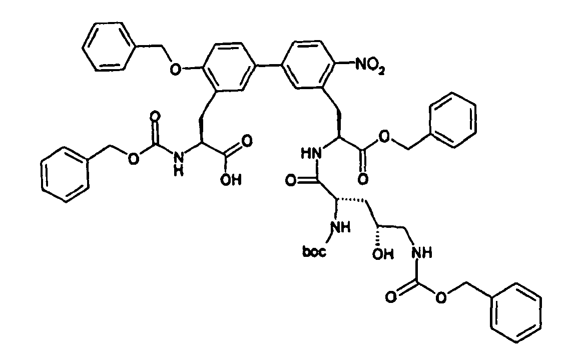 | 20B aus Beispiel 19I | LC-MS (Methode 14): Rₜ = 4.07 min. MS (ES): m/z = 1068 (M+H)⁺ |
| **20J** | 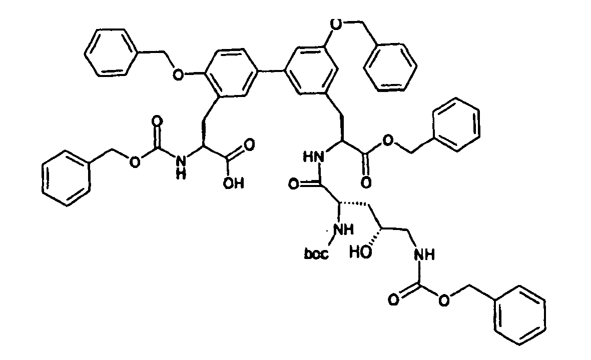 | 20B aus Beispiel 19J | LC-MS (Methode 14): Rₜ = 4.16 min. MS (ES): m/z = 1129 (M+H)⁺ |
| **20K** | 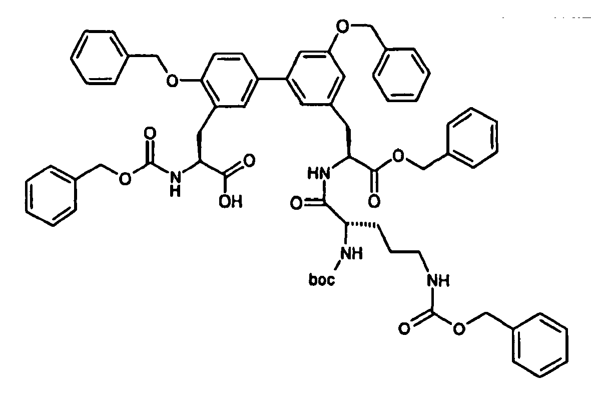 | 20B aus Beispiel 19K | LC-MS (Methode 14): Rₜ = 4.21 min. MS (ES): m/z = 1113 (M+H)⁺ |
| **20L** | 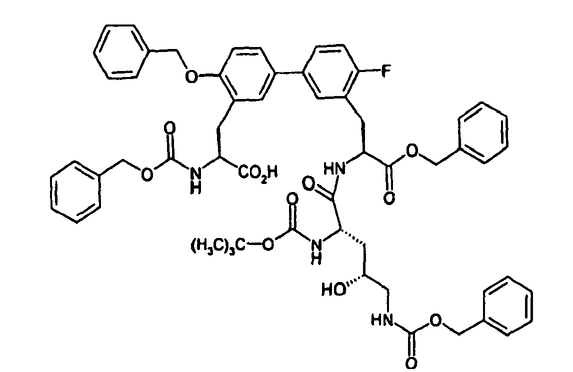 | 20B aus Beispiel 19L | LC-MS (Methode 14): Rₜ = 4.08 min. MS (EI): m/z = 1041 (M+H)⁺ |
| **20M** | 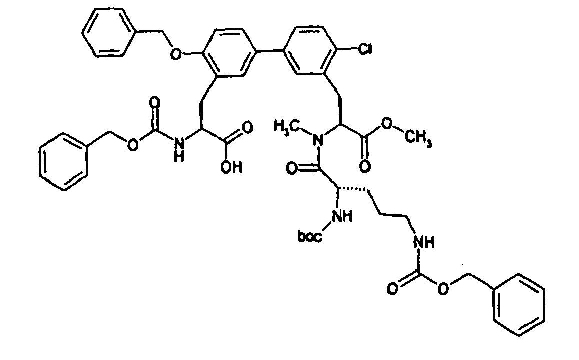 | 20B aus Beispiel 19M | LC-MS (Methode 2): Rₜ = 3.04 min. MS (ES): m/z = 979 (M+H)⁺ |
| **20N** | 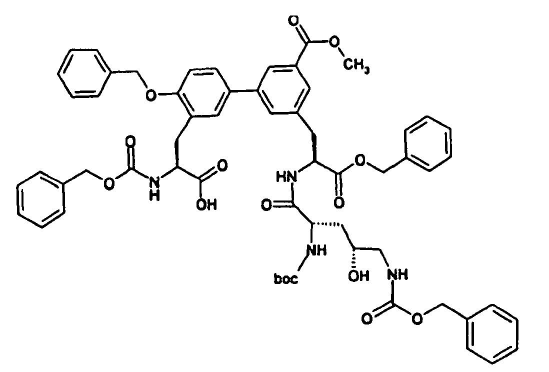 | 20B aus Beispiel 19N | LC-MS (Methode 5): Rₜ = 2.82 min. MS (ES): m/z = 1081 (M+H)⁺ |
| **20P** | 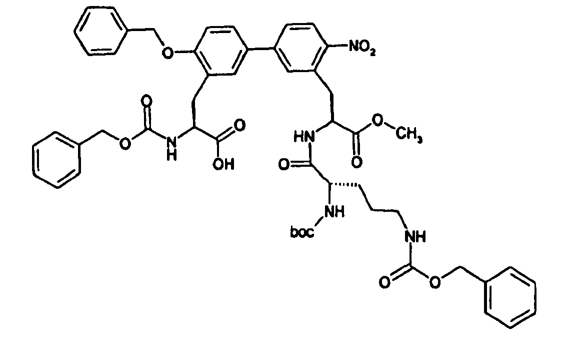 | 20B aus Beispiel 19P | LC-MS (Methode 3): Rₜ = 3.03 min. MS (ES): m/z = 976 (M+H)⁺ |

### Beispiel 20O

### (2S)-3-(4-(Benzyloxy)-3'-{(2S)-2-[{(2R)-5-{[(benzyloxy)carbonyl]amino}-2-[(tert-butoxycarbonyl)amino]pentanoyl}(methyl)amino]-3-methoxy-3-oxopropyl}-4'-fluorbiphenyl-3-yl)-2-{[(benzyloxy)carbonyl]amino}propionsäure

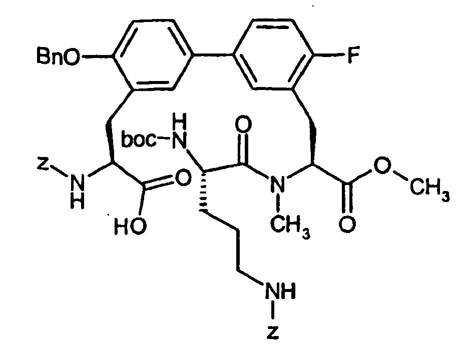

Zu einer Lösung von 980 mg (0.922 mmol) 2-(Trimethylsilyl)ethyl-(2*S*)-3-(4-(benzyloxy)-3'-{(2*S*)-2-[{(2*R*)-5-{[(benzyloxy)carbonyl]amino}-2-[(*tert-*butoxycarbonyl)amino]pentanoyl}(methyl)-amino]-3-methoxy-3-oxopropyl}-4'-fluorbiphenyl-3-yl)-2- {[(benzyloxy)carbonyl]amino}propanoat (Beispiel 190) in 20 ml absolutem DMF werden tropfenweise 1.9 ml 1N Tetrabutylammoniumfluorid in THF hinzugegeben. Nach 60 min bei RT wird auf 0°C abgekühlt und mit Eiswasser versetzt. Es wird sofort mit Dichlormethan extrahiert. Die organische Phase wird über Magnesiumsulfat getrocknet, im Vakuum eingeengt und im Hochvakuum getrocknet. Das Rohprodukt wird ohne weitere Reinigung umgesetzt.
Ausbeute: quant.
LC-MS (Methode 2): Rₜ = 2.91 min.
MS (EI): m/z = 964 (M+H)⁺.

### Beispiel 21B

### Pentafluorphenyl-(2S)-3-(4-(benzyloxy)-3'-{(2S)-2-[2-[{(2S,4S)-5-{[(benzyloxy)carbonyl]amino}-2-[(tert-butoxycarbonyl)amino]-4-hydroxypentanoyl}(methyl)amino]-3-methoxy-3-oxopropyl-4'chlorbiphenyl-3-yl)-2-{[(benzyloxy)carbonyl]amino} propanoat

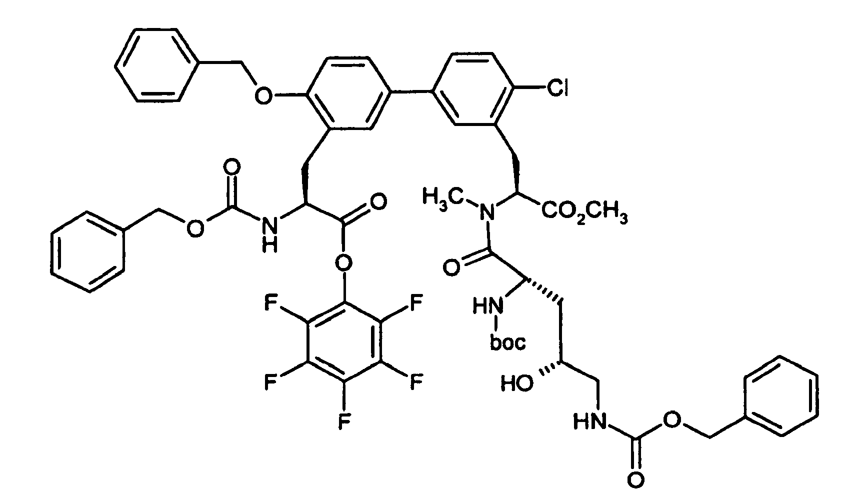

235 mg (0.24 mmol) der Verbindung aus Beispiel 20B werden in 10 ml abs. Dichlormethan gelöst, auf-20°C abgekühlt und unter Rühren mit 217 mg (1.18 mmol) Pentafluorphenyl, 2.9 mg (0.02 mmol) DMAP und 49.8 mg (0.26 mmol) EDC versetzt. Man lässt die Temperatur langsam auf RT ansteigen und rührt über Nacht nach. Es wird im Vakuum eingeengt und das Rohprodukt im Hochvakuum bis zur Gewichtskonstanz getrocknet.
Ausbeute: 219 mg (57% d.Th.)
LC-MS (Methode 2): Rₜ = 3.25 min.
MS (EI): m/z = 1160 [M+H]⁺

Analog zu den angegebenen Vorschriften werden die in der folgenden Tabelle aufgeführten Beispiele 21A, 21C bis 21N und 21P aus den entsprechenden Edukten hergestellt:

| Beispiel-Nr. | Struktur | Hergestellt analog Beispiel-Nr. | Analytische Daten |
|---|---|---|---|
| **21A** | 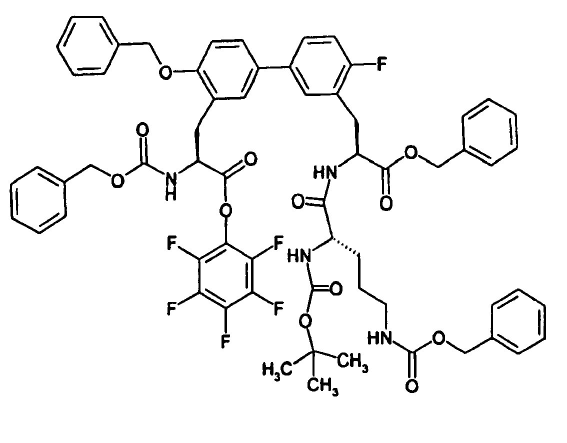 | 21B aus Beispiel 20B | LC-MS (Methode 2): Rₜ = 3.27 min. MS (EI): m/z = 1190 (M+H)⁺ |
| **21C** | 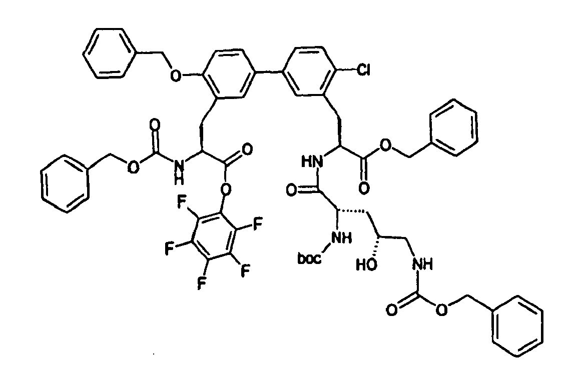 | 21B aus Beispiel 20C | LC-MS (Methode 5): Rₜ = 3.28 min. MS (ES): m/z = 1223 (M+H)⁺ |
| **21D** | 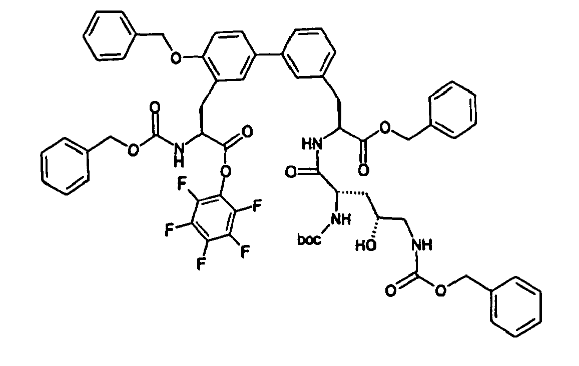 | 21B aus Beispiel 20D | LC-MS (Methode 8): R, = 4.72 min. MS (ES): m/z = 1189 (M+H)⁺ |
| **21E** | 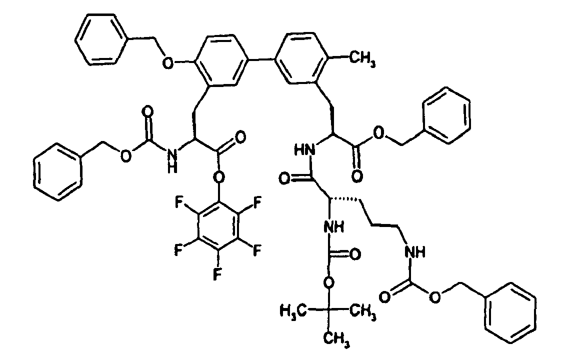 | 21B aus Beispiel 20E | LC-MS (Methode 5): Rₜ = 3.32 min. MS (ES): m/z = 1187 (M+H)⁺ |
| **21F** | 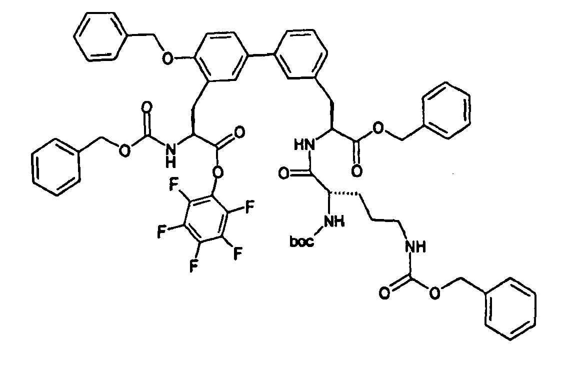 | 21B aus Beispiel 20F | LC-MS (Methode 6): Rₜ = 3.59 min. MS (ES): m/z = 1173 (M+H)⁺ |
| **21G** | 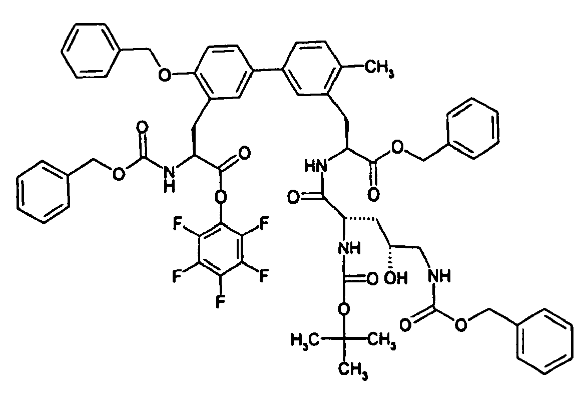 | 21B aus Beispiel 20G | |
| **21H** | 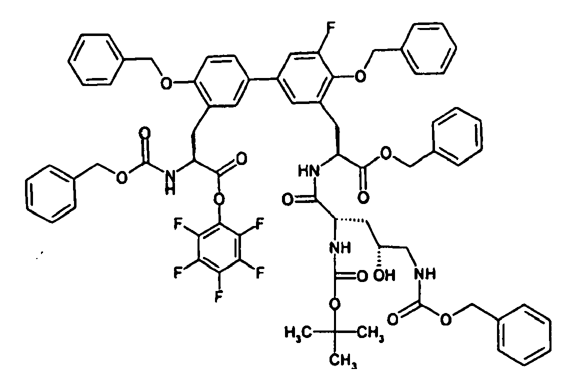 | 21B aus Beispiel 20H | |
| **21I** | 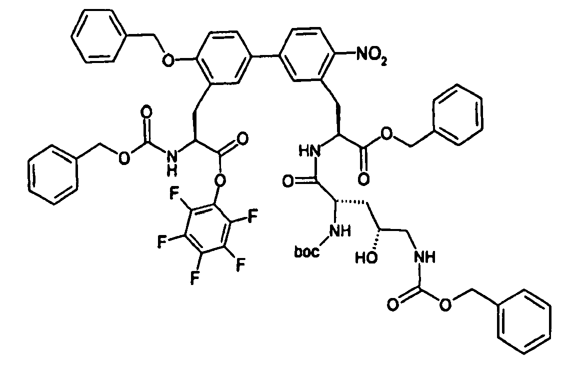 | 21B aus Beispiel 20I | LC-MS (Methode 14): Rₜ = 4.39 min. MS (ES): m/z = 1234 (M+H)⁺ |
| **21J** | 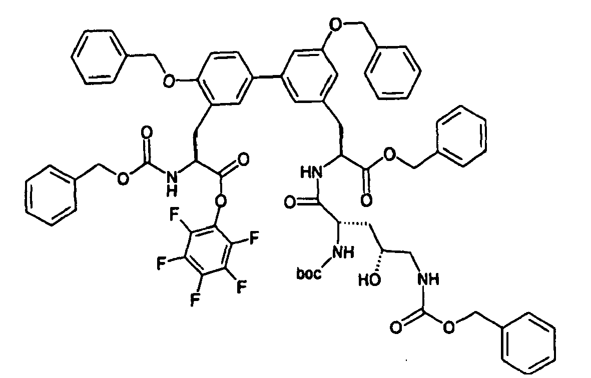 | 21B aus Beispiel 20J | |
| **21K** | 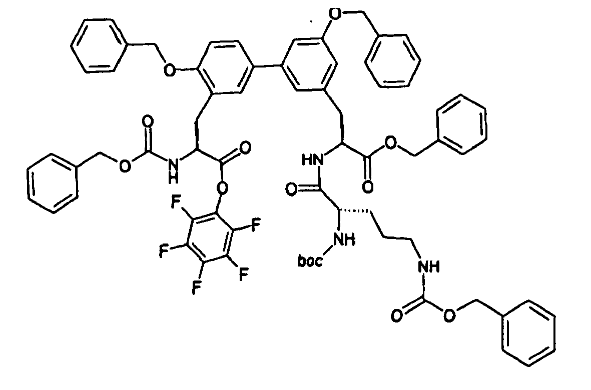 | 21B aus Beispiel 20K | |
| **21L** | 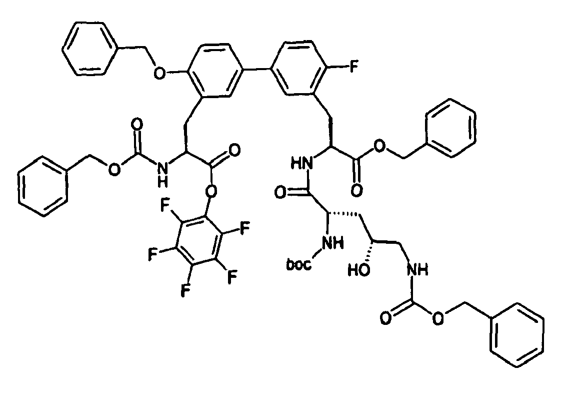 | 21B aus Beispiel 20L | LC-MS (Methode 1): Rₜ = 3.42 min. MS (EI): m/z = 1207 (M+H)⁺ |
| **21M** | 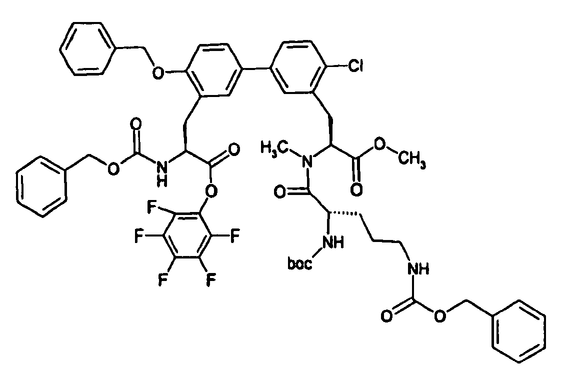 | 21B aus Beispiel 20M | LC-MS (Methode 3): Rₜ = 3.45 min. MS (ES): m/z = 1145 (M+H)⁺ |
| **21N** | 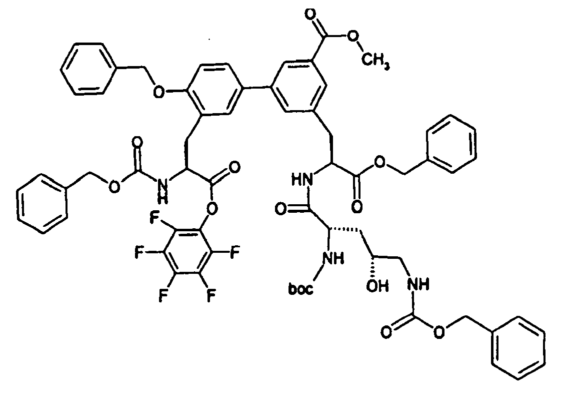 | 21B aus Beispiel 20N | LC-MS (Methode 4): Rₜ = 4.01 min. MS (ES): m/z = 1247 (M+H)⁺ |
| **21P** | 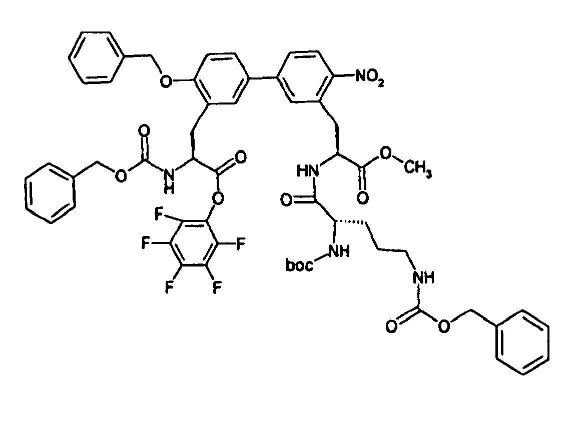 | 21B aus Beispiel 20P | LC-MS (Methode 3): Rₜ = 3.37 min. MS (ES): m/z = 1142 (M+H)⁺ |

### Beispiel 21O

### Pentafluorphenyl-(2S)-3-(4-(benzyloxy)-3'-{(2S)-2-[{(2R)-S{[(benzyloxy)carbonyl]amino}-2-[(tert-butoxycarbonyl)amino]pentanoyl}(methyl)amino]-3-methoxy-3-oxopropyl}-4'-fluorbiphenyl-3-yl)-2-{[(benzyloxy)carbonyl]amino}propanoat

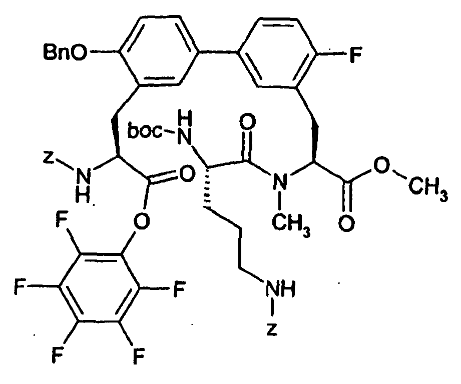

0.89 g (Rohprodukt, ca. 0.922 mmol) (2*S*)-3-(4-(Benzyloxy)-3'-{(2*S*)-2-[{(2*R*)-5-{[(benzyloxy)-carbonyl]amino}-2-[(*tert-*butoxycarbonyl)amino]pentanoyl}(methyl)amino]-3-methoxy-3-oxo-propyl}-4'-fluorbiphenyl-3-yl)-2-{[(benzyloxy)carbonyl]amino}propionsäure (Beispiel 200) werden in 50 ml Dichlormethan vorgelegt. Bei -25°C werden 0.85 g (4.61 mmol) Pentafluorphenol, 0.21 g (1.11 mmol) EDC und 45 mg (0.37 mmol) DMAP unter Argon hinzugefügt. Die Mischung erwärmt sich über Nacht langsam auf RT. Die Reaktionsmischung wird im Vakuum eingeengt und im Hochvakuum kurz getrocknet. Das Rohprodukt wird ohne weitere Reinigung umgesetzt.
Ausbeute: quant.
LC-MS (Methode 3): Rₜ = 3.41 min.
MS (EI): m/z = 1130 (M+H)⁺.

### Beispiel 22B

### Methyl-(2S)-2-[{(2S,4S)-2-amino-5-{[(benzyloxy)carbonyl]amino}-4-hydroxypentanoyl}(methyl)-amino]-3-{4'-(benzyloxy)-3'-[(2S)-2-{[(benzyloxy)carbonyl]amino}-3-oxo-3-(pentafluorphenoxy)propyl]-4-chlorbiphenyl-3-yl}propanoat-Hydrochlorid

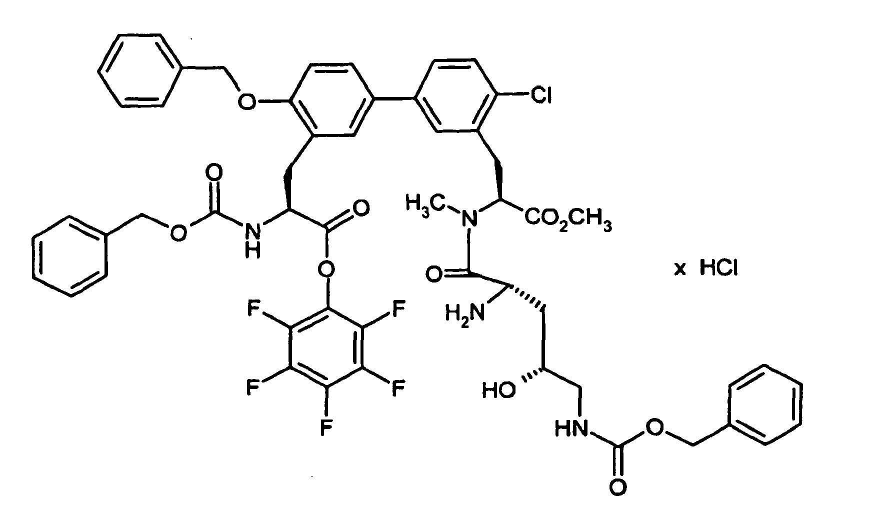

219 mg (0.14 mmol) der Verbindung aus Beispiel 21B werden in 2.3 ml Dioxan gelöst und unter Rühren bei 0°C mit 6 ml einer 4N Chlorwasserstoff-Dioxan-Lösung versetzt. Man rührt 30 min bei dieser Temperatur, lässt die Temperatur auf RT ansteigen, rührt eine weitere Stunde und dampft dann alles im Vakuum zur Trockne ein. Nach Trocknen im Hochvakuum bis zur Gewichtskonstanz erhält man das Produkt.
Ausbeute: 207 mg (quantitativ)
LC-MS (Methode 2): Rₜ = 3.25 min.
MS (EI): m/z = 1060 (M-HCl+H)⁺

Analog zu den angegebenen Vorschriften werden die in der folgenden Tabelle aufgeführten Beispiele 22A, 22C bis 22N und 22P aus den entsprechenden Edukten hergestellt:

| Beispiel-Nr. | Struktur | Hergestellt analog Beispiel-Nr. | Analytische Daten |
|---|---|---|---|
| **22A** | 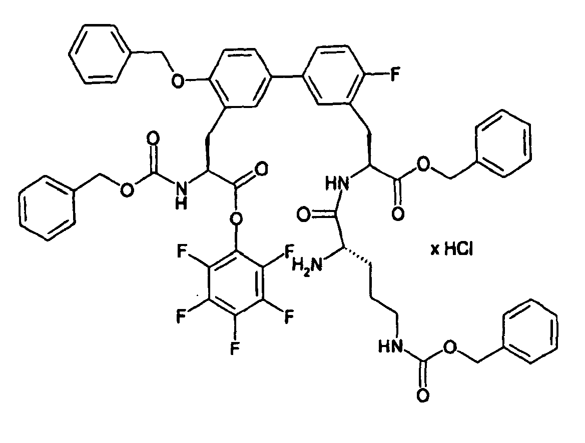 | 22B aus Beispiel 21A | LC-MS (Methode 6): Rₜ = 0.35 min. MS (EI): m/z = 1090 (M-HCl+H)⁺ |
| **22C** | 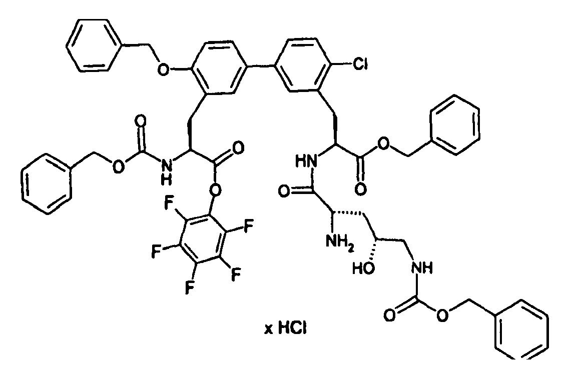 | 22B aus Beispiel 21C | LC-MS (Methode 5): Rₜ = 2.54 min. MS (ES): m/z = 1123 (M-HCl+H)⁺ |
| **22D** | 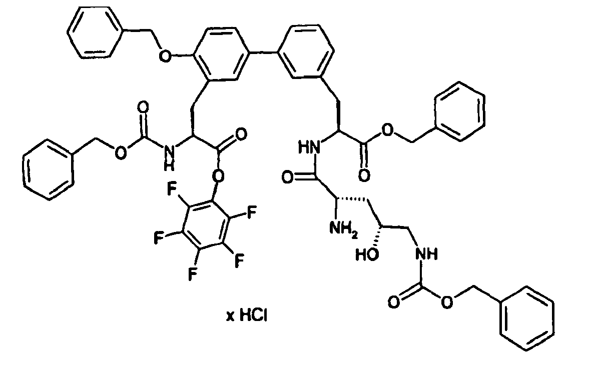 | 22B aus Beispiel 21D | LC-MS (Methode 14): Rₜ = 3.45 min. MS (ES): m/z = 1189 (M-HCl+H)⁺ |
| **22E** | 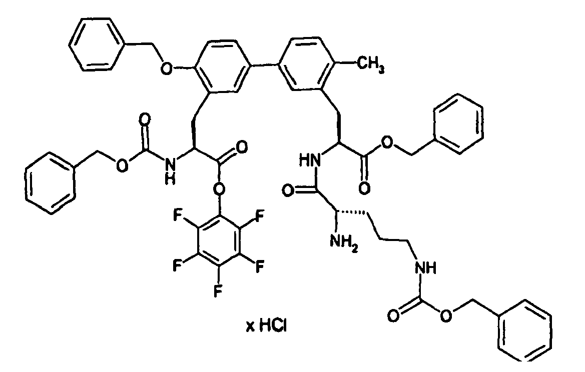 | 22B aus Beispiel 21E | LC-MS (Methode 5): Rₜ = 3.32 min. MS (ES): m/z = 1087 (M-HCl+H)⁺ |
| **22F** | 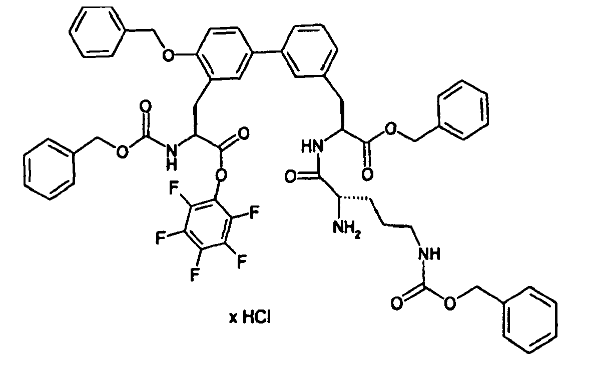 | 22B aus Beispiel 21F | LC-MS (Methode 5): Rₜ = 2.51 min. MS (ES): m/z = 1073 (M-HCl+H)⁺ |
| **22G** | 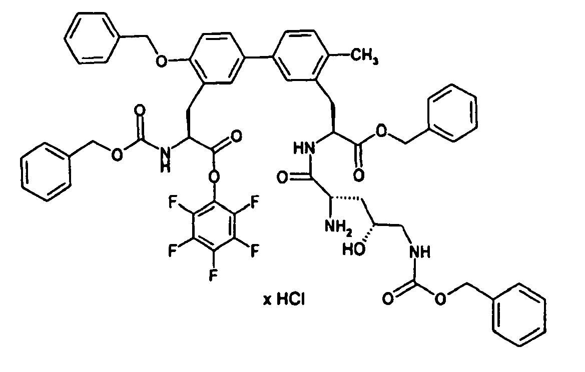 | 22B aus Beispiel 21G | LCMS (Methode 5): Rₜ = 2.60 min. MS (ES): m/z=1 1104 (M-HCl+H)⁺ |
| **22H** | 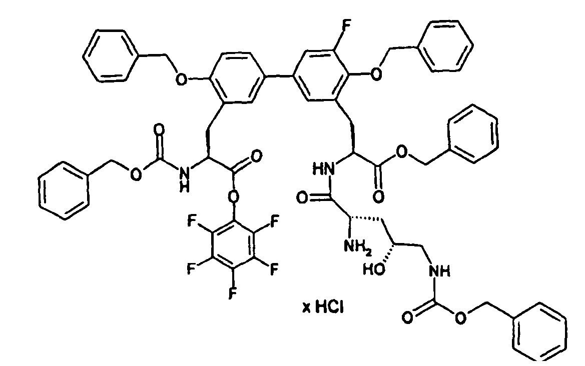 | 22B aus Beispiel 21H | |
| **22I** | 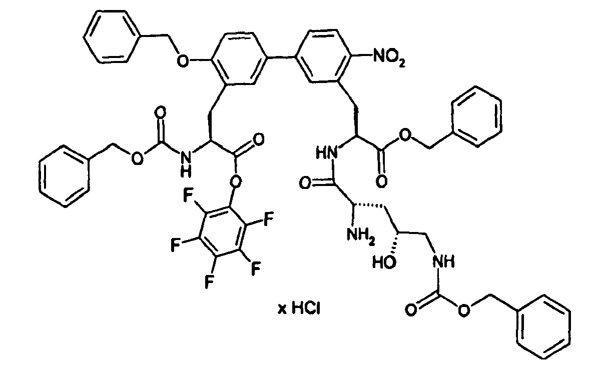 | 22B aus Beispiel 21I | LC-MS (Methode 14): Rₜ = 3.46 min. MS (ES): m/z= 1134 (M-HCl+H)⁺ |
| **22J** | 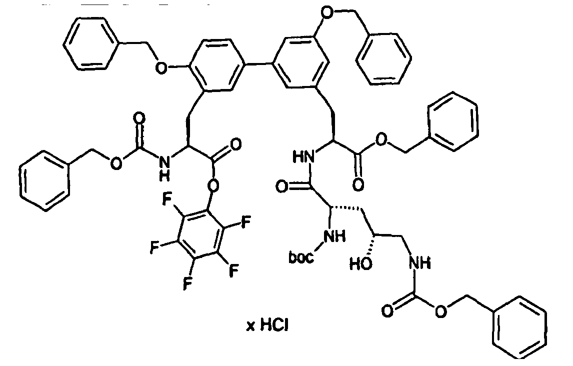 | 22B aus Beispiel 21J | |
| **22K** | 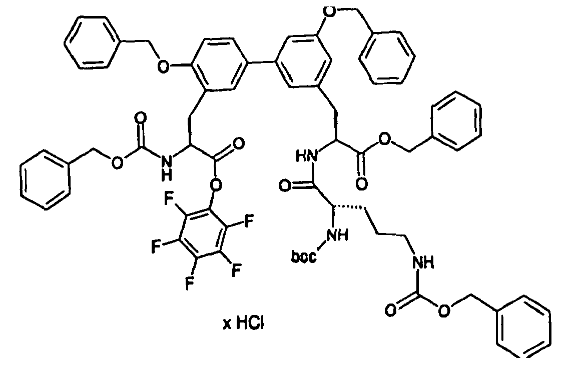 | 22B aus Beispiel 21K | |
| **22L** | 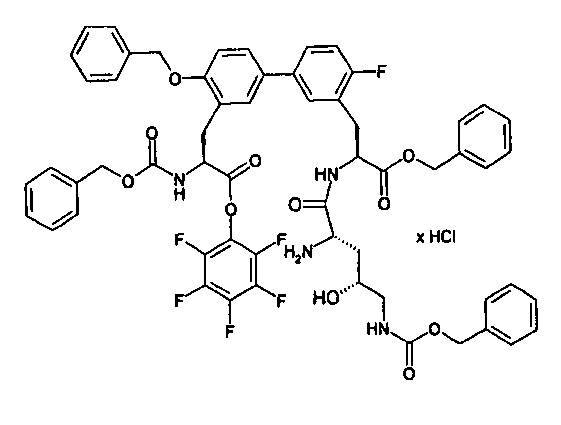 | 22B aus Beispiel 21L | LC-MS (Methode 2): Rₜ = 2.40 min. MS (EI): m/z =1107 (M-HCl+H)⁺ |
| **21M** | 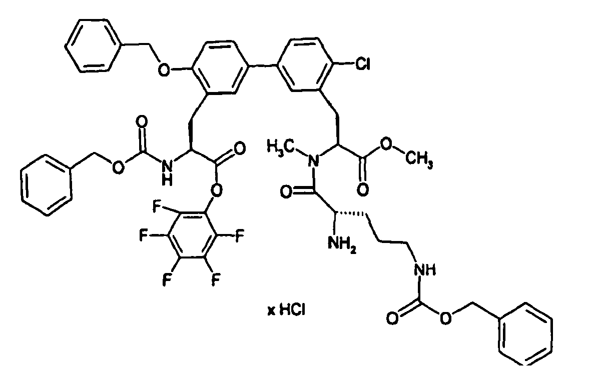 | 22B aus Beispiel 21M | LC-MS (Methode 3): Rₜ = 2.99 min. MS (ES): m/z =1045 (M-HCl+H)⁺ |
| **22N** | 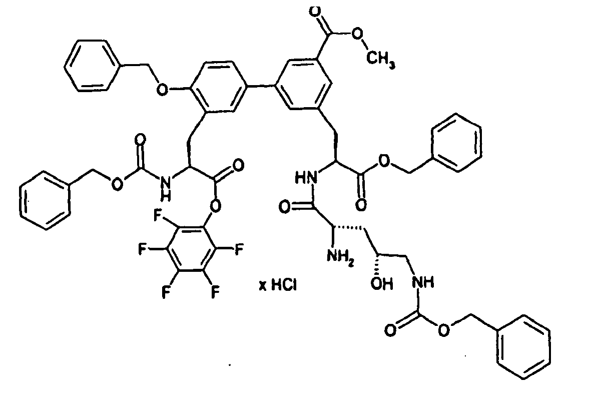 | 22B aus Beispiel 21N | LC-MS (Methode 5): Rₜ = 2.53 min. MS (ES): m/z =1147 (M-HCl+H)⁺ |
| **22P** | 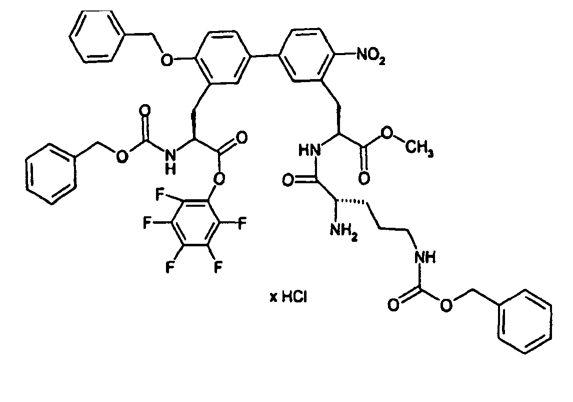 | 22B aus Beispiel 21P | LC-MS (Methode 3): Rₜ = 2.77 min. MS (ES): m/z = 1042 (M-HCl+H)⁺ |

### Beispiel 22O

### Methyl-(2S)-2-[((2R)-2-amino-5-{[(benzyloxy)carbonyl]amino}pentanoyl)(methyl)amino]-3-{4'-(benzyloxy)-3'-[(2S)-2-{[(benzyloxy)carbonyl]amino}-3-oxo-3-(pentafluorphenoxy)propyl]-4-fluorbiphenyl-3-yl}propanoat-Hydrochlorid

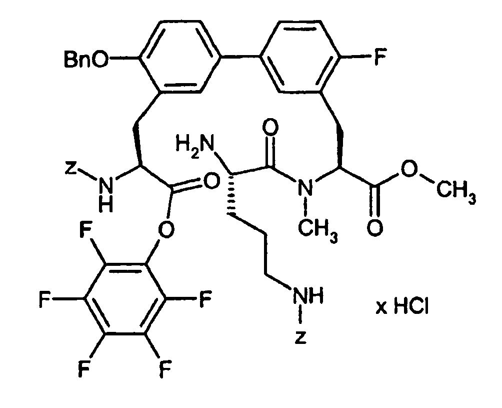

Zu einer Lösung von 1.038 g (Rohprodukt, ca. 0.92 mmol) Pentafluorphenyl-(2*S*)-3-(4-(benzyloxy)-3'-{(2*S*)-2-[{(2*R*)-5-{[(benzyloxy)carbonyl]amino}-2-[(-butoxycarbonyl)amino]-pentanoyl}(methyl)amino]-3-methoxy-3-oxopropyl}-4'-fluorbiphenyl-3-yl)-2-{[(benzyloxy)-carbonyl]amino}propanoat (Beispiel 210) in 19 ml wasserfreiem Dioxan werden bei 0°C 37 ml einer 4M Chlorwasserstoff-Dioxan-Lösung hinzugegeben. Nach 1 h bei 0°C wird die Reaktionslösung im Vakuum eingeengt, mehrmals mit Dichlormethan coevaporiert und im Hochvakuum getrocknet. Das Rohprodukt wird ohne weitere Reinigung umgesetzt.
Ausbeute: quant.
LC-MS (Methode 2): Rₜ = 2.54 min.
MS (EI): m/z = 1029 (M+H)⁺.

### Beispiel 23B

### Methyl-(8S,11S,14S)-17-(benzyloxy)-14-{[(benzyloxy)carbonyl]amino}-11-((2R)-3-{[(benzyloxy)-carbonyl]amino}-2-hydroxypropyl)-5-chlor-10,13-dioxo-9,12-diazatricyclo[14.3.1.1^{2,6}]henicosa-1(20,2(21),3,5,16,18-hexaen-8-carboxylat

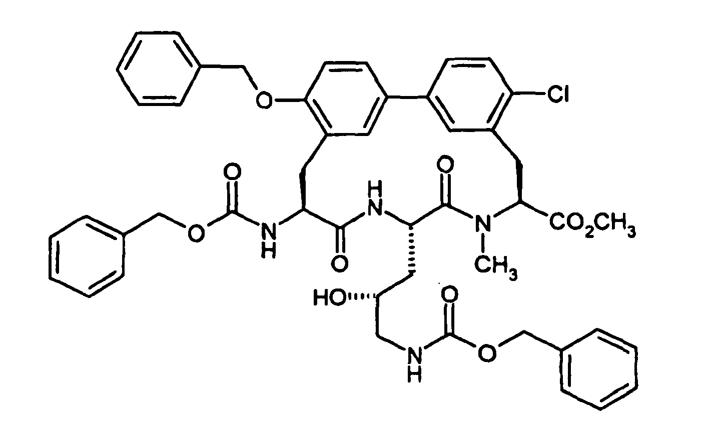

207 mg (0.18 mmol) der Verbindung aus Beispiel 22B werden in 250 ml abs. Chloroform gelöst und unter kräftigem Rühren tropfenweise in 20 min mit 1.8 ml (1.3 g, 12.9 mmol) Triethylamin in 30 ml Chloroform versetzt. Man lässt über Nacht weiterrühren und dampft alles im Vakuum ein (Badtemperatur ≤ 40°C). Der Rückstand wird über präparative HPLC getrennt (Acetonitril / Wasser).
Ausbeute: 77 mg (46% d.Th.)
LC-MS (Methode 2): Rₜ = 2.96 min.
MS (EI): m/z = 876 (M+H)⁺

Analog zu den angegebenen Vorschriften werden die in der folgenden Tabelle aufgeführten Beispiele 23A, 23C bis 23N und 23P aus den entsprechenden Edukten hergestellt:

| Beispiel-Nr. | Struktur | Hergestellt analog Beispiel-Nr. | Analytische Daten |
|---|---|---|---|
| **23A** | 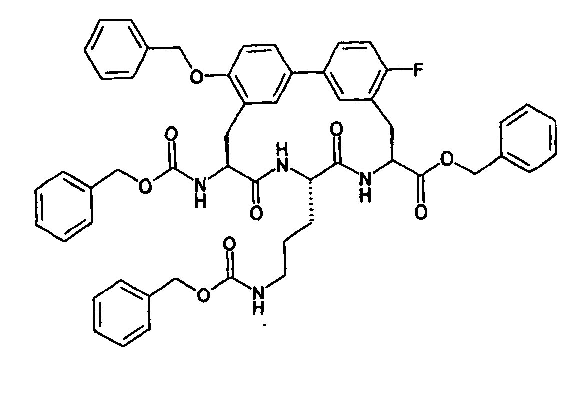 | 23B aus Beispiel 22A | LC-MS (Methode 8): Rₜ = 4.35 min. MS (EI): m/z = 906 (M+H)⁺ |
| **23C** | 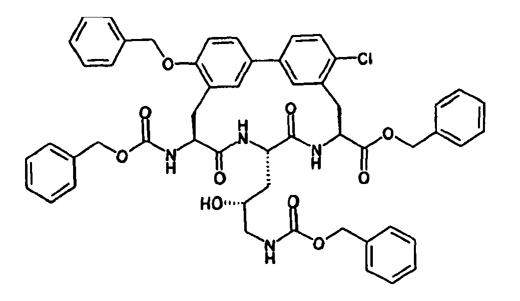 | 23B aus Beispiel 22C | LC-MS (Methode 8): Rₜ = 4.03 min. MS (EI): m/z = 939 (M+H)⁺ |
| **23D** | 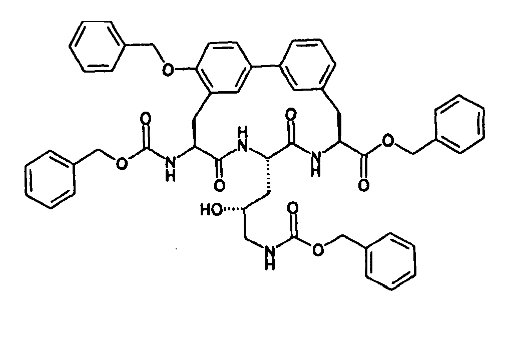 | 23B aus Beispiel 22D | LC-MS (Methode 8): Rₜ = 3.85 min. MS (EI): m/z = 905 (M+H)⁺ |
| **23E** | 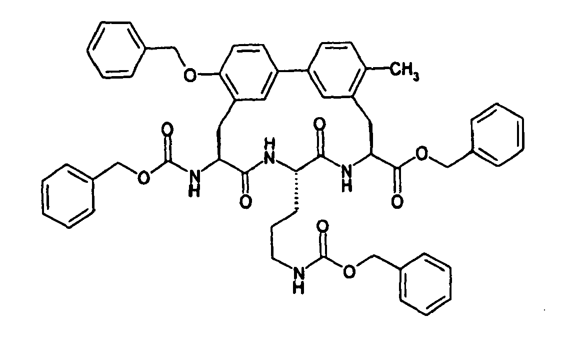 | 23B aus Beispiel 22E | LC-MS (Methode 1): Rₜ = 3.23 min. MS (EI): m/z = 903 (M+H)⁺ |
| **23F** | 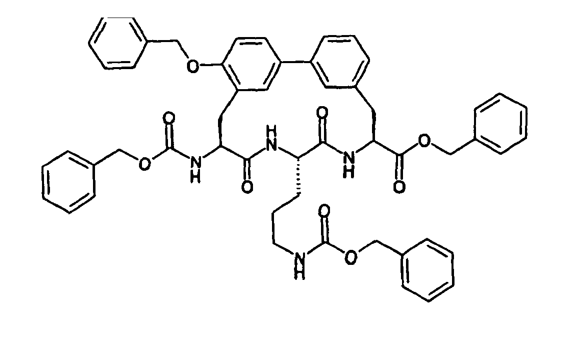 | 23B aus Beispiel 22F | LC-MS (Methode 6): Rₜ = 1.51 min. MS (EI): m/z = 889 (M+H)⁺ |
| **23G** | 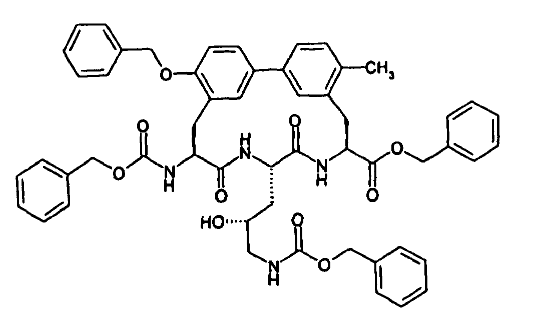 | 23B aus Beispiel 22G | LC-MS (Methode 5): Rₜ = 2.96 min. MS (EI): m/z = 919 (M+H)⁺ |
| **23H** | 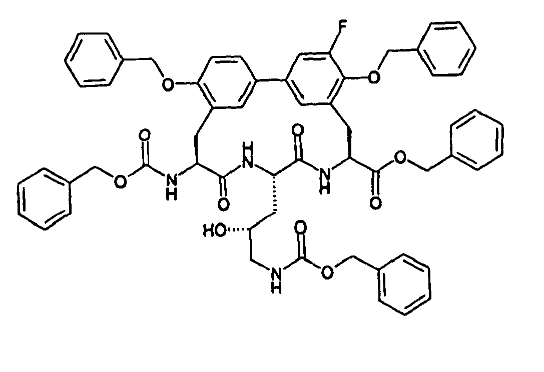 | 23B aus Beispiel 22H | LC-MS (Methode 6): Rₜ = 2.80 min. MS (EI): m/z = 1029 (M+H)⁺ |
| **23I** | 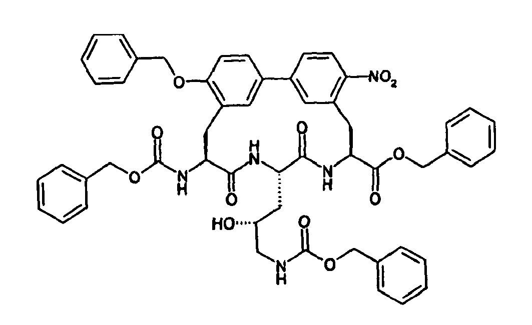 | 23B aus Beispiel 22I | LC-MS (Methode 7): Rₜ = 0.99 min. MS (EI): m/z = 950 (M+H)⁺ |
| **23J** | 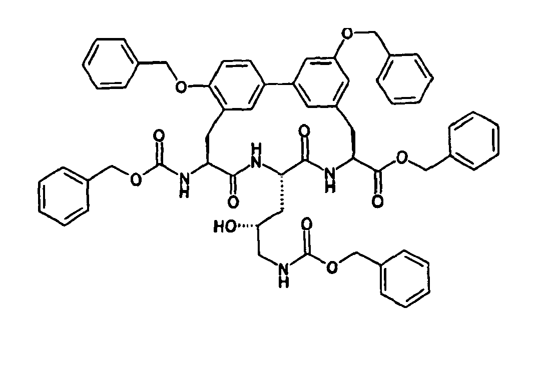 | 23B aus Beispiel 22J | LC-MS (Methode 14): Rₜ = 4.18 min. MS (EI): m/z =1011 (M+H)⁺ |
| **23K** | 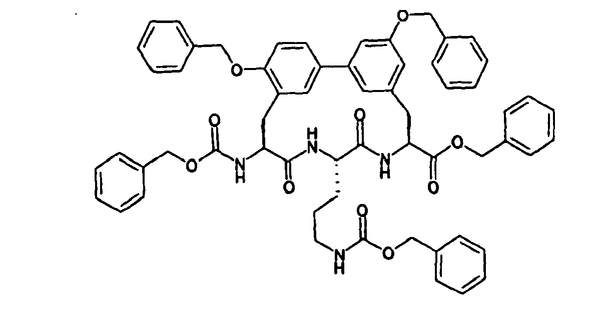 | 23B aus Beispiel 22K | LC-MS (Methode 14): Rₜ = 4.22 min. MS (EI): m/z = 995 (M+H)⁺ |
| **23L** | 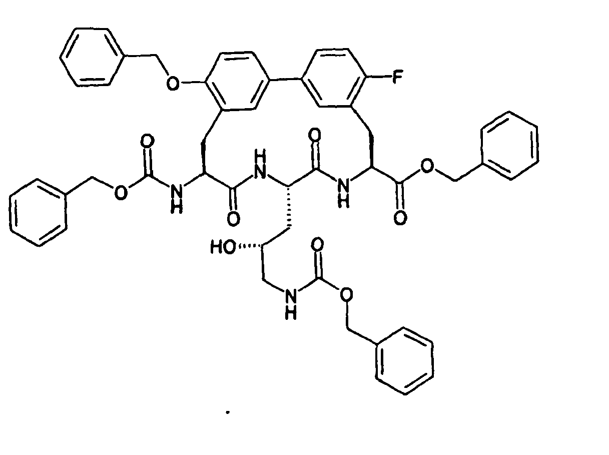 | 23B aus Beispiel 22L | LC-MS (Methode 2): Rₜ = 3.05 min. MS (EI): m/z = 923 (M+H)⁺ |
| **23M** | 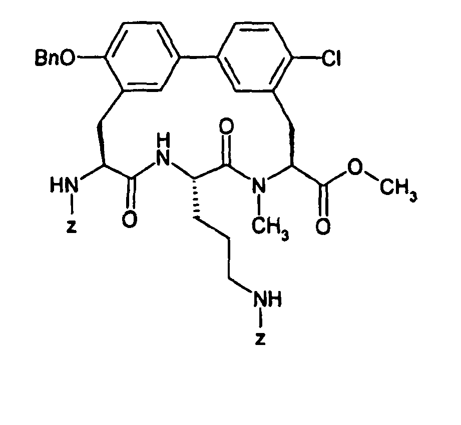 | 23B aus Beispiel 22M | LC-MS (Methode 3): Rₜ = 3.26 min. MS (ES): m/z = 861 (M+H)⁺ |
| **23N** | 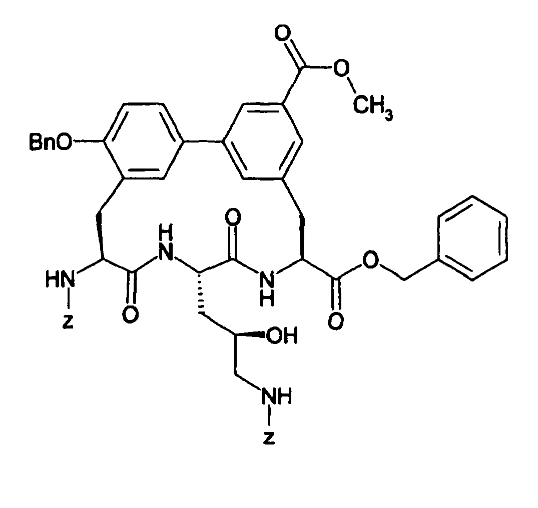 | 23B aus Beispiel 22N | LC-MS (Methode 5): Rₜ = 2.86 min. MS (ES): m/z = 963 (M+H)⁺ |
| 23P | 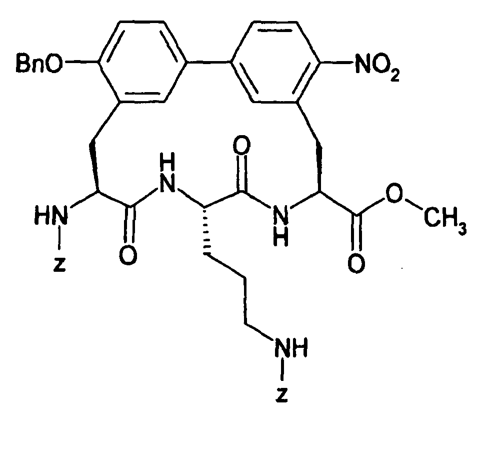 | 23B aus Beispiel 22P | LC-MS (Methode 2): Rₜ = 2.84 min. MS (ES): m/z = 858 (M+H)⁺ |

### Beispiel 23O

### Methyl-(8S,11S,14S)-17-(benzyloxy)-14-{[(benzyloxy)carbonyl]amino}-11-(3-{[(benzyloxy)-carbonyl]amino}propyl)-5-fluor-9-methyl-10,13-dioxo-9,12-diazatricyclo[14.3.1.1^{2,6}]henicosa-1(20),2(21),3,5,16,18-hexaen-8-carboxylat

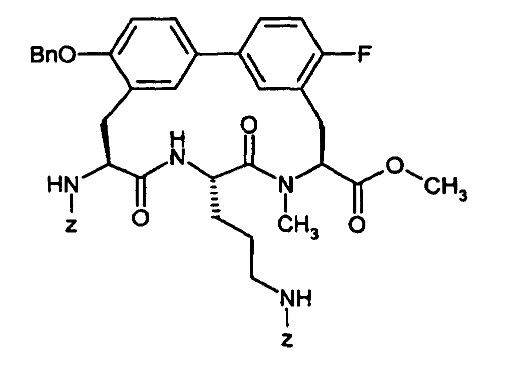

777 mg (Rohprodukt, ca. 0.92 mmol) Methyl-(2*S*')-2-[((2*R*)-2-amino-5-{[(benzyloxy)carbonyl]-amino}pentanoyl)(methyl)amino]-3-{4'-(benzyloxy)-3'-[(2*S*)-2-{[(benzyloxy)carbonyl]amino}-3-oxo-3-(pentafluorphenoxy)propyl]-4-fluorbiphenyl-3-yl}propanoat-Hydrochlorid (Beispiel 220) werden in 1.4 1 Dichlormethan gelöst und tropfenweise mit 14 ml Triethylamin versetzt. Es wird über Nacht bei RT gerührt. Zur Aufarbeitung wird das Gemisch schonend im Vakuum einrotiert und in Dichlormethan aufgenommen. Es wird mit Wasser versetzt, und durch Zugabe von 0,1N Natronlauge wird auf pH 10 gestellt. Die organische Phase wird mit Wasser gewaschen, über Natriumsulfat getrocknet und eingeengt. Das Rohprodukt wird durch präparative HPLC gereinigt.
Ausbeute: 450 mg (57 % d. Th., über vier Stufen ausgehend von Beispiel 190).
LC-MS (Methode 3): Rₜ = 3.15 min.
MS (EI): m/z = 845 (M+H)⁺.

### Beispiel 24B

### Methyl-(8S,11S,14S)-14-amino-11-[(2R)-3-amino-2-hydroxypropyl]-5-chlor-17-hydroxy-10,13-dioxo-9,12-diazatricyclo[14.3.1.1^{2,6}]henicosa-1(20),2(21),3,5,16,18-hexaen-8-carboxylat-Dihydrobromid

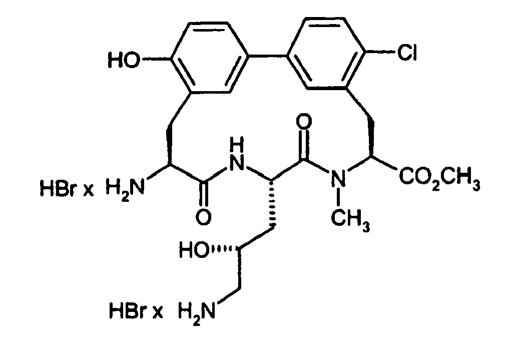

73 mg (0.08 mmol) der Verbindung aus Beispiel 23B werden 15 min bei RT mit 2 ml Eisessig / Bromwasserstoff (33%ig) gerührt. Anschließend dampft man alles vorsichtig (Badtemperatur max. 40°C) zur Trockne ein, nimmt mit 3 ml Toluol auf, dampft zur Trockne ein und wiederholt diese Prozedur noch einmal. Man trocknet im Hochvakuum bis zur Gewichtskonstanz.
Ausbeute: 69 mg eines Gemisches aus 58% Produkt und 19% des O-Acetyl-Produktes
LC-MS (Methode 1): Rₜ = 1.36 min. (Produkt) bzw. 1.44 min. (O-Acetyl-Produkt)
MS (EI): m/z = 518 (M-2HBr+H)⁺ bzw. 560 (MOAc-2HBr+H)⁺

### Beispiel 24M

### Methyl-(8S,11S,14S)-14-amino-11-(3-aminopropyl)-5-chlor-17-hydroxy-10,13-dioxo-9,12-diazatricyclo[14.3.1.12,6]henicosa-1(20),2(21),3,5,16,16-hexaen-8-carboxylat-Dihydrobromid

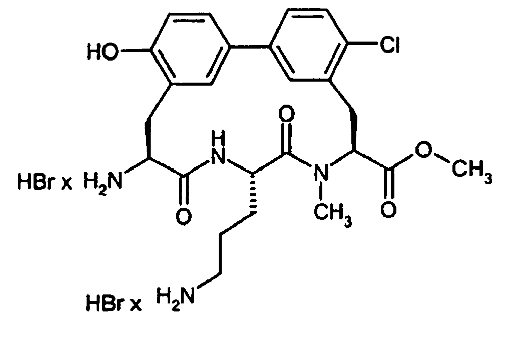

200 mg (0.23 mmol) der Verbindung aus Beispiel 23M werden 45 min bei RT mit 2 ml Eisessig / Bromwasserstoff (33%ig) gerührt. Anschließend dampft man alles vorsichtig (Badtemperatur max. 40°C) zur Trockne ein und trocknet den Rückstand im Hochvakuum bis zur Gewichtskonstanz.
Ausbeute: quant.
LC-MS (Methode 17): Rₜ = 2.76 min
MS (EI): m/z = 503 (M-2HBr+H)⁺

### Beispiel 25C

### Benzyl-(8S,11S,14S)-17-(benzyloxy)-14-{[(benzyloxy)carbonyl]amino}-11-((2R)-3-{[(benzyloxy)carbonyl]amino}-2-{[tert-butyl(dimethyl)silyl]oxy}propyl)-5-chlor-10,13-dioxo-9,12-diazatricyclo[14.3.1.1^{2,6}]henicosa-1(20),2(21),3,5,16,18-hexaen-8-carboxylat

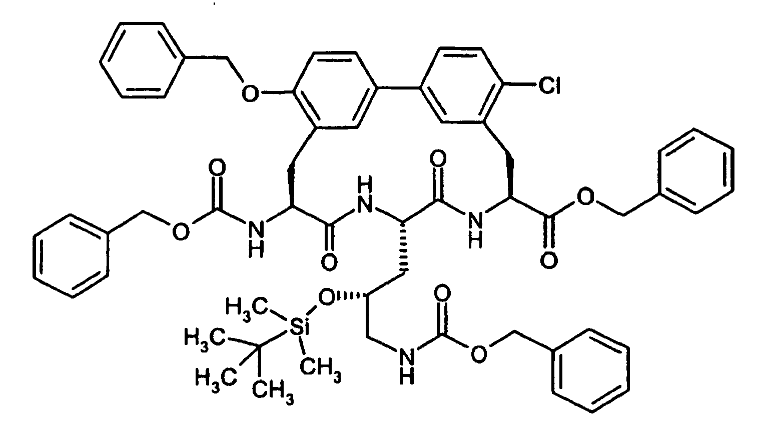

200 mg (0.21 mmol) der Verbindung aus Beispiel 23C werden in 27 ml absolutem DMF gelöst und bei 0°C mit 230 mg (0.85 mmol) Trifluormethansulfonsäure-*tert*-butyldimethylsilylester, 0.12 ml (0.85 mmol) Triethylamin und 30 mg (0.21 mmol) DMAP versetzt. Es wird 1 Tag bei RT gerührt. Im Vakuum wird vorsichtig auf ein Volumen von 1 ml eingeengt. Nach Zugabe von 20 ml Methylenchlorid wäscht man die organische Phase vorsichtig mit 10 ml gesättigter Natriumhydrogencarbonat-Lösung und 10 ml Wasser. Die organische Phase wird zur Trockne eingeengt und der Rückstand im Hochvakuum getrocknet.
Ausbeute: 220 mg (99% d. Th.)
LC-MS (Methode 1): Rₜ = 3.55 min.
MS (EI): m/z = 1053 (M+H)⁺

### Beispiel 26C1

### (8S,11S,14S)-17-(Benzyloxy)-14-{[(benzyloxy)carbonyl]amino}-11-((2R)-3-{[(benzyloxy)-carbonyl]amino}-2-{[tert-butyl(dimethyl)silyl]oxy}propyl)-5-chlor-10,13-dioxo-9,12-diazatricyclo[14.3.1.1^{2,6}]henicosa-1 (20),2(21),3,5,16,18-hexaen-8-carbonsäure

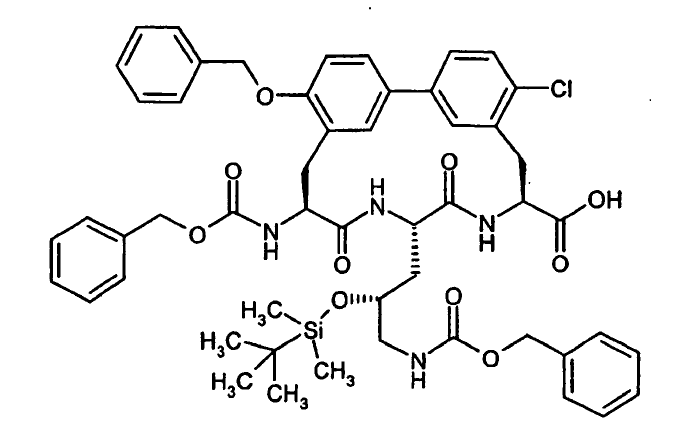

220 mg (0.21 mmol) der Verbindung aus Beispiel 25C werden in 11 ml THF gelöst und mit je 3 ml Wasser und Methanol versetzt. Nach Zugabe von 10.2 mg (0.43 mmol) Lithiumhydroxid wird 24 h bei RT gerührt. Anschließend wird die Reaktionslösung im Vakuum eingeengt und das Rohprodukt im Hochvakuum getrocknet.
Ausbeute: 200 mg (99% d. Th.)
LC-MS (Methode 2): Rₜ = 3.18 min.
MS (EI): m/z = 963 (M+H)⁺

### Beispiel 26C2

### (8S,11S,14S)-17-(Benzyloxy)-14-{[(benzyloxy)carbonyl]amino}-11-{(2R)-3-{[(benzyloxy)-carbonyl]amino}-2-hydroxypropyl}-5-chlor-10,13-dioxo-9,12-diazatricyclo[14.3.1.1^{2,6}]henicosa-1 (20),2(21),3,5,16,18-hexaen-8-carbonsäure

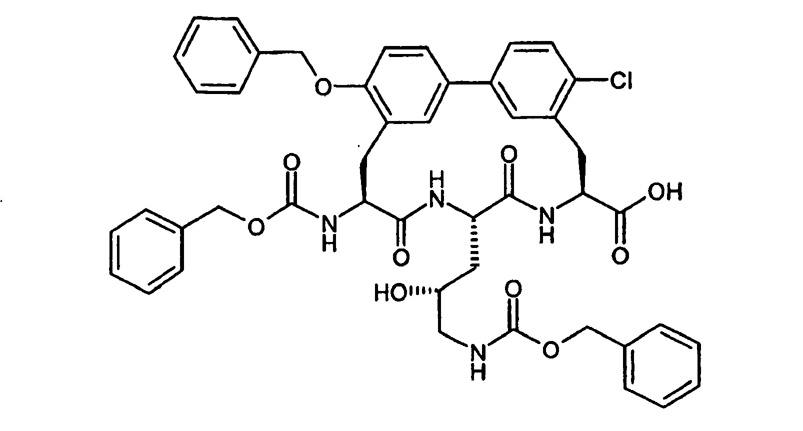

Die Herstellung folgt analog zu Beispiel 26C1 aus 169 mg (0.18 mmol) der Verbindung aus Beispiel 23C und 7.7 mg (0.32 mmol) Lithiumhydroxid in 12 ml THF:Methanol:Wasser 4:1:1.
Ausbeute: 135 mg (99% d. Th.)
LC-MS (Methode 1): Rₜ = 2.85 min.
MS (EI): m/z = 849 (M+H)⁺

### Beispiel 26E

### (8S,11S,14S)-17-(Benzyloxy)-14-{[(benzyloxy)carbonyl]amino}-11-(3-{[(benzyloxy)carbonyl]-amino}propyl)-5-methyl-10,13-dioxo-9,12-diazatricyclo[14.3.1.1^{2,6}]henicosa1(20),2(21),3,5,16,18-hexaen-8-carbonsäure

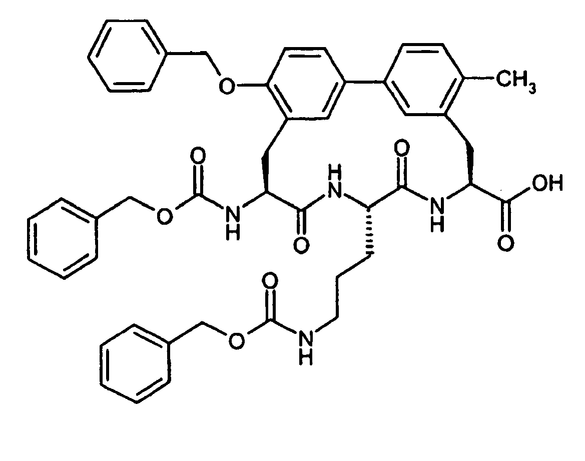

0.21 g (0.23 mmol) der Verbindung aus Beispiel 23E werden bei RT in einer Mischung aus THF:Methanol:Wasser (28 ml, 4:1:2) suspendiert und mit 11.0 mg (0.47 mmol) Lithiumhydroxid versetzt. Nach 12 h bei Raumtemperatur wird im Vakuum eingeengt und der Rückstand in 100 ml Wasser suspendiert. Man stellt mit 1N Salzsäure auf pH = 3 ein, wodurch das Produkt in kristalliner Form ausfällt. Das Produkt wird abfiltriert und im Vakuum getrocknet.
Ausbeute: 179 mg (94% d. Th.)
LC-MS (Methode 2): Rₜ = 2.70 min.
MS (EI): m/z = 813 [M+H]⁺

### Beispiel 26F1

### (8S,11S,14S)-17-(Benzyloxy)-14-{[(benzyloxy)carbonyl]amino}-11-(3-{[(benzyloxy)carbonyl]-amino}propyl)-10,13-dioxo-9,12-diazatricyclo[14.3.1.1^{2,6}]henicosa-1(20),2(21),3,5,16,18-hexaen-8-carbonsäure

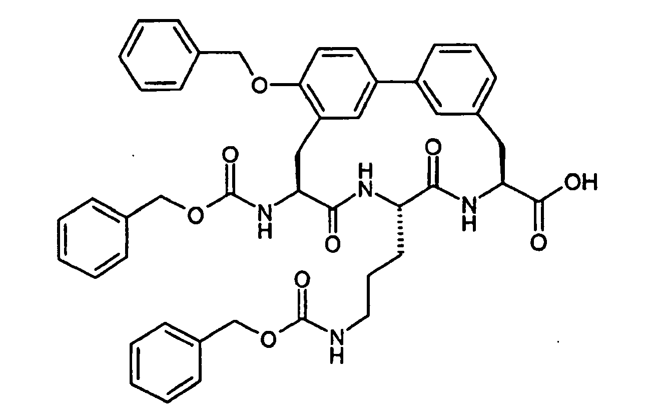

Die Herstellung folgt analog zu Beispiel 26E aus 250 mg (0.28 mmol) der Verbindung aus Beispiel 23F und 13.5 mg (0.56 mmol) Lithiumhydroxid in THF:Methanol:Wasser.
Ausbeute: 194 mg (86% d. Th.)
LC-MS (Methode 5): Rₜ = 2.61 min.
MS (EI): m/z = 799 (M+H)⁺

### Beispiel 26F2

### O-Benzyl-N-{[(8S,11S,14S)-17-(benzyloxy)-14-{[(benzyloxy)carbonyl]amino}-11-(3-{[(benzyloxy)carbonyl]amino} propyl)-10,13-dioxo-9,12-diazatricyclo[ 14.3.1.1^{2,6}]henicosa-1(20),2(21),3,5,16,18-hexaen-8-yl]carbonyl}-L-tyrosin

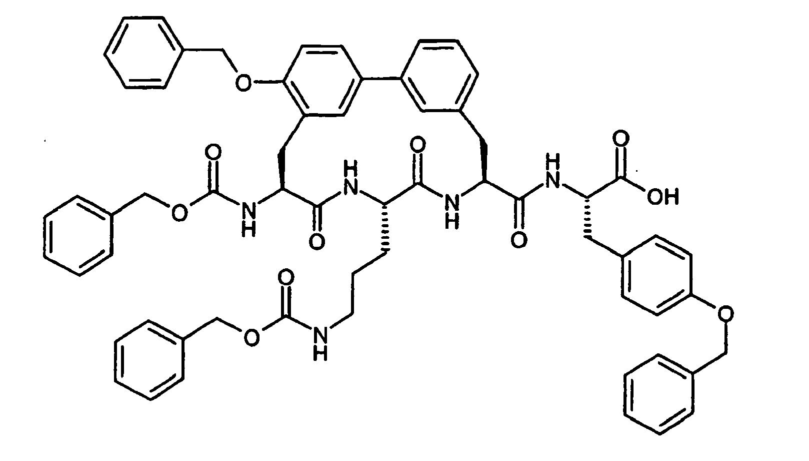

Die Herstellung folgt analog zu Beispiel 26F1 aus 58 mg (0.05 mmol) der Verbindung aus Beispiel 27F3 und 2.4 mg (0.1 mmol) Lithiumhydroxid in 12 ml THF:Methanol:Wasser 4:1: 1.
Ausbeute: 53 mg (99% d. Th.)
LC-MS (Methode 5): Rₜ = 2.86 min.
MS (EI): m/z = 1052 (M+H)⁺

### Beispiel 26O

### (8S,11S, 14S)-17-(Benzyloxy)-14-{[(benzyloxy)carbonyl]amino}-11-(3-{[(benzyloxy)carbonyl]-amino}propyl)-5-fluor-9-methyl-10,13-dioxo-9,12-diazabicyclo[14.3.1.1^{2,6}]henicosa-1 (20),2(21),3,5,16,18- hexaen-8-carbonsäure

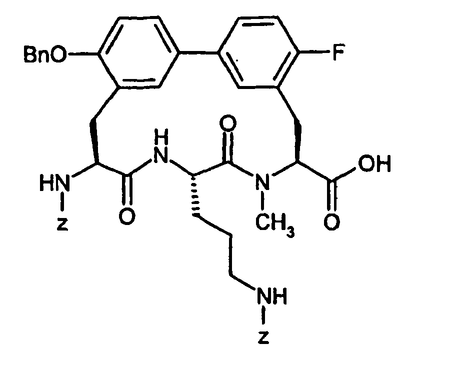

Zu einer Suspension aus 280 mg (0.331 mmol) Methyl-(8*S*,11*S*,14*S*-17-benzyloxy)-14-{[(benzyloxy)carbonyl]amino}-11-(3-{[(benzyloxy)carbonyl]amino}propyl)-5-fluor-9-methyl-10,13-dioxo-9,12-diazatricyclo[14.3.1.1^{2,6}]henicosa-1(20),2(21),3,5,16,18-hexaen-8-carboxylat (Beispiel 230) in 125 ml Dioxan/Wasser (4:1) wird 6.6 ml (0.66 mmol) einer 0.1N wässrigen Lithiumhydroxidlösung bei 0 °C hinzugegeben. Es wird 12 h bei RT gerührt. Durch Zugabe von 0.1N Salzsäure wird auf pH 2 gestellt. Die Reaktionsmischung wird im Vakuum eingeengt und im Hochvakuum getrocknet. Das Rohprodukt wird ohne weitere Reinigung umgesetzt.
LC-MS (Methode 2): Rₜ = 2.75 min.
MS (EI): m/z = 831 (M+H)⁺.

### Beispiel 27E

Benzyl-{3-[(8*S*,11*S*,14*S*)-17-(benzyloxy)-14-{[(benzyloxy)carbonyl]amino}-8-{[(2-{[(benzyloxy)-carbonyl]amino} ethyl)amino]carbonyl}-5-methyl-10,13-dioxo-9,12-diazatricyclo[14.3.1.1^{2,6}]-henicosa-1(20),2(21),3,5,16,18-hexaen-11-yl]propyl}carbamat 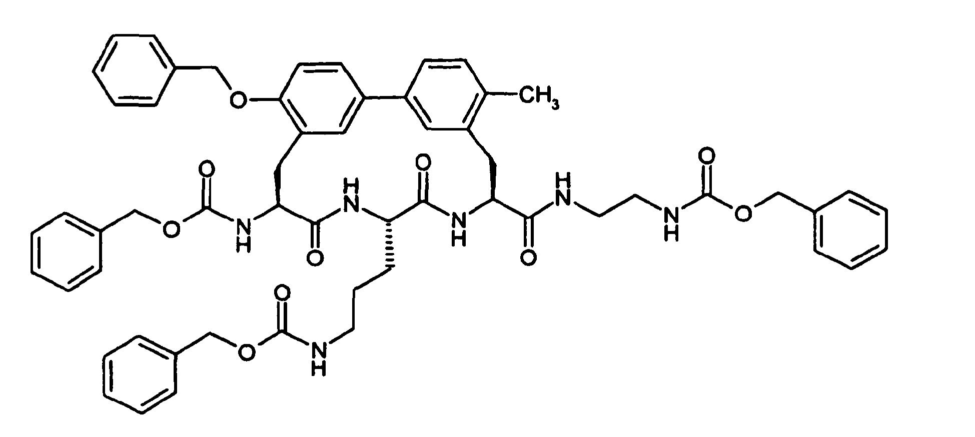 25 mg (0.030 mmol) der Verbindung aus Beispiel 26E werden bei RT unter Argon in DMF (2.0 ml) suspendiert und mit 18.0 mg (0.090 mmol) Benzyl-(2-aminoethyl)carbamat, 8 mg (0.06 mmol) *N,N*-Diisopropylethylamin und 23 mg (0.060 mmol) HATU versetzt. Nach 12 h bei Raumtemperatur wird erneut mit 18.0 mg (0.090 mmol) Benzyl-(2-aminoethyl)carbamat, 8 mg (0.06 mmol) *N,N-*Diisopropylethylamin und 23 mg (0.060 mmol) HATU versetzt und 4 h bei RT gerührt. Man versetzt mit 50 ml Wasser, wodurch das Produkt in kristalliner Form ausfällt. Das Produkt wird abfiltriert, mit Wasser gewaschen und in 50 ml Acetonitril:Methanol ausgeführt. Man trocknet im Vakuum bis zur Gewichtskonstanz.
Ausbeute: 19 mg (62% d. Th.)
LC-MS (Methode 2): Rₜ = 2.90 min.
MS (EI): m/z = 989 [M+H]⁺

Analog zu obiger Vorschrift werden die in der folgenden Tabelle aufgeführten Beispiele 27C1 bis 27C10 und 27F1 bis 27F4 aus den entsprechenden Edukten hergestellt:

| Beispiel-Nr. | Struktur | Hergestellt analog Beispiel-Nr. | Analytische Daten |
|---|---|---|---|
| **27C1** | 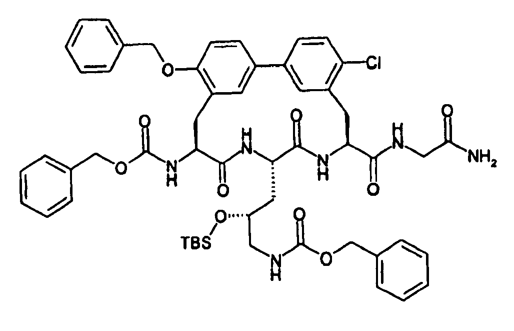 | 27E aus Beispiel 26C1 und Glycinamid | LC-MS (Methode 16): Rₜ = 2.21 min. MS (EI): m/z = 1019 (M+H)⁺ |
| **27C2** | 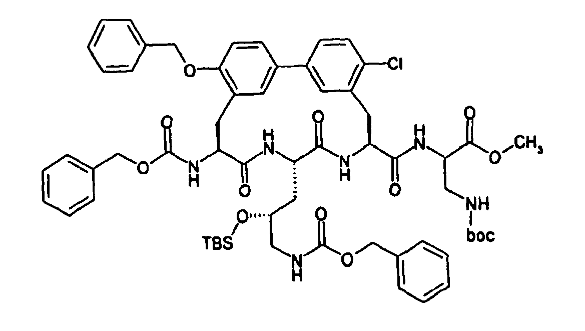 | 27E aus Beispiel 26C1 und Methyl-3-[(*tert*-butoxycarbonyl)-amino]-*L-*alaninat | LC-MS (Methode 2): Rₜ = 3.28 min. MS (EI): m/z = 1163 (M+H)⁺ |
| **27C3** | 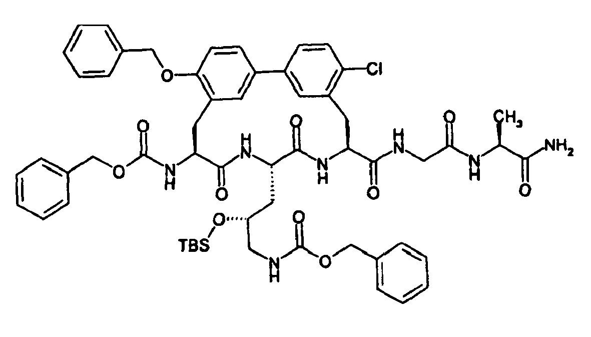 | 27E aus Beispiel 26C 1 und Glycyl-*L-*alaninamid | LC-MS (Methode 2): Rₜ = 3.01 min. MS (EI): m/z = 1090 (M+H)⁺ |
| **27C4** | 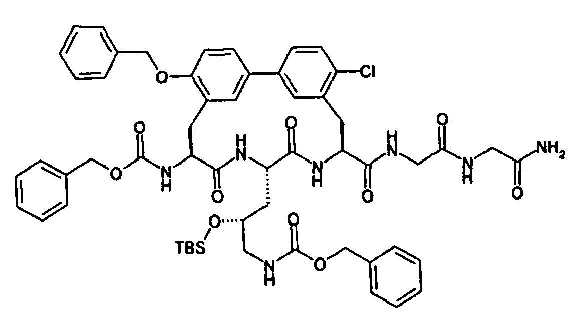 | 27E aus Beispiel 26C 1 und Glycylglycinamid | LC-MS (Methode 2): Rₜ = 2.97 min. MS (EI): m/z = 1076 (M+H)⁺ |
| **27C5** | 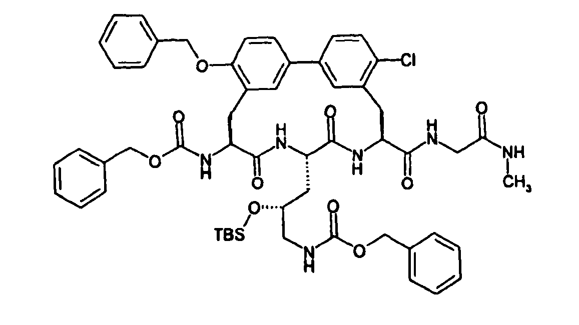 | 27E aus Beispiel 26C1 und *N¹*-Methylglycinamide | LC-MS (Methode 2): Rₜ = 3.12 min. MS (EI): m/z = 1033 (M+H)⁺ |
| **27C6** | 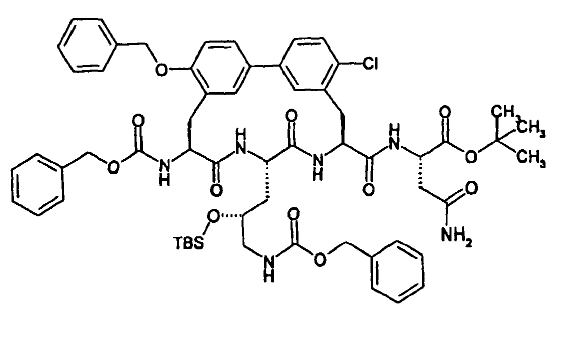 | 27E aus Beispiel 26C 1 und *tert*-Butyl-*L*-asparaginat | LC-MS (Methode 3): Rₜ = 3.35 min. MS (EI): m/z =1133 (M+H)⁺ |
| **27C7** | 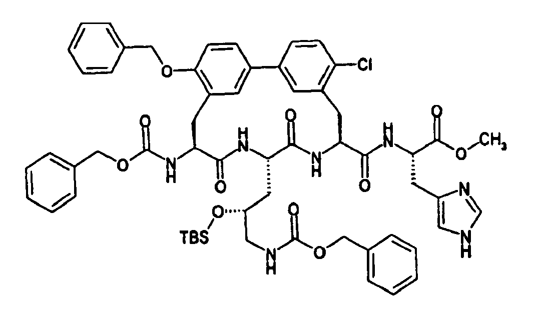 | 27E aus Beispiel 26C 1 und Methyl-*L-*histidinat | LC-MS (Methode 3): Rₜ = 2.94 min. MS (EI): m/z = 1114 (M+H)⁺ |
| **27C8** | 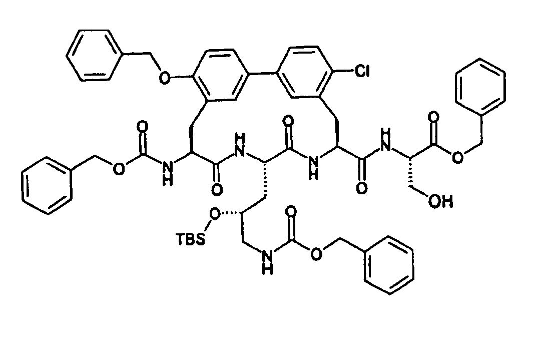 | 27E aus Beispiel 26C1 und Benzyl-*L-*serinat | LC-MS (Methode 2): Rₜ = 3.28 min. MS (EI): m/z = 1140 (M+H)⁺ |
| **27C9** | 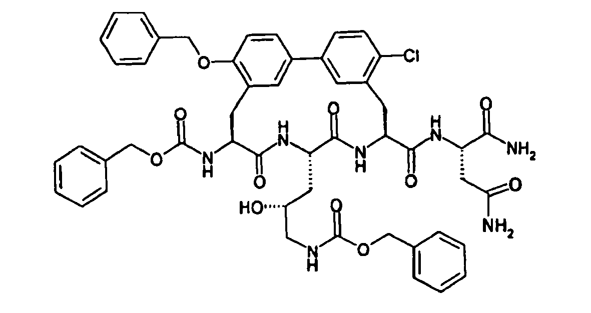 | 27E aus Beispiel 26C2 und L-Aspartamid | LC-MS (Methode 1): Rₜ = 2.61 min. MS (EI): m/z = 962 (M+H)⁺ |
| **27C1**0 | 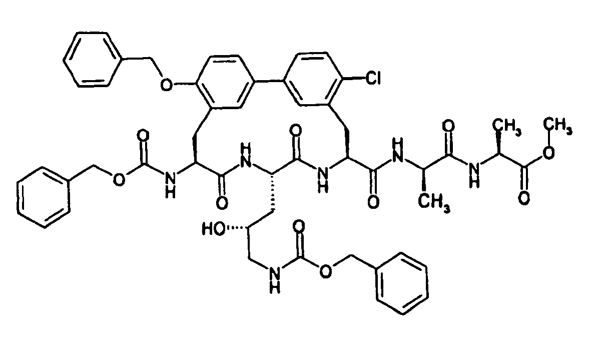 | 27E aus Beispiel 26C2 und Methyl-D-alanyl-d-alanylat | LC-MS (Methode 1): Rₜ = 2.89 min. MS (EI): m/z = 1005 (M+H)⁺ |
| **27C11** | 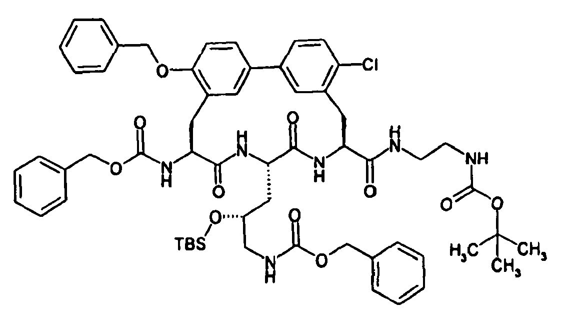 | 27E aus Beispiel 26C1 und tert: Butyl-(2-aminoethyl)-carbamat | LC-MS (Methode 1): Rₜ = 3.43 min. MS (EI): m/z = 1105 (M+H)⁺ |
| **27F1** | 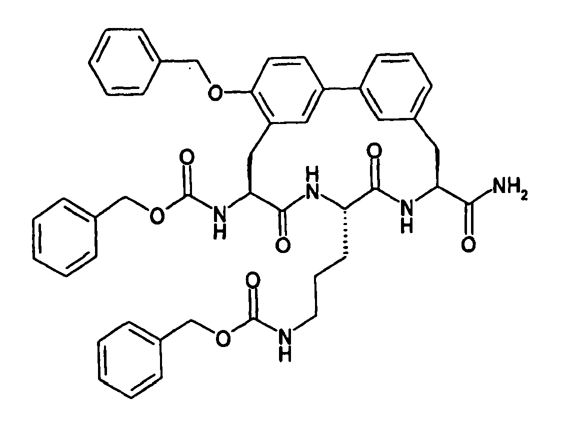 | 27E aus Beispiel 26F und Ammoniak | LC-MS (Methode 5): Rₜ = 2.55 min. MS (EI): m/z = 798 (M+H)⁺ |
| **27F2** | 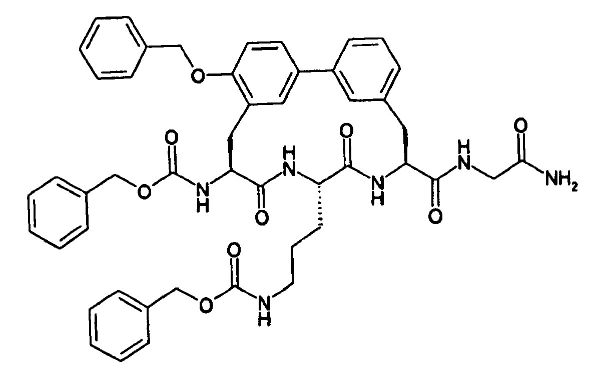 | 27E aus Beispiel 26F und Glycinamid | LC-MS (Methode 5): Rₜ = 2.47 min. MS (EI): m/z = 855 (M+H)⁺ |
| **27F3** | 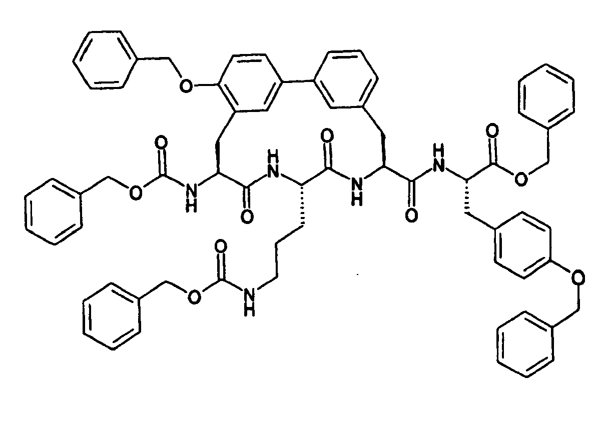 | 27E aus Beispiel 26F1 und Benzyl-O-benzyl-*L-*tyrosinat | LC-MS (Methode 5): Rₜ = 3.14 min. MS (EI): m/z = 1142 (M+H)⁺ |
| **27F4** | 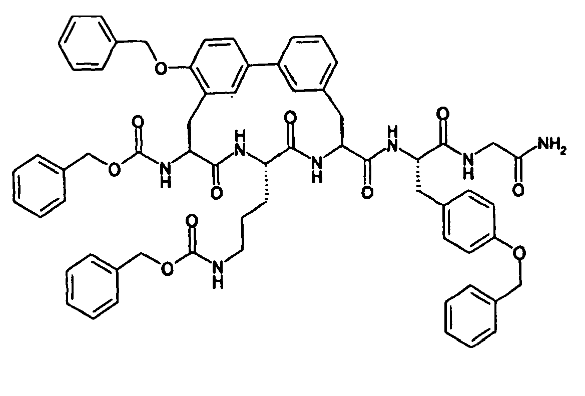 | 27E aus Beispiel 26F2 und Glycinamid | LC-MS (Methode 3): Rₜ = 3.00 min. MS (EI): m/z = 1109 (M+H)⁺ |

### Beispiel 28A

### (8S,11S,14S)-14-[(tert-Butoxycarbonyl)amino]-11-{3-[(tert-butoxycarbonyl)amino]propyl}-5-fluor-17-hydroxy-10,13-dioxo-9,12-diazatricyclo[14.3.1.1^{2,6}]henicosa-1(20),2(21),3,5,16,18-hexaen-8-carbonsäure

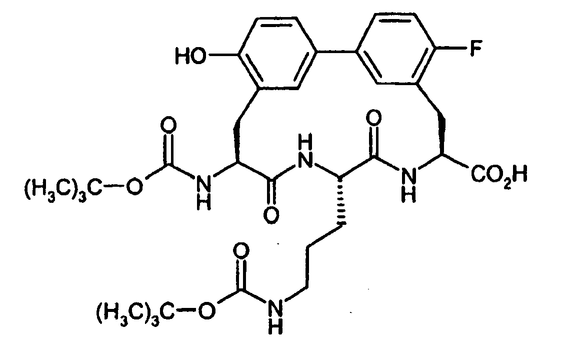

33 mg (0.06 mmol) der Verbindung aus Beispiel 1 werden in 0.28 ml 0.1N Natronlauge gelöst, unter Rühren mit 40.7 mg (0.19 mmol) Di-*tert*-butyldicarbonat versetzt und über Nacht gerührt. Mit 0.1N Salzsäure stellt man pH 4 ein und schüttelt zweimal mit Essigsäureethylester aus. Die organischen Phasen werden vereinigt, mit Natriumsulfat getrocknet und im Vakuum zur Trockne eingedampft.
Ausbeute: 33 mg (77% d. Th.)
LC-MS (Methode 3): Rₜ = 2.24 min.
MS (EI): m/z = 658 (M+H)⁺

Es ist ein Produktgemisch aus Zielverbindung und den entsprechenden Phenylcarbonaten (m/z = 757 bzw m/z = 857) entstanden, welches ohne weitere Auftrennung für Folgeumsetzungen verwendet wird.

### Beispiel 28J

### (8S,11S,14S)-14-[(tert-Butoxycarbonyl)amino]-11-{(2R)-3-[(tert-butoxycarbonyl)amino]-2-hydroxypropyl}-4,17-dihydroxy-10,13-dioxo-9,12-diazatricyclo[14.3.1.1^{2,6}]henicosa-1(20),2(21),3,5,16,18-hexaen-8-carbonsäure

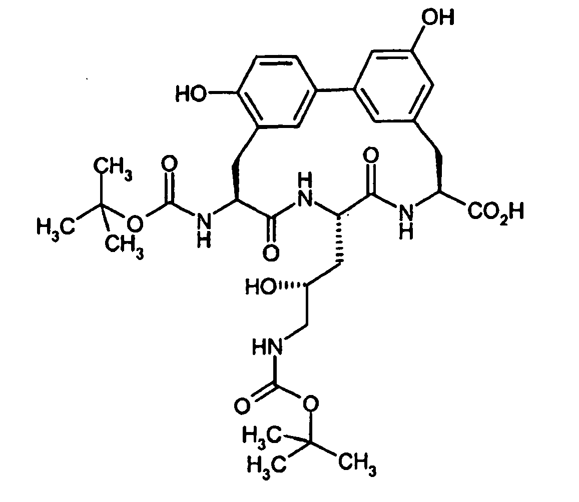

17 mg (0.03 mmol) der Verbindung aus Beispiel 34 werden in 1.0 ml Methanol:Wasser (9:1) vorgelegt, mit 1.0 ml einer gesättigter wässriger Natriumhydrogencarbonat-Lösung und anschließend mit 27.5 mg (0.12 mmol) Di-*tert*-butylcarbonat in 0.15 ml Methanol:Wasser (9:1) versetzt und 12 h bei RT gerührt. Die Reaktionslösung wird im Vakuum eingeengt, mit Wasser versetzt und mit Essigsäureethylester extrahiert. Nach Phasentrennung wird die wässrige Phase durch Zugabe von 0.1N Salzsäure auf pH = 4 gestellt und mehrfach mit Essigsäureethylester extrahiert. Die vereinten organischen Phasen werden über Natriumsulfat getrocknet und im Vakuum zur Trockne eingedampft.
Ausbeute: 21 mg (quantitativ),
LC-MS (Methode 15): Rₜ = 2.44 min.
MS (EI): m/z = 673 (M+H)⁺

Es ist ein Produktgemisch aus Zielverbindung und den entsprechenden Phenylcarbonaten (m/z = 772 bzw m/2 = 872) entstanden, welches ohne weitere Auftrennung für Folgeumsetzungen verwendet wird.

Analog zu den angegebenen Vorschriften werden die in der folgenden Tabelle aufgeführten Beispiele 28B, 28D und 28K bis 28N aus den entsprechenden Edukten hergestellt:

| Beispiel-Nr. | Struktur | Hergestellt analog Beispiel-Nr. | Analytische Daten |
|---|---|---|---|
| **28B** | 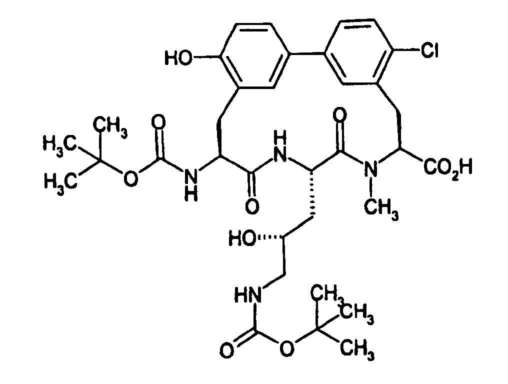 | 28A aus Beispiel 4 | LC-MS (Methode 2): Rₜ = 2.08 min. MS (EI): m/z = 704 (M+H)⁺ |
| **28D** | 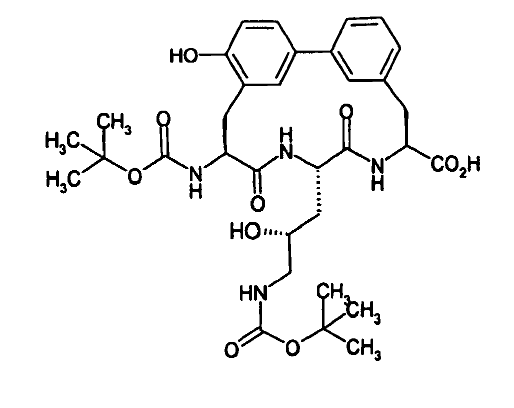 | 28J aus Beispiel 25 | |
| **28F** | 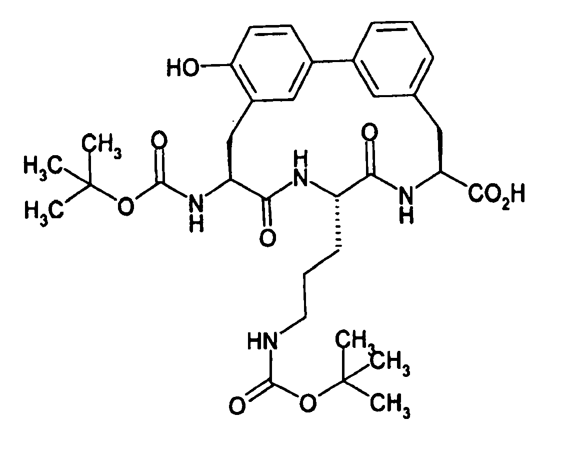 | 28A aus Beispiel 35 | LC-MS (Methode 1): Rₜ = 2.19 min. MS (EI): m/z = 640 (M+H)⁺ |
| **28K** | 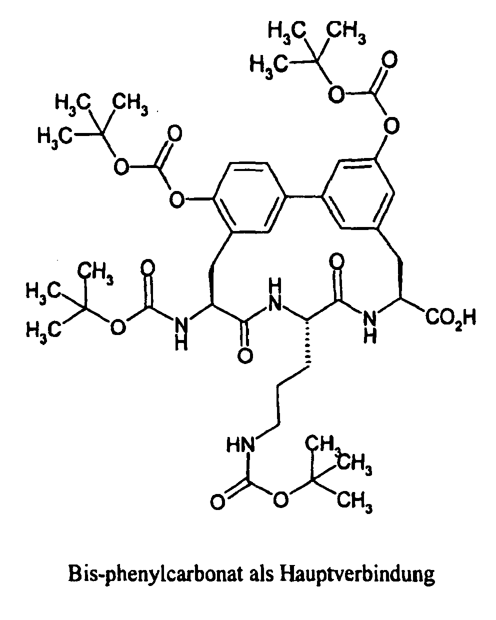 | 28J aus Beispiel 2 | LC-MS (Methode 15): Rₜ = 3.33 min. MS (EI): m/z = 857 (M+H)⁺ |
| **28L** | 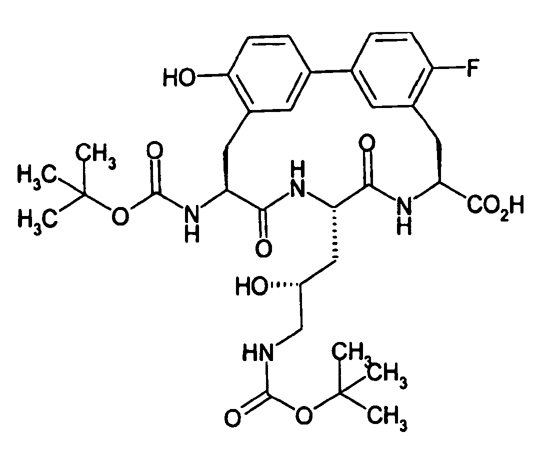 | 28A aus Beispiel 3 | LC-MS (Methode 17): Rₜ = 3.20 min. MS (EI): m/z = 675 (M+H)⁺ |
| **28M** | 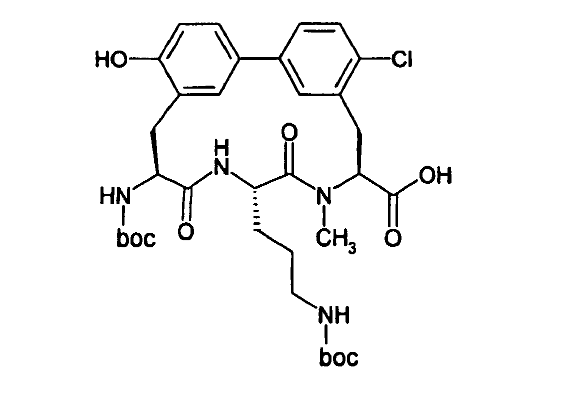 | 280 aus Beispiel 24M | LC-MS (Methode 1): Rₜ = 2.39 min. MS (ES): m/z = 689 (M+H)⁺ |
| **28N** | 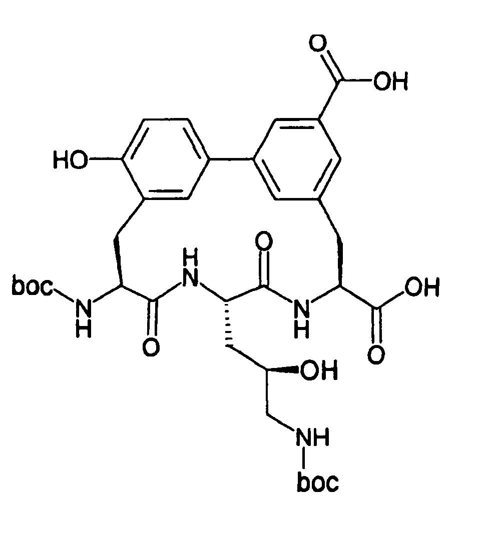 | 280 aus Beispiel 31N | LC-MS (Methode 3): Rₜ = 1.89 min. MS (ES): m/z = 701 (M+H)⁺ |

### Beispiel 28O

### (8S,11S,14S)-14-[(tert-Butoxycarbonyl)amino]-11-{3-[(tert-butoxycarbonyl)amino]propyl -5-fluor-17-hydroxy-9-methyl-10,13-dioxo-9,12-diazatricyclo[ 14.3.1.1^{2,6}]henicosa-1(20),2(21),3,5,16,18- hexaen-8-carbonsäure

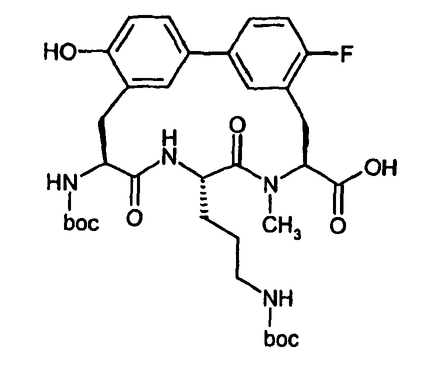

180 mg (0.330 mmol) (8*S*,11*S*,14*S*)-14-Amino-11-(3-aminopropyl)-5-fluor-17-hydroxy-9-methyl-10,13-dioxo-9,12-diazatricyclo[14.3.1.1^{2,6}]henicosa-1 (20),2(21),3,5,16,18-hexaen-8-carbonsäure-Dihydrochlorid (Beispiel 40) werden in 1.6 ml (1.6 mmol) 1N Natronlauge und 2 ml Wasser gelöst und bei Raumtemperatur unter Rühren mit 216 mg (0.99 mmol) Di-*tert*-butyldicarbonat, gelöst in 1 ml Methanol, versetzt. Die Mischung wird über Nacht bei RT gerührt. Durch Zutropfen von 0.1N Salzsäure wird auf pH = 3 gestellt. Man extrahiert die wässrige Phase mit Essigsäureethylester, trocknet die organische Phase über Magnesiumsulfat und dampft im Vakuum bis zur Gewichtskonstanz ein. Das Produkt wird ohne weitere Reinigung umgesetzt.
LC-MS (Methode 2): Rₜ = 2.11 min.
MS (EI): m/z = 673 (M+H)⁺.

### Beispiel 28P1

### (8S,11S,14S)-5,14-Bis[(tert-butoxycarbonyl)amino]-11-{3-[(tert-butoxycarbonyl)amino]propyl}-17-hydroxy-10,13-dioxo-9,12-diazatricyclo[14.3.1.1^{2,6}]henicosa-1 (20),2(21),3,5,16,18-hexaen-8-carbonsäure

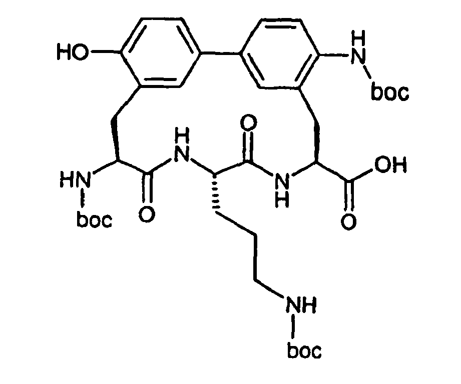

170 mg (0.26 mmol) der Verbindung aus Beispiel 31P werden in 1.29 ml (1.29 mmol) 1N Natronlauge und 0.85 ml Wasser gelöst und bei Raumtemperatur unter Rühren mit170 mg (0.78 mmol) Di-*tert-*butyldicarbonat, gelöst in 0.3 ml Methanol, versetzt. Die Mischung wird 1 h bei RT gerührt, danach nochmals mit 170 mg (0.78 mmol) Di-*tert*-butyldicarbonat und 1.29 ml (1.29 mmol) 1N Natronlauge versetzt und weitere 2 h bei RT gerührt. Durch Zutropfen von 0.1N Salzsäure wird auf pH = 3 gestellt, wobei ein Niederschlag entsteht. Man extrahiert zunächst mit Essigsäureethylester, und trennt die organische Phase ab. Man trocknet die organische Phase über Magnesiumsulfat und dampft im Vakuum bis zur Gewichtskonstanz ein. Das Produkt wird ohne weitere Reinigung umgesetzt. Die verbleibende wässrige Phase wird filtriert und der Rückstand im Vakuum bis zur Gewichtskonstanz getrocknet (siehe Beispiel 28P2).
Ausbeute: 140 mg (70% d. Th.)
LC-MS (Methode 2): Rₜ = 2.32 min.
MS (ES): m/z = 756 (M+H)⁺

### Beispiel 28P2

### (8S,11S,14S)-5-Amino-14-[(tert-butoxycarbonyl)amino]-11-{3-[(tert-butoxycarbonyl)amino]-propyl}-17-hydroxy-10,13-dioxo-9,12-diazatricyclo[14.3.1.1^{2,6}]henicosa-1(20),2(21),3,5,16,18-hexaen-8-carbonsäure-Hydrochlorid

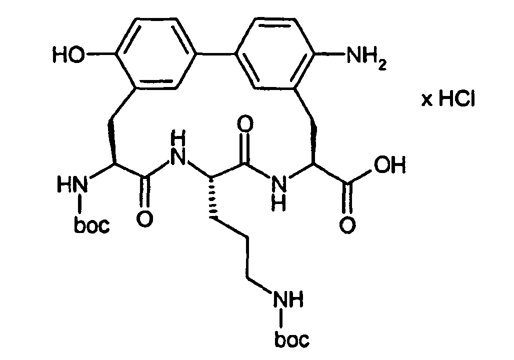

Beispiel 28P2 entsteht als Nebenprodukt bei der Darstellung von Beispiel 28P1.
Ausbeute: 20 mg (12% d. Th.)
LC-MS (Methode 2): Rₜ = 1.67 min.
MS (ES): m/z = 656 (M-HCl+H)⁺

### Beispiel 29A1

### tert-Butyl-{3-[(8S,11S,14S)-14-[(tert-butoxycarbonyl)amino]-8-[({2-[(tert-butoxycarbonyl)-amino]ethyl}amino)carbonyl]-5-fluor-17-hydroxy-10,13-dioxo-9,12-diazatricyclo[14.3.1.^{2,6}]-henicosa-1(20),2(21),3,5,16,18-hexaen-11-yl]propyl}carbamat

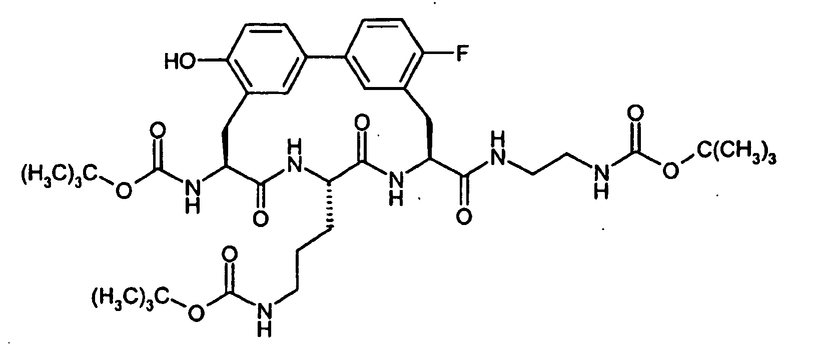

15 mg (0.02 mmol) der Verbindung aus Beispiel 28A und 4.38 mg (0.03 mmol) *tert*-Butyl-(2-aminoethyl)-carbarnat werden in 1 ml abs. DMF gelöst, auf 0°C abgekühlt und mit 10.4 mg (0.03 mmol) HATU und 3.4 mg (0.03 mmol) Hünig-Base versetzt. Man rührt 30 min bei 0°C, lässt die Temperatur dann auf RT ansteigen, versetzt mit weiteren 6.8 mg (0.05 mmol) Hünig-Base und lässt über Nacht nachreagieren. Es wird alles im Vakuum zur Trockne eindampft und der Rückstand mittels präparativer HPLC (Acetonitril / Wasser) getrennt.
Ausbeute: 6.5 mg (36% d.Th.)
LC-MS (Methode 3): Rₜ = 2.48 min.
MS (EI): m/z = 800 (M+H)⁺

Analog den angegebenen Vorschriften werden die in der folgenden Tabelle aufgeführten Beispiele 29A2, 29B1, 29B2, 29D, 29F1, 29F2, 29J, 29K, 29L, 29F4, 29M1, 29M2, 29N, 2901, 29P1 und 29P2 aus den entsprechenden Edukten hergestellt:

| Beispiel-Nr. | Struktur | Hergestellt analog Beispiel-Nr. | Analytische Daten |
|---|---|---|---|
| **29A2** | 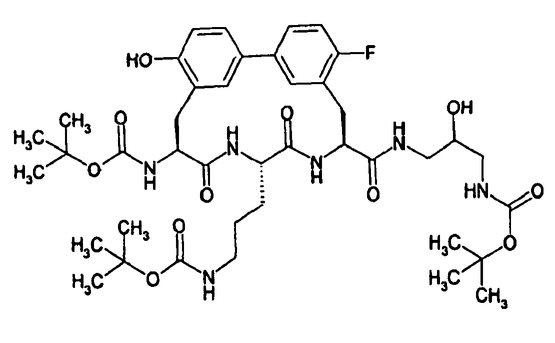 | 29A1 aus Beispiel 28A und *tert*-Butyl-(3- amino-2-hydroxypropyl)-carbamat | LC-MS (Methode 3): Rₜ = 2.39 min. MS (EI): m/z = 830 (M+H)⁺ |
| **29B1** | 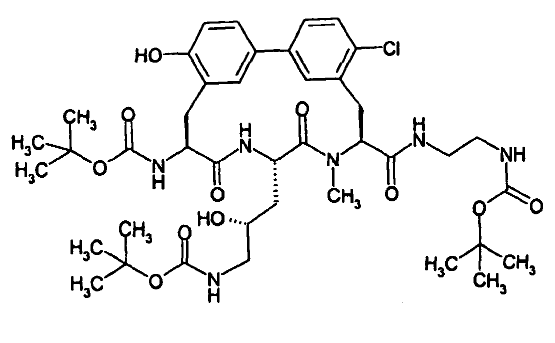 | 29A1 aus Beispiel 28B und *tert-*Butyl-(2-aminoethyl)-carbamat | LC-MS (Methode 2): Rₜ = 2.41 min. MS (EI): m/z = 846 ([M+H]⁺ |
| **29B2** | 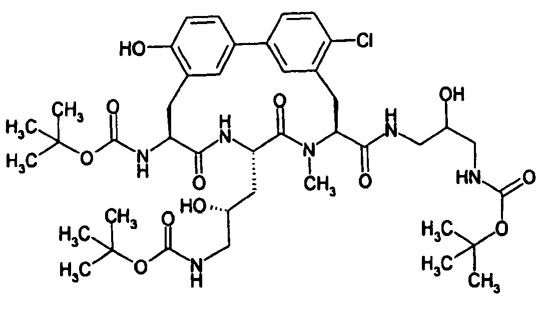 | 29A1 aus Beispiel 28B und *tert-*Butyl-(3-amino-2-hydroxypropyl carbamat | LC-MS (Methode 2): Rₜ = 2.30 min. MS (EI): m/z = 876 [M+H]⁺ |
| **29D** | 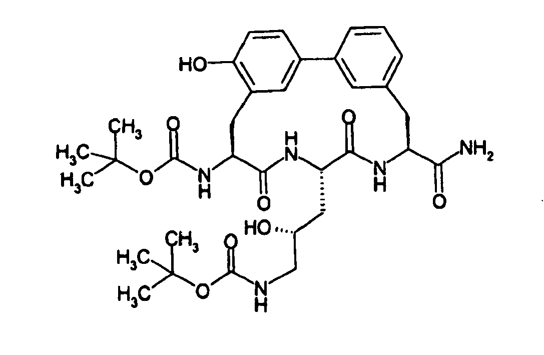 | 29A1 aus Beispiel 28D und Ammoniak | LC-MS (Methode 8): Rₜ = 2.65 min. MS (EI): m/z = 656 (M+H)⁺ |
| **29F1** | 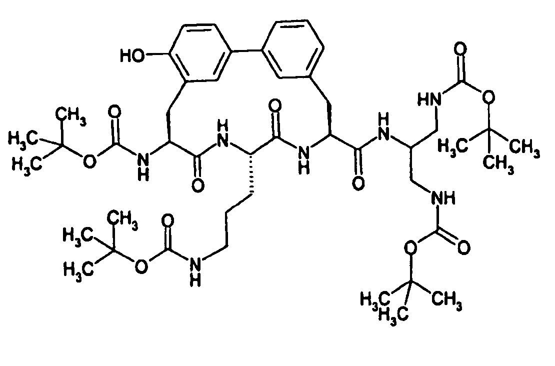 | 29A1 aus Beispiel 28F und di-*tert-*Butyl-(2-aminopropan-1,3-diyl)-biscarbamat Darstellung siehe Tetrahedron, 1995, 51, 2875-94. | LC-MS (Methode 1): Rₜ = 2.63 min. MS (EI): m/z = 912 (M+H)⁺ |
| **29F2** | 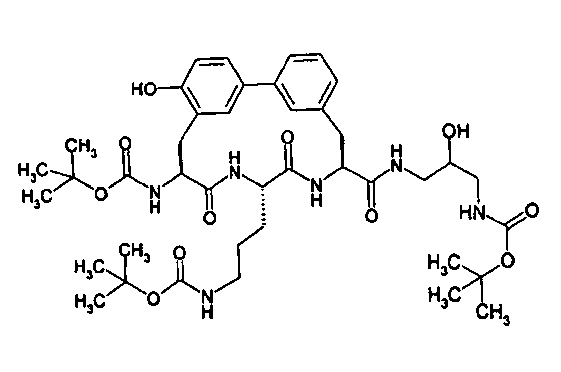 | 29A1 aus Beispiel 28F und *tert-*Butyl-(3-amino-2-hydroxypropyl carbamat | LC-MS (Methode 2): Rₜ = 2.17 min. MS (EI): m/z = 813 (M+H)⁺ |
| **29J** | 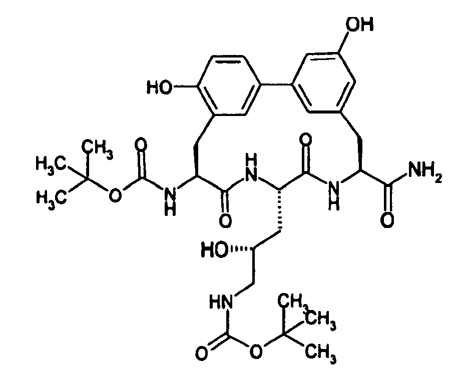 | 29A1 aus Beispiel 28J und Ammoniak | LC-MS (Methode 8): Rₜ = 2.40 min. MS (EI): m/z = 672 (M+H)⁺ |
| **29K** | 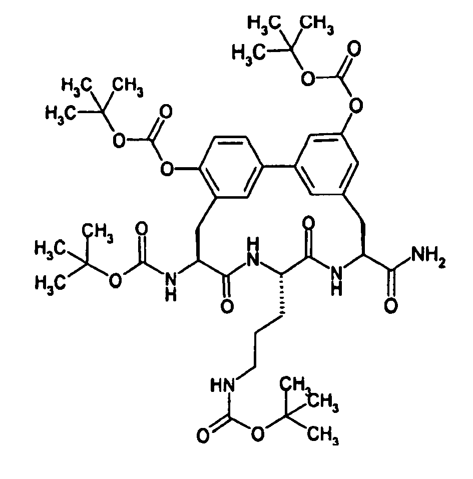 | 29A1 aus Beispiel 28K und Ammoniak | LC-MS (Methode 8): Rₜ = 3.58 min. MS (EI): m/z = 856 (M+H)⁺ |
| **29L** | 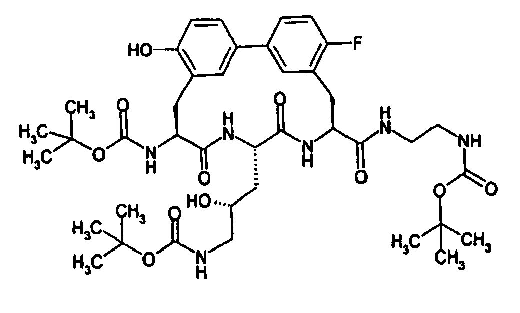 | 29A1 aus Beispiel 28L und *tert-*Butyl-(2-aminoethyl)-carbamat | LC-MS (Methode 2): Rₜ = 2.23 min. MS (EI): m/z = 817 (M+H)⁺ |
| **29F4** | 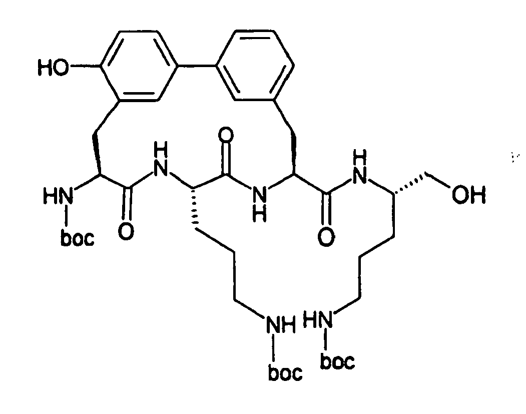 | 29F3 aus Beispiel 28F und Beispiel Z5 | LC-MS (Methode 3): Rₜ = 2.37 min. MS (EI): m/z = 841 (M+H)⁺ |
| **29M1** | 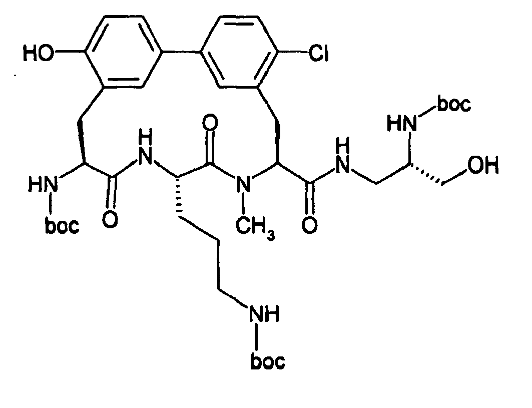 | 29F3 aus Beispiel 28M und Beispiel Z11 | LC-MS (Methode 2): Rₜ = 2.33 min. MS (EI): m/z = 861 (M+H)⁺ |
| **29M2** | 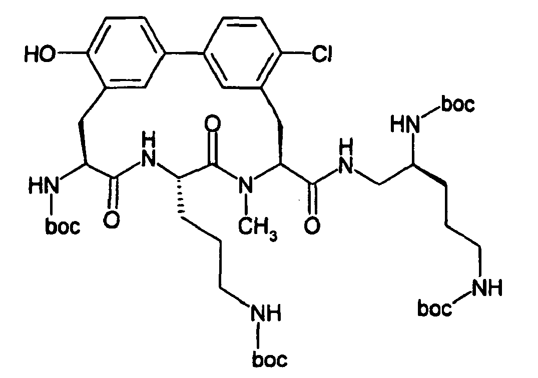 | 29F3 aus Beispiel 28M und Beispiel Z9 | LC-MS (Methode 3): Rₜ = 2.91 min. MS (EI): m/z = 988 (M+H)⁺ |
| **29N** | 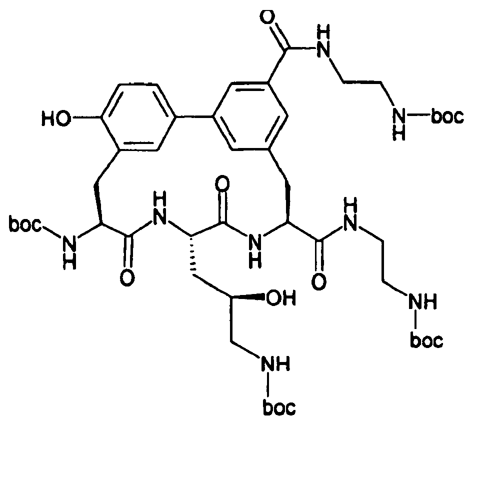 | 29F3 aus Beispiel 28N und 2 eq *tert-*Butyl-(2-aminoethyl)-carbamat | LC-MS (Methode 3): Rₜ = 2.35 min. MS (ES): m/z = 985 (M+H)⁺ |
| **2901** | 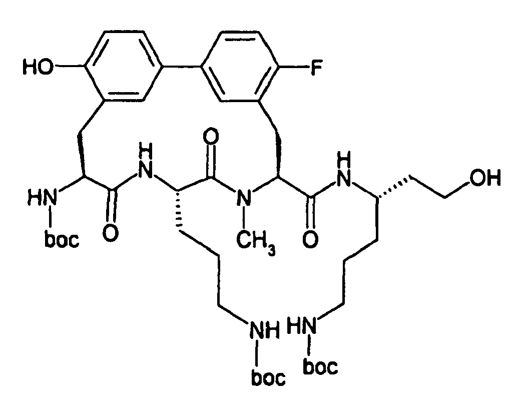 | 29F3 aus Beispiel 280 und Beispiel Z3 | LC-MS (Methode 3): Rₜ = 2.49 min. MS (EI): m/z = 887 (M+H)⁺ |
| **29P1** | 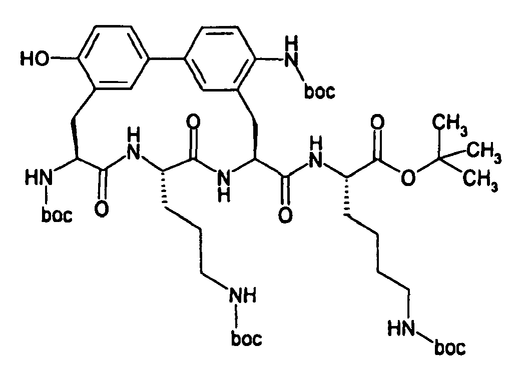 | 29F3 aus Beispiel 28P und *tert*-Butyl-N⁶-(*tert-*butoxycarbonyl)-*L-*lysinat | LC-MS (Methode 2): Rₜ = 2.91 min. MS (EI): m/z =1040 (M+H)⁺ |
| **29P2** | 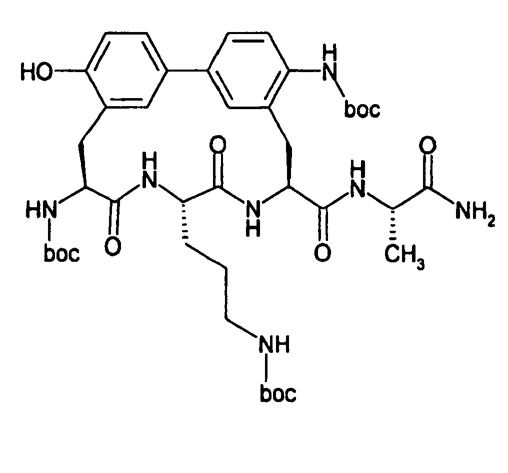 | 29F3 aus Beispiel 28P und *L-*Alaninamid | LC-MS (Methode 2): Rₜ = 2.49 min. MS (EI): m/z = 826 (M+H)⁺ |

### Beispiel 29F3

### tert-Butyl-{3-[(8S,11S,14S)-14-[(tert-butoxycarbonyl)amino]-8-[({2-[(tert-butoxycarbonyl)amino]-ethyl}amino)carbonyl]-17-hydroxy-10,13-dioxo-9,12-diazatricyclo[14.3,1.1^{2,6}]henicosa-1(20),2(21),3,5,16,18-hexaen-11-yl]propyl}carbamat

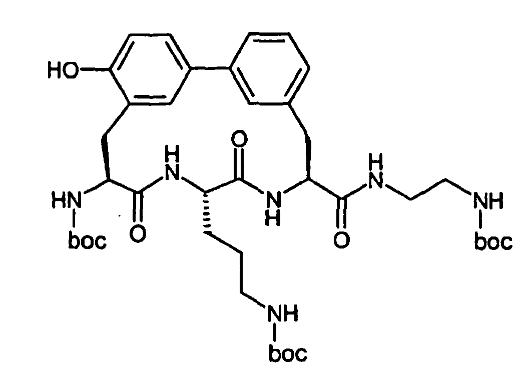

Unter Argon werden 24 mg (0.037 mmol) der Verbindung aus Beispiel 28F und 7.8 mg (0.048 mmol) *tert-*Butyl-(2-aminoethyl)carbamat in 1 ml Dimethylformamid gelöst. Bei 0°C (Eisbad) werden dann 9.2 mg (0.048 mmol) EDC und 1.5 mg (0.011 mmol) HOBt zugegeben. Es wird langsam auf RT erwärmt und für 12 h bei RT gerührt. Die Lösung wird im Vakuum eingeengt und der Rückstand wird mit Wasser verrührt. Der verbleibende Feststoff wird abgesaugt und im Hochvakuum getrocknet.
Ausbeute: 18 mg (63% d. Th.)
LC-MS (Methode 17): Rₜ = 2.41 min.
MS (EI): m/z = 783 (M+H)⁺

### Beispiel 29P3

### (8S,11S,14S)-14-[(tert-Butoxycarbonyl)amino]-11-{3-[(tert-butoxycarbonyl)amino]propyl}-5-{[N-(tert-butoxycarbonyl)glycyl]amino}-17-hydroxy-10,13-dioxo-9,12-diazatricyclo[14.3.1.1^{2,6}]-henicosa-1(20),2(21),3,5,16,18-hexaen-8-carbonsäure

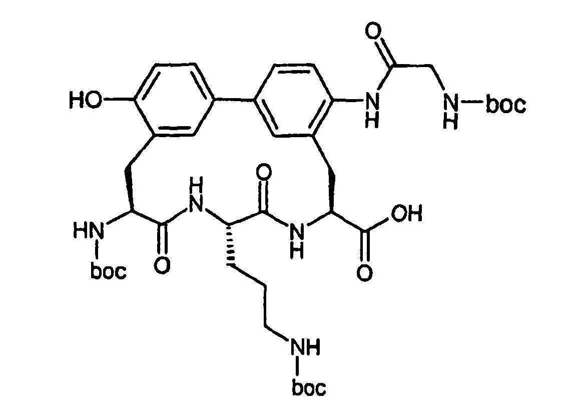

Unter Argon werden 4.4 mg (0.025 mmol) N-(*tert-*Butoxycarbonyl)glycin in 1.7 ml Dimethylformamid gelöst. Bei 0°C (Eisbad) werden dann 5.3 mg (0.027 mmol) EDC und 1.0 mg (0.007 mmol) HOBt zugegeben. Anschließend werden 20 mg (0.032 mmol) der Verbindung aus Beispiel 28P2 zugegeben. Es wird langsam auf RT erwärmt und für 12 h bei RT gerührt. Die Lösung wird im Vakuum eingeengt und der Rückstand chromatographisch an Sephadex-LH20 gereinigt (Laufmittel: Methanol / Essigsäure (0.25%)).
Ausbeute: 8.8 mg (44% d. Th.)
LC-MS (Methode 1): Rₜ = 2.17 min.
MS (EI): m/z = 813 (M+H)⁺

### Beispiel 30M1

### Benzyl-{3-[(8S,11S,14S)-17-(benzyloxy)-14-{[(benzyloxy)carbonyl]amino}-8-[({2-[bis(2-aminoethyl)amino]ethyl}amino)carbonyl]-5-chlor-9-methyl-10,13-dioxo-9,12-diazatricyclo[14.3.1.1^{2,6}]-henicosa-1(20),2(21),3,5,16,18-hexaen-11-yl]propyl}carbamat

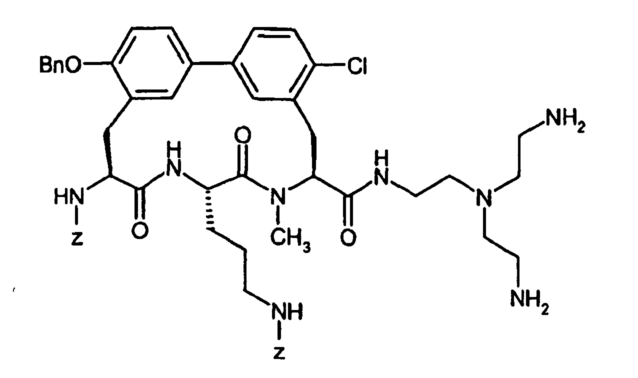

Zu einer Lösung aus 100 mg (0.036 mmol) der Verbindung aus Beispiel 23M in 0.25 ml N,N-Bis(2-aminoethyl)ethan-1,2-diamin werden 0.76 mg (0.012 mmol) Kaliumcyanid hinzugegeben. Es wird 16 h bei RT gerührt. Die Mischung wird mit Wasser versetzt. Der ausgefallene Feststoff wird abfiltriert und im Hochvakuum getrocknet.
Ausbeute: 102 mg (87 % d. Th.)
LC-MS (Methode 3): Rₜ = 2.03 min.
MS (EI): m/z = 975 (M+H)⁺.

### Beispiel 300

### Benzyl-{3-[(8S,11S,14S)-8-{[(2-aminoethyl)amino]carbonyl}-17-(benzyloxy)-14-{[(benzyloxy)-carbonyl]amino}-5-fluor-9-methyl-10,13-dioxo-9,12-diazatricyclo[14.3.1.1^{2,6}]henicosa-1(20),2(21),3,5,16,18-hexaen-11-yl]propyl}carbamat

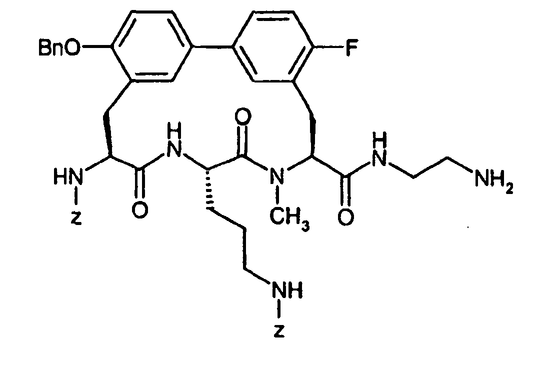

Zu einer Lösung aus 30 mg (0.036 mmol) Methyl-(8*S*,11*S*,14*S*)-17-(benzyloxy)-14-{[(benzyloxy)-carbonyl]amino}-11-(3-{[(benzyloxy)carbonyl]amino}propyl)-5-fluor-9-methyl-10,13-dioxo-9,12-diazatricyclo[14.3.1.1^{2,6}]henicosa-1(20),2(21),3,5,16,18-hexaen-8-carboxylat (Beispiel 230) in 0.6 ml 1,2-Diaminoethan werden 0.46 mg (0.007 mmol) Kaliumcyanid hinzugegeben. Es wird 16 h bei RT gerührt. Die Mischung wird mit Wasser versetzt. Der ausgefallene Feststoff wird abfiltriert und im Hochvakuum getrocknet.
Ausbeute: 28 mg (89% d. Th.)
LC-MS (Methode 2): Rₜ = 2.13 min.
MS (EI): m/z = 873 (M+H)⁺.

Analog den angegebenen Vorschriften werden die in der folgenden Tabelle aufgeführten Beispiele 30M2 und 30P aus den entsprechenden Edukten hergestellt:

| **Beispiel-Nr.** | **Struktur** | **Hergestellt analog Beispiel-Nr.** | **Analytische Daten** |
|---|---|---|---|
| **30M2** | 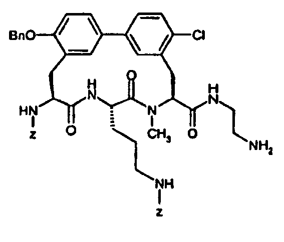 | 300 aus Beispiel 23M | LC-MS (Methode 1): Rₜ = 2.51 min. MS (ES): m/z = 889 (M+H)⁺ |
| **30P** | 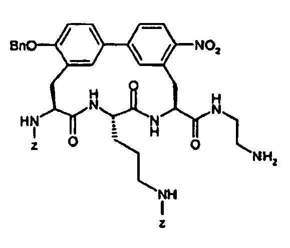 | 300 aus Beispiel 23P | LC-MS (Methode 1): Rₜ = 2.30 min. MS (ES): m/z = 886 (M+H)⁺ |

### Beispiel 31N

### (8S,11S,14S)-14-Amino-11-[(2R)-3-amino-2-hydroxypropyl]-17-hydroxy-4-(methoxycarbonyl)-10,13-dioxo-9,12-diazatricyclo[14.3.1.1^{2,6}]henicosa-1(20),2(21),3,5,16,18-hexaen-8-carbonsäure-Di(hydrotrifluoracetat)

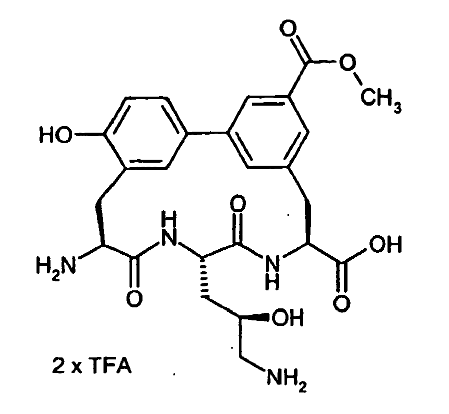

Die Herstellung erfolgt in Analogie zu Beispiel 39 aus Beispiel 23N.
LC-MS (Methode 3): Rₜ = 1.08 min.
MS (ES): m/z = 515 (M-2TFA+H)⁺
¹H-NMR (400 MHz, D₂O): δ = 1.8 (m_{c}, 1H), 1.96 (m_{c}, 1H), 2.75-3.2 (m, 5H), 3.48 (m_{c}, 1H), 3.84 (s, 3H), 3.86 (m_{c}, 1H), 4.38 (m_{c}, 1H), 4.57 (m_{c}, 1H), 4.80 (m_{c}, 1H), 6.83 (s, 1H), 6.88 (d, 1H), 7.37 (d, 1H), 7.40 (s, 1H), 7.65 (s, 1H), 7.84 (s, 1H).

### Beispiel 31O

### Methyl-(8S,11S,14S)-14-amino-11-(3-aminopropyl)-5-fluor-17-hydroxy-9-methyl-10,13-dioxo-9,12-diazatricyclo[14.3.1.1^{2,6}]henicosa-1(20),2(21),3,5,16,18-hexaen-8-carboxylat

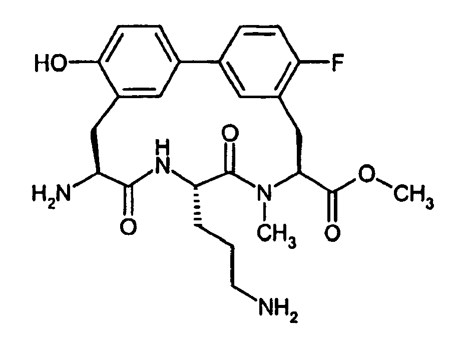

Eine Lösung von 50 mg (0.059 mmol) Methyl-(8*S*,11*S*,14*S*)-17-(benzyloxy)-14-{[(benzyloxy)-carbonyl]amino}-11-(3-{[(benzyloxy)carbonyl]amino}propyl)-5-fluor-9-methyl-10,13-dioxo-9,12-diazatricyclo[14.3.1.1^{2,6}]henicosa-1(20),2(21),3,5,16,18-hexaen-8-carboxylat (Beispiel 230) in Ethanol wird nach Zugabe von 5 mg Palladium auf Aktivkohle (10%ig) 12 h bei RT und Normaldruck hydriert. Es wird über Kieselgur filtriert und der Rückstand mit Ethanol gewaschen. Das Filtrat wird im Vakuum zur Trockne eingeengt.
Ausbeute: quant.
LC-MS (Methode 3): Rₜ = 1.34 min.
MS (EI): m/z = 487 (M+H)⁺.

### Beispiel 31P

### Methyl-(8S,11S,14S)-5,14-diamino-11-(3-aminopropyl)-17-hydroxy-10,13-dioxo-9,12-diazatricyclo[14.3.1.1^{2,6}]henicosa-1(20),2(21),3,5,16,18-hexaen-8-carboxylat-Trihydroacetat

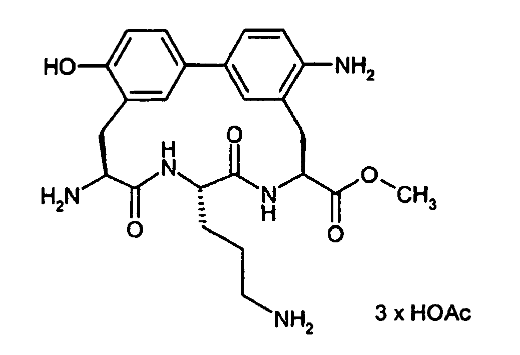

Es werden 150 mg der Verbindung aus Beispiel 23P in ein Gemisch aus 17 ml Essigsäure/Wasser/Ethanol (4:1:1) gegeben. Dazu gibt man 30 mg Palladium auf Aktivkohle (10%ig) und hydriert anschließend 72 h bei RT und Normaldruck. Das Reaktionsgemisch wird über vorgewaschenem Kieselgur filtriert, mit Ethanol gewaschen und das Filtrat im Vakuum einrotiert. Der zurückgebliebene Feststoff wird im Hochvakuum getrocknet.
Ausbeute: 100 mg (88% d. Th.)
LC-MS (Methode 17): Rₜ = 2.16 min.
MS (EI): m/z = 470 (M-3HOAc+H)⁺.
¹H-NMR (400 MHz, D₂O): δ = 1.6-1.9 (m, 4H), 2.85-3.25 (m, 5H), 3.48 (m_{c}, 1H), 3.75 (s, 3H), 4.37 (m_{c}, 1H), 4.6-4.7 (m, 1H, unter D₂O), 4.92 (m_{c}, 1H), 6.75-6.95 (m, 3H), 7.09 (s, 1H), 7.32 (d, 1H), 7.42 (d, 1H).

### Beispiel Z1

### (3S)-3-{[(Benzyloxy)carbonyl]amino}-6-[(tert-butoxycarbonyl)amino]hexanoyl-methylcarbonat

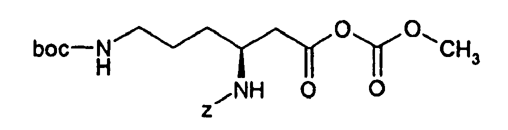

Unter Argon werden 2 g (5.26 mmol) (3*S*)-3-{[(Benzyloxy)carbonyl]amino}-6-[(*tert-*butoxycarbonyl)amino]hexansäure und 0.56 g (5.73 mmol) Triethylamin in 30 ml THF gelöst und auf 0°C abgekühlt. Dazu gibt man 0.59 g (5.73 mmol) Chlorameisensäureethylester und lässt 3 Stunden bei 0°C nachrühren. Die Reaktionsmischung wird über Kieselgur filtriert und das Filtrat wird direkt umgesetzt.

### Beispiel Z2

### Benzyl-[(1S)-4-[(tert-butoxycarbonyl)amino]-1-(2-hydroxyethyl)butyl]carbamat

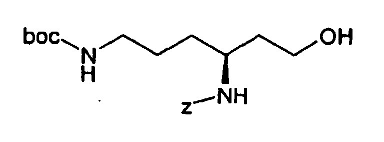

Das Filtrat von (3*S*)-3-{[(Benzyloxy)carbonyl]amino}-6-[(*tert-*butoxycarbonyl)amino]hexanoyl-methylcarbonat (Beispiel Z1) wird zu einer Suspension von 0.49 g (13.14 mmol) Natriumborhydrid in 0.6 ml Wasser bei 0°C tropfenweise hinzugegeben. Die Mischung erwärmt sich langsam auf RT und wird über Nacht gerührt. Die Reaktionslösung wird eingeengt, und der Rückstand wird mit Essigsäureethylester und Wasser versetzt. Die organische Phase wird über Magnesiumsulfat getrocknet, eingeengt und im Hochvakuum getrocknet. Das Rohprodukt wird ohne weitere Reinigung umgesetzt.
Ausbeute: 570 mg (30 % d.Th., über zwei Stufen)
LC-MS (Methode 1): Rₜ = 2.09 min.
MS (EI): m/z = 367 (M+H)⁺

### Beispiel Z3

### tert-Butyl-[(4S)-4-amino-6-hydroxyhexyl]carbamat

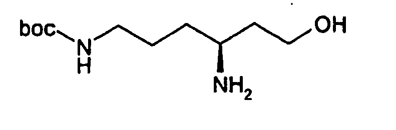

620 mg (1.69 mmol) Benzyl-[(1S)-4-[(*tert-*butoxycarbonyl)amino]-1-(2-hydroxyethyl)butyl]-carbamat (Beispiel Z2) werden in 60 ml Ethanol gelöst. Dazu gibt man 100 mg Palladium auf Aktivkohle (10%ig) und hydriert anschließend 15 h bei RT und Normaldruck. Das Reaktionsgemisch wird über vorgewaschenem Kieselgur filtriert, mit Ethanol gewaschen, und das Filtrat wird einrotiert. Das Produkt wird ohne weitere Reinigung umgesetzt.
Ausbeute: quant.
¹H-NMR (400 MHz, D₂O): δ = 1.2-1.6 (m, 6H), 1.4 (s, 9H), 2.6-3.0 (m, 1H), 3.0-3.2 (m, 2H), 3.7-3.9 (m, 2H), 4.6 (br.s, 1H)

### Beispiel Z4

### Benzyl-[(1S)-4-[(tert-butoxycarbonyl)amino]-1-(hydroxymethyl)butyl]carbamat

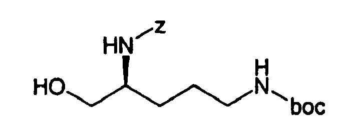

Eine Lösung von 570 mg (1.56 mmol) *N*²-[(Benzyloxy)carbonyl]-*N*⁵-(*tert-*butoxycarbonyl)-*L-*omithin in 10 ml Tetrahydrofuran wird bei -10°C mit 157 mg (1.56 mmol) 4-Methylmorpholin und 169 mg (1.56 mmol) Chlorameisensäureethylester versetzt und 30 min gerührt. Bei dieser Temperatur werden 3.11 ml (3.11 mmol) einer 1M Lösung von Lithiumaluminiumhydrid in Tetrahydrofuran langsam zugetropft. Es wird langsam auf RT erwärmt und für 12 h bei RT gerührt. Unter Eiskühlung gibt man vorsichtig 0.2 ml Wasser und 0.3 ml 4.5%ige Natriumhydroxid-Lösung hinzu und rührt weitere 3 h bei RT. Der Ansatz wird filtriert und das Filtrat wird im Vakuum eingeengt. Der Rückstand wird in Essigsäureethylester gelöst, mit Wasser gewaschen, über Magnesiumsulfat getrocknet und erneut im Vakuum zur Trockne eingeengt. Das Produkt wird ohne weitere Reinigung umgesetzt.
Ausbeute: 170 mg (31% d. Th.)
LC-MS (Methode 2): Rₜ = 1.88 min.
MS (EI): m/z = 353 (M+H)⁺.

### Beispiel Z5

### tert-Butyl-[(4S)-4-amino-5-hydroxypentyl]carbamat

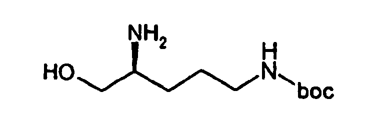

Eine Lösung von 169 mg (0.48 mmol) Benzyl-[(1*S*)-4-[(*tert-*butoxycarbonyl)amino]-1-(hydroxymethyl)butyl]carbamat (Beispiel Z4) in 50 ml Ethanol wird nach Zugabe von 17 mg Palladium auf Aktivkohle (10%ig) 4 h bei RT und Normaldruck hydriert. Es wird über Kieselgur filtriert und der Rückstand mit Ethanol gewaschen. Das Filtrat wird im Vakuum zur Trockne eingeengt. Das Produkt wird ohne weitere Reinigung umgesetzt.
Ausbeute: 104 mg (99% d. Th.)
MS (DCI): m/z = 219 (M+H)⁺

### Beispiel Z6

### tert-Butyl-[(1S)-4-[(tert-butoxycarbonyl)amino]-1-(hydroxymethyl)butyl]carbamat

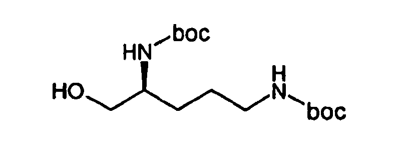

Eine Lösung von 300 mg (0.90 mmol) *N*²,*N*⁵-Bis-(*tert-*butoxycarbonyl)-*L-*ornithin in 10 ml Tetrahydrofuran wird bei -10°C mit 91 mg (0.90 mmol) 4-Methylmorpholin und 98 mg (0.90 mmol) Chlorameisensäureethylester versetzt und 30 min gerührt. Bei dieser Temperatur werden 1.81 ml (1.81 mmol) einer 1M Lösung von Lithiumaluminiumhydrid in Tetrahydrofuran langsam zugetropft. Es wird langsam auf RT erwärmt und für 12 h bei RT gerührt. Unter Eiskühlung gibt man vorsichtig 0.1 ml Wasser und 0.15 ml 4.5%ige Natriumhydroxid-Lösung hinzu und rührt weitere 3 h bei RT. Der Ansatz wird filtriert und das Filtrat wird im Vakuum eingeengt. Der Rückstand wird in Essigsäureethylester gelöst, mit Wasser gewaschen, über Magnesiumsulfat getrocknet und erneut im Vakuum zur Trockne eingeengt. Das Produkt wird ohne weitere Reinigung umgesetzt.
Ausbeute: 239 mg (83% d. Th.)
MS (ESI): m/z = 319 (M+H)⁺; 341 (M+Na)⁺.

### Beispiel Z7

### (2S)-2,5-Bis[(tert-butoxycarbonyl)amino]pentyl-methansulfonat

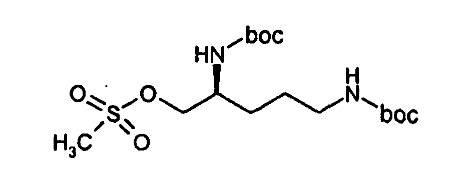

Eine Lösung von 240 mg (0.75 mmol) *tert-*Butyl-[(1*S*)-4-[(*tert-*butoxycarbonyl)amino]-1-(hydroxymethyl)butyl]carbamat (Beispiel Z6) in 20 ml Dichlormethan wird mit 103 mg (0.90 mmol) Methansulfonsäurechlorid und 0.21 ml (1.5 mmol) Triethylamin versetzt und für 16 h bei RT gerührt. Es wird mit Dichlormethan verdünnt und zweimal mit 0.1N Salzsäure gewaschen. Die organische Phase wird über Magnesiumsulfat getrocknet und im Vakuum bis zur Trockne eingeengt. Das Produkt wird ohne weitere Reinigung umgesetzt.
Ausbeute: 218 mg (73% d. Th.)
MS (ESI): m/z = 419 (M+Na)⁺.

### Beispiel Z8

### tert-Butyl-{(4S)-5-azido-4-[(tert-butoxycarbonyl)amino]pentyl}carbamat

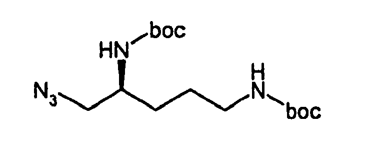

Eine Lösung von 218 mg (0.55 mmol) (2*S*)-2,5-Bis[(*tert-*butoxycarbonyl)amino]pentyl-methansulfonat (Beispiel Z7) in 15 ml Dimethylformamid wird mit 36 mg (0.55 mmol) Natriumazid versetzt und 12 h bei 70°C gerührt. Ein Großteil des Lösungsmittel wird im Vakuum abdestilliert und der Rückstand wird mit Essigsäureethylester verdünnt. Es wird mehrmals mit gesättigter Natriumhydrogencarbonat-Lösung gewaschen, über Magnesiumsulfat getrocknet und im Vakuum zur Trockne eingeengt. Das Produkt wird ohne weitere Reinigung umgesetzt.
Ausbeute: 188 mg (99% d. Th.)
MS (ESI): m/z = 344 (M+H)⁺.

### Beispiel Z9

### tert-Butyl-{(4S)-5-amino-4-[(tert-butoxycarbonyl)amino]pentyl}carbamat

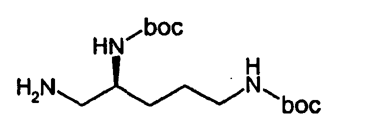

Eine Lösung von 188 mg (0.55 mmol) *tert-*Butyl-{(4*S*)-5-azido-4-[(*tert-*butoxycarbonyl)amino]-pentyl}carbamat (Beispiel Z8) in Ethanol wird nach Zugabe von 20 mg Palladium auf Aktivkohle (10%ig) 12 h bei RT und Normaldruck hydriert. Es wird über Kieselgur filtriert und der Rückstand mit Ethanol gewaschen. Das Filtrat wird im Vakuum zur Trockne eingeengt. Das Produkt wird ohne weitere Reinigung umgesetzt.
Ausbeute: 102 mg (59% d. Th.)
MS (ESI): m/z = 318 (M+H)⁺; 340 (M+Na)⁺.

Analog den angegebenen Vorschriften werden die in der folgenden Tabelle aufgeführten Beispiele Z10 und Z11 aus den entsprechenden Edukten hergestellt:

| **Bsp.-Nr.** | **Struktur** | **Hergestellt analog Beispiel-Nr.** | **Analytische Daten** |
|---|---|---|---|
| **Z10** | 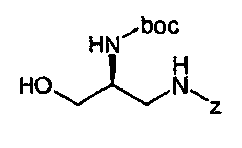 | Z4 aus 3-{[(Benzyl-oxy)-carbonyl]-amino}-*N*-(*tert-*butoxy-carbonyl)-*L-*alanin | LC-MS (Methode 12): Rₜ = 1.79 min. MS (EI): m/z = 325 (M+H)⁺ |
| **Z11** | 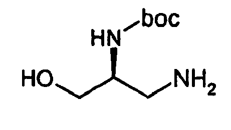 | Z5 aus Beispiel Z10 | MS (DCI): m/z = 191 (M+H)⁺ |

### Ausführungsbeispiele

### Beispiel 1

### (8S,11S,14S)-14-Amino-11-(3-aminopropyl)-5-fluor-17-hydroxy-10,13-dioxo-9,12-diazatricyclo[14.3.1.1^{2,6}]henicosa-1(20),2(21),3,5,16,18-hexaen-8-carbonsäure-Dihydrochlorid

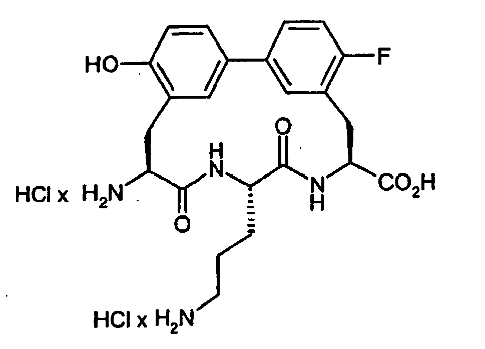

30 mg (0.03 mmol) der Verbindung aus Beispiel 23A werden in 30 ml Eisessig / Wasser / Ethanol = 4/1/1 suspendiert, mit 15 mg Pd/C-Katalysator (10%ig) versetzt und 24 h bei RT hydriert. Der Katalysator wird abfiltriert, das Filtrat mit 5 ml 0.1N Salzsäure versetzt und im Vakuum eingedampft und im Hochvakuum getrocknet.
Ausbeute: 18 mg (quantitativ)
LC-MS (Methode 14): Rₜ = 1.78 min.
MS (EI): m/z = 458 (M-2HCl+H)⁺

### Beispiel 2

### (8S,11S,14S)-14-Amino-11-(3-aminopropyl)-4,17-dihydroxy-10,13-dioxo-9,12-diazatricyclo[14.3.1.1^{2,6}]henicosa-1(20),2(21),3,5,16,18-hexaen-8-carbonsäure-Dihydrochlorid

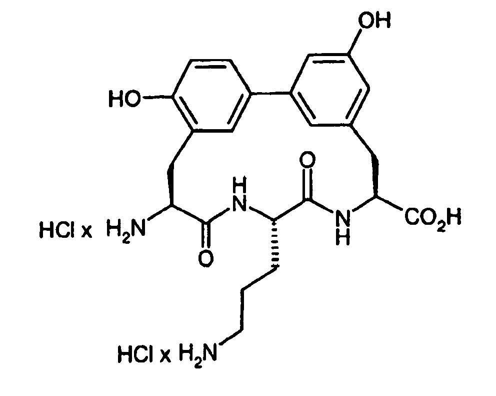

Die Herstellung erfolgt analog zu Beispiel 1 aus 22 mg (0.02 mmol) der Verbindung aus Beispiel 23K in 4 ml Eisessig / Wasser / Ethanol = 4/1/1 mit 8 mg Pd/C-Katalysator (10%ig).
Ausbeute: 11 mg (97% d. Th.)
LC-MS (Methode 15): Rₜ = 1.24 min.
MS (EI): m/z = 457 (M-2HCl+H)⁺

### Beispiel 3

### (8S,11S,14S)-14-Amino-11-[(2R)-3-amino-2-hydroxypropyl]-5-fluor-17-hydroxy-10,13-dioxo-9,12-diazatricyclo[14.3.1.1^{2,6}]henicosa-1(20),2(21),3,5,16,18-hexaen-8-carbonsäure-Dihydrochlorid

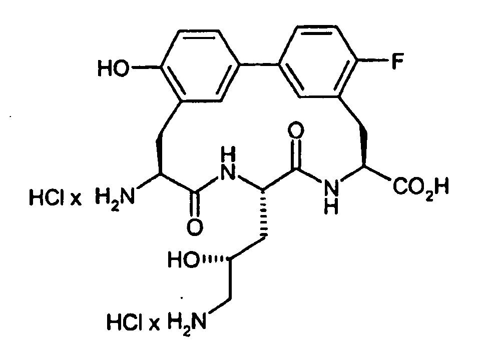

Die Herstellung erfolgt analog zu Beispiel 1 aus 55 mg (0.06 mmol) der Verbindung aus Beispiel 23L in 55 ml Eisessig / Wasser / Ethanol = 4/1/1 mit 34 mg Pd/C-Katalysator (10%ig).
Ausbeute: 33 mg (quantitativ)
LC-MS (Methode 2): Rₜ = 1.37 min.
MS (EI): m/z = 475 (M-2HCl+H)⁺

### Beispiel 4

### (8S,11S,14S)-14-Amino-11-[(2R)-3-amino-2-hydroxypropyl]-5-chlor-17-hydroxy-9-methyl-10,13-dioxo-9,12-diazatricyclo[14.3.1.1^{2,6}]henicosa-1(20),2(21),3,5,16,18-hexaen-8-carbonsäure

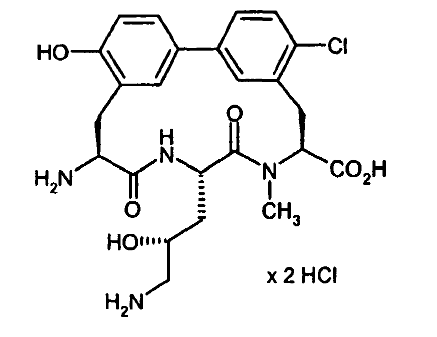

69 mg (ca. 0.077 mmol) des Gemisches aus Beispiel 24B werden in 2 ml THF / Methanol (1/1) suspendiert und unter Rühren mit 4.85 mg (0.2 mmol) Lithiumhydroxid versetzt. Es entsteht eine Lösung. Man rührt noch eine weitere halbe Stunde, versetzt mit 0.22 ml 0.1N Salzsäure und dampft dann alles im Vakuum zur Trockne ein.
Ausbeute: 80 mg eines Gemisches aus 44% Produkt und 18% des O-Acetyl-Produktes
LC-MS (Methode 3): Rₜ = 1.33 min. (Produkt) bzw. 1.51 min. (O-Acetyl-Produkt)
MS (EI): m/z = 504 (M-2HCl+H)⁺ bzw. 546 (MOAc-2HCl+H)⁺

### Beispiel 5

### (8S,11S,14S)-14-Amino-N-(2-aminoethyl)-11-(3-aminopropyl)-5-fluor-17-hydroxy-10,13-dioxo-9,12-diazatricyclo[14.3.1.1^{2,6}]henicosa-1(20),2(21),3,5,16,18-hexaen-8-carboxamid-Trihydrochlorid

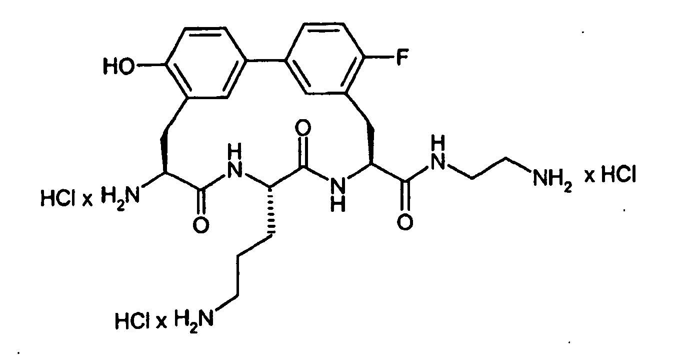

6.5 mg (0.01 mmol) der Verbindung aus Beispiel 29A1 werden bei RT mit 1 ml 4N Dioxan / Chlorwasserstoff-Lösung übergossen und 2 h gerührt. Man dampft alles im Vakuum zur Trockne , ein und trocknet im Hochvakuum bis zur Gewichtskonstanz.
Ausbeute: 5 mg (quantitativ)
LC-MS (Methode 3): Rₜ = 0.27 min.
MS (EI): m/z = 500 (M-3HCl+H)⁺

### Beispiel 6

### (8S,11S,14S)-14-Amino-11-[(2R)-3-amino-2-hydroxypropyl]-17-hydroxy-10,13-dioxo-9,12-diazatricyclo[14.3.1.1^{2,6}]henicosa-1(20),2(21),3,5,16,18-hexaen-8-carboxamid-Di(hydrotrifluoracetat)

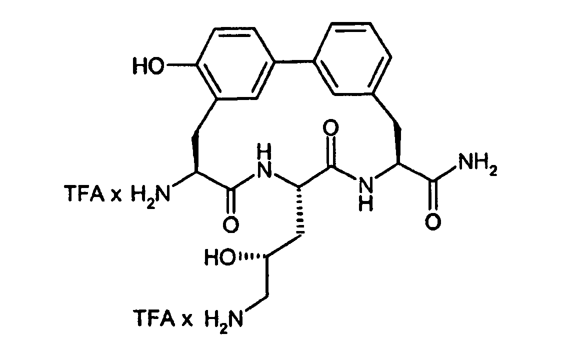

Die Herstellung erfolgt analog zu Beispiel 5 aus 16.1 mg (0.025 mmol) der Verbindung aus Beispiel 29D mit 0.4 ml 4N Dioxan / Chlorwasserstoff-Lösung. Das Rohprodukt wird durch HPLC (Kromasil 100C18, Laufmittel Acetonitril / 0.2% wässrige TFA 1:3) gereinigt.
Ausbeute: 1 mg (5% d. Th.)
LC-MS (Methode 5): Rₜ = 1.14 min.
MS (EI): m/z = 456 (M-2TFA+H)⁺
¹H-NMR (400 MHz, D₂O): δ = 1.83 (m_{c}, 1H), 1.95 (m_{c}, 1H), 2.85 (m_{c}, 1H), 2.9-3.2 (m, 4H), 3.54 (m_{c}, 1H), 3.80 (m_{c}, 1H), 4.38 (m_{c}, 1H), 4.6 (m, 1H, unter D₂O-Signal), 4.84 (m_{c}, 1H), 6.85-7.0 (m, 2H), 7.14 (d, 1H), 7.24-7.48 (m, 4H).

Analog zu den oben aufgeführten Vorschriften werden die in der folgenden Tabelle aufgeführten Beispiele 7 bis 12 aus den entsprechenden Edukten hergestellt:

| Beispiel-Nr. | Struktur | Hergestellt analog Beispiel-Nr. | Analytische Daten |
|---|---|---|---|
| **7** | 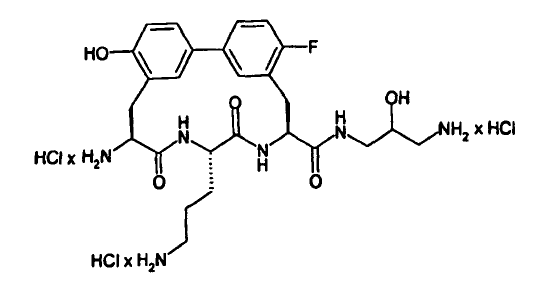 | 5 aus Beispiel 29A2 Ausbeute: 99% d. Th. | LC-MS (Methode 3): Rₜ = 0.25 min. MS (EI): m/z = 530 (M-3HCl+H)⁺ |
| **8** | 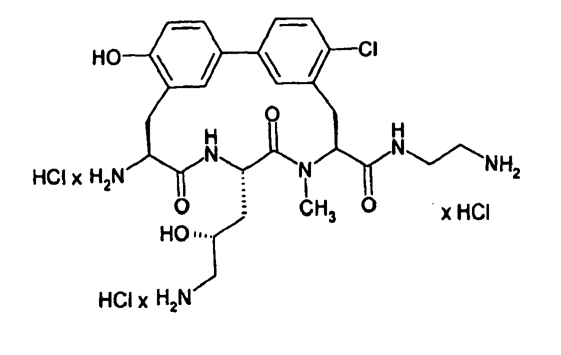 | 5 aus Beispiel 29B1 Ausbeute: 99% d. Th. | LC-MS (Methode 2): Rₜ = 0.48 min. MS (EI): m/z= 546 (M-3HCl+H)⁺ |
| **9** | 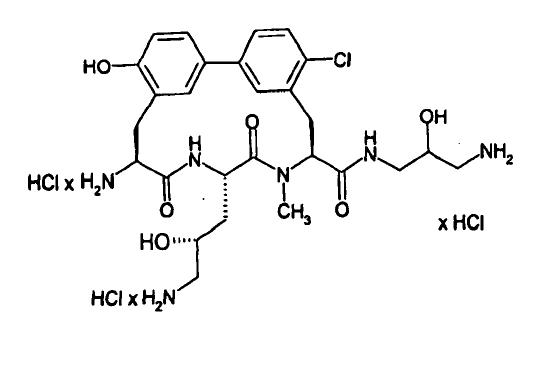 | 5 aus Beispiel 29B2 Ausbeute: 97% d. Th. | LC-MS (Methode 3): Rₜ = 0.29 min. MS (EI): m/z = 576 (M-3HCl+H)⁺ |
| **10** | 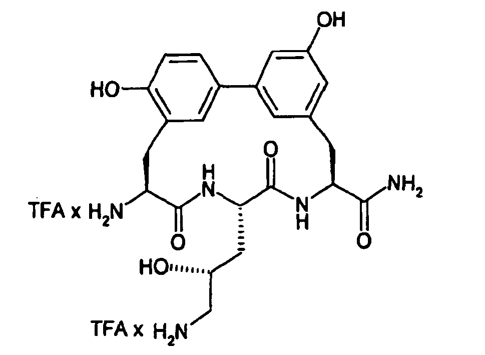 | 6 aus Beispiel 29J Ausbeute: 38% d. Th. | LC-MS (Methode 5): Rₜ = 0.77 min. MS (EI): m/z = 470 (M-2TFA-H)⁻ |
| **11** | 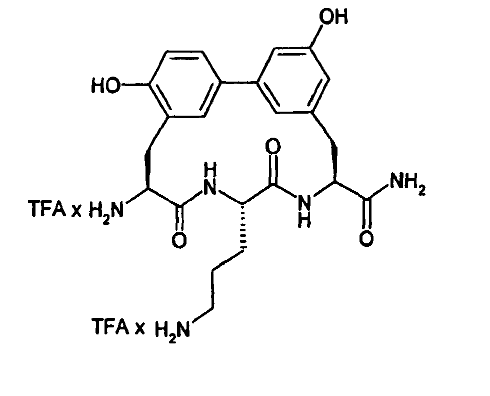 | 6 aus Beispiel 29K Ausbeute: 45% d. Th. | LC-MS (Methode 8): Rₜ =1.41 min. MS (EI): m/z = 456 (M-2TFA+H)⁺ |
| **12** | 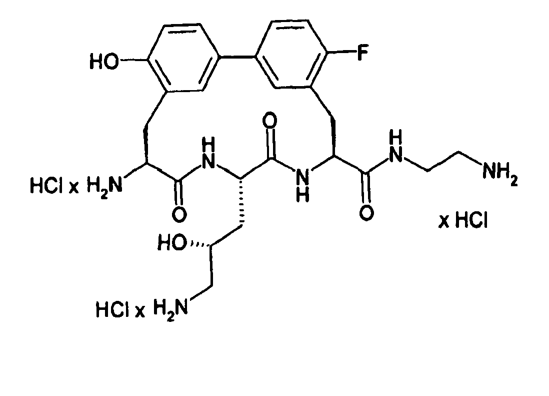 | 5 aus Beispiel 29L Ausbeute: 99% d. Th. | LC-MS (Methode 17): Rₜ = 2.20 min. MS (EI): m/z = 517 (M-3HCl+H)⁺ |

### Beispiel 13

### (8S,11S,14S)-14-Amino-11-[(2R)-3-amino-2-hydroxypropyl]-N-(2-amino-2-oxoethyl)-5-chlor-17-hydroxy,- 10,13-dioxo-9,12-diazatricyclo[14.3.1.12.6]henicosa-1(20),2(21),3,5,16,18-hexaen-8-carboxamid-Di(hydrotrifluoracetat)

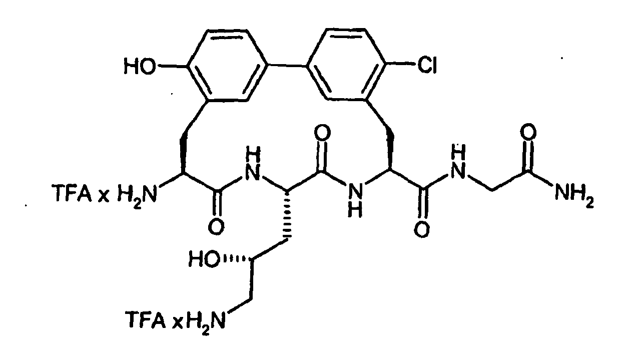

41 mg (0.04 mmol) der Verbindung aus Beispiel 27C1 werden in 0.5 ml einer 33%igen Bromwasserstoff-Lösung in Essigsäure gelöst und 45 min bei RT gerührt. Anschließend dampft man alles vorsichtig (Badtemperatur max. 40°C) zur Trockne ein und nimmt das Rohprodukt in 7 ml THF / Methanol / Wasser (4:2:1) auf. Man versetzt mit 2 mg (0.08 mmol) Lithiumhydroxid und rührt für 12 h bei RT nach. Der Ansatz wird im Vakuum eingeengt, in 3 ml 0.1N Salzsäure aufgenommen und 15 min bei RT gerührt. Das Lösungsmittel wird im Vakuum eingedampft und das Rohprodukt wird durch HPLC (Kromasil 100C18, Laufmittel Acetonitril / 0.2%ige wässrige TFA, Gradient) gereinigt.
Ausbeute: 8.25 mg (29% d. Th.)
LC-MS (Methode 1): Rₜ = 1.15 min
MS (EI): m/z = 547 (M-2TFA+H)⁺
¹H-NMR (400 MHz, D₂O): δ = 1.83 (m_{c}, 1H), 1.97 (m_{c}, 1H), 2.84 (m_{c}, 1H), 2.93-3.08 (m, 3H), 3.40 (m_{c}, 1H), 3.53 (m_{c}, 1H), 3.75-4.0 (m, 3H), 4.38 (m_{c}, 1H), 4.78 (m_{c}, 1H), 4.85 (m_{c}, 1H), 6.90 (d, 1H), 6.97 (s, 1H), 7.30 (s, 1H), 7.33-7.50 (m, 3H).

### Beispiel 14

### Methyl-3-amino-N-{[(8S,11S,14S)-14-amino-11-[(2R)-3-amino-2-hydroxypropyl]-5-chlor-17-hydroxy-10,13-dioxo-9,12-diazatricyclo[14.3.1.1^{2,6}]henicosa-1(20),2(21),3,5,16,18-hexaen-8-yl]carbonyl}-L-alaninat-Tri(hydrotrifluoracetat)

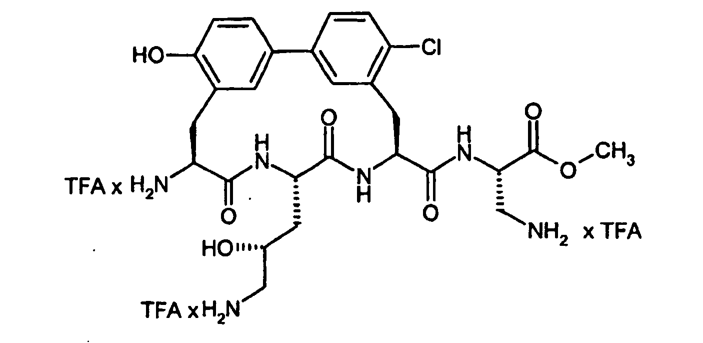

51 mg (0.044 mmol) der Verbindung aus Beispiel 27C2 werden in 1 ml einer 33%igen Bromwasserstoff-Lösung in Essigsäure gelöst und 45 min bei RT gerührt. Anschließend wird im Vakuum eingeengt, das Rohprodukt in 2 ml DMF gelöst und mit 2 mg Lithiumhydroxid versetzt. Es wird 12 h bei RT gerührt. Das Lösungsmittel wird im Vakuum eingedampft und das Rohprodukt wird durch HPLC (Kromasil 100C18, Laufmittel Acetonitril / 0.2%ige wässrige TFA, Gradient) gereinigt.
Ausbeute: 17.8 mg (43% d. Th.)
LC-MS (Methode 3): Rₜ = 0.57 min
MS (EI): m/z = 591 (M-3TFA+H)⁺
¹H-NMR (400 MHz, D₂O): δ = 1.85 (m_{c}, 1H), 1.97 (m_{c}, 1H), 2.84 (m_{c}, 1H), 2.95-3.12 (m, 3H), 3.30 (m_{c}, 1H), 3.4-3.6 (m, 4H), 3.75 (s, 3H), 3.85 (m_{c}, 1H), 4.38 (m_{c}, 1H), 4.78 (m_{c}, 1H), 4.87 (m_{c}, 1H), 6.90 (d, 1H), 7.0 (s, 1H), 7.34 (s, 1H), 7.37-7.55 (m, 3H).

### Beispiel 15

### 3-Amino-N-{[(8S,11S,14S)-14-amino-11-[(2R)-3-amino-2-hydroxypropyl]-5-chlor-17-hydroxy-10,13-dioxo-9,12-diazatricyclo[14.3.1.1^{2,6}]henicosa-1(20),2(21),3,5,16,18-hexaen-8-yl]carbonyl}-L-alanin-Tri(hydrotrifluoracetat)

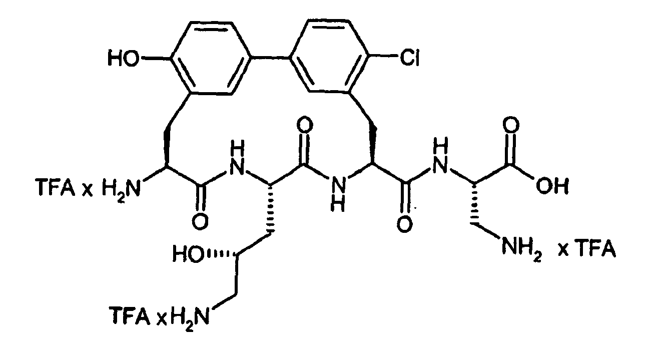

Beispiel 15 entsteht als Nebenprodukt der Umsetzung zu Beispiel 14 und wird ebenfalls durch HPLC (Kromasil 100C18, Laufmittel Acetonitril / 0.2%ige wässrige TFA, Gradient) isoliert.
Ausbeute: 3.8 mg (8% d. Th.)
LC-MS (Methode 3): Rₜ = 0.76 min
MS (EI): m/z = 577 (M-3TFA+H)⁺
¹H-NMR (400 MHz, D₂O): δ = 1.85 (m_{c}, 1H), 1.96 (m_{c}, 1H), 2.86 (m_{c}, 1H), 2.95-3.14 (m, 3H), 3.23 (m_{c}, 1H), 3.27-3.6 (m, 4H), 3.85 (m_{c}, 1H), 4.38 (m_{c}, 1H), 4.78 (m_{c}, 1H), 4.87 (m_{c}, 1H), 6.90 (d, 1H), 6.98 (s, 1H), 7.28 (s, 1H), 7.33-7.53 (m, 3H).

### Beispiel 16

*N*-{[(8*S*,11*S*,14*S*)-14-Amino-11-[(2*R*)-3-amino-2-hydroxypropyl]-5-chlor-17-hydroxy-10,13-dioxo-9,12-diazatricyclo[14.3.1.1^{2,6}]henicosa-1(20),2(21),3,5,16,18-hexaen-8-yl]carbonyl}-*L-*serin-Di(hydrotrifluoracetat) 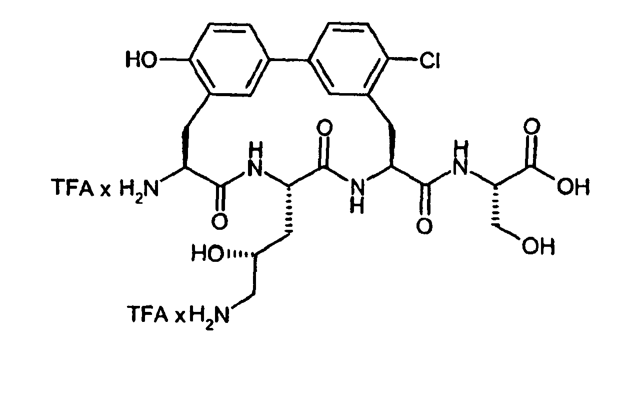 51 mg (0.044 mmol) der Verbindung aus Beispiel 27C8 werden in 1 ml einer 33%igen Bromwasserstoff-Lösung in Essigsäure gelöst und 45 min bei RT gerührt. Anschließend wird im Vakuum eingeengt, das Rohprodukt in 2 ml DMF gelöst und mit 2 mg Lithiumhydroxid versetzt. Es wird 12 h bei 60°C gerührt. Das Lösungsmittel wird im Vakuum eingedampft und das Rohprodukt wird durch HPLC (Kromasil 100C18, Laufmittel Acetonitril / 0.2%ige wässrige TFA, Gradient) gereinigt.
Ausbeute: 3.86 mg (11% d. Th.)
LC-MS (Methode 2): Rₜ = 0.91 min
MS (EI): m/z = 578 (M-2TFA+H)⁺
¹H-NMR (400 MHz, D₂O): δ = 1.84 (m_{c}, 1H), 1.97 (m_{c}, 1H), 2.68-3.10 (m, 3H), 3.37 (m_{c}, 1H), 3.53 (m_{c}, 1H), 3.85 (m_{c}, 1H), 4.38 (m_{c}, 1H), 4.47 (m_{c}, 1H), 4.76 (m_{c}, 1H), 4.96 (m_{c}, 1H), 6.86 (d, 1H), 6.94 (s, 1H), 7.27 (s, 1H), 7.3-7.5 (m, 3H).

Analog zu den oben aufgeführten Vorschriften werden die in der folgenden Tabelle aufgeführten Beispiele 17 bis 24 aus den entsprechenden Edukten hergestellt:

| **Beispiel-Nr.** | **Struktur** | **Hergestellt analog Beispiel-Nr.** | **Analytische Daten** |
|---|---|---|---|
| **17** | 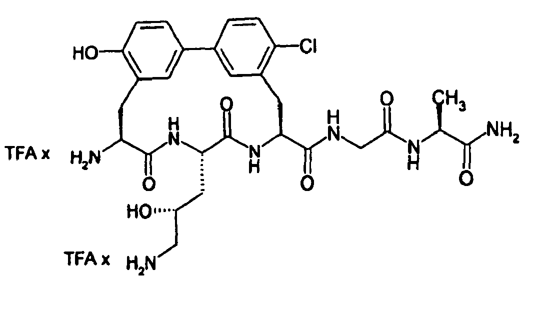 | 13 | LC-MS (Methode 2): Rₜ = 0.92 min. |
| | | aus Beispiel 27C3 | MS (EI): m/z = 618 (M-2TFA+H)⁺ |
| | | | ¹H-NMR (400 MHz, D₂O): δ = 1.33 (d, 3H), 1.83 (m_{c}, |
| | | Ausbeute: 25% d. Th. | 1H), 1.98 (m_{c}, 1H), 2.85 (m_{c}, 1H), 2.95-3.1 (m, 3H), 3.4 (m_{c}, 1H), 3.53 (m_{c}, 1H), 3.83 (m_{c}, 1H), 3.95 (m_{c}, 2H), 4.22 (q, 1H), 4.38 (m_{c}, 1H), 4.77 (m_{c}, 1H), 4.84 (m_{c}, 1H), 6.90 (d, 1H), 6.96 (s, 1H), 7.3 (s, 1H), 7.35-7.5 (m, 3H). |
| **18** | 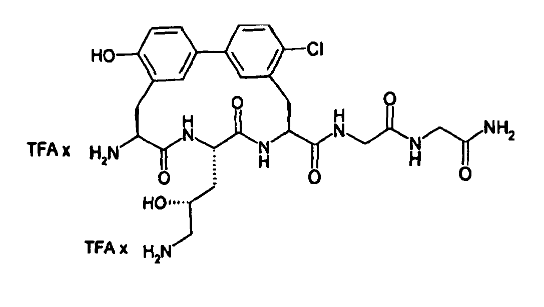 | 14 | LC-MS (Methode 3): Rₜ =1.13 min. |
| | | aus Beispiel | MS (EI): m/z = 604 (M-2TFA+H)⁺ |
| | | 27C4 Ausbeute: 59% d. Th. | ¹H-NMR (400 MHz, D₂O): δ = 1.83 (m_{c}, 1H), 1.98 (m_{c}, 1H), 2.83 (m_{c}, 1H), 2.92-3.08 (m, 3H), 3.37 (m_{c}, 1H), 3.52 (m_{c}, 1H), 3.75-4.05 (m, 5H), 4.38 (m_{c}, 1H), 4.77 (m_{c}, 1H), 4.84 (m_{c}, 1H), 6.87 (d, 1H), 6.95 (s, 1H), 7.27 (s, 1H), 7.3-7.5 (m, 3H). |
| **19** | 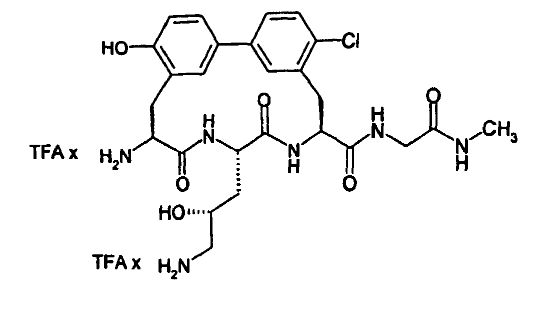 | 14 | LC-MS (Methode 3): Rₜ =1.19 min. |
| | | aus Beispiel | MS (EI): m/z = 561 (M-2TFA+H)⁺ |
| | | 27C5 | |
| | | Ausbeute: 59% d. Th. | ¹H-NMR (400 MHz, D₂O): δ =1.83 (m_{c}, 1H), 1.98 (m_{c}, 1H), 2.66 (s, 3H), 2.85 (m_{c}, 1H), 2.92-3.08 (m, 3H), 3.4 (m_{c}, 1H), 3.52 (m_{c}, 1H), 3.75-3.95 (m, 3H), 4.37 (m_{c}, 1H), 4.76 (m_{c}, 1H), 4.84 (m_{c}, 1H), 6.88 (d, 1H), 6.95 (s, 1H), 7.29 (s, 1H), 7.35-7.5 (m, 3H). |
| **20** | 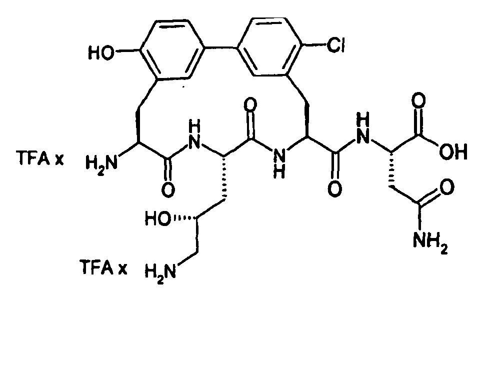 | 14 | LC-MS (Methode 2): Rₜ = 0.91 min. |
| | | aus Beispiel | MS (EI): m/z = 605 (M-2TFA+H)⁺ |
| | | 27C6 Ausbeute: 40% d. Th. | ¹H-NMR (400 MHz, D₂O): δ = 1.83 (m_{c}, 1H), 1.98 (m_{c}, 1H), 2.66-3.08 (m, 6H), 3.32 (m_{c}, 1H), 3.53 (m_{c}, 1H), 3.82 (m_{c}, 1H), 4.37 (m_{c}, 1H), 4.56 (m_{c}, 1H), 4.76 (m_{c}, 1H), 4.84 (m_{c}, 1H), 6.8-6.95 (m, 2H), 7.21 (s, 1H), 7.3-7.45 (m, 3H). |
| **21** | 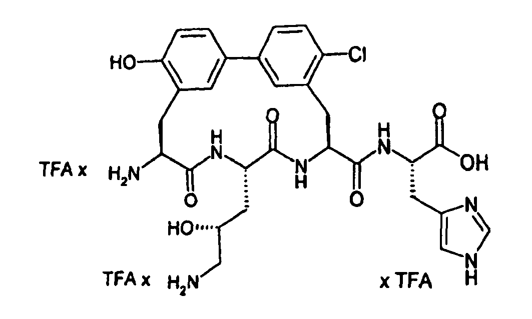 | 14 | MS (EI): m/z = 628 (M-3TFA+H)⁺ |
| | | aus Beispiel 27C7 | ¹H-NMR (400 MHz, D₂O): δ = 1.80 (m_{c}, 1H), 1.95 (m_{c}, 1H), 2.75-3.2 (m, 6H), 3.27 (m_{c}, 1H), 3.50 (m_{c}, 1H), |
| | | Ausbeute: 21% d. Th. | 3.81 (m_{c}, 1H), 4.37 (m_{c}, 1H), 4.48 (m_{c}, 1H), 4.68 (m_{c}, 1H), 4.75 (m_{c}, 1H), 6.83-6.97 (m, 2H), 7.21 (s, 1H), 7.26 (s, 1H), 7.3-7.5 (m, 3H), 8.57 (s, 1H). |
| **22** | 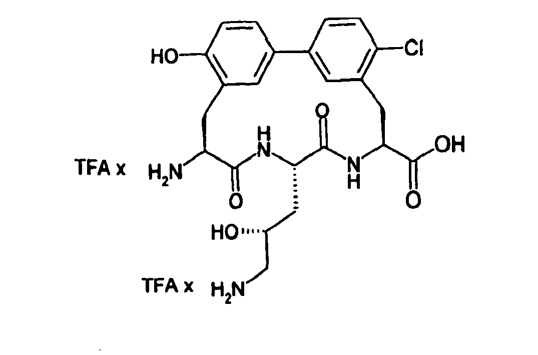 | 14 | LC-MS (Methode 8): Rₜ = 1.67 min. |
| | | aus Beispiel 23C | MS (EI): m/z = 491 (M.2TFA+H)⁺ |
| | | Ausbeute: | ¹H-NMR (400 MHz, D₂O): δ = 1.8 (m_{c}, 1H), 1.97 (m_{c}, 1H), 2.83 (m_{c}, 2H), 2.96 (m_{c}, 1H), 3.05 (m_{c}, 1H), 3.48 (m_{c}, 2H), 3.88 (m_{c}, 1H), 4.35 (m_{c}, 1H), 4.57 (m_{c}, 1H), 4.75 (m_{c}, 1H), 6.8-6.93 (m, 2H), 7.20 (s, 1H), 7.25-7.45 (m, 3H). |
| | | 25% d. Th. | |
| **23** | 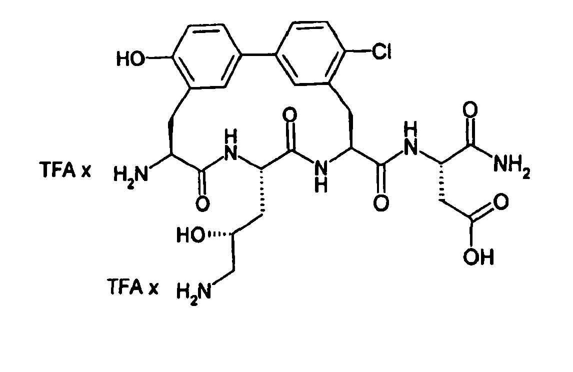 | 14 | LC-MS (Methode 2): Rₜ = 0.92 min. |
| | | aus Beispiel 27C9 | MS (EI): m/z = 605 (M-2TFA+H)⁺ |
| | | Ausbeute: 9% d. Th. | ¹H-NMR (400 MHz, D₂O): δ = 1.83 (m_{c}, 1H), 1.98 (m_{c}, 1H), 2.66-3.1 (m, 6H), 3.28 (m_{c}, 1H), 3.54 (m_{c}, 1H), 3.82 (m_{c}, 1H), 4.40 (m_{c}, 1H), 4.7-4.8 (m, 2H, unter D₂O-Signal), 4.85 (m_{c}, 1H), 6.8-6.93 (m, 2H), 7.20 (s, 1H), 7.25-7.47 (m, 3H). |
| **24** | 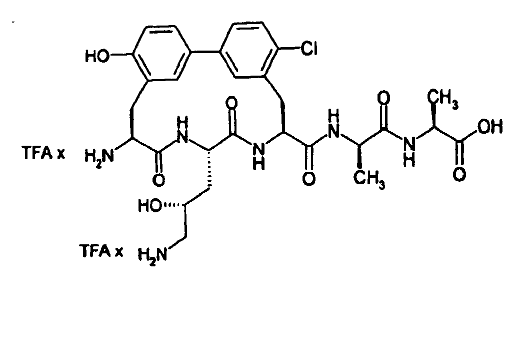 | 14 | LC-MS (Methode 3): Rₜ = 0.32 min. |
| | | aus Beispiel 27C10 | MS (EI): m/z = 633 (M-2TFA+H)⁺ |
| | | Ausbeute: 18% d. Th. | ¹H-NMR (400 MHz, D₂O): δ = 1.28 (d, 3H), 1.35 (d, 3H), 1.80 (m_{c}, 1H), 1.98 (m_{c}, 1H), 2.84 (m_{c}, 1H), 2.93-3.1 (m, 3H), 3.35 (m_{c}, 1H), 3.53 (m_{c}, 1H), 3.77 (m_{c}, 1H), 4.06 (q, 1H), 4.24 (q, 1H), 4.37 (m_{c}, 1H), 4.67-4.85 (m, 2H, unter D₂O-Signal), 6.90 (d, 1H), 7.0 (s, 1H), 7.31 (s, 1H), 7.35-7.52 (m, 3H). |

### Beispiel 25

### (8S,11S,14S)-14-Amino-11-[(2R)-3-amino-2-hydroxypropyl]-17-hydroxy-10,13-dioxo-9,12-diazatricyclo[14.3.1.1^{2,6}]henicosa-1(20),2(21),3,5,16,18-hexaen-8-carbonsäure-Di(hydrotrifluoracetat)

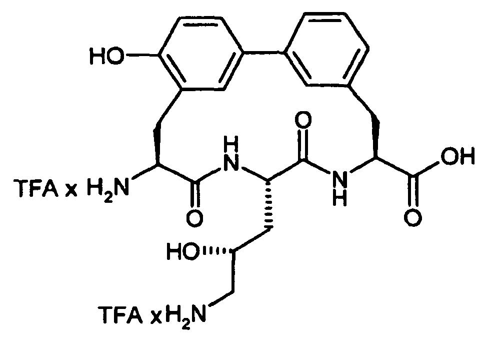

30 mg (0.03 mmol) der Verbindung aus Beispiel 23D werden in 50 ml Eisessig: Wasser:THF =4:1:1 suspendiert, mit 10 mg Pd/C (10%-ig) Katalysator versetzt und für 1 Tag bei RT unter Normaldruck hydriert. Das Rohprodukt wird durch HPLC (Kromasil 100C18, Laufmittel Acetonitril / 0.2%ige wässrige TFA 1:3) gereinigt.
Ausbeute: 25 mg (95% d. Th.)
LC-MS (Methode 14): Rₜ = 1.69 min
MS (EI): m/z = 457 (M-2TFA+H)⁺
¹H-NMR (400 MHz, D₂O): δ = 1.83 (m_{c}, 1H), 1.95 (m_{c}, 1H), 2.83 (m_{c}, 1H), 2.9-3.1 (m, 3H), 3.16 (m_{c}, 1H), 3.52 (m_{c}, 1H), 3.88 (m_{c}, 1H), 4.38 (m_{c}, 1H), 4.52 (m_{c}, 1H), 4.72 (m_{c}, 1H), 6.85-7.0 (m, 2H), 7.14 (d, 1H), 7.24-7.48 (m, 4H).

### Beispiel 26

### (8S,11S,14S)-14-Amino-N-(2-aminoethyl)-11-(3-aminopropyl)-17-hydroxy-5-methyl-10,13-dioxo-9,12-diazatricyclo[14.3.1.1^{2,6}]henicosa-1(20),2(21),3,5,16,18-hexaen-8-carboxamid-Trihydrochlorid

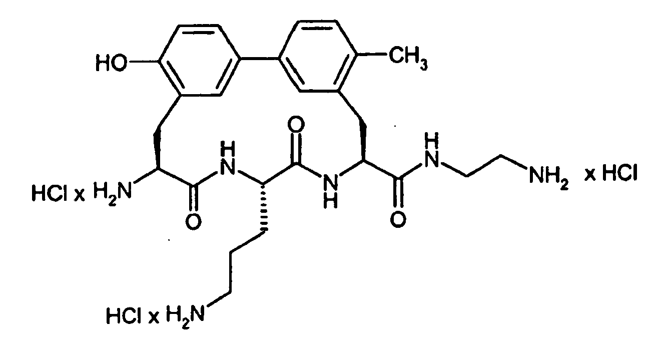

19 mg (0.020 mmol) der Verbindung aus Beispiel 27E werden in 12 ml Eisessig:Wasser :Ethanol = 4:1:1 suspendiert, mit 10 mg Pd/C (10%-ig) Katalysator versetzt und 2 Tage bei Raumtemperatur unter Normaldruck hydriert. Der Katalysator wird abfiltriert, das Filtrat mit 0.5 ml 0.1N Salzsäure versetzt, im Vakuum eingedampft und im Hochvakuum getrocknet. Man rührt mit 2.5 ml Dioxan:Methanol (4:1) aus und filtriert das Produkt ab.
Ausbeute: 2.9 mg (5% d. Th.)
LC-MS (Methode 2): Rₜ = 0.31 min.
MS (EI): m/z = 497 (M-3HCl+H)⁺

Analog zu den oben aufgeführten Vorschriften werden die in der folgenden Tabelle aufgeführten Beispiele 27 bis 34 aus den entsprechenden Edukten hergestellt:

| Beispiel-Nr. | Struktur | Hergestellt analog Beispiel-Nr. | Analytische Daten |
|---|---|---|---|
| **27** | 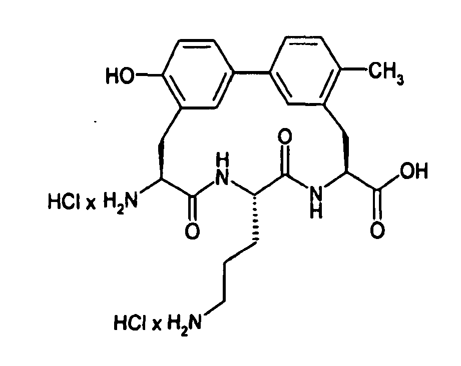 | 26 | LC-MS (Methode 5): Rₜ = 1.23 min. |
| | | Aus Beispiel 23E | MS (EI): m/z = 455 (M-2HCl+H)⁺ |
| | | Ausbeute: 72% d. Th. | |
| **28** | 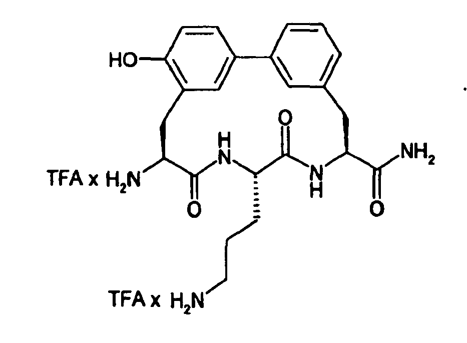 | 25 | MS (EI): m/z = 440 (M-2TFA+H)⁺ |
| | | Aus Beispiel 27F1 | ¹H-NMR (400 MHz, D₂O): δ = 1.5-1.8 (m, 4H), 2.8-3.2 (m, 5H), 3.54 (m_{c}, 1H), 4.42 (m_{c}, 1H), 4.65-4.8 (m, 2H, unter D₂O-Signal), 6.85-7.0 (m, 2H), 7.13 (d, 1H), 7.25-7.48 (m, 4H). |
| | | Ausbeute: 15% d. Th. | |
| **29** | | 25 | MS (EI): m/z = 497 (M-2TFA+H)⁺ |
| | 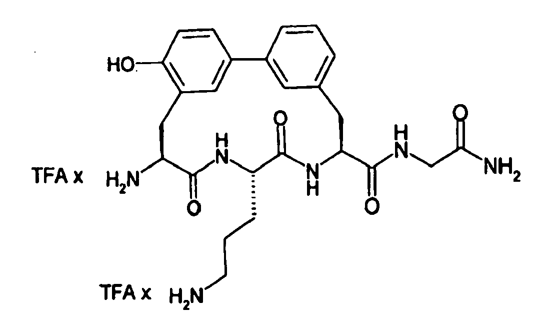 | aus Beispiel 27F2 | ¹H-NMR (400 MHz, D₂O): δ = 1.5-1.9 (m, 4H), 2.85-3.15 (m, 5H), 3.54 (m_{c}, 1H), 3.88 (m_{c}, 2H), 4.42 (m_{c}, 1H), 4.7 (m, 1H, unter D₂O-Signal), 4.78 (m_{c}, 1H), 6.85-7.0 (m, 2H), 7.13 (d, 1H), 7.25-7.48 (m, 4H). |
| | | Ausbeute: 68% d. Th. | |
| **30** | 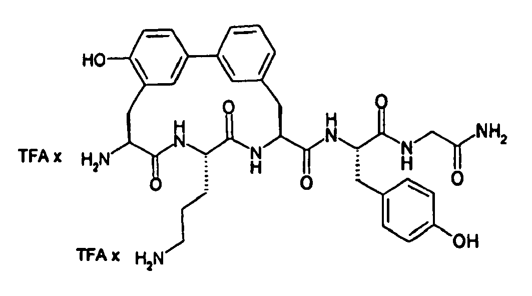 | 25 | MS (EI): m/z = 660 (M-2TFA+H)⁺ |
| | | aus Beispiel 27F4 | ¹H-NMR (400 MHz, D₂O): δ = 1.4-1.7 (m, 4H), 2.75-3.1 (m, 7H), 3.54 (m_{c}, 1H), 3.80 (m_{c}, 2H), 4.42 (m_{c}, 1H), 4.53 (m_{c}, 1H), 4.58 (m_{c}, 1H), 4.7 (m, 1H, unter D₂O-Signal), 6.73 (d, 2H), 6.85-7.0 (m, 2H), 7.03-7,13 (m, 3H), 7.22 (s, 1H), 7.28 (t, 1H), 7.33-7.46 (m, 2H). |
| | | Ausbeute: 21% d. Th. | |
| **31** | 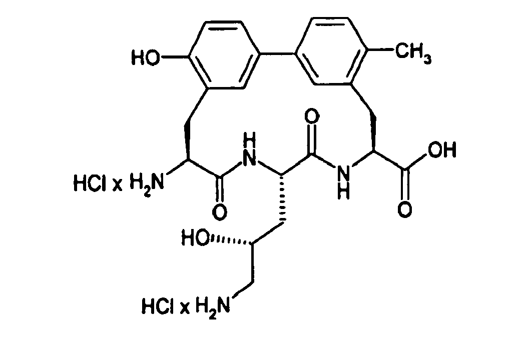 | 26 | LC-MS (Methode 5): Rₜ = 1.24 min. |
| | | aus Beispiel 23G | MS (EI): m/z = 471 (M-2HCl+H)⁺ |
| | | Ausbeute: 57% d. Th. | |
| **32** | 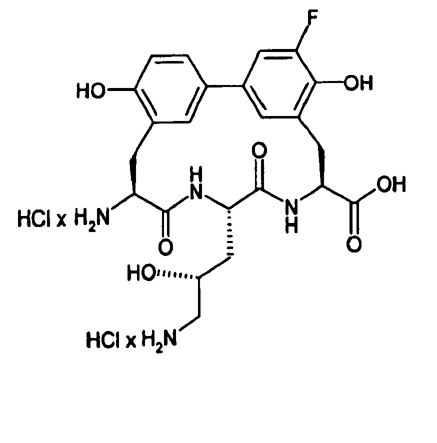 | 26 | LC-MS (Methode 5): Rₜ = 0.98 min. |
| | | aus Beispiel 23H | MS (EI): m/z =491 [M+H]⁺ |
| | | Ausbeute: 76% d. Th. | |
| **33** | 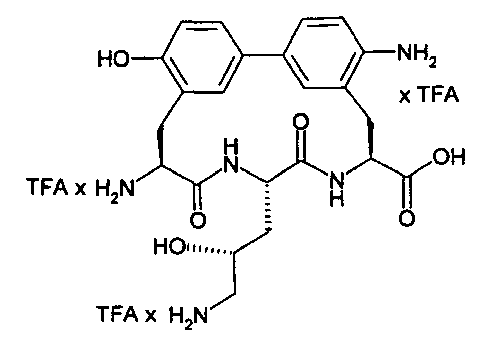 | 25 | MS (EI): m/z = 472 (M-3TFA+H)⁺ |
| | | aus Beispiel 231 Ausbeute: 49% | ¹H-NMR (400 MHz, D₂O): δ = 1.83 (m_{c}, 1H), 1.96 (m_{c}, 1H), 2.75-3.1 (m, 4H), 3.22 (m_{c}, 1H), 3.54 (m_{c}, 1H), 3.85 (m_{c}, 1H), 4.38 (m_{c}, 1H), 4.53 (m_{c}, 1H), 4.78 (m_{c}, 1H), 6.90 (d, 1H), 6.97 (s, 1H), 7.29 (d, 1H), 7.38-7.53 (m, 3H). |
| | | d. Th. | |
| **34** | 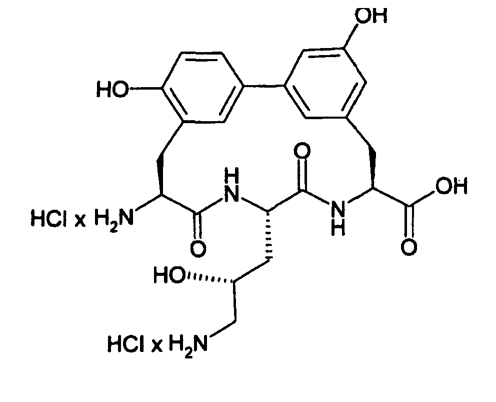 | 26 | LC-MS (Methode 8): Rₜ = 1.42 min. |
| | | aus Beispiel 23J | MS (EI): m/z = 473 (M-2HCl+H)⁺ |
| | | Ausbeute: 98% d. Th. | |

### Beispiel 35

### (8S,11S,14S)-14-Amino-11-(3-aminopropyl)-17-hydroxy-10,13-dioxo-9,12-diazatricyclo[14.3.1.1^{2,6}]henicosa-1(20),2(21),3,5,16,18-hexaen-8-carbonsäure-Dihydrochlorid

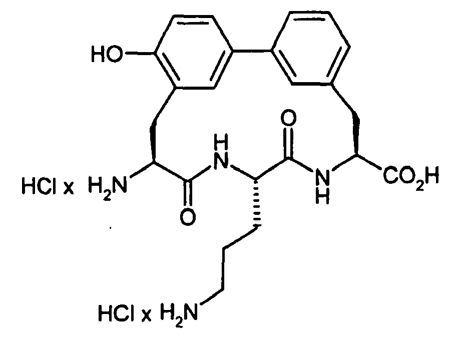

Die Herstellung erfolgt analog zu Beispiel 1 aus 140 mg (0.16 mmol) der Verbindung aus Beispiel 23F in 150 ml Eisessig / Wasser / Ethanol = 4/1/1 mit 50 mg Pd/C-Katalysator (10%ig).
Ausbeute: 80 mg (95% d. Th.)
LC-MS (Methode 14): Rₜ = 1.53 min.
MS (EI): m/z = 441 (M-2HCl+H)⁺
¹H-NMR (400 MHz, D₂O): δ = 1.79 (m_{c}, 3H), 1.94 (m_{c}, 1H), 2.8-3.3 (m, 5H), 3.63 (m_{c}, 1H), 4.51 (m_{c}, 1H), 4.65-4.85 (m, 2H), 7.0 (d, 1H), 7.05 (s, 1H), 7.25 (d, 1H), 7.35-7.54 (m, 4H).

### Beispiel 36

### (8S,11S,14S)-14-Amino-N-(2-aminoethy)-11-[(2R)-3-amino-2-hydroxypropyl]-17-hydroxy-10,13-dioxo-9,12-diazatricyclo[14.3.1.1^{2,6}]henicosa-1(20),2(21),3,5,16,18-hexaen-8-carboxamid-Tri(hydrotrifluoracetat)

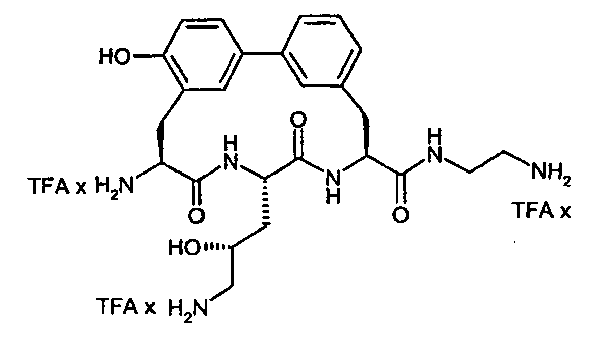

43 mg (0.04 mmol) der Verbindung aus Beipiel 27C11 werden in 23 ml Eisessig / Wasser / Ethanol (4/1/1) suspendiert, mit 8 mg Pd/C-Katalysator (10%ig) versetzt und 24 h bei RT unter Normaldruck hydriert. Der Katalysator wird über einen Membranfilter abgetrennt und das Filtrat wird im Vakuum eingeengt. Das Rohprodukt wird in 0.1 ml 4N Dioxan / Chlorwasserstoff-Lösung auf genommen und für 2 h bei RT gerührt. Das Lösungsmittel wird im Vakuum eingedampft und das Rohprodukt wird durch HPLC (Kromasil 100C18, Laufmittel Acetonitril / 0.2%ige wässrige TFA, Gradient) gereinigt.
Ausbeute: 6.1 mg (29% d. Th.)
LC-MS (Methode 17): Rₜ = 2.19 min
MS (EI): m/z = 499 (M-3TFA+H)⁺
¹H-NMR (400 MHz, D₂O): δ = 1.87 (m_{c}, 1H), 1.97 (m_{c}, 1H), 2.86 (m_{c}, 1H), 3.0-3.15 (m, 6H), 3.50 (m_{c}, 2H), 3.55 (m_{c}, 1H), 3.85 (m_{c}, 1H), 4.42 (m_{c}, 1H), 4.73 (m_{c,} 1H), 4.85 (m_{c}, 1H), 6.94 (d, 1H), 6.98 (s, 1H), 7.13 (s, 1H). 7.25-7.37 (m, 2H), 7.4-7.48 (m, 2H).

Analog zu den oben aufgeführten Vorschriften werden die in der folgenden Tabelle aufgeführten Beispiele 37 und 38 aus den entsprechenden Edukten hergestellt:

| Beispiel-Nr. | Struktur | Hergestellt analog Beispiel-Nr. | Analytische Daten |
|---|---|---|---|
| **37** | 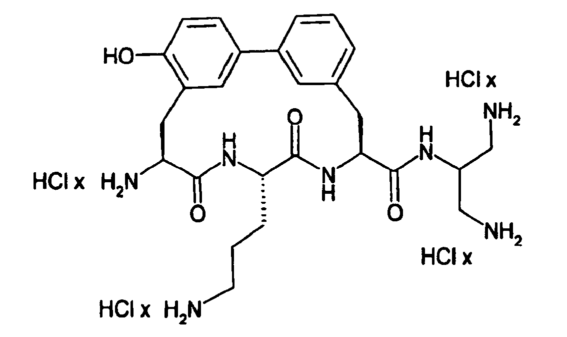 | 5 | LC-MS (Methode 2): Rₜ = 0.24 min. |
| | | aus Beispiel 29F1 | MS (EI): m/z = 512 (M-4HCl+H)⁺ |
| **38** | 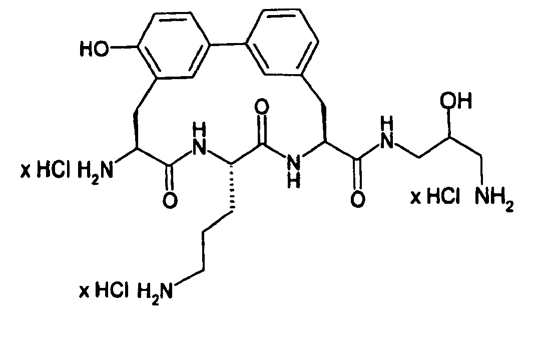 | 5 | LC-MS (Methode 17): Rₜ = 2.32 min. |
| | | aus Beispiel 29F2 | MS (EI): m/z = 513 (M-3HCl+H)⁺ |

### Beispiel 39

### (8S,11S,14S)-14-Amino-N-(2-aminoethyl)-11-(3-aminopropyl)-5-fluor-17-hydroxy-9-methyl-10,13-dioxo-9,12-diazatricyclo[14.3.1.1^{2,6}]henicosa-1(20),2(21),3,5,16,18-hexaen-8-carboxamid-Tri(hydrotrifluoracetat)

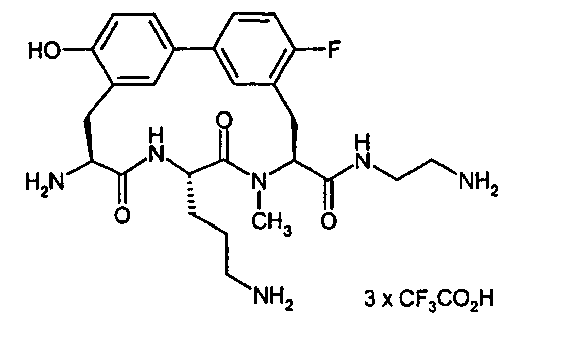

Es werden 25 mg (0.029 mmol) Benzyl-{3-[(8*S*,11*S*,14*S*)-8-{[(2-aminoethyl)amino]carbonyl}-17-(benzyloxy)-14- {[(benzyloxy)carbonyl]amino}-5-fluor-9-methyt-10,13-dioxo-9,12-diazatri*-cyclo[14.3.1.1^{2,6}]henicosa-1(20),2(21),3,5,16,18-hexaen-11-yl]propyl}carbamat (Beispiel 30O) in ein Gemisch aus 5 ml Essigsäure/Wasser/Ethanol (4:1:1) gegeben. Dazu gibt man 10 mg Palladium auf Aktivkohle (10%ig) und hydriert anschließend 15 h bei RT und Normaldruck Das Reaktionsgemisch wird Ober vorgewaschenem Kieselgur filtriert, mit Ethanol gewaschen und das Filtrat im Vakuum einrotiert. Der Rückstand wird mit 0.1N Salzsäure versetzt und im Vakuum eingeengt. Der zurückgebliebene Feststoff wird im Hochvakuum getrocknet. Das Trihydrochlorid wird durch präparative HPLC (Kromasil 100C18, Laufmittel Acetonitril/0.2% wässrige Trifluoressigsäure 5:95 → 95:5) in das Tri(hydrotrifluoracetat) überführt.
Ausbeute: 11 mg (45% d. Th.)
LC-MS (Methode 17): Rₜ = 2.18 min.
MS (EI): m/z = 515 (M-3CF₃CO₂H+H)⁺.
¹H-NMR (400 MHz, D₂O): δ = 1.56-1.94 (m, 8H), 2.85-3.60 (m, 9H), 2.92 (s, 3H), 5.70 (m, 1H), 6.90 (d, 1H), 6.96 (s, 1H), 7.10-7.20 (m, 2H), 7.40-7.60 (m, 2H).

### Beispiel 40

### (8S,11S,14S)-14-Amino-11-(3-aminopropyl)-5-fluor-17-hydroxy-9-methyl-10,13-dioxo-9,12-diazatricyclo[14.3.1.1^{2,6}]henicosa-1(20),2(21),3,5,16,18-hexaen-8-carbonsäure-Dihydrochlorid

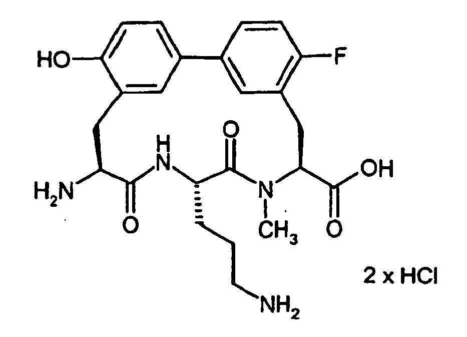

Es werden 275 mg (0.331 mmol) (8*S*,11*S*,14*S*)-17-(Benzyloxy)-14-{[(benzyloxy)carbonyl]-amino}-11-(3-{[(benzyloxy)carbonyl]amino}propyl)-5-fluor-9-methyl-10,13-dioxo-9,12-diazatricyclo[14.3.1.1^{2,6}]yhenicosa-1(20),2(21),3,5,16,18-hexaen-8-carbonsäure (Beispiel 26O) in ein Gemisch aus 90 ml Essigsäure/Wasser/Ethanol (4:1:1) gegeben. Dazu gibt man 300 mg Palladium auf Aktivkohle (10%ig) und hydriert anschließend 15 h bei RT und Normaldruck. Das Reaktionsgemisch wird über vorgewaschenem Kieselgur filtriert, mit Ethanol gewaschen und das Filtrat im Vakuum einrotiert. Der Rückstand wird mit 0.1N Salzsäure versetzt und eingeengt. Der zurückgebliebene Feststoff wird im Hochvakuum getrocknet.
Ausbeute: quant.
LC-MS (Methode 17): Rₜ = 2.47 min.
MS (EI): m/z = 473 (M-2HCl+H)⁺.

### Beispiel 41

### (8S,11S,14S)-14-Amino-11-(3-aminopropyl)-5-chlor-N-[(2S)-2,5-diaminopentyl]-17-hydroxy-9-methyl-10,13-dioxo-9,12-diazatricyclo[14.3.1.1^{2,6}]henicosa-1(20),2(21),3,5,16,18-hexaen-8-carboxamid-Tetrahydrochlorid

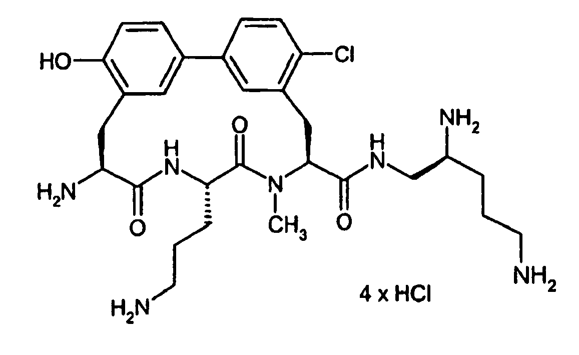

11.4 mg (0.012 mmol) der Verbindung aus Beispiel 29M2 werden bei RT in 0.05 ml Dioxan gelöst und mit 0.175 ml 4N Chlorwasserstoff-Dioxan-Lösung versetzt und 2 h gerührt. Man dampft alles im Vakuum zur Trockne ein und trocknet im Hochvakuum bis zur Gewichtskonstanz.
Ausbeute: 7.9 mg (93% d. Th.)
MS (ESI): m/z = 588 (M-4HCl+H)⁺
¹H-NMR (400 MHz, D₂O): δ = 1.55-1.95 (m, 8H), 2.85 (s, 3H), 2.9-3.1 (m, 6H), 3.25-3.75 (m, 7H), 4.45 (m_{c}, 1H), 4.78 (m_{c}, 1H), 5.70 (m_{c}, 1H), 6.92 (d, 1H), 7.0 (s, 1H), 7.15 (s, 1H), 7.45 (d, 1H), 7.51 (d, 1H), 7.57 (d, 1H).

### Beispiel 42

### (8S,11S,14S)-14-Amino-11-(3-aminopropyl)-N-{2-[bis(2-aminoethyl)amino]ethyl}-5-chlor-17-hydroxy-9-methyl-10,13-dioxo-9,12-diazatricyclo[14.3.1.1^{2,6}]henicosa-1(20),2(21),3,5,16,18-hexaen-8-carboxamid-Penta(hydrotrifluoracetat)

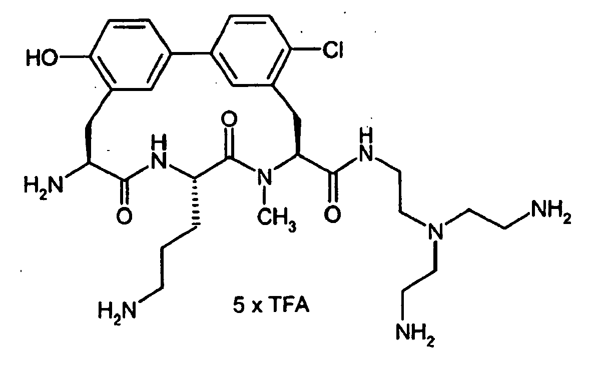

110 mg (0.113 mmol) der Verbindung aus Beispiel 30Ml werden in 2 ml einer 33%igen Bromwasserstoff-Lösung in Essigsäure gelöst und 45 min bei RT gerührt. Anschließend wird im Vakuum eingeengt und das Rohprodukt wird durch HPLC (Kromasil 100C18, Laufmittel Acetonitril/0.2%ige wässrige Trifluoressigsäure, Gradient) gereinigt.
Ausbeute: 64.1 mg (53% d. Th.)
MS (ESI): m/z = 617 (M-5TFA+H)⁺
¹H-NMR (400 MHz, D₂O): δ = 1.57-1.74 (m, 3H), 1.78-1.91 (m, 1H), 2.68 (t, 2H), 2.79 (m_{c}, 4H), 2.88 (s, 3H), 2.92 (m_{c}, 2H), 3.0-3.1 (m, 5H), 3.15-3.5 (m, 4H), 3.57 (m_{c}, 1H), 4.44 (m_{c}, 1H), 4.80 (m_{c}, 1H), 5.63 (m_{c}, 1H), 6.91 (d, 1H), 6.98 (s, 1H), 7.13 (s, 1H), 7.43 (d, 1H), 7.50 (d, 1H), 7.56 (d, 1H).

### Beispiel 43

### (8S,11S,14S)-5,14-Diamino-N-(2-aminoethyl)-11-(3-aminopropyl)-17-hydroxy-10,13-dioxo-9,12-diazatricyclo[14.3.1.1^{2,6}]henicosa-1(20),2(21),3,5,16,18-hexaen-8-carboxamid-Tetrahydrochlorid

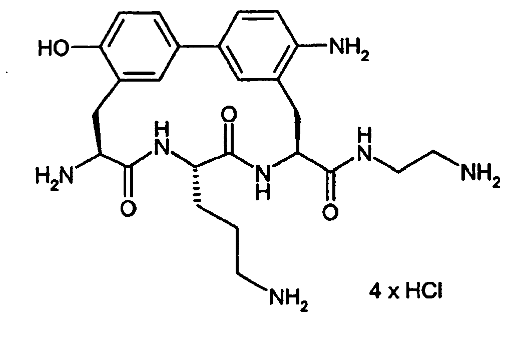

Es werden 30 mg der Verbindung aus Beispiel 30P in ein Gemisch aus 28 ml Essigsäure/Wasser/Ethanol (4:1:1) gegeben. Dazu gibt man 16 mg Palladium auf Aktivkohle (10%ig) und hydriert anschließend 48 h bei RT und Normaldruck. Das Reaktionsgemisch wird über vorgewaschenem Kieselgur filtriert, mit Ethanol gewaschen und das Filtrat im Vakuum einrotiert. Der Rückstand wird mit 1.2 ml 0.1N Salzsäure versetzt und erneut im Vakuum eingeengt. Der zurückgebliebene Feststoff wird im Hochvakuum getrocknet.
Ausbeute: 21 mg (96% d. Th.)
LC-MS (Methode 17): Rₜ = 0.73 min.
MS (EI): m/z = 498 (M-4HCl+H)⁺.
¹H-NMR (400 MHz, D₂O): δ = 1.5-1.9 (m, 4H), 2.85-3.2 (m, 7H), 3.3-3.6 (m, 3H), 4.42 (m_{c}, 1H), 4.6-4.7 (m, 1H, unter D₂O), 4.87 (m_{c}, 1H), 6.90 (d, 1H), 7.01 (s, 1H), 7.23 (d, 1H), 7.45 (s, 1H), 7.45-7.55 (m, 2H).

Das Trihydrochlorid wird durch präparative HPLC (Kromasil 100C18, Laufmittel Acetonitril/0.2%ige wässrige Trifluoressigsäure 5:95 → 95:5) in das Tri(hydrotrifluoracetat) überführt.

| Beispiel-Nr. | Struktur | Hergestellt analog Beispiel-Nr. | Analytische Daten |
|---|---|---|---|
| **44** | 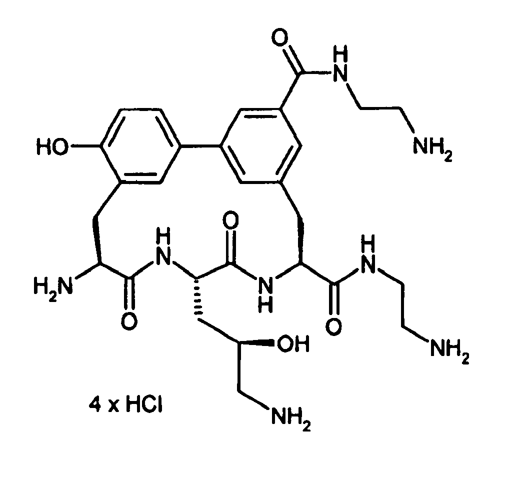 | 5 | LC-MS (Methode 17): Rₜ = 1.88 min. |
| | | aus Beispiel | MS (ES): m/z = 585 (M-4HCl+H)⁺ |
| | | 29N | |
| | | | ¹H-NMR (400 MHz, D₂O): δ = 1.8-2.0 (m, 2H), 2.75 (m_{c}, 1H), 2.95-3.23 (m, 6H), 3.4-3.75 (m, 6H), 3.86 (m_{c}, 1H), 4.40 (m_{c}, 1H), 4.7 (m, 1H, unter D₂O), 4.83 (m_{c}, 1H), 6.9-7.0 (m, 2H), 7.4-7.55 (m, 3H), 7.78 (s, 1H). |
| **45** | 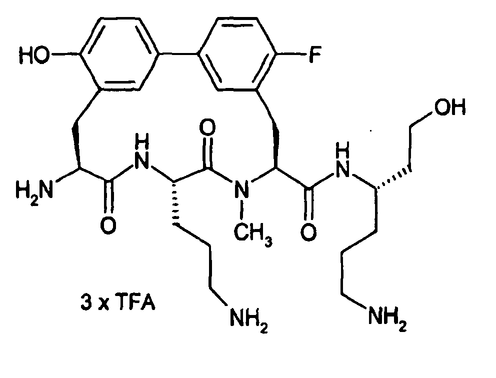 | 39 | LC-MS (Methode 17): Rₜ = 2.18 min. |
| | | aus Beispiel | MS (EI): m/z = 587 (M-3 TFA+H)⁺ |
| | | 2901 | |
| | | | ¹H-NMR (400 MHz, D₂O): δ = 1.53-1.87 (m, 10H), 2.64-3.62 (m, 12H), |
| | | | 4.53 (m, 1H), 4.92-4.97 (m, 1H), 5.66 (dd, 1H), 6.94 (d, 1H), 6.99 (s, 1H), 7.11-7.19 (m, 2H), 7.48-7.58 (m, 2H). |
| **46** | 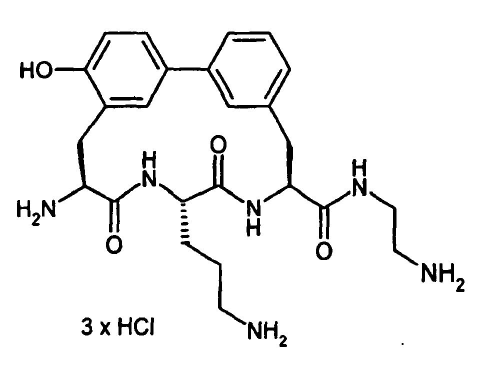 | 5 | LC-MS (Methode 17): Rₜ = 2.04 min. |
| | | aus Beispiel | MS (EI): m/z = 486 (M.3HCl+H)⁺ |
| | | 29F3 | |
| | | | ¹H-NMR (400 MHz, D₂O): δ = 1.55-1.9 (m, 4H), 2.93 (m_{c}, 2H), 2.97-3.17 (m, 5H), 3.45-3.6 (m, 3H), 4.41 (m_{c}, 1H), 4.6-4.8 (m, 2H unter D₂O), 6.85-6.97 (m, 2H), 7.12 (m_{c}, 1H), 7.24-7.36 (m, 2H), 7.37-7.48 (m, 2H). |
| **47** | 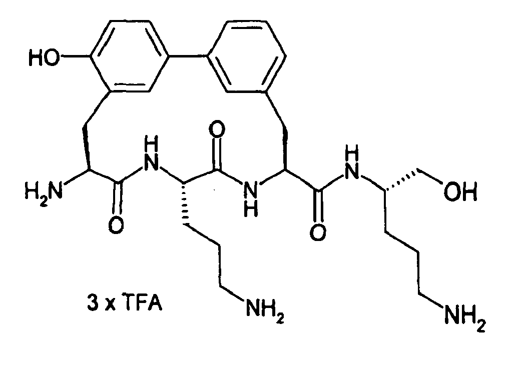 | 39 | LC-MS (Methode 17): Rₜ = 1.85 min. |
| | | aus Beispiel | MS (EI): m/z = 541 (M-3TFA+H)⁺ |
| | | 29F4 | |
| | | | ¹H-NMR (400 MHz, D₂O): δ = 1.5-1.9 (m, 8H), 2.85-3.15 (m, 7H), 3.45-3.9 (m, 4H), 4.41 (m_{c}, 1H), 4.6-4.8 (m, 2H unter D₂O), 6.85-6.97 (m, 2H), 7.12 (m_{c}, 1H), 7.22-7.48 (m, 4H). |
| **48** | 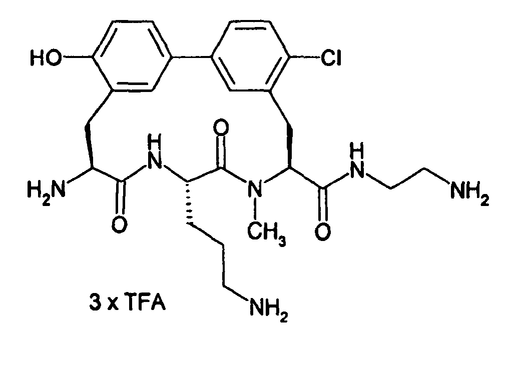 | 45 | LC-MS (Methode 17): Rₜ = 2.30 min. |
| | | aus Beispiel | MS (EI): m/z = 531 (M-3TFA+H)⁺ |
| | | 30M2 | |
| | | | ¹H-NMR (400 MHz, D₂O): δ =1.5-1.9 (m, 4H), 2.87 (s, 3H), 2.95 (m_{c}, 2H), 3.03-3.15 (m, 3H), 3.26 (m_{c}, 1H), 3.38-3.6 (m, 4H), 4.43 (m_{c}, 1H), 4.88 (m_{c}, 1H), 5.66 (m_{c}, 1H), 6.87 (d, 1H), 6.98 (s, 1H), 7.12 (s, 1H), 7.38-7.6 (m, 3H). |
| **49** | 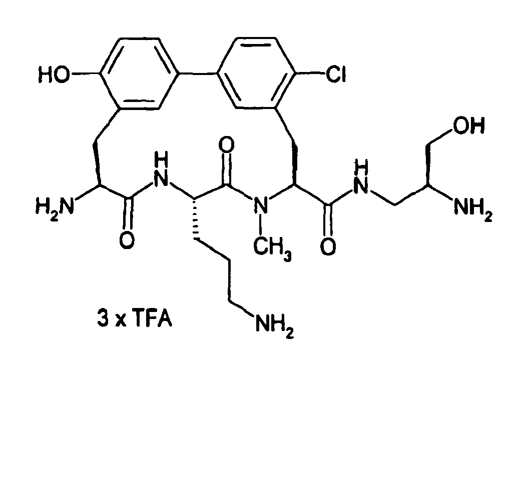 | 39 | LC-MS (Methode 17): Rₜ = 2.40 min. |
| | | aus Beispiel | MS (EI): m/z = 561 (M-3TFA+H)⁺ |
| | | 29M1 | |
| | | | ¹H-NMR (400 MHz, D₂O): δ = 1.58-1.75 (m, 3H), 1.78-1.92 (m, 1H), 2.85 (s, 3H), 2.94 (m_{c}, 2H), 3.05 (m_{c}, 1H), 3.28 (m_{c}, 1H), 3.38-3.68 (m, 7H), 3.75 (m_{c}, 1H), 4.44 (m_{c}, 1H), 4.80 (m_{c}, 1H), 5.70 (m_{c}, 1H), 6.90 (d, 1H), 7.0 (s, 1H), 7.15 (s, 1H), 7.43 (d, 1H), 7.50 (d, 1H), 7.56 (d, 1H). |
| **50** | 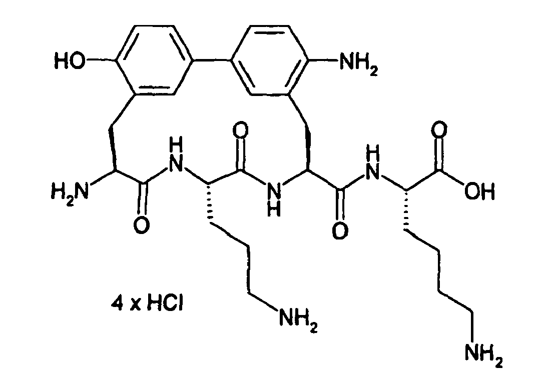 | 5 aus Beispiel 29P1 | MS (ESI): m/z = 584 (M-4HCl+H)^{+ 1}H-NMR (400 MHz, D₂O): δ = 1.5-1.9 (m, 4H), 2.6-3.1 (m, 8H), 3.15-3.35 (m, 3H), 3.45-3.75 (m, 3H), 3.9 (m_{c}, 1H), 4.40 (m_{c}, 1H), 4.6-4.7 (m, 1H, unter D₂O), 4.78 (m_{c}, 1H), 6.85-6.93 (m, 2H), 6.96 (s, 1H), 7.25 (s, 1H), 7.33 (d, 1H), 7.40 (d, 1H) |
| **51** | 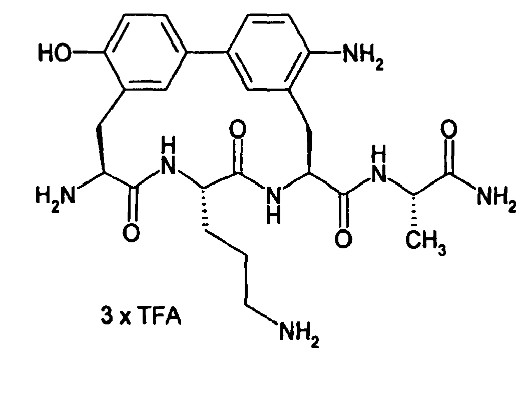 | 39 aus Beispiel 29P2 | LC-MS (Methode 17): Rₜ = 1.90 min. MS (EI): m/z = 526 (M-3TFA+H)⁺ |
| **52** | 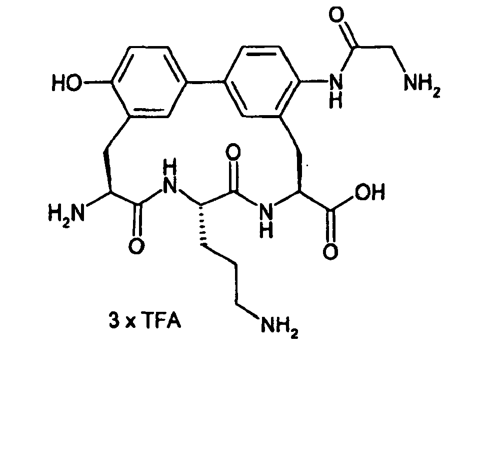 | 39 | LC-MS (Methode 17): Rₜ = 1,37 min. |
| | | aus Beispiel | MS (EI): m/z = 513 (M-3TFA+H)⁺ |
| | | 28P2 | |
| | | | ¹H-NMR (400 MHz, D₂O): δ = 1.63-1.95 (m, 4H), 2.85 (m_{c},1H), 2.98 (m_{c}, 2H), 3.10 (m_{c}, 1H), 3.25 (m_{c}, 1H), 3.58 (m_{c}, 1H), 4.04 (s, 2H), 4.45 (m_{c}, 1H), 4.57 (m_{c}, 1H), 4.6-4.8 (m, 1H, unter D₂O), 6.96 (d, 1H), 7.07 (s, 1H), 7.30 (d, 1H), 7.4-7.6 (m, 3H). |

### B. Bewertung der physiologischen Wirksamkeit

### Verwendete Abkürzungen:

- AMP: Adenosinmonophosphat
- ATP: Adenosintriphosphat
- BHI Medium: Brain heart infusion medium
- CoA: Coenzym A
- DMSO: Dimethylsulfoxid
- DTT: Dithiothreitol
- EDTA: Ethylendiamintetraessigsäure
- KCl: Kaliumchlorid
- KH₂PO₄: Kaliumdihydrogenphosphat
- MgSO₄: Magnesiumsulfat
- MHK: Minimale Hemmkonzentration
- MTP: Mikrotiterplatte
- NaCl: Natriumchlorid
- Na₂HPO₄: Dinatriumhydrogenphosphat
- NH₄Cl: Ammoniumchlorid
- NTP: Nukleotidtriphosphat
- PBS: Phosphat Buffered Saline
- PCR: Polymerase Chain Reaction
- PEG: Polyethylenglykol
- PEP: Phosphoenolpyruvat
- Tris: Tris[hydroxymethyl]aminomethan

Die *in vitro*-Wirkung der erfindungsgemäßen Verbindungen kann in folgenden Assays gezeigt werden:

### In vitro Transkription-Translation mit E. coli Extrakten

Zur Herstellung eines S30-Extraktes werden logarithmisch wachsende *Escherichia coli* MRE 600 (M. Müller; University Freiburg) geerntet, gewaschen und wie beschrieben für den *in vitro* Transkriptions-Translations-Test eingesetzt (Müller, M. and Blobel, G. Proc Natl Acad Sci U S A (1984) 81, pp.7421-7425).

Dem Reaktionsmix des *in vitro* Transkriptions-Translations-Tests werden zusätzlich 1 µl cAMP (11.25 mg/ml) je 50 µl Reaktionsmix zugegeben. Der Testansatz beträgt 105 µl, wobei 5 µl der zu testenden Substanz in 5%igem DMSO vorgelegt werden. Als Transkriptionsmatrize werden 1 µg/100µl Ansatz des Plasmides pBESTLuc (Promega, Deutschland) verwendet. Nach Inkubation für 60 min bei 30°C werden 50 µl Luziferinlösung (20 mM Tricine, 2.67 mM MgSO₄, 0.1 mM EDTA, 33.3 mM DTT pH 7.8, 270 µM CoA, 470 µM Luziferin, 530 µM ATP) zugegeben und die entstehende Biolumineszenz für 1 Minute in einem Luminometer gemessen. Als IC₅₀ wird die Konzentration eines Inhibitors angegeben, die zu einer 50%igen Inhibition der Translation von Firefly Luziferase führt.

### In vitro Transkription-Translation mit S. aureus Extrakten

### Konstruktion eines S. aureus Luziferase Reporterplasmids

Zur Konstruktion eines Reporterplasmids, welches in einem *in vitro* Transkriptions-Translations-Assay aus *S. aureus* verwendet werden kann, wird das Plasmid pBESTluc (Promega Corporation, USA) verwendet. Der in diesem Plasmid vor der Firefly Luziferase vorhandene *E. coli tac* Promoter wird gegen den *capA1* Promoter mit entsprechender Shine-Dalgarno Sequence aus *S. aureus* ausgetauscht. Dazu werden die Primer CAPFor 5'-CGGCC-AAGCTTACTCGGATCCAGAGTTTGCAAAATATACAGGGGATTATATATAATGGAAAAC AAGAAAGGAAAATAGGAGGTTTATATGGAAGACGCCA-3' und CAPRev 5'-GTCATCGTCGGGAAGACCTG-3' verwendet. Der Primer CAPFor enthält den *capA1* Promotor, die Ribosomenbindestelle und die 5'-Region des Luziferase Gens. Nach PCR unter Verwendung von pBESTluc als Template kann ein PCR-Produkt isoliert werden, welches das Firefly Luziferase Gen mit dem fusionierten *capA1* Promotor enthält. Dieses wird nach einer Restriktion mit ClaI und HindIII in den ebenfalls mit ClaI und HindIII verdauten Vektor pBESTluc ligiert. Das entstandene Plasmid pla kann in *E. coli* repliziert werden und als Template im *S. aureus in vitro* Transkriptions-Translations-Test verwendet werden.

### Herstellung von S30 Extrakten aus S. aureus

Sechs Liter BHI Medium werden mit einer 250 ml Übemachtkultur eines S. aureus Stammes inokuliert und bei 37°C bis zu einer OD600nm von 2-4 wachsen gelassen. Die Zellen werden durch Zentrifugation geerntet und in 500 ml kaltem Puffer A (10 mM Tris-acetat, pH 8.0, 14 mM Magnesiumacetat, 1 mM DTT, 1M KCl) gewaschen. Nach erneutem Abzentrifugieren werden die Zellen in 250 ml kaltem Puffer A mit 50 mM KCl gewaschen und die erhaltenen Pellets bei -20°C für 60 min eingefroren. Die Pellets werden in 30 bis 60 min auf Eis aufgetaut und bis zu einem Gesamtvolumen von 99 ml in Puffer B (10 mM Tris-acetat, pH 8.0, 20 mM Magnesiumacetat, 1 mM DTT, 50 mM KCl) aufgenommen. Je 1.5 ml Lysostaphin (0.8 mg/ml) in Puffer B werden in 3 vorgekühlte Zentrifugenbecher vorgelegt und mit je 33 ml der Zellsuspension vermischt. Die Proben werden für 45 bis 60 min bei 37°C unter gelegentlichem Schütteln inkubiert, bevor 150 µl einer 0.5M DTT Lösung zugesetzt werden. Die lysierten Zellen werden bei 30.000 x g 30 min bei 4°C abzentrifugiert. Das Zellpellet wird nach Aufnahme in Puffer B unter den gleichen Bedingungen nochmals zentrifugiert und die gesammelten Überstände werden vereinigt. Die Überstände werden nochmals unter gleichen Bedingungen zentrifugiert und zu den oberen 2/3 des Überstandes werden 0.25 Volumen Puffer C (670 mM Tris-acetat, pH 8.0, 20 mM Magnesiumacetat, 7 mM Na₃-Phosphoenolpyruvat, 7 mM DTT, 5.5 mM ATP, 70 µM Aminosäuren (complete von Promega), 75 µg Pyruvatkinase (Sigma, Deutschland))/ml gegeben. Die Proben werden für 30 min bei 37°C inkubiert. Die Überstände werden über Nacht bei 4°C gegen 21 Dialysepuffer (10 mM Tris-acetat, pH 8.0, 14 mM Magnesiumacetat, 1 mM DTT, 60 mM Kaliumacetat) mit einem Pufferwechsel in einem Dialyseschlauch mit 3500 Da Ausschluss dialysiert. Das Dialysat wird auf eine Proteinkonzentration von etwa 10 mg/ml konzentriert, indem der Dialyseschlauch mit kaltem PEG 8000 Pulver (Sigma, Deutschland) bei 4°C bedeckt wird. Die S30 Extrakte können aliquotiert bei -70°C gelagert werden.

### Bestimmung der IC₅₀ im S. aureus in vitro Transcriptions-Translations-Assay

Die Inhibition der Proteinbiosynthese der Verbindungen kann in einem *in vitro* Transkriptions-Translations-Assay gezeigt werden. Der Assay beruht auf der zellfreien Transkription und Translation von Firefly Luziferase unter Verwendung des Reporterplasmids p1a als Template und aus *S*. *aureus* gewonnenen zellfreien S30 Extrakten. Die Aktivität der entstandenen Luziferase kann durch Lumineszenzmessung nachgewiesen werden.

Die Menge an einzusetzenden S30 Extrakt bzw. Plasmid p1a muss für jede Präparation erneut ausgetestet werden, um eine optimale Konzentration im Test zu gewährleisten. 3 µl der zu testenden Substanz gelöst in 5 % DMSO werden in eine MTP vorgelegt. Anschließend werden 10 µl einer geeignet konzentrierten Plasmidlösung p1a zugegeben. Anschließend werden 46 µl eines Gemisches aus 23 µl Premix (500 mM Kaliumacetat, 87.5 mM Tris-acetat, pH 8.0, 67.5 mM Ammoniumacetat, 5 mM DTT, 50 µg Folsäure/ml, 87.5 mg PEG 8000/ml, 5 mM ATP, 1.25 mM je NTP, 20 µM je Aminosäure, 50 mM PEP (Na₃-Salz), 2.5 mM cAMP, 250 µg je *E. coli* tRNA/ml) und 23 µl einer geeigneten Menge *S*. *aureus* S30 Extrakt zugegeben und vermischt. Nach Inkubation für 60 min bei 30°C werden 50 µl Luziferinlösung (20 mM Tricine, 2.67 mM MgSO₄, 0.1 mM EDTA, 33.3 mM DTT pH 7.8, 270 µM CoA, 470 µM Luziferin, 530 µM ATP) und die entstehende Biolumineszenz für 1 min in einem Luminometer gemessen. Als IC₅₀ wird die Konzentration eines Inhibitors angegeben, die zu einer 50 %igen Inhibition der Translation von Firefly Luziferase führt.

### Bestimmung der Minimalen Hemmkonzentration (MHK)

Die minimale Hemmkonzentration (MHK) ist die minimale Konzentration eines Antibiotikums, mit der ein Testkeim in seinem Wachstum über 18-24 h inhibiert wird. Die Hemmstoffkonzentration kann dabei nach mikrobiologischen Standardverfahren bestimmt werden (siehe z.B. The National Committee for Clinical Laboratory Standards. Methods for dilution antimicrobial susceptibility tests for bacteria that grow aerobically; approved standard-fifth edition. NCCLS document M7-A5 [ISBN 1-56238-394-9]. NCCLS, 940 West Valley Road, Suite 1400, Wayne, Pennsylvania 19087-1898 USA, 2000). Die MHK der erfindungsgemäßen Verbindungen wird im Flüssigdilutionstest im 96er-Mikrotiter-Platten-Maßstab bestimmt. Die Bakterienkeime werden in einem Minimalmedium (18.5 mM Na₂HPO₄, 5.7 mM KH₂PO₄, 9.3 mM NH₄Cl, 2.8 mM MgSO₄, 17.1 mM NaCl, 0.033 µg/ml Thiaminhydrochlorid, 1.2 µg/ml Nicotinsäure, 0.003 µg/ml Biotin, 1% Glucose, 25 µg/ml von jeder proteinogenen Aminosäure mit Ausnahme von Phenylalanin; [H.-P. Kroll; unver-öffentlicht]) unter Zusatz von 0.4 % BH-Bouillon kultiviert (Testmedium). Im Fall von *Enterococcus faecium* L4001 wird dem Testmedium hitzeinaktiviertes fötales Kälberserum (FCS; GibcoBRL, Deutschland) in einer Endkonzentration von 10% zugesetzt. Übernachtkulturen der Testkeime werden auf eine OD₅₇₈ von 0.001 (im Falle der Enterokokken auf 0.01) in frisches Testmedium verdünnt und 1:1 mit Verdünnungen der Testsubstanzen (Verdünnungsstufen 1:2) in Testmedium inkubiert (200 µl Endvolumen). Die Kulturen werden bei 37°C für 18-24 Stunden inkubiert; Enterokokken in Gegenwart von 5 % CO₂.

Die jeweils niedrigste Substanzkonzentration, bei der kein sichtbares Bakterienwachstum mehr auftrat, wird als MHK definiert. Die MHK-Werte in µM einiger erfindungsgemäßer Verbindungen gegenüber einer Reihe von Testkeimen sind in der nachstehenden Tabelle beispielhaft aufgeführt. Die Verbindungen zeigen eine abgestufte antibakterielle Wirkung gegen die meisten der Testkeime.

**Tabelle A (mit Vergleichsbeispiel Biphenomycin B)**

| **Beispiel- Nr.** | **MHK *S. aureus* 133** | **MHK *S. aureus* T17** | **MHK *E. faecium* L4001** | **IC₅₀ *S. aureus* 133 Translation** |
|---|---|---|---|---|
| **9** | 0.35 | 0.7 | 11 | 0.3 |
| **12** | 0.16 | 0.6 | 20 | 0.8 |
| **36** | 0.4 | 0.8 | >25 | 0.36 |
| **Biphenomycin B** | 0.1 | >25 | >25 | 1.5 |

| | | | | |
|---|---|---|---|---|
| Alle Konzentrationsangaben in µM. **Alternative Bestimmungsmethode der Minimalen Hemmkonzentration (MHK)** | | | | |

Die minimale Hemmkonzentration (MHK) ist die minimale Konzentration eines Antibiotikums, mit der ein Testkeim in seinem Wachstum über 18-24 h inhibiert wird. Die Hemmstoffkonzentration kann dabei nach mikrobiologischen Standardverfahren mit modifiziertem Medium im Rahmen eines Agardilutionstests bestimmt werden (siehe z.B. The National Committee for Clinical Laboratory Standards. Methods for dilution antimicrobial susceptibility tests for bacteria that grow aerobically; approved standard-fifth edition. NCCLS document M7-A5 [ISBN 1-56238-394-9]. NCCLS, 940 West Valley Road, Suite 1400, Wayne, Pennsylvania 19087-1898 USA, 2000). Die Bakterienkeime werden auf 1.5%igen Agarplatten kultiviert, die 20% defibriniertes Pferdeblut enthalten. Die Testkeime, die über Nacht auf Columbia-Blutagarplatten (Becton-Dickinson) inkubiert werden, werden in PBS verdünnt, auf eine Keimzahl von ca. 5x10⁵ Keime/ml eingestellt und auf Testplatten getropft (1-3 µl). Die Testsubstanzen enthalten unterschiedliche Verdünnungen der Testsubstanzen (Verdünnungsstufen 1:2). Die Kulturen werden bei 37°C für 18-24 Stunden in Gegenwart von 5% CO2 inkubiert.

Die jeweils niedrigste Substanzkonzentration, bei der kein sichtbares Bakterienwachstum mehr auftritt, wird als MHK definiert und in µg/ml angegeben.

**Tabelle B (mit Vergleichsbeispiel Biphenomycin B)**

| **Bsp.- Nr.** | **MHK *S. aureus 133*** | **MHK *S. aureus T17*** | **MHK *E. faecium L4001*** | **IC₅₀ *S. aureus* 133 Translation** |
|---|---|---|---|---|
| **9** | 2 | 4 | 16 | 0.3 |
| **12** | 1 | 1 | 16 | 0.8 |
| **36** | 1 | 2 | 8 | 0.36 |
| **42** | 2 | 2 | 32 | 0.08 |
| **43** | 2 | 4 | >32 | 0.9 |
| **Biphenomycin B** | <0.03 | >32 | 0.5 | 1.5 |

| | | | | |
|---|---|---|---|---|
| Konzentrationsangaben: MHK in µg/ml; IC₅₀ in µM. **Systemische Infektion mit *S. aureus* 133** | | | | |

Die Eignung der erfindungsgemäßen Verbindungen zur Behandlung von bakteriellen Infektionen kann in verschiedenen Tiermodellen gezeigt werden. Dazu werden die Tiere im allgemeinen mit einem geeigneten virulenten Keim infiziert und anschließend mit der zu testenden Verbindung, die in einer an das jeweilige Therapiemodell angepassten Formulierung vorliegt, behandelt. Speziell kann die Eignung der erfindungsgemäßen Verbindungen zur Behandlung von bakteriellen Infektionen in einem Sepsismodell an Mäusen nach Infektion mit *S. aureus* demonstriert werden.

Dazu werden *S. aureus* 133 Zellen über Nacht in BH-Bouillon (Oxoid, Deutschland) angezüchtet. Die Übernachtkultur wurde 1:100 in frische BH-Bouillon verdünnt und für 3 Stunden hochgedreht. Die in der logarithmischen Wachstumsphase befindlichen Bakterien werden abzentrifugiert und zweimal mit gepufferter, physiologischer Kochsalzlösung gewaschen. Danach wird am Photometer (Dr. Lange LP 2W) eine Zellsuspension in Kochsalzlösung mit einer Extinktion von 50 Einheiten eingestellt. Nach einem Verdünnungsschritt (1: 15) wird diese Suspension 1:1 mit einer 10 %-igen Mucinsuspension gemischt. Von dieser Infektionslösung wird 0.2 ml/20 g Maus i.p. appliziert. Dies entspricht einer Zellzahl von etwa 1-2 x 10⁶ Keimen/Maus. Die i.v.-Therapie erfolgt 30 Minuten nach der Infektion. Für den Infektionsversuch werden weibliche CFW 1-Mäuse verwendet. Das Überleben der Tiere wird über 6 Tage protokolliert. Das Tiermodell ist so eingestellt, dass unbehandelte Tiere innerhalb von 24 h nach der Infektion versterben.

### Bestimmung der Spontanresistenzfrequenzen gegen S. aureus

Die Spontanresistenzraten der erfindungsgemäßen Verbindungen werden wie folgt bestimmt: die Bakterienkeime werden in 30 ml eines Minimalmediums (18.5 mM Na₂HPO₄, 5.7 mM KH₂PO₄, 9.3 mM NH₄Cl, 2.8 mM MgSO₄, 17.1 mM NaCl, 0.033 µg/ml Thiaminhydrochlorid, 1.2 µg/ml Nicotinsäure, 0.003 µg/ml Biotin, 1 % Glucose, 25 µg/ml von jeder proteinogenen Aminosäure unter Zusatz von 0,4% BH Bouillon) bei 37°C über Nacht kultiviert, 10 min bei 6.000xg abzentrifugiert und in 2 ml phosphat-gepufferter physiologischer Natriumchlorid-Lösung resuspendiert (ca. 2 x 10⁹ Keime/ml). 100 ul dieser Zellsuspension bzw. 1:10 und 1:100 Verdünnungen werden auf vorgetrockneten Agarplatten (1.5 % Agar, 20 % defibriniertes Pferdeblut bzw. 1.5 % Agar, 20 % Rinderserum in 1/10 Müller-Hinton-Medium verdünnt mit PBS), welche die zu testende erfindungsgemäße Verbindung in einer Konzentration entsprechend 5xMHK bzw. 10xMHK enthalten, ausplattiert und 48 h bei 37°C bebrütet. Die entstehenden Kolonien (cfu) werden ausgezählt.

**Tabelle C**

| | | | |
|---|---|---|---|
| **Bsp.-Nr.** | **12** | **42** | **Bipehnomycin B** |
| **(Konzentration)** | **(5 x MHK)** | **(5 x MHK)** | **(10 x MHK)** |
| ***S. aureus* 133** | 1.3 x 10⁻¹⁰ | 5.3 x 10⁻¹⁰ | 1.7 x 10⁻⁶ |

Die Spontanresistenzrate für Beispiel 42 wird zusätzlich wie folgt bestimmt: die Bakterienkeime werden auf Columbia-Blut-Agarplatten bei 37°C mikroaerophil über Nacht kultiviert und in phosphat-gepufferter physiologischer NaCl-Lösung resuspendiert (ca. 1.5 x 10¹⁰ Keime/ml). 50 µl dieser Zellsuspension werden auf vorgetrockneten Agarplatten (1.5% Agar, 20% defibriniertes Pferdeblut), welche die zu testende erfindungsgemäße Verbindung in einer Konzentration entsprechend 10xMHK enthält, ausplattiert und 48 h bei 37°C bebrütet. Die entstehenden Kolonien (cfu) werden ausgezählt. Für Beispiel 42 wurde eine Spontanresistenzrate von 6.7 x 10⁻¹¹ für *S. aureus* 133 ermittelt.

### Isolierung der Biphenomycin-resistenten S. aureus Stämme RN4220Bi^{R} und T17

Der *S. aureus* Stamm RN4220Bi^{R} wird *in vitro* isoliert. Dazu werden jeweils 100 µl einer *S. aureus* RN4220 Zellsuspension (ca. 1.2x10⁸ cfu/ml) auf einer antibiotikafreien Agarplatte (18.5 mM Na₂HPO₄, 5.7 mM KH₂PO₄, 9.3 mM NH₄Cl, 2.8 mM MgSO₄, 17.1 mM NaCl, 0.033 µg/ml Thiaminhydrochlorid, 1.2 µg/ml Nicotinsäure, 0.003 µg/ml Biotin, 1 % Glucose, 25 µg/ml von jeder proteinogenen Aminosäure unter Zusatz von 0.4 % BH-Bouillon und 1% Agarose) und einer A-garplatte, die 2 µg/ml Biphenomycin B (10xMHK) enthält, ausplattiert und über Nacht bei 37°C bebrütet. Während auf der antibiotikafreien Platte ca. 1x10⁷ Zellen wachsen, wachsen auf der antibiotikahaltigen Platte ca. 100 Kolonien, entsprechend einer Resistenzfrequenz von 1x10-⁵. Einige der auf der antibiotikahaltigen Platte gewachsenen Kolonien werden auf MHK gegen Biphenomycin B getestet. Eine Kolonie mit einer MHK > 50 µM wird zur weiteren Verwendung ausgewählt und der Stamm mit RN4220Bi^{R} bezeichnet.

Der *S. aureus* Stamm T17 wird *in vivo* isoliert. CFW1-Mäuse werden mit 4x10⁷ *S*. *aureus* 133-Zellen pro Maus intraperitoneal infiziert. 0.5 Std. nach der Infektion werden die Tiere mit 50 mg/kg Biphenomycin B intravenös behandelt. Den überlebenden Tieren werden am Tag 3 nach der Infektion die Nieren entnommen. Nach dem Homogenisieren der Organe werden die Homogenate, wie bei RN4220Bi^{R} beschrieben, auf antibiotikafreien und antibiotikahaltigen Agarplatten, ausplattiert und über Nacht bei 37°C bebrütet. Etwa die Hälfte der aus der Niere isolierten Kolonien zeigen ein Wachstum auf den antibiotikahaltigen Platten (2.2x10⁶ Kolonien), was die Anreicherung von Biphenomycin B resistenten *S*. *aureus* Zellen in der Niere der behandelten Tiere belegt. Ca. 20 dieser Kolonien werden auf MHK gegen Biphenomycin B getestet und eine Kolonie mit einer MHK > 50 µM wird zur Weiterkultivierung ausgewählt und der Stamm mit T17 bezeichnet.

### C. Ausführungsbeispiele für pharmazeutische Zusammensetzungen

Die erfindungsgemäßen Verbindungen können folgendermaßen in pharmazeutische Zubereitungen überführt werden:

### Intravenös applizierbare Lösung:

### Zusammensetzung:

1 mg der Verbindung von Beispiel 1,15 g Polyethylenglykol 400 und 250 g Wasser für Injektionszwecke.

### Herstellung:

Die erfindungsgemäße Verbindung wird zusammen mit Polyethylenglykol 400 in dem Wasser unter Rühren gelöst. Die Lösung wird sterilfiltriert (Porendurchmesser 0,22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 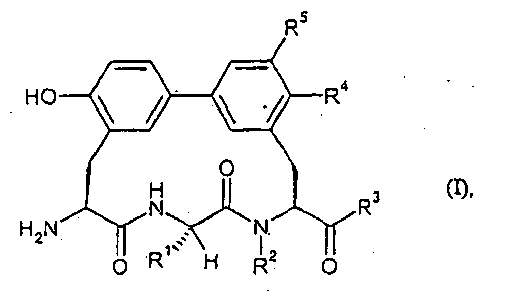 worin
R¹ gleich Alkyl ist, wobei Alkyl substituiert ist mit 1 bis 3 Substituenten, wobei die Substituenten unabhängig voneinander ausgewählt werden aus der Gruppe, beste- hend aus Amino, Hydroxy, Aminocarbonyl, Hydroxycarbonyl, (C₁-C₆)-Alkoxy, (C₁-C₆)-Alkylamino, 5- bis 7-gliedriges Heterocyclyl, 5- bis 7-gliedriges Heteroa- ryl, (C₁-C₆)-Alkylaminocarbonyl, Guanidino und Amidino, worin Heterocyclyl und Heteroaryl substituiert sein können mit 1 bis 2 Substituenten, wobei die Substituenten unabhängig voneinander ausge- wählt werden aus der Gruppe, bestehend aus Amino und (C₁-C₆).Akyl,
R² gleich Wasserstoff, (C₁-C₆)-Alkyl oder (C₃-C₇)Cycloalkyl ist,
R³ gleich "NR⁶R⁷ ist,
R⁴ gleich Wasserstoff, Halogen, Amino, Hydroxy, Aminocarbonyl, Hydroxycarbonyl, Nitro, Trifluormethyl, Trifluormethoxy, (C₁-C₄)-Alkyl, (C₁-C₄).Alkoxy, (C₁-C₆)- Alkylamino, Amino oder mono-(C₁-C₄)-Alkylamino substituiertes mono-(C₁-C₆)- Alkylaminocarbonyl oder Amino oder mono-(C₁-C₄)-Alkylamino substituiertes (C₁-C₆)-Alkylcarbonylamino ist,
R⁵ gleich Wasserstoff, Halogen, Amino, Hydroxy, Aminocarbonyl, Hydroxycarbonyl, Nitro, Trifluormethyl, Trifluormethoxy, (C₁-C₄)-Alkyl, (C₁-C₄)-Alkoxy, (C₁-C₆)- Alkylamino, Amino oder mono-(C₁-C₄)-Alkylamino substituiertes mono-(C₁-C₆)- Alkylaminocarbonyl oder Amino oder mono-(C₁-C₄)-Alkylamino substituiertes (C₁-C₆)-Alkylcarbonylamino ist, wobei R⁵ gleich Halogen, Amino, Hydroxy, Aminocarbonyl, Hydroxycarbonyl, Nitro, Trifluormethyl, Trifluormethoxy, (C₁-C₄)-Alkyl, (C₁-C₄)-Alkoxy, (C₁-C₆)- Alkylamino, Amino oder mono-(C₁-C₄)-Alkylamino substituiertes mono-(C₂-C₆). Alkylaminocarbonyl oder Amino oder mono-(C₁-C₄)-Alkylamino substituiertes (C₁-C₆)-Alkylcarbonylamino ist, wenn R⁴ gleich Hydroxy ist,
R⁶ gleich Wasserstoff, (C₁-C₆)-Alkyl, (C₃-C₇)-Cycloalkyl, 5- bis 7-gliedriges Hetero- cyclyl, (C₆-C₁₀)-Aryl oder 5- bis 7-gliedriges Heteroaryl ist, wobei Alkyl, Cycloalkyl, Heterocyclyl, Aryl und Heteroaryl substituiert sein kön- nen mit 1 bis 3 Substituenten, wobei die Substituenten unabhängig voneinander ausgewählt werden aus der Gruppe, bestehend aus Halogen, Amino, Hydroxy, A- minocarbonyl, Hydroxycarbonyl, Nitro, Cyano, Trifluormethyl, Trifluormethoxy, (C₁-C₆)-Alkyl, (C₁-C₆)-Alkoxy, (C₁-C₆)-Alkylamino, (C₃-C₇)-Cycloalkyl, 5- bis 7- gliedriges Heterocyclyl, (C₆-C₁₀)-Aryl, 5- bis 7-gliedriges Heteroaryl, (C₁-C₆)- Alkoxycarbonyl, (C₁-C₆)-Alkylaminocarbonyl, (C₁-C₆)-Alkylsulfonylamino und (C₆-C₁₀)-Arylsulfonylamino, worin Alkyl, Alkylamino, Cycloalkyl, Heterocyclyl, Aryl, Heteroaryl, Al- kylaminocarbonyl, Alkylsulfonylamino und Arylsulfonylamino substituiert sein können mit 1 bis 3 Substituenten, wobei die Substituenten unabhängig voneinander ausgewählt werden aus der Gruppe, bestehend aus Amino, Hydroxy, Aminocarbonyl und Hydroxycarbonyl,
R⁷ gleich Wasserstoff, (C₁-C₆)-Alkyl oder (C₃-C₁)-Cycloalkyl ist, wobei Alkyl substituiert sein kann mit 1 bis 2 Substituenten, wobei die Substituen- ten unabhängig voneinander ausgewählt werden aus der Gruppe, bestehend aus Amino, Hydroxy und (C₁-C₆)-Alkylamino,
oder
R⁶ und R⁷ zusammen mit dem Stickstoffatom, an das sie gebunden sind, ein Piperidinyl, Morpholinyl, Piperazinyl oder Pyrrolidinyl bilden, wobei Piperidinyl, Morpholi- nyl, Piperazinyl und Pyrrolidinyl substituiert sein können mit 1 bis 3 Substituen- ten, wobei die Substituenten unabhängig voneinander ausgewählt werden aus der Gruppe, bestehend aus Amino, Hydroxy, Aminocarbonyl, Hydroxycarbonyl, gege- benenfalls Amino oder Hydroxy substituierten (C₁-C₆)-Alkyl, (C₁-C₆)-Alkoxy. (C₁- C₆)-Alkylamino und (C₁-C₆)-Alkoxycarbonyl,
oder eines ihrer Salze, ihrer Solvate oder der Solvate ihrer Salze.

2. Verbindung nach Anspruch 1, **dadurch gekennzeichnet, dass**
R¹ gleich Aminomethyl, 2-Aminoethyl, 3-Aminoprop-1-yl, 4-Aminobut-1-yl, Hydro- xymethyl, 2-Hydroxy-ethyl, Aminocarbonylmethyl, 2-Aminocarbonylethyl, 2- Hydroxycarbonylethyl, 3-Guanidinoprop-1-yl, 3-Amino-2-hydroxyprop-1-yl oder 4-Amino-3-hydroxybut-1-yl ist,
R² gleich Wasserstoff, Methyl, Ethyl oder Cyclopropyl ist,
R³ gleich -NR⁶R⁷ ist,
R⁴ gleich Wasserstoff, Halogen, Amino, Hydroxy, Hydroxycarbonyl, Aminocarbonyl, Nitro oder Methyl ist,
R⁵ gleich Wasserstoff, Halogen, Amino, Hydroxy, Aminocarbonyl, Hydroxycarbonyl, Nitro oder Methyl ist, wobei R⁵ gleich Halogen, Amino, Hydroxy, Aminocarbonyl, Hydroxycarbonyl, Nitro oder Methyl ist, wenn R⁴ gleich Hydroxy ist,
R⁶ gleich Wasserstoff, (C₁-C₆)-Alkyl, (C₅-C₆)-Cycloalkyl, 5- bis 7-gliedriges Hetero- cyclyl oder Phenyl ist, wobei Alkyl, Cycloalkyl, Heterocyclyl und Phenyl substituiert sein können mit 1 bis 3 Substituenten, wobei die Substituenten unabhängig voneinander ausgewählt werden aus der Gruppe, bestehend aus Amino, Hydroxy, Aminocarbonyl, Hydro- xycarbonyl, (C₁-C₄)-Alkyl, (C₁-C₆)-Alkylamino, 5- bis 7-gliedriges Heterocyclyl, Phenyl, 5- oder 6-gliedriges Heteroaryl, (C₁-C₆)-Alkoxycarbonyl und (C₁-C₆)- Alkylaminocarbonyl, worin Alkyl, Alkylamino, Heterocyclyl, Aryl, Heteroaryl und Alkyl- aminocarbonyl substituiert sein können mit 1 bis 3 Substituenten, wobei die Substituenten unabhängig voneinander ausgewählt werden aus der Gruppe, bestehend aus Amino, Hydroxy, Aminocarbonyl und Hydroxy- carbonyl,
R⁷ gleich Wasserstoff oder (C₁-C₄)-Alkyl ist,
wobei Alkyl substituiert sein kann mit 1 bis 2 Substituenten, wobei die Substituen- ten unabhängig voneinander ausgewählt werden aus der Gruppe, bestehend aus Amino, Hydroxy und (C₁-C₆)-Alkylamino,
oder
R⁶ und R⁷ zusammen mit dem Stickstoffatom, an das sie gebunden sind, ein Piperazinyl bilden, wobei Piperazinyl substituiert sein kann mit 1 bis 3 Substituenten, wobei die Substituenten unabhängig voneinander ausgewählt werden aus der Gruppe, be- stehend aus Amino, Hydroxy, gegebenenfalls Amino substituiertem (C₁-C₆)-Alkyl, und (C₁-C₆)-Alkylamino.

3. Verbindung nach Anspruch 1 oder 2, **dadurch gekennzeichnet, dass**
R¹ gleich 2-Aminoethyl, 3-Aminoprop-1-yl, 4-Aminobut-1-yl oder 3-Amino-2- hydroxyprop-1-yl ist,
R² gleich Wasserstoff, Methyl oder Ethyl ist,
R³ gleich -NR⁶R⁷ ist,
R⁴ gleich Wasserstoff, Fluor, Chlor, Amino, Hydroxy oder Methyl ist,
R⁵ gleich Wasserstoff, Fluor oder Hydroxy ist, wobei R⁵ gleich Fluor ist, wenn R⁴ gleich Hydroxy ist,
R⁶ gleich eine Gruppe der Formel 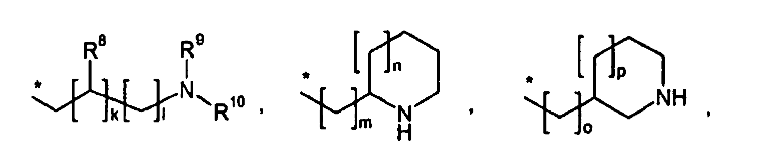 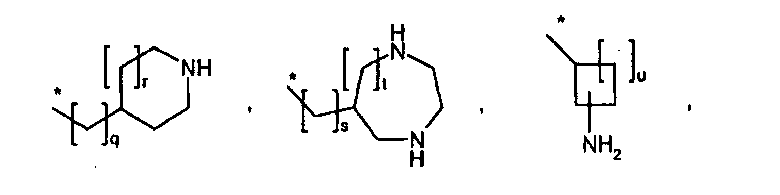 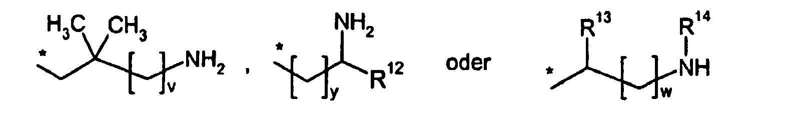
ist,
wobei
R⁸ gleich Wasserstoff oder Hydroxy ist,
R⁹ und R¹⁴ unabhängig voneinander Wasserstoff, Methyl oder eine Gruppe der Formel 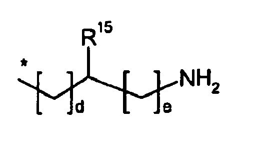 sind,
worin
* die Anknüpfstelle an das Stickstoffatom ist,
R¹⁵ gleich Wasserstoff oder *-(CH₂)₁-NH₂ ist,
worin
f eine Zahl 1, 2 oder 3 ist,
d eine Zahl 0, 1, 2 oder 3 ist
und
e eine Zahl 1, 2 oder 3 ist,
R¹⁰ gleich Wasserstoff oder Aminoethyl ist,
oder
R⁹ und R¹⁰ bilden zusammen mit dem Stickstoffatom an das sie gebunden sind einen Piperazin-Ring,
R¹² und R¹³ unabhängig voneinander eine Gruppe der Formel *-(CH₂)_{Z1}-OH oder
*-(CH₂)_{z2}-NH₂ sind,
worin
* die Anknüpfstelle an das Stickstoffatom ist,
Z1 und Z2 unabhängig voneinander eine Zahl 1, 2, 3 oder 4 sind,
k und t unabhängig voneinander eine Zahl 0 oder 1 sind,
1, w und y unabhängig voneinander eine Zahl 1, 2, 3 oder 4 ist,
m, r, s und v unabhängig voneinander eine Zahl 1 oder 2 sind, n, o, p und q unabhängig voneinander eine Zahl 0, 1 oder 2 sind,
u eine Zahl 0, 1, 2 oder 3 ist, 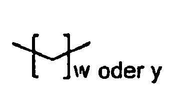
unabhängig voneinander bei w oder y gleich 3 eine Hydroxy-Gruppe am mittleren Kohlenstoffatom der Dreierkette tragen kann,
* die Anknüpfstelle an das Stickstoffatom ist,
R⁷ gleich Wasserstoff ist.

4. Verbindung nach Anspruch 1, 2 oder 3, **dadurch gekennzeichnet, dass**
R¹ gleich 3-Aminoprop-1-yl oder 3-Amino-2-hydroxyprop-1-yl ist,
R² gleich Wasserstoff oder Methyl ist,
R³ gleich -NR⁶R⁷ ist,
R⁴ gleich Wasserstoff, Fluor, Chlor oder Methyl ist,
R⁵ gleich Wasserstoff ist,
R⁶ gleich eine Gruppe der Formel 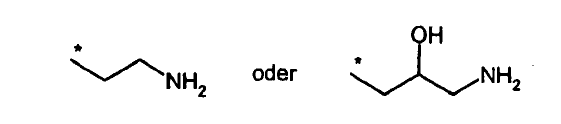
ist,
wobei
* gleich die Anknüpfstelle an das Stickstoffatom ist,
R⁷ gleich Wasserstoff ist.

5. Verfahren zur Herstellung einer Verbindung der Formel (I) nach Anspruch 1, wobei eine Verbindung der Formel (I) eine Verbindung der Formel (Ia) oder (Ib) ist, oder eines ihrer Salze, ihrer Solvate oder der Solvate ihrer Salze, **dadurch gekennzeichnet, dass**
[A] eine Verbindung der Formel 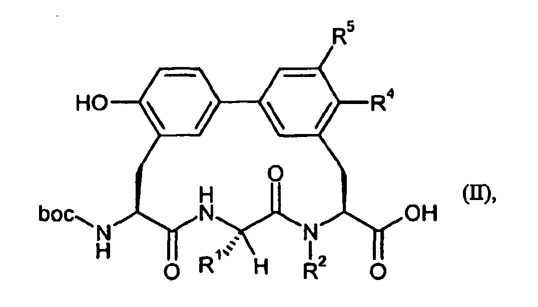 worin R¹, R², R⁴ und R⁵ die in Anspruch 1 angegebene Bedeutung haben und boc gleich *tert*-Butoxycarbonyl ist,
in einem zweistufigen Verfahren zunächst in Gegenwart von einem oder mehreren Dehydratisierungsreagenzien mit einer Verbindung der Formel
HNR⁶R⁷ (III),
worin R⁶ und R⁷ die oben angegebene Bedeutung haben,
und anschließend mit einer Säure zu einer Verbindung der Formel 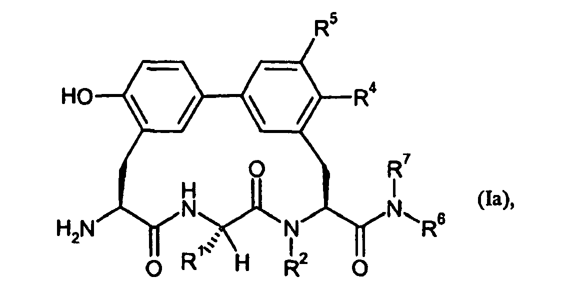 worin R¹, R², R⁴ und R⁵ die oben angegebene Bedeutung haben,
oder
[B] eine Verbindung der Formel 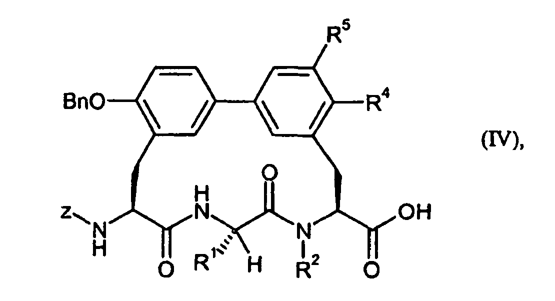 worin R¹, R², R⁴ und R⁵ die in Anspruch 1 angegebene Bedeutung haben und Z gleich Benzyloxycarbonyl ist,
in einem zweistufigen Verfahren zunächst in Gegenwart von einem oder mehreren Dehydratisierungsreagenzien mit Verbindungen der Formel (III) und anschließend mit einer Säure oder durch Hydrogenolyse zu einer Verbindung der Formel (Ia),
oder
[C] eine Verbindung der Formel (IV) mit einer Säure oder durch Hydrogenolyse zu einer Verbindung der Formel 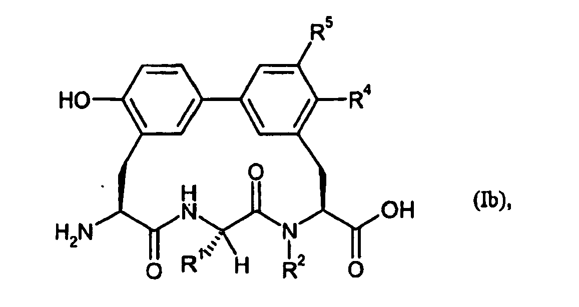 worin R', R², R⁴ und R⁵ die in Anspruch 1 angegebene Bedeutung haben, oder
[D] eine Verbindung der Formel 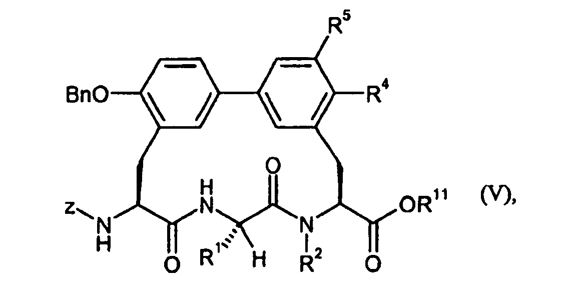 worin R¹, R², R⁴ und R⁵ die in Anspruch 1 angegebene Bedeutung haben, und
R¹¹ gleich Benzyl, Methyl oder Ethyl ist,
mit einer Säure oder durch Hydrogenolyse, gegebenenfalls durch anschließende Umsetzung mit einer Base zur Verseifung des Methyl- oder Ethylesters, zu einer Verbindung der Formel (Ib), umgesetzt wird.

6. Verfahren zur Herstellung einer Verbindung der Formel (I) nach Anspruch 1 oder eines ihrer Solvate, **dadurch gekennzeichnet, dass** ein Salz der Verbindung oder ein Solvat eines Salzes der Verbindung durch Chromatographie unter Zusatz einer Base in die Verbindung überführt wird.

7. Verbindung nach einem der Ansprüche 1 bis 4 zur Behandlung und/oder Prophylaxe von Krankheiten.

8. Verwendung einer Verbindung nach einem der Ansprüche 1 bis 4 zur Herstellung eines Arzneimittels zur Behandlung und/oder Prophylaxe von Krankheiten.

9. Verwendung einer Verbindung nach einem der Ansprüche 1 bis 4 zur Herstellung eines Arzneimittels zur Behandlung und/oder Prophylaxe von bakteriellen Erkrankungen.

10. Arzneimittel enthaltend mindestens eine Verbindung nach einem der Ansprüche 1 bis 4 in Kombination mit mindestens einem inerten, nichttoxischen, pharmazeutisch geeigneten Hilfsstoff.

11. Arzneimittel nach Anspruch 10 zur Behandlung und/oder Prophylaxe von bakteriellen Infektionen.

12. Verwendung einer antibakteriell wirksamen Menge mindestens einer Verbindung nach einem der Ansprüche 1 bis 4 oder eines Arzneimittels nach Anspruch 10 oder 11 zur Herstellung eines Medikaments zur Bekämpfung von bakteriellen Infektionen in Menschen und Tieren.

## Claims

1. Compound of formula 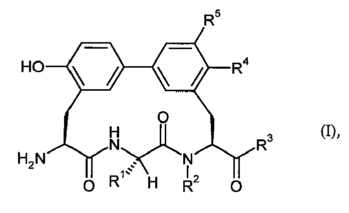 in which
R¹ is alkyl, whereby alkyl is substituted with 1 to 3 substituents, whereby the substituents are selected independently of one another from the group consisting of amino, hydroxy, aminocarbonyl, hydroxycarbonyl, (C₁-C₆)-alkoxy, (C₁-C₆)-alkylamino, 5- to 7-membered heterocyclyl, 5- to 7-membered heteroaryl, (C₁-C₆)-alkylaminocarbonyl, guanidino and amidino, in which heterocyclyl and heteroaryl may be substituted with 1 to 2 substituents, whereby the substituents are selected inde- pendently of one another from the group consisting of amino and (C₁-C₆)-alkyl,
R² is hydrogen, (C₁-C₆)-alkyl or (C₃-C₇)-cycloalkyl,
R³ is -NR⁶R⁷,
R⁴ is hydrogen, halogen, amino, hydroxy, aminocarbonyl, hydroxycar- bonyl, nitro, trifluoromethyl, trifluoromethoxy, (C₁-C₄)-alkyl, (C₁-C₄)- alkoxy, (C₁-C₆)-alkylamino, amino or mono-(C₁-C₄)-alkylamino substi- tuted mono-(C₂-C₆)-alkylaminocarbonyl or amino or mono-(C₁-C₄)- alkylamino substituted (C₁-C₆)-alkylcarbonylamino,
R⁵ is hydrogen, halogen, amino, hydroxy, aminocarbonyl, hydroxycar- bonyl, nitro, trifluoromethyl, trifluoromethoxy, (C₁-C₄)-alkyl, (C₁-C₄)- alkoxy, (C₁-C₆)-alkylamino, amino or mono-(C₁-C₄)-alkylamino substi- tuted mono-(C₂-C₆)-alkylaminocarbonyl or amino or mono-(C₁-C₄)- alkylamino substituted (C₁-C₆)-alkylcarbonylamino, whereby R⁵ is halogen, amino, hydroxy, aminocarbonyl, hydroxycar- bonyl, nitro, trifluoromethyl, trifluoromethoxy, (C₁-C₄)-alkyl, (C₁-C₄)- alkoxy, (C₁-C₆)-alkylamino, amino or mono-(C₁-C₄)-alkylamino substi- tuted mono-(C₂-C₆)-alkylaminocarbonyl or amino or mono-(C₁-C₄)- alkylamino substituted (C₁-C₆)-alkylcarbonylamino if R⁴ is hydroxy,
R⁶ is hydrogen, (C₁-C₆)-alkyl, (C₃-C₇)-cycloalkyl, 5- to 7-membered hetero- cyclyl, (C₆-C₁₀)-aryl or 5- to 7-membered heteroaryl, whereby alkyl, cycloalkyl, heterocyclyl, aryl and heteroaryl may be sub- stituted with 1 to 3 substituents, whereby the substituents are selected independently of one another from the group consisting of halogen, amino, hydroxy, aminocarbonyl, hydroxycarbonyl, nitro, cyano, tri- fluoromethyl, trifluoromethoxy, (C₁-C₆)-alkyl, (C₁-C₆)-alkoxy, (C₁-C₆)- alkylamino, (C₃-C₇)-cycloalkyl, 5- to 7-membered heterocyclyl, (C₆-C₁₀)- aryl, 5- to 7-membered heteroaryl, (C₁-C₆)-alkoxycarbonyl, (C₁-C₅)- alkylaminocarbonyl, (C₁-C₆)-alkylsulfonylamino and (C₆-C₁₀)-arylsulf- onylamino, in which alkyl, alkylamino, cycloalkyl, heterocyclyl, aryl, het- eroaryl, alkylaminocarbonyl, alkylsulfonylamino and arylsulf- onylamino may be substituted with 1 to 3 substituents, whereby the substituents are selected independently of one another from the group consisting of amino, hydroxy, aminocarbonyl and hydroxycarbonyl,
R⁷ is hydrogen, (C₁-C₆)-alkyl or (C₃-C₇)-cycloalkyl, whereby alkyl may be substituted with 1 to 2 substituents, whereby the substituents are selected independently of one another from the group consisting of amino, hydroxy and (C₁-C₆)-alkylamino,
or
R⁶ and R⁷ together with the nitrogen atom to which they are bonded form a piperidinyl, morpholinyl, piperazinyl or pyrrolidinyl, whereby piperidinyl, morpholinyl, piperazinyl and pyrrolidinyl may be substituted with 1 to 3 substituents, whereby the substituents are selected independently of one another from the group consisting of amino, hydroxy, aminocarbonyl, hydroxycarbonyl, optionally amino or hydroxy substituted (C₁-C₆)-alkyl, (C₁-C₆)-alkoxy, (C₁-C₆)-alkylamino and (C₁ C₆)-alkoxycarbonyl,
or one of the salts thereof, the solvates thereof or the solvates of the salts thereof.

2. Compound according to Claim 1, **characterized in that**
R¹ is aminomethyl, 2-aminoethyl, 3-aminoprop-1-yl, 4-aminobut-1-yl, hydroxymethyl, 2-hydroxy-ethyl, aminocarbonylmethyl, 2-aminocarb- onylethyl, 2-hydroxycarbonylethyl, 3-guanidinoprop-1-yl, 3-amino-2- hydroxyprop-1-yl or 4-amino-3-hydroxybut-1-yl,
R² is hydrogen, methyl, ethyl or cyclopropyl,
R³ is -NR⁶R⁷,
R⁴ is hydrogen, halogen, amino, hydroxy, hydroxycarbonyl, aminocarb- onyl, nitro or methyl,
R⁵ is hydrogen, halogen, amino, hydroxy, aminocarbonyl, hydroxycar- bonyl, nitro or methyl, whereby R⁵ is halogen, amino, hydroxy, aminocarbonyl, hydroxycar- bonyl, nitro or methyl, if R⁴ is hydroxy,
R⁶ is hydrogen, (C₁-C₆)-alkyl, (C₅-C₆)-cycloalkyl, 5- to 7-membered hetero- cyclyl or phenyl, whereby alkyl, cycloalkyl, heterocyclyl and phenyl may be substituted with 1 to 3 substituents, whereby the substituents are selected inde- pendently of one another from the group consisting of amino, hy- droxy, aminocarbonyl, hydroxycarbonyl, (C₁-C₄)-alkyl, (C₁-C₆)-alkyl- amino, 5- to 7-membered heterocyclyl, phenyl, 5- or 6-membered het- eroaryl, (C₁-C₆)-alkoxycarbonyl and (C₁-C₆)-alkylaminocarbonyl, in which alkyl, alkylamino, heterocyclyl, aryl, heteroaryl and al- kylaminocarbonyl may be substituted with 1 to 3 substituents, whereby the substituents are selected independently of one an- other from the group consisting of amino, hydroxy, aminocarb- onyl and hydroxycarbonyl,
R⁷ is hydrogen or (C₁-C₄)-alkyl, whereby alkyl may be substituted with 1 to 2 substituents, whereby the substituents are selected independently of one another from the group consisting of amino, hydroxy and (C₁-C₆)-alkylamino,
or
R⁶ and R⁷ together with the nitrogen atom to which they are bonded form a piperazinyl, whereby piperazinyl may be substituted with 1 to 3 substituents, whereby the substituents are selected independently of one another from the group consisting of amino, hydroxy, optionally amino substituted (C₁-C₆)-alkyl, and (C₁-C₆)-alkylamino.

3. Compound according to Claim 1 or 2, **characterized in that**
R¹ is 2-aminoethyl, 3-aminoprop-1-yl, 4-aminobut-1-yl or 3-amino-2- hydroxyprop-1-yl,
R² is hydrogen, methyl or ethyl,
R³ is -NR⁶R⁷,
R⁴ is hydrogen, fluorine, chlorine, amino, hydroxy or methyl,
R⁵ is hydrogen, fluorine or hydroxy, whereby R⁵ is fluorine if R⁴ is hydroxy,
R⁶ is a group of the formula 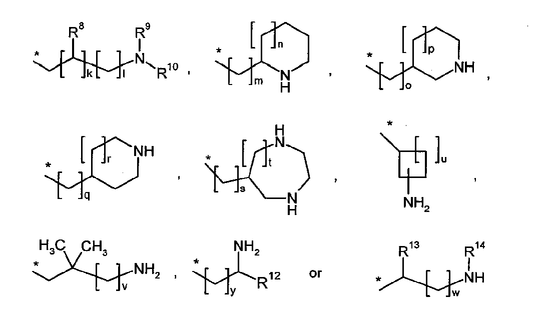
whereby
R⁸ is hydrogen or hydroxy,
R⁹ and R¹⁴ are independently of one another hydrogen, methyl or a group of the formula 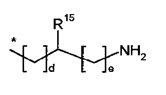 in which
* is the point of attachment to the nitrogen atom,
R¹⁵ is hydrogen or *-(CH₂)_{f-}NH₂,
in which
f is a number 1, 2 or 3,
d is a number 0, 1, 2 or 3,
and e is a number 1, 2 or 3,
R¹⁰ is hydrogen or aminoethyl,
or
R⁹ and R¹⁰ form together with the nitrogen atom to which they are bonded a piperazine ring,
R¹² and R¹³ are independently of one another a group of the formula *-(CH₂)_{Z1}-OH or *-(CH₂)_{Z2}-NH₂,
in which
* is the point of attachment to the nitrogen atom,
Z1 and Z2 are independently of one another a number 1, 2, 3 or 4,
k and t are independently of one another a number 0 or 1, 1, w and y are independently of one another a number 1, 2, 3 or 4, m, r, s and v are independently of one another a number 1 or 2, n, o, p and q are independently of one another a number 0, 1 or 2,
u is a number 0, 1, 2 or 3, 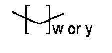 may independently of one another if w or y is 3 carry a hydroxy group on the middle carbon atom of the three-membered chain,
* is the point of attachment to the nitrogen atom,
R⁷ is hydrogen.

4. Compound according to Claim 1, 2 or 3, **characterized in that**
R¹ is 3-aminoprop-1-yl or 3-amino-2-hydroxyprop-1-yl,
R² is hydrogen or methyl,
R³ is -NR⁶R⁷,
R⁴ is hydrogen, fluorine, chlorine or methyl,
R⁵ is hydrogen,
R⁶ is a group of the formula 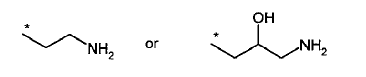
whereby
* is the point of attachment to the nitrogen atom,
R⁷ is hydrogen.

5. Process for preparing a compound of formula (I) according to Claim 1, whereby a compound of formula (I) is a compound of formula (Ia) or (Ib), or one of the salts thereof, the solvates thereof or the solvates of the salts thereof, **characterized in that**
[A] a compound of formula 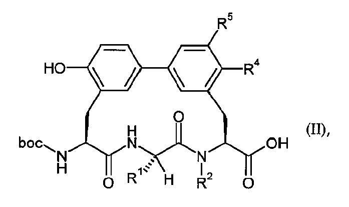 in which R¹, R², R⁴ and R⁵ have the meaning indicated in Claim 1, and boc is tert-butoxycarbonyl,
is reacted in a two-stage process first in the presence of one or more dehydrating reagents with a compound of formula
HNR⁶R⁷ (III),
in which R⁶ and R⁷ have the meaning indicated above,
and then with an acid to give a compound of formula 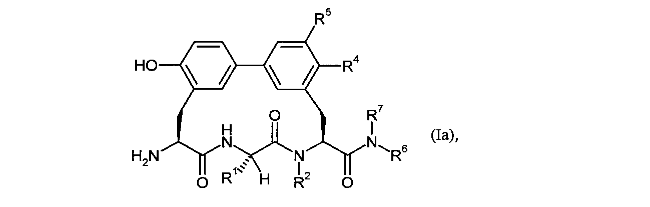 in which R¹, R², R⁴ and R⁵ have the meaning indicated above,
or
[B] a compound of formula 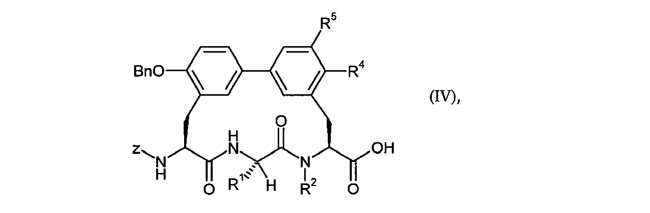 in which R¹, R², R⁴ and R⁵ have the meaning indicated in Claim 1, and Z is benzyloxycarbonyl,
is reacted in a two-stage process first in the presence of one or more dehydrating reagents with compounds of the formula (III) and then with an acid or by hydrogenolysis to give a compound of formula (Ia),
or
[C] a compound of formula (IV) is reacted with an acid or by hydrogenolysis to give a compound of formula 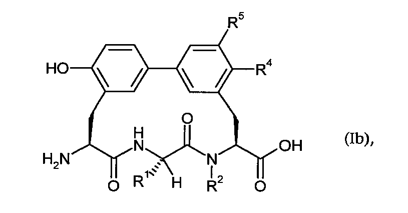 in which R¹, R², R⁴ and R⁵ have the meaning indicated in Claim 1,
or
[D] a compound of formula 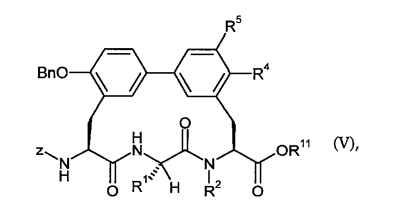
in which R¹, R², R⁴ and R⁵ have the meaning indicated in Claim 1, and
R¹¹ is benzyl, methyl or ethyl,
is reacted with an acid or by hydrogenolysis, optionally by subsequent reaction with a base to hydrolyse the methyl or ethyl ester, to give a compound of formula (Ib).

6. Process for preparing a compound of formula (I) according to Claim 1 or one of its solvates, **characterized in that** a salt of the compound or a solvate of a salt of the compound is converted into the compound by chromatography with addition of a base.

7. Compound according to any of Claims 1 to 4 for the treatment and/or prophylaxis of diseases.

8. Use of a compound according to any of Claims 1 to 4 for the production of a medicament for the treatment and/or prophylaxis of diseases.

9. Use of a compound according to any of Claims 1 to 4 for the production of a medicament for the treatment and/or prophylaxis of bacterial diseases.

10. Medicament comprising at least one compound according to any of Claims 1 to 4 in combination with at least one inert, nontoxic, pharmaceutically acceptable excipient.

11. Medicament according to Claim 10 for the treatment and/or prophylaxis of bacterial infections.

12. Use of an antibacterially effective amount of at least one compound according to any of Claims 1 to 4 or of a medicament according to Claim 10 or 11 for the production of a medicament for controlling bacterial infections in humans and animals

## Revendications

1. Composé de la formule : 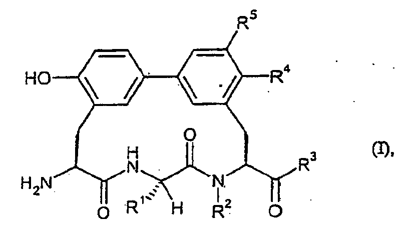 où
R¹ est alkyle, qui est substitué par 1 à 3 substituants, où les substituants sont choisis indépendamment l'un de l'autre parmi le groupe consistant en amino, hydroxy, aminocarbonyle, hydroxycarbonyle, alcoxy (C₁-C₆), alkylamino (C₁-C₆), hétérocyclyle ayant 5 à 7 membres, hétéroaryle ayant 5 à 7 membres, alkyl (C₁-C₆)aminocarbonyle, guanidino et amidino,
où hétérocyclyle et hétéroaryle peuvent être substitués par 1 à 2 substituants, où les substituants sont choisis indépendamment parmi le groupe consistant en amino et alkyle (C₁-C₆),
R² est hydrogène, alkyle (C₁-C₆) ou cycloalkyle (C₃-C₇),
R³ est -NR⁶R⁷,
R⁴ est hydrogène, halogène, amino, hydroxy, aminocarbonyle, hydroxycarbonyle, nitro, trifluorométhyle, trifluorométhoxy, alkyle (C₁-C₄), alcoxy (C₁-C₄), alkylamino (C₁-C₆), mono[alkyl (C₂-C₆)]aminocarbonyle substitué par amino ou mono[alkyl (C₁-C₄)]amino, ou mono[alkyl (C₁-C₆)]carbonylamino substitué par amino ou mono[alkyl (C₁-C₄)]amino,
R⁵ est hydrogène, halogène, amino, hydroxy, aminocarbonyle, hydroxycarbonyle, nitro, trifluorométhyle, trifluorométhoxy, alkyle (C₁-C₄), alcoxy (C₁-C₄), alkylamino (C₁-C₆), mono[alkyl (C₂-C₆)]aminocarbonyle substitué par amino ou mono[alkyl (C₁-C₄)]amino, ou mono[alkyl (C₁-C₆)]carbonylamino substitué par amino ou mono[alkyl (C₁-C₄)]amino,
où R⁵ est halogène, amino, hydroxy, aminocarbonyle, hydroxycarbonyle, nitro, trifluorométhyle, trifluorométhoxy, alkyle (C₁-C₄), alcoxy (C₁-C₄), alkylamino (C₁-C₆), mono[alkyl (C₂-C₆)]aminocarbonyle substitué par amino ou mono[alkyl (C₁-C₄)lamino, ou mono[alkyl (C₁-C₆)]carbonylamino substitué par amino ou mono[alkyl (C₁-C₄)]amino, lorsque R⁴ est hydroxy,
R⁵ est hydrogène, alkyle (C₁-C₆), cycloalkyle (C₃-C₇), hétérocyclyle ayant 5 à 7 membres, aryle (C₆-C₁₀) ou hétéroaryle ayant 5 à 7 membres,
où alkyle, cycloalkyle, hétérocyclyle, aryle et hétéroaryle peuvent être substitués par 1 à 3 substituants, où les substituants sont choisis indépendamment parmi le groupe consistant en halogène, amino, hydroxy, aminocarbonyle, hydroxycarbonyle, nitro, cyano, trifluorométhyle, trifluorométhoxy, alkyle (C₁-C₆), alcoxy (C₁-C₆), alkylamino (C₁-C₆), cycloalkyle (C₃-C₇), hétérocyclyle ayant 5 à 7 membres, aryle (C₆-C₁₀), hétéroaryle ayant 5 à 7 membres, alcoxy (C₁-C₆)carbonyle, alkyl (C₁-C₆)aminocarbonyle, alkyl (C₁-C₆)sulfonylamino, et aryl (C₆-C₁₀)sulfonylamino,
où alkyle, alkylamino, cycloalkyle, hétérocyclyle, aryle, hétéroaryle, alkylaminocarbonyle, alkylsulfonylamino et arylsulfonylamino peuvent être substitués par 1 à 3 substituants, où les substituants sont choisis indépendamment parmi le groupe consistant en amino, hydroxy, aminocarbonyle et hydroxycarbonyle
R⁷ est hydrogène, alkyle (C₁-C₆) ou cycloalkyle (C₃-C₇),
où alkyle peut être substitué par 1 à 2 substituants, où les substituants sont choisis indépendamment parmi le groupe consistant en amino, hydroxy et alkylamino (C₁-C₆),
ou
R⁶ et R⁷ avec l'atome d'azote auquel ils sont liés, forment un pipéridinyle, morpholinyle, pipérazinyle ou pyrrolidinyle, où pipéridinyle, morpholinyle, pipérazinyle et pyrrolidinyle peuvent être substitués par 1 à 3 substituants, où les substituants sont choisis indépendamment parmi le groupe consistant en amino, hydroxy, aminocarbonyle, hydroxycarbonyle, alkyle (C₁-C₆) le cas échéant substitué par amino ou hydroxy, alcoxy (C₁-C₆), alkylamino (C₁-C₆), et alcoxy (C₁-C₆)carbonyle,
ou un de leurs sels, leurs solvates ou les solvates de leurs sels.

2. Composé selon la revendication 1, **caractérisé en ce que**
R¹ est aminométhyle, 2-aminoéthyle, 3-aminoprop-1-yle, 4-aminobut-1-yle, hydroxyméthyle, 2-hydroxyéthyle, aminocarbonylméthyle, 2-amino-carbonyléthyle, 2-hydroxycarbonyléthyle, 3-guanidinoprop-1-yle, 3-amino-2-hydroxyprop-1-yle ou 4-amino-3-hydroxybut-1-yle,
R² est hydrogène, méthyle, éthyle ou cyclopropyle,
R³ est -NR⁶R⁷,
R⁴ est hydrogène, halogène, amino, hydroxy, hydroxycarbonyle, aminocarbonyle, nitro ou méthyle,
R⁵ est hydrogène, halogène, amino, hydroxy, aminocarbonyle, hydroxycarbonyle, nitro ou méthyle,
où R⁵ est halogène, amino, hydroxy, aminocarbonyle, hydroxycarbonyle, nitro ou méthyle, lorsque R⁴ est hydroxy,
R⁶ est hydrogène, alkyle (C₁-C₆), cycloalkyle (C₅-C₆), hétérocyclyle ayant 5 à 7 membres ou phényle,
où alkyle, cycloalkyle, hétérocyclyle et phényle peuvent être substitués par 1 à 3 substituants, où les substituants sont choisis indépendamment parmi le groupe consistant en amino, hydroxy, aminocarbonyle, hydroxycarbonyle, alkyle (C₁-C₄), alkylamino (C₁-C₆), hétérocyclyle ayant 5 à 7 membres, phényle, hétéroaryle ayant 5 ou 6 membres, alcoxy (C₁-C₆)carbonyle et alkyl (C₁-C₆)aminocarbonyle,
où alkyle, alkylamino, hétérocyclyle, aryle, hétéroaryle et alkylaminocarbonyle peuvent être substitués par 1 à 3 substituants, où les substituants sont choisis indépendamment parmi le groupe consistant en amino, hydroxy, aminocarbonyle et hydroxycarbonyle
R⁷ est hydrogène ou alkyle (C₁-C₄),
où alkyle peut être substitué par 1 à 2 substituants, où les substituants sont choisis indépendamment parmi le groupe consistant en amino, hydroxy et alkylamino (C₁-C₆),
ou
R⁶ et R⁷ avec l'atome d'azote auquel ils sont liés, forment un pipérazinyle, où pipérazinyle peut être substitué par 1 à 3 substituants, où les substituants sont choisis indépendamment parmi le groupe consistant en amino, hydroxy, alkyle (C₁-C₆) le cas échéant substitué par amino et alkylamino (C₁-C₆).

3. Composé selon la revendication 1 ou 2, **caractérisé en ce que**
R¹ est 2-aminoéthyle, 3-aminoprop-1-yle, 4-aminobut-1-yle, ou 3-amino-2-hydroxyprop-1-yle,
R² est hydrogène, méthyle ou éthyle,
R³ est -NR⁶R⁷,
R⁴ est hydrogène, fluor, chlore, amino, hydroxy ou méthyle,
R⁵ est hydrogène, fluor ou hydroxy,
où R⁵ est fluor, lorsque R⁴ est hydroxy,
R⁶ est un groupe de formule : 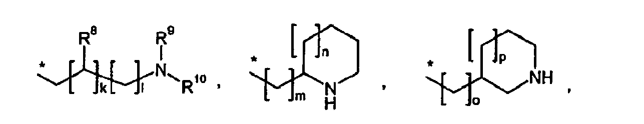 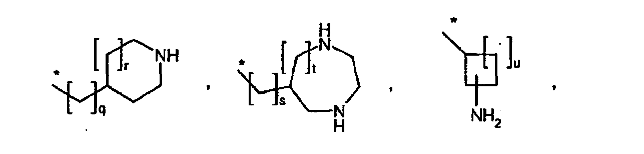 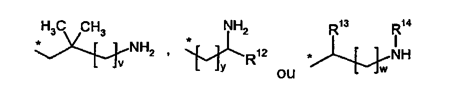 où R⁸ est hydrogène ou hydroxy,
R⁹ et R¹⁴ sont indépendamment l'un de l'autre, hydrogène, méthyle ou un groupe de formule : 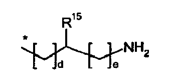 où
* est le site de liaison sur l'atome d'azote,
R¹⁵ est hydrogène ou *-(CH₂)_{f}-NH₂,
où
f est le nombre 1, 2 ou 3,
d est le nombre 0, 1, 2 ou 3,
et
e est le nombre 1, 2 ou 3,
R¹⁰ est hydrogène ou aminoéthyle,
ou
R⁹ et R¹⁰ forment avec l'atome d'azote auquel ils sont liés, un cycle pipérazine,
R¹² et R¹³ sont indépendamment l'un de l'autre, un groupe de formule *-(CH₂)_{Z1}-OH ou *-(CH₂)_{Z2}-NH₂,
où
* est le site de liaison sur l'atome d'azote,
Z1 et Z2 sont indépendamment l'un de l'autre, le nombre 1, 2, 3 ou 4, k et t sont indépendamment l'un de l'autre, le nombre 0 ou 1, l, w et y sont indépendamment l'un de l'autre, le nombre 1, 2, 3 ou 4, m, r, s et v sont indépendamment l'un de l'autre, le nombre 1 ou 2, n, o, p et q sont indépendamment l'un de l'autre, le nombre 0, 1 ou 2, u est le nombre 0, 1, 2 ou 3, 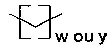 indépendamment l'un de l'autre lorsque w ou y est 3, peut porter un groupe hydroxy sur l'atome de carbone central de la chaine à trois éléments,
* est le site de liaison sur l'atome d'azote,
R⁷ est hydrogène.

4. Composé selon la revendication 1, 2 ou 3, **caractérisé en ce que**
R¹ est 3-aminoprop-1-yle ou 3-amino-2-hydroxyprop-1-yle,
R² est hydrogène ou méthyle,
R³ est -NR⁶R⁷,
R⁴ est hydrogène, fluor, chlore ou méthyle,
R⁵ est hydrogène,
R⁶ est un groupe de formule : 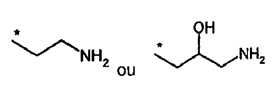 où
* est le site de liaison sur l'atome d'azote,
R⁷ est hydrogène.

5. Procédé de préparation d'un composé de formule (I) selon la revendication 1, où un composé de formule (I) est un composé de formule (Ia) ou (Ib), ou un de leurs sels, leurs solvates ou les solvates de leurs sels, **caractérisé en ce que**
[A] un composé de formule : 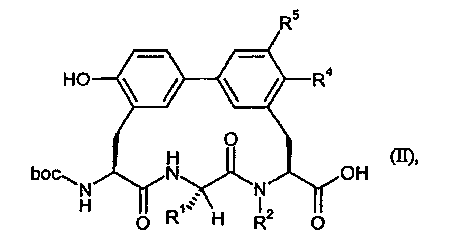 où R¹, R², R⁴ et R⁵ ont la signification donnée à la revendication 1, et boc est t-butoxycarbonyle,
est mis à réagir selon un procédé en deux étapes, d'abord en présence d'un ou de plusieurs agents de déshydratation, avec un composé de formule :
HNR⁶R⁷ (III)
où R⁶ et R⁷ ont la signification donnée ci-dessus,
et ensuite avec un acide pour donner le composé de formule : 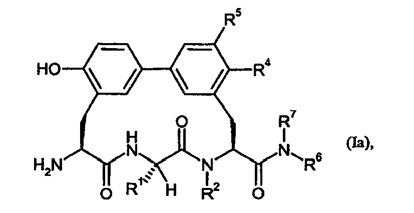 où R¹, R², R⁴ et R⁵ ont la signification donnée ci-dessus,
ou
[B] un composé de formule : 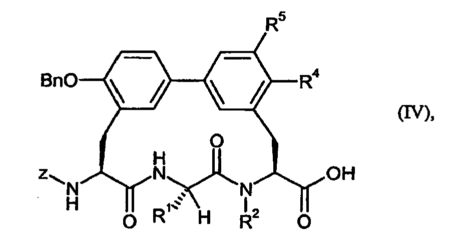 où R¹, R², R⁴ et R⁵ ont la signification donnée à la revendication 1 et Z est benzyloxycarbonyle,
est mis a réagir selon un procédé en deux étapes, d'abord en présence d'un ou de plusieurs agents de déshydratation avec les composés de la formule (III) et ensuite, avec un acide ou par hydrogénolyse, pour donner un composé de formule (Ia),
ou
[C] un composé de formule (IV) est mis à réagir avec un acide ou par hydrogénolyse, en un composé de formule : 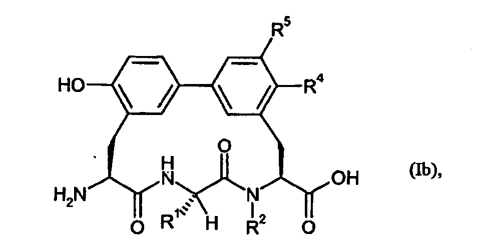 où R¹, R², R⁴ et R⁵ ont la signification donnée à la revendication 1, ou
[D] un composé de formule : 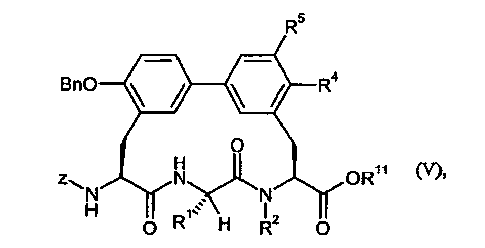 où R¹, R², R⁴ et R⁵ ont la signification donnée à la revendication 1, et R¹¹ est benzyle, méthyle ou éthyle,
est mis à réagir avec un acide ou par hydrogénolyse, le cas échéant avec réaction suivante avec une base pour la saponification de l'ester méthylique ou éthylique, pour donner un composé de formule (Ib).

6. Procédé de préparation d'un composé de formule (I) selon la revendication 1, ou d'un de ses solvates, **caractérisé en ce qu'**un sel du composé ou un solvate d'un sel du composé est transformé en le composé par chromatographie avec addition d'une base.

7. Composé selon l'une des revendications 1 à 4, pour le traitement et/ou la prophylaxie de maladies.

8. Utilisation d'un composé selon l'une des revendications 1 à 4, pour la préparation d'un agent pharmaceutique pour le traitement et/ou la prophylaxie de maladies.

9. Utilisation d'un composé selon l'une des revendications 1 à 4, pour la préparation d'un agent pharmaceutique pour le traitement et/ou la prophylaxie de maladies bactériennes.

10. Agent pharmaceutique contenant au moins un composé selon l'une des revendications 1 à 4, en combinaison avec au moins un auxiliaire inerte, non toxique, pharmaceutiquement approprié.

11. Agent pharmaceutique selon la revendication 10, pour le traitement et/ou la prophylaxie d'infections bactériennes.

12. Utilisation d'une quantité active de manière antibactérienne d'au moins un composé selon l'une des revendications 1 à 4 ou d'un agent pharmaceutique selon la revendication 10 ou 11, pour la préparation d'un médicament pour lutter contre des infections bactériennes chez l'homme et l'animal.
